# EUROPEAN PATENT SPECIFICATION

(11) **EP 2 214 690 B1**
(45) Date of publication and mention of the grant of the patent: **10.05.2017**
(21) Application number: 08874142.6
(22) Date of filing: 30.10.2008
(51) Int. Cl.: A61K 38/34, C07K 16/28, C07K 14/68, C12N 5/10, A61P 3/04, A61P 15/10, A61P 29/00, A61P 43/00

(54) **MELANOCORTIN RECEPTOR BINDING MIMETIBODIES, COMPOSITIONS, METHODS AND USES**
DEN MELANOCORTINREZEPTOR BINDENDE MIMETIKÖRPER, ZUSAMMENSETZUNGEN, VERFAHREN UND VERWENDUNGEN
CORPS MIMÉTIQUES SE LIANT AU RÉCEPTEUR DE LA MÉLANOCORTINE, COMPOSITIONS, PROCÉDÉS ET UTILISATIONS

(30) Priority: 30.10.2007 US 929046
(43) Date of publication of application: 11.08.2010
(73) Proprietor: Janssen Biotech, Inc., Horsham, PA 19044 (US)
(72) Inventor: CUNNINGHAM, Mark, Radnor, Pennsylvania 19087 (US); STOJANOVIC-SUSULIC, Vedrana, Radnor, PA 19087 (US); O'NEIL, Karyn, Radnor, Pennsylvania 19087 (US); HUANG, Chichi, Radnor, Pennsylvania 19087 (US); LUO, Jeffrey, Radnor, Pennsylvania 19087 (US)
(74) Representative: Goodfellow, Hugh Robin
(86) International application number: PCT/US2008/081734
(87) International publication number: WO 2009/134281

(56) References cited:
- WO-A2-2005/032460
- WO-A2-2006/047535
- US-A1- 2007 082 843
- Helena Strömbergsson ET AL: "Rough set-based proteochemometrics modeling of G-protein-coupled receptor-ligand interactions", Proteins: Structure, Function, and Bioinformatics, vol. 63, no. 1, 1 April 2006 (2006-04-01), pages 24-34, XP055105231, ISSN: 0887-3585, DOI: 10.1002/prot.20777
- Helena Strömbergsson ET AL: "Proteochemometrics modelling of receptor ligand interactions using rough sets", Proceedings of the German conference on Bioinformatics, 4 October 2004 (2004-10-04), XP55105253, ISBN: 978-3-88-579382-3
- Mike Clark: "An alignment of IgG sequences from Human, Mouse and Rat Sequences Human IgG1", , 1 April 2002 (2002-04-01), XP055105427, Retrieved from the Internet: URL:http://www.path.cam.ac.uk/~mrc7/lectur enotes/handout1a.pdf [retrieved on 2014-03-04]

### SEQUENCE LISTING

<110> CENTOCOR, INC.
<120> MELANOCORTIN RECEPTOR BINDING MIMETIBODIES, COMPOSITIONS, METHODS AND USES
<130> CEN5080PCT1
<140> To Be Assigned
   <141> 2008-10-30
<150> 11/929046
   <151> 2007-10-30
<150> 11/257851
   <151> 2005-10-25
<150> 60/621960
   <151> 2004-10-25
<150> 60/972018
   <151> 2007-09-13
<160> 282
<170> FastSEQ for Windows Version 4.0
<210> 1
   <211> 39
   <212> DNA
   <213> Homo sapiens
<400> 1
   tcctactcca tggagcactt ccgctggggc aagccggtg 39
<210> 2
   <211> 13
   <212> PRT
   <213> Homo sapiens
<400> 2
   Ser Tyr Ser Met Glu His Phe Arg Trp Gly Lys Pro Val
   1 5 10
<210> 3
   <211> 39
   <212> DNA
   <213> Artificial Sequence
<220>
   <223> In Vitro synthesized DNA encoding a constrained peptide sequence
<400> 3
   agctatagct gcgaacattt tcgctggtgc aaaccggtg 39
<210> 4
   <211> 13
   <212> PRT
   <213> Artificial Sequence
<220>
   <223> Constrained peptide encoded by an in vitro synthesized DNA
<400> 4
   Ser Tyr Ser Cys Glu His Phe Arg Trp Cys Lys Pro Val
   1 5 10
<210> 5
   <211> 39
   <212> DNA
   <213> Artificial Sequence
<220>
   <223> In Vitro synthesized DNA encoding a constrained peptide sequence
<400> 5
   agctattgca tggaacattt tcgctggtgc aaaccggtg 39
<210> 6
   <211> 13
   <212> PRT
   <213> Artificial Sequence
<220>
   <223> Constrained peptide encoded by an In Vitro synthesized DNA
<400> 6
   Ser Tyr Cys Met Glu His Phe Arg Trp Cys Lys Pro Val
   1 5 10
<210> 7
   <211> 39
   <212> DNA
   <213> Artificial Sequence
<220>
   <223> In Vitro synthesized DNA encoding a constrained peptide sequence
<400> 7
   agctgcagca tggaacattt tcgctggtgc aaaccggtg 39
<210> 8
   <211> 13
   <212> PRT
   <213> Artificial Sequence
<220>
   <223> Constrained peptide encoded by an In Vitro synthesized DNA
<400> 8
   Ser Cys Ser Met Glu His Phe Arg Trp Cys Lys Pro Val
    1               5                  10
<210> 9
   <211> 39
   <212> DNA
   <213> Artificial Sequence
<220>
   <223> In Vitro synthesized DNA encoding a constrained peptide sequence
<400> 9
   tgctatagca tggaacattt tcgctggggc tgcccggtg 39
<210> 10
   <211> 13
   <212> PRT
   <213> Artificial Sequence
<220>
   <223> Constrained peptide encoded by an In Vitro synthesized DNA
<400> 10
   Cys Tyr Ser Met Glu His Phe Arg Trp Gly Cys Pro Val
    1               5                  10
<210> 11
   <211> 45
   <212> DNA
   <213> Artificial Sequence
<220>
   <223> In Vitro synthesized DNA encoding a constrained peptide sequence
<400> 11
   agctggagct gggaacattt tcgctggggc aaatggacct ggaaa 45
<210> 12
   <211> 15
   <212> PRT
   <213> Artificial Sequence
<220>
   <223> Constrained peptide encoded by an In Vitro synthesized DNA
<400> 12
   Ser Trp Ser Trp Glu His Phe Arg Trp Gly Lys Trp Thr Trp Lys
    1               5                   10                  15
<210> 13
   <211> 51
   <212> DNA
   <213> Artificial Sequence
<220>
   <223> In Vitro synthesized DNA encoding a constrained peptide sequence
<400> 13
   agctggagct ggggcgaaca ttttcgctgg ggcaaaggct ggacctggaa a 51
<210> 14
   <211> 16
   <212> PRT
   <213> Artificial Sequence
<220>
   <223> Constrained peptide encoded by an In Vitro synthesized DNA
<400> 14
   Ser Trp Ser Trp Gly Glu His Phe Arg Trp Gly Lys Gly Trp Thr Trp
    1               5                   10                  15
<210> 15
   <211> 57
   <212> DNA
   <213> Artificial Sequence
<220>
   <223> In Vitro synthesized DNA encoding a constrained peptide sequence
<400> 15
   agctggagct ggggcggcga acattttcgc tggggcaaag gcggctggac ctggaaa 57
<210> 16
   <211> 16
   <212> PRT
   <213> Artificial Sequence
<220>
   <223> Constrained peptide encoded by an In Vitro synthesized DNA
<400> 16
   Ser Trp Ser Trp Gly Gly Glu His Phe Arg Trp Gly Lys Gly Gly Trp
    1               5                   10                  15
<210> 17
   <211> 57
   <212> DNA
   <213> Artificial Sequence
<220>
   <223> In Vitro synthesized DNA encoding a constrained peptide sequence
<400> 17
   agctggagct ggagcatgga acattttcgc tggggcaaac cggtgtggac ctggaaa 57
<210> 18
   <211> 16
   <212> PRT
   <213> Artificial Sequence
<220>
   <223> Constrained peptide encoded by an In Vitro synthesized DNA
<400> 18
   Ser Trp Ser Trp Ser Met Glu His Phe Arg Trp Gly Lys Pro Val Trp
   1               5                   10                  15
<210> 19
   <211> 12
   <212> DNA
   <213> Artificial Sequence
<220>
   <223> In Vitro synthesized DNA encoding a flexible peptide sequence
<400> 19
   ggcagcggca gc 12
<210> 20
   <211> 4
   <212> PRT
   <213> Artificial Sequence
<220>
   <223> Flexible peptide encoded by an In Vitro synthesized DNA
<400> 20
   Gly Ser Gly Ser
   1
<210> 21
   <211> 12
   <212> DNA
   <213> Artificial Sequence
<220>
   <223> In Vitro synthesized DNA encoding a flexible peptide sequence
<400> 21
   ggcggcagcg gc 12
<210> 22
   <211> 4
   <212> PRT
   <213> Artificial Sequence
<220>
   <223> Flexible peptide encoded by an In Vitro synthesized DNA
<400> 22
   Gly Gly Ser Gly
   1
<210> 23
   <211> 18
   <212> DNA
   <213> Artificial Sequence
<220>
   <223> In Vitro synthesized DNA encoding a flexible peptide sequence
<400> 23
   ggcagcggcg gcggcagc 18
<210> 24
   <211> 6
   <212> PRT
   <213> Artificial Sequence
<220>
   <223> Flexible peptide encoded by an In Vitro synthesized DNA
<400> 24
   Gly Ser Gly Gly Gly Ser
   1 5
<210> 25
   <211> 21
   <212> DNA
   <213> Artificial Sequence
<220>
   <223> In Vitro synthesized DNA encoding a flexible peptide sequence
<400> 25
   ggcagcggcg gcggcagcgg c 21
<210> 26
   <211> 7
   <212> PRT
   <213> Artificial Sequence
<220>
   <223> Flexible peptide encoded by an In Vitro synthesized DNA
<400> 26
   Gly Ser Gly Gly Gly Ser Gly
   1 5
<210> 27
   <211> 12
   <212> DNA
   <213> Artificial Sequence
<220>
   <223> In Vitro synthesized DNA encoding a flexible peptide sequence
<400> 27
   ggcagcagcg gc 12
<210> 28
   <211> 4
   <212> PRT
   <213> Artificial Sequence
<220>
   <223> Flexible peptide encoded by an In Vitro synthesized DNA
<400> 28
   Gly Ser Ser Gly
   1
<210> 29
   <211> 18
   <212> DNA
   <213> Artificial Sequence
<220>
   <223> In Vitro synthesized DNA encoding a flexible peptide sequence
<400> 29
   ggcagcggcg gcggcagc 18
<210> 30
   <211> 6
   <212> PRT
   <213> Artificial Sequence
<220>
   <223> Flexible peptide encoded by an In Vitro synthesized DNA
<400> 30
   Gly Ser Gly Gly Gly Ser
   1 5
<210> 31
   <211> 24
   <212> DNA
   <213> Homo sapiens
<400> 31
   ggcaccctgg tgaccgtgag cagc 24
<210> 32
   <211> 8
   <212> PRT
   <213> Homo sapiens
<400> 32
   Gly Thr Leu Val Thr Val Ser Ser
   1 5
<210> 33
   <211> 21
   <212> DNA
   <213> Artificial Sequence
<220>
   <223> In Vitro mutagenized homo sapien DNA encoding a V2 peptide sequence comprising a T to A substitution to limit O-linked glycosylation
<400> 33
   accctggtgg cggtgagcag c 21
<210> 34
   <211> 7
   <212> PRT
   <213> Artificial Sequence
<220>
   <223> Mutagenized homo sapien V2 peptide sequence comprising a T to A substitution to limit O-linked glycosylation and encoded by an In Vitro mutagenized homo sapien DNA
<400> 34
   Thr Leu Val Ala Val Ser Ser
   1 5
<210> 35
   <211> 45
   <212> DNA
   <213> Homo sapeins
<400> 35
   gaaccgaaaa gctgcgataa aacccatacc tgcccgccgt gcccg 45
<210> 36
   <211> 15
   <212> PRT
   <213> Homo sapiens
<400> 36
   Glu Pro Lys Ser Cys Asp Lys Thr His Thr Cys Pro Pro Cys Pro
   1 5 10 15
<210> 37
   <211> 45
   <212> DNA
   <213> Homo sapiens
<400> 37
   gaaccgaaaa gcgcggataa aacccatacc tgcccgccgt gcccg 45
<210> 38
   <211> 15
   <212> PRT
   <213> Homo sapiens
<400> 38
   Glu Pro Lys Ser Ala Asp Lys Thr His Thr Cys Pro Pro Cys Pro
   1 5 10 15
<210> 39
   <211> 36
   <212> DNA
   <213> Homo sapiens
<400> 39
   gaaagcaaat atggcccgcc gtgcccgagc tgcccg 36
<210> 40
   <211> 12
   <212> PRT
   <213> Homo sapiens
<400> 40
   Glu Ser Lys Tyr Gly Pro Pro Cys Pro Ser Cys Pro
   1 5 10
<210> 41
   <211> 36
   <212> DNA
   <213> Homo sapiens
<400> 41
   gaaagcaaat atggcccgcc gtgcccgccg tgcccg 36
<210> 42
   <211> 12
   <212> PRT
   <213> Homo sapiens
<400> 42
   Glu Ser Lys Tyr Gly Pro Pro Cys Pro Pro Cys Pro
   1 5 10
<210> 43
   <211> 15
   <212> DNA
   <213> Homo sapiens
<400> 43
   tgcccgccgt gcccg 15
<210> 44
   <211> 5
   <212> PRT
   <213> Homo sapiens
<400> 44
   Cys Pro Pro Cys Pro
   1 5
<210> 45
   <211> 12
   <212> DNA
   <213> Homo sapiens
<400> 45
   tgcccgagct gc 12
<210> 46
   <211> 4
   <212> PRT
   <213> Homo sapiens
<400> 46
   Cys Pro Ser Cys
   1
<210> 47
   <211> 330
   <212> DNA
   <213> Homo sapiens
<400> 47
   gcgccggaac tgctgggcgg cccgagcgtg tttctgtttc cgccgaaacc gaaagatacc 60
   ctgatgatta gccgcacccc ggaagtgacc tgcgtggtgg tggatgtgag ccatgaagat 120
   ccggaagtga aatttaactg gtatgtggat ggcgtggaag tgcataacgc gaaaaccaaa 180
   ccgcgcgaag aacagtataa cagcacctat cgcgtggtga gcgtgctgac cgtgctgcat 240
   caggattggc tgaacggcaa agaatataaa tgcaaagtga gcaacaaagc gctgccggcg 300
   ccgattgaaa aaaccattag caaagcgaaa                                  330
<210> 48
   <211> 110
   <212> PRT
   <213> Homo sapiens
<400> 48
   Ala Pro Glu Leu Leu Gly Gly Pro Ser Val Phe Leu Phe Pro Pro Lys
    1              5                   10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys
               100                 105                 110
<210> 49
   <211> 330
   <212> DNA
   <213> Homo sapiens
<400> 49
   gcgccggaag cggcgggcgg cccgagcgtg tttctgtttc cgccgaaacc gaaagatacc 60
   ctgatgatta gccgcacccc ggaagtgacc tgcgtggtgg tggatgtgag ccatgaagat 120
   ccggaagtga aatttaactg gtatgtggat ggcgtggaag tgcataacgc gaaaaccaaa 180
   ccgcgcgaag aacagtataa cagcacctat cgcgtggtga gcgtgctgac cgtgctgcat 240
   caggattggc tgaacggcaa agaatataaa tgcaaagtga gcaacaaagc gctgccggcg 300
   ccgattgaaa aaaccattag caaagcgaaa                                  330
<210> 50
   <211> 110
   <212> PRT
   <213> Homo sapiens
<400> 50
   Ala Pro Glu Ala Ala Gly Gly Pro Ser Val Phe Leu Phe Pro Pro Lys
    1              5                   10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys
               100                 105                 110
<210> 51
   <211> 330
   <212> DNA
   <213> Homo sapiens
<400> 51
   gcgccggaat ttctgggcgg cccgagcgtg tttctgtttc cgccgaaacc gaaagatacc 60
   ctgatgatta gccgcacccc ggaagtgacc tgcgtggtgg tggatgtgag ccaggaagat 120
   ccggaagtgc agtttaactg gtatgtggat ggcgtggaag tgcataacgc gaaaaccaaa 180
   ccgcgcgaag aacagtttaa cagcacctat cgcgtggtga gcgtgctgac cgtgctgcat 240
   caggattggc tgaacggcaa agaatataaa tgcaaagtga gcaacaaagg cctgccgagc 300
   agcattgaaa aaaccattag caaagcgaaa                                  330
<210> 52
   <211> 110
   <212> PRT
   <213> Homo sapiens
<400> 52
   Ala Pro Glu Phe Leu Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser Gln Glu Asp Pro Glu Val Gln Phe Asn Trp Tyr
           35                  40                  45
   Val Asp Gly Val Glu Val His Asn Ala Lys Thr Lys Pro Arg Glu Glu
       50                  55                  60
   Gln Phe Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Gly Leu Pro Ser Ser Ile Glu Lys Thr Ile Ser Lys Ala Lys
               100                 105                 110
<210> 53
   <211> 330
   <212> DNA
   <213> Homo sapiens
<400> 53
   gcgccggaag cggcgggcgg cccgagcgtg tttctgtttc cgccgaaacc gaaagatacc 60
   ctgatgatta gccgcacccc ggaagtgacc tgcgtggtgg tggatgtgag ccaggaagat 120
   ccggaagtgc agtttaactg gtatgtggat ggcgtggaag tgcataacgc gaaaaccaaa 180
   ccgcgcgaag aacagtttaa cagcacctat cgcgtggtga gcgtgctgac cgtgctgcat 240
   caggattggc tgaacggcaa agaatataaa tgcaaagtga gcaacaaagg cctgccgagc 300
   agcattgaaa aaaccattag caaagcgaaa                                  330
<210> 54
   <211> 110
   <212> PRT
   <213> Homo sapiens
<400> 54
   Ala Pro Glu Ala Ala Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser Gln Glu Asp Pro Glu Val Gln Phe Asn Trp Tyr
           35                  40                  45
   Val Asp Gly Val Glu Val His Asn Ala Lys Thr Lys Pro Arg Glu Glu
       50                  55                  60
   Gln Phe Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Gly Leu Pro Ser Ser Ile Glu Lys Thr Ile Ser Lys Ala Lys
               100                 105                 110
<210> 55
   <211> 321
   <212> DNA
   <213> Homo sapiens
<400> 55
   ggccagccgc gcgaaccgca ggtgtatacc ctgccgccga gccgcgatga actgaccaaa 60
   aaccaggtga gcctgacctg cctggtgaaa ggcttttatc cgagcgatat tgcggtggaa 120
   tgggaaagca acggccagcc ggaaaacaac tataaaacca ccccgccggt gctggatagc 180
   gatggcagct tttttctgta tagcaaactg accgtggata aaagccgctg gcagcagggc 240
   aacgtgttta gctgcagcgt gatgcatgaa gcgctgcata accattatac ccagaaaagc 300
   ctgagcctga gcccgggcaa a                                           321
<210> 56
   <211> 107
   <212> PRT
   <213> Homo sapiens
<400> 56
   Gly Gln Pro Arg Glu Pro Gln Val Tyr Thr Leu Pro Pro Ser Arg Asp
    1               5                  10                  15
   Glu Leu Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Lys Leu Thr Val Asp Lys Ser Arg Trp Gln Gln Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Pro Gly Lys
               100                 105
<210> 57
   <211> 321
   <212> DNA
   <213> Homo sapiens
<400> 57
   ggccagccgc gcgaaccgca ggtgtatacc ctgccgccga gccaggaaga aatgaccaaa 60
   aaccaggtga gcctgacctg cctggtgaaa ggcttttatc cgagcgatat tgcggtggaa 120
   tgggaaagca acggccagcc ggaaaacaac tataaaacca ccccgccggt gctggatagc 180
   gatggcagct tttttctgta tagccgcctg accgtggata aaagccgctg gcaggaaggc 240
   aacgtgttta gctgcagcgt gatgcatgaa gcgctgcata accattatac ccagaaaagc 300
   ctgagcctga gcctgggcaa a                                          321
<210> 58
   <211> 107
   <212> PRT
   <213> Homo sapiens
<400> 58
   Gly Gln Pro Arg Glu Pro Gln Val Tyr Thr Leu Pro Pro Ser Gln Glu
    1               5                  10                  15
   Glu Met Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Arg Leu Thr Val Asp Lys Ser Arg Trp Gln Glu Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Leu Gly Lys
               100                 105
<210> 59
   <211> 777
   <212> DNA
   <213> Artificial Sequence
<220>
   <223> In vitro synthesized DNA encoding a melanocortin receptor binding alpha-MSH mimetibody without secretory signal (p3024)
<400> 59
   tcctactcca tggagcactt ccgctggggc aagccggtgg gatccggtgg aggctccggt 60
   accttagtca ccgtctcctc agagcccaaa tcttgtgaca aaactcacac gtgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccggg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 60
   <211> 259
   <212> PRT
   <213> Artificial Sequence
<220>
   <223> Melanocortin receptor binding alpha-MSH Mimetibody without secretory signal encoded by an in vitro synthesized DNA (p3024).
<400> 60
   Ser Tyr Ser Met Glu His Phe Arg Trp Gly Lys Pro Val Gly Ser Gly
    1               5                  10                  15
   Gly Gly Ser Gly Thr Leu Val Thr Val Ser Ser Glu Pro Lys Ser Cys
               20                  25                  30
   Asp Lys Thr His Thr Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 61
   <211> 843
   <212> DNA
   <213> Artificial Sequence
<220>
   <223> In Vitro synthesized DNA encoding a Melanocortin receptor binding alpha-MSH mimetibody with secretory signal and V1
<400> 61
   atggcttggg tgtggacctt gctattcctg atggcggccg cccaaagtat acaggcccag 60
   atccagtcct actccatgga gcacttccgc tggggcaagc cggtgggatc cggtggaggc 120
   tccggtacct tagtcaccgt ctcctcagag cccaaatctt gtgacaaaac tcacacgtgc 180
   ccaccgtgcc cagcacctga actcctgggg ggaccgtcag tcttcctctt ccccccaaaa 240
   cccaaggaca ccctcatgat ctcccggacc cctgaggtca catgcgtggt ggtggacgtg 300
   agccacgaag accctgaggt caagttcaac tggtacgtgg acggcgtgga ggtgcataat 360
   gccaagacaa agccgcggga ggagcagtac aacagcacgt accgggtggt cagcgtcctc 420
   accgtcctgc accaggactg gctgaatggc aaggagtaca agtgcaaggt ctccaacaaa 480
   gccctcccag cccccatcga gaaaaccatc tccaaagcca aagggcagcc ccgagaacca 540
   caggtgtaca ccctgccccc atcccgggat gagctgacca agaaccaggt cagcctgacc 600
   tgcctggtca aaggcttcta tcccagcgac atcgccgtgg agtgggagag caatgggcag 660
   ccggagaaca actacaagac cacgcctccc gtgctggact ccgacggctc cttcttcctc 720
   tacagcaagc tcaccgtgga caagagcagg tggcagcagg ggaacgtctt ctcatgctcc 780
   gtgatgcatg aggctctgca caaccactac acgcagaaga gcctctccct gtctccgggt 840
   aaa                                                               843
<210> 62
   <211> 281
   <212> PRT
   <213> Artificial Sequence
<220>
   <223> Melanocortin receptor binding alpha-MSH mimetibody with secretory signal and V1 encoded by an in vitro synthesized DNA
<400> 62
   Met Ala Trp Val Trp Thr Leu Leu Phe Leu Met Ala Ala Ala Gln Ser
    1               5                  10                  15
   Ile Gln Ala Gln Ile Gln Ser Tyr Ser Met Glu His Phe Arg Trp Gly
               20                  25                  30
   Lys Pro Val Gly Ser Gly Gly Gly Ser Gly Thr Leu Val Thr Val Ser
           35                  40                  45
   Ser Glu Pro Lys Ser Cys Asp Lys Thr His Thr Cys Pro Pro Cys Pro
       50                  55                  60
   Ala Pro Glu Leu Leu Gly Gly Pro Ser Val Phe Leu Phe Pro Pro Lys
   65                  70                  75                  80
   Pro Lys Asp Thr Leu Met Ile Ser Arg Thr Pro Glu Val Thr Cys Val
                   85                  90                  95
   Val Val Asp Val Ser His Glu Asp Pro Glu Val Lys Phe Asn Trp Tyr
               100                 105                 110
   Val Asp Gly Val Glu Val His Asn Ala Lys Thr Lys Pro Arg Glu Glu
           115                 120                 125
   Gln Tyr Asn Ser Thr Tyr Arg Val Val Ser Val Leu Thr Val Leu His
       130                 135                 140
   Gln Asp Trp Leu Asn Gly Lys Glu Tyr Lys Cys Lys Val Ser Asn Lys
   145                 150                 155                 160
   Ala Leu Pro Ala Pro Ile Glu Lys Thr Ile Ser Lys Ala Lys Gly Gln
                   165                 170                 175
   Pro Arg Glu Pro Gln Val Tyr Thr Leu Pro Pro Ser Arg Asp Glu Leu
               180                 185                 190
   Thr Lys Asn Gln Val Ser Leu Thr Cys Leu Val Lys Gly Phe Tyr Pro
           195                 200                 205
   Ser Asp Ile Ala Val Glu Trp Glu Ser Asn Gly Gln Pro Glu Asn Asn
       210                 215                 220
   Tyr Lys Thr Thr Pro Pro Val Leu Asp Ser Asp Gly Ser Phe Phe Leu
   225                 230                 235                 240
   Tyr Ser Lys Leu Thr Val Asp Lys Ser Arg Trp Gln Gln Gly Asn Val
                   245                 250                 255
   Phe Ser Cys Ser Val Met His Glu Ala Leu His Asn His Tyr Thr Gln
               260                 265                 270
   Lys Ser Leu Ser Leu Ser Pro Gly Lys
           275                 280
<210> 63
   <211> 11978
   <212> DNA
   <213> Artificial Sequence
<220>
   <223> In vitro synthesized DNA with expression vector functions that encodes an alha-MSH mimetibody
<400> 63
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcccag atccagtcct actccatgga 1680
   gcacttccgc tggggcaagc cggtgggatc cggtggaggc tccggtacct tagtcaccgt 1740
   ctcctcagag cccaaatctt gtgacaaaac tcacacgtgc ccaccgtgcc cagcacctga 1800
   actcctgggg ggaccgtcag tcttcctctt ccccccaaaa cccaaggaca ccctcatgat 1860
   ctcccggacc cctgaggtca catgcgtggt ggtggacgtg agccacgaag accctgaggt 1920
   caagttcaac tggtacgtgg acggcgtgga ggtgcataat gccaagacaa agccgcggga 1980
   ggagcagtac aacagcacgt accgggtggt cagcgtcctc accgtcctgc accaggactg 2040
   gctgaatggc aaggagtaca agtgcaaggt ctccaacaaa gccctcccag cccccatcga 2100
   gaaaaccatc tccaaagcca aagggcagcc ccgagaacca caggtgtaca ccctgccccc 2160
   atcccgggat gagctgacca agaaccaggt cagcctgacc tgcctggtca aaggcttcta 2220
   tcccagcgac atcgccgtgg agtgggagag caatgggcag ccggagaaca actacaagac 2280
   cacgcctccc gtgctggact ccgacggctc cttcttcctc tacagcaagc tcaccgtgga 2340
   caagagcagg tggcagcagg ggaacgtctt ctcatgctcc gtgatgcatg aggctctgca 2400
   caaccactac acgcagaaga gcctctccct gtctccgggt aaatgagggc ccgtttaaac 2460
   agatccagac atgataagat acattgatga gtttggacaa accacaacta gaatgcagtg 2520
   aaaaaaatgc tttatttgtg aaatttgtga tgctattgct ttatttgtaa ccattataag 2580
   ctgcaataaa caagttaaca acaacaattg cattcatttt atgtttcagg ttcaggggga 2640
   ggtgtgggag gttttttaaa gcaagtaaaa cctctacaaa tgtggtatgg ctgattatga 2700
   tccggctgcc tcgcgcgttt cggtgatgac ggtgaaaacc tctgacacat gcagctcccg 2760
   gagacggtca cagcttgtct gtaagcggat gccgggagca gacaagcccg tcaggcgtca 2820
   gcgggtgttg gcgggtgtcg gggcgcagcc atgaggtcga ctctagagga tcgatgcccc 2880
   gccccggacg aactaaacct gactacgaca tctctgcccc ttcttcgcgg ggcagtgcat 2940
   gtaatccctt cagttggttg gtacaacttg ccaactgggc cctgttccac atgtgacacg 3000
   gggggggacc aaacacaaag gggttctctg actgtagttg acatccttat aaatggatgt 3060
   gcacatttgc caacactgag tggctttcat cctggagcag actttgcagt ctgtggactg 3120
   caacacaaca ttgcctttat gtgtaactct tggctgaagc tcttacacca atgctggggg 3180
   acatgtacct cccaggggcc caggaagact acgggaggct acaccaacgt caatcagagg 3240
   ggcctgtgta gctaccgata agcggaccct caagagggca ttagcaatag tgtttataag 3300
   gcccccttgt taaccctaaa cgggtagcat atgcttcccg ggtagtagta tatactatcc 3360
   agactaaccc taattcaata gcatatgtta cccaacggga agcatatgct atcgaattag 3420
   ggttagtaaa agggtcctaa ggaacagcga tatctcccac cccatgagct gtcacggttt 3480
   tatttacatg gggtcaggat tccacgaggg tagtgaacca ttttagtcac aagggcagtg 3540
   gctgaagatc aaggagcggg cagtgaactc tcctgaatct tcgcctgctt cttcattctc 3600
   cttcgtttag ctaatagaat aactgctgag ttgtgaacag taaggtgtat gtgaggtgct 3660
   cgaaaacaag gtttcaggtg acgcccccag aataaaattt ggacgggggg ttcagtggtg 3720
   gcattgtgct atgacaccaa tataaccctc acaaacccct tgggcaataa atactagtgt 3780
   aggaatgaaa cattctgaat atctttaaca atagaaatcc atggggtggg gacaagccgt 3840
   aaagactgga tgtccatctc acacgaattt atggctatgg gcaacacata atcctagtgc 3900
   aatatgatac tggggttatt aagatgtgtc ccaggcaggg accaagacag gtgaaccatg 3960
   ttgttacact ctatttgtaa caaggggaaa gagagtggac gccgacagca gcggactcca 4020
   ctggttgtct ctaacacccc cgaaaattaa acggggctcc acgccaatgg ggcccataaa 4080
   caaagacaag tggccactct tttttttgaa attgtggagt gggggcacgc gtcagccccc 4140
   acacgccgcc ctgcggtttt ggactgtaaa ataagggtgt aataacttgg ctgattgtaa 4200
   ccccgctaac cactgcggtc aaaccacttg cccacaaaac cactaatggc accccgggga 4260
   atacctgcat aagtaggtgg gcgggccaag ataggggcgc gattgctgcg atctggagga 4320
   caaattacac acacttgcgc ctgagcgcca agcacagggt tgttggtcct catattcacg 4380
   aggtcgctga gagcacggtg ggctaatgtt gccatgggta gcatatacta cccaaatatc 4440
   tggatagcat atgctatcct aatctatatc tgggtagcat aggctatcct aatctatatc 4500
   tgggtagcat atgctatcct aatctatatc tgggtagtat atgctatcct aatttatatc 4560
   tgggtagcat aggctatcct aatctatatc tgggtagcat atgctatcct aatctatatc 4620
   tgggtagtat atgctatcct aatctgtatc cgggtagcat atgctatcct aatagagatt 4680
   agggtagtat atgctatcct aatttatatc tgggtagcat atactaccca aatatctgga 4740
   tagcatatgc tatcctaatc tatatctggg tagcatatgc tatcctaatc tatatctggg 4800
   tagcataggc tatcctaatc tatatctggg tagcatatgc tatcctaatc tatatctggg 4860
   tagtatatgc tatcctaatt tatatctggg tagcataggc tatcctaatc tatatctggg 4920
   tagcatatgc tatcctaatc tatatctggg tagtatatgc tatcctaatc tgtatccggg 4980
   tagcatatgc tatcctcatg catatacagt cagcatatga tacccagtag tagagtggga 5040
   gtgctatcct ttgcatatgc cgccacctcc caagggggcg tgaattttcg ctgcttgtcc 5100
   ttttcctgct ggttgctccc attcttaggt gaatttaagg aggccaggct aaagccgtcg 5160
   catgtctgat tgctcaccag gtaaatgtcg ctaatgtttt ccaacgcgag aaggtgttga 5220
   gcgcggagct gagtgacgtg acaacatggg tatgcccaat tgccccatgt tgggaggacg 5280
   aaaatggtga caagacagat ggccagaaat acaccaacag cacgcatgat gtctactggg 5340
   gatttattct ttagtgcggg ggaatacacg gcttttaata cgattgaggg cgtctcctaa 5400
   caagttacat cactcctgcc cttcctcacc ctcatctcca tcacctcctt catctccgtc 5460
   atctccgtca tcaccctccg cggcagcccc ttccaccata ggtggaaacc agggaggcaa 5520
   atctactcca tcgtcaaagc tgcacacagt caccctgata ttgcaggtag gagcgggctt 5580
   tgtcataaca aggtccttaa tcgcatcctt caaaacctca gcaaatatat gagtttgtaa 5640
   aaagaccatg aaataacaga caatggactc ccttagcggg ccaggttgtg ggccgggtcc 5700
   aggggccatt ccaaagggga gacgactcaa tggtgtaaga cgacattgtg gaatagcaag 5760
   ggcagttcct cgccttaggt tgtaaaggga ggtcttacta cctccatata cgaacacacc 5820
   ggcgacccaa gttccttcgt cggtagtcct ttctacgtga ctcctagcca ggagagctct 5880
   taaaccttct gcaatgttct caaatttcgg gttggaacct ccttgaccac gatgcttttc 5940
   caaaccaccc tccttttttg cgccctgcct ccatcaccct gaccccgggg tccagtgctt 6000
   gggccttctc ctgggtcatc tgcggggccc tgctctatcg ctcccggggg cacgtcaggc 6060
   tcaccatctg ggccaccttc ttggtggtat tcaaaataat cggcttcccc tacagggtgg 6120
   aaaaatggcc ttctacctgg agggggcctg cgcggtggag acccggatga tgatgactga 6180
   ctactgggac tcctgggcct cttttctcca cgtccacgac ctctccccct ggctctttca 6240
   cgacttcccc ccctggctct ttcacgtcct ctaccccggc ggcctccact acctcctcga 6300
   ccccggcctc cactacctcc tcgaccccgg cctccactgc ctcctcgacc ccggcctcca 6360
   cctcctgctc ctgcccctcc tgctcctgcc cctcctcctg ctcctgcccc tcctgcccct 6420
   cctgctcctg cccctcctgc ccctcctgct cctgcccctc ctgcccctcc tgctcctgcc 6480
   cctcctgccc ctcctcctgc tcctgcccct cctgcccctc ctcctgctcc tgcccctcct 6540
   gcccctcctg ctcctgcccc tcctgcccct cctgctcctg cccctcctgc ccctcctgct 6600
   cctgcccctc ctgctcctgc ccctcctgct cctgcccctc ctgctcctgc ccctcctgcc 6660
   cctcctgccc ctcctcctgc tcctgcccct cctgctcctg cccctcctgc ccctcctgcc 6720
   cctcctgctc ctgcccctcc tcctgctcct gcccctcctg cccctcctgc ccctcctcct 6780
   gctcctgccc ctcctgcccc tcctcctgct cctgcccctc ctcctgctcc tgcccctcct 6840
   gcccctcctg cccctcctcc tgctcctgcc cctcctgccc ctcctcctgc tcctgcccct 6900
   cctcctgctc ctgcccctcc tgcccctcct gcccctcctc ctgctcctgc ccctcctcct 6960
   gctcctgccc ctcctgcccc tcctgcccct cctgcccctc ctcctgctcc tgcccctcct 7020
   cctgctcctg cccctcctgc tcctgcccct cccgctcctg ctcctgctcc tgttccaccg 7080
   tgggtccctt tgcagccaat gcaacttgga cgtttttggg gtctccggac accatctcta 7140
   tgtcttggcc ctgatcctga gccgcccggg gctcctggtc ttccgcctcc tcgtcctcgt 7200
   cctcttcccc gtcctcgtcc atggttatca ccccctcttc tttgaggtcc actgccgccg 7260
   gagccttctg gtccagatgt gtctcccttc tctcctaggc catttccagg tcctgtacct 7320
   ggcccctcgt cagacatgat tcacactaaa agagatcaat agacatcttt attagacgac 7380
   gctcagtgaa tacagggagt gcagactcct gccccctcca acagcccccc caccctcatc 7440
   cccttcatgg tcgctgtcag acagatccag gtctgaaaat tccccatcct ccgaaccatc 7500
   ctcgtcctca tcaccaatta ctcgcagccc ggaaaactcc cgctgaacat cctcaagatt 7560
   tgcgtcctga gcctcaagcc aggcctcaaa ttcctcgtcc ccctttttgc tggacggtag 7620
   ggatggggat tctcgggacc cctcctcttc ctcttcaagg tcaccagaca gagatgctac 7680
   tggggcaacg gaagaaaagc tgggtgcggc ctgtgaggat cagcttatcg atgataagct 7740
   gtcaaacatg agaattcttg aagacgaaag ggcctcgtga tacgcctatt tttataggtt 7800
   aatgtcatga taataatggt ttcttagacg tcaggtggca cttttcgggg aaatgtgcgc 7860
   ggaaccccta tttgtttatt tttctaaata cattcaaata tgtatccgct catgagacaa 7920
   taaccctgat aaatgcttca ataatattga aaaaggaaga gtatgagtat tcaacatttc 7980
   cgtgtcgccc ttattccctt ttttgcggca ttttgccttc ctgtttttgc tcacccagaa 8040
   acgctggtga aagtaaaaga tgctgaagat cagttgggtg cacgagtggg ttacatcgaa 8100
   ctggatctca acagcggtaa gatccttgag agttttcgcc ccgaagaacg ttttccaatg 8160
   atgagcactt ttaaagttct gctatgtggc gcggtattat cccgtgttga cgccgggcaa 8220
   gagcaactcg gtcgccgcat acactattct cagaatgact tggttgagta ctcaccagtc 8280
   acagaaaagc atcttacgga tggcatgaca gtaagagaat tatgcagtgc tgccataacc 8340
   atgagtgata acactgcggc caacttactt ctgacaacga tcggaggacc gaaggagcta 8400
   accgcttttt tgcacaacat gggggatcat gtaactcgcc ttgatcgttg ggaaccggag 8460
   ctgaatgaag ccataccaaa cgacgagcgt gacaccacga tgcctgcagc aatggcaaca 8520
   acgttgcgca aactattaac tggcgaacta cttactctag cttcccggca acaattaata 8580
   gactggatgg aggcggataa agttgcagga ccacttctgc gctcggccct tccggctggc 8640
   tggtttattg ctgataaatc tggagccggt gagcgtgggt ctcgcggtat cattgcagca 8700
   ctggggccag atggtaagcc ctcccgtatc gtagttatct acacgacggg gagtcaggca 8760
   actatggatg aacgaaatag acagatcgct gagataggtg cctcactgat taagcattgg 8820
   taactgtcag accaagttta ctcatatata ctttagattg atttaaaact tcatttttaa 8880
   tttaaaagga tctaggtgaa gatccttttt gataatctca tgaccaaaat cccttaacgt 8940
   gagttttcgt tccactgagc gtcagacccc gtagaaaaga tcaaaggatc ttcttgagat 9000
   cctttttttc tgcgcgtaat ctgctgcttg caaacaaaaa aaccaccgct accagcggtg 9060
   gtttgtttgc cggatcaaga gctaccaact ctttttccga aggtaactgg cttcagcaga 9120
   gcgcagatac caaatactgt ccttctagtg tagccgtagt taggccacca cttcaagaac 9180
   tctgtagcac cgcctacata cctcgctctg ctaatcctgt taccagtggc tgctgccagt 9240
   ggcgataagt cgtgtcttac cgggttggac tcaagacgat agttaccgga taaggcgcag 9300
   cggtcgggct gaacgggggg ttcgtgcaca cagcccagct tggagcgaac gacctacacc 9360
   gaactgagat acctacagcg tgagctatga gaaagcgcca cgcttcccga agggagaaag 9420
   gcggacaggt atccggtaag cggcagggtc ggaacaggag agcgcacgag ggagcttcca 9480
   gggggaaacg cctggtatct ttatagtcct gtcgggtttc gccacctctg acttgagcgt 9540
   cgatttttgt gatgctcgtc aggggggcgg agcctatgga aaaacgccag caacgcggcc 9600
   tttttacggt tcctggcctt ttgctggcct tgaagctgtc cctgatggtc gtcatctacc 9660
   tgcctggaca gcatggcctg caacgcgggc atcccgatgc cgccggaagc gagaagaatc 9720
   ataatgggga aggccatcca gcctcgcgtc gcgaacgcca gcaagacgta gcccagcgcg 9780
   tcggccccga gatgcgccgc gtgcggctgc tggagatggc ggacgcgatg gatatgttct 9840
   gccaagggtt ggtttgcgca ttcacagttc tccgcaagaa ttgattggct ccaattcttg 9900
   gagtggtgaa tccgttagcg aggtgccgcc ctgcttcatc cccgtggccc gttgctcgcg 9960
   tttgctggcg gtgtccccgg aagaaatata tttgcatgtc tttagttcta tgatgacaca 10020
   aaccccgccc agcgtcttgt cattggcgaa ttcgaacacg cagatgcagt cggggcggcg 10080
   cggtccgagg tccacttcgc atattaaggt gacgcgtgtg gcctcgaaca ccgagcgacc 10140
   ctgcagcgac ccgcttaaca gcgtcaacag cgtgccgcag atcccggggg gcaatgagat 10200
   atgaaaaagc ctgaactcac cgcgacgtct gtcgagaagt ttctgatcga aaagttcgac 10260
   agcgtctccg acctgatgca gctctcggag ggcgaagaat ctcgtgcttt cagcttcgat 10320
   gtaggagggc gtggatatgt cctgcgggta aatagctgcg ccgatggttt ctacaaagat 10380
   cgttatgttt atcggcactt tgcatcggcc gcgctcccga ttccggaagt gcttgacatt 10440
   ggggaattca gcgagagcct gacctattgc atctcccgcc gtgcacaggg tgtcacgttg 10500
   caagacctgc ctgaaaccga actgcccgct gttctgcagc cggtcgcgga ggccatggat 10560
   gcgatcgctg cggccgatct tagccagacg agcgggttcg gcccattcgg accgcaagga 10620
   atcggtcaat acactacatg gcgtgatttc atatgcgcga ttgctgatcc ccatgtgtat 10680
   cactggcaaa ctgtgatgga cgacaccgtc agtgcgtccg tcgcgcaggc tctcgatgag 10740
   ctgatgcttt gggccgagga ctgccccgaa gtccggcacc tcgtgcacgc ggatttcggc 10800
   tccaacaatg tcctgacgga caatggccgc ataacagcgg tcattgactg gagcgaggcg 10860
   atgttcgggg attcccaata cgaggtcgcc aacatcttct tctggaggcc gtggttggct 10920
   tgtatggagc agcagacgcg ctacttcgag cggaggcatc cggagcttgc aggatcgccg 10980
   cggctccggg cgtatatgct ccgcattggt cttgaccaac tctatcagag cttggttgac 11040
   ggcaatttcg atgatgcagc ttgggcgcag ggtcgatgcg acgcaatcgt ccgatccgga 11100
   gccgggactg tcgggcgtac acaaatcgcc cgcagaagcg cggccgtctg gaccgatggc 11160
   tgtgtagaag tactcgccga tagtggaaac cgacgcccca gcactcgtcc ggatcgggag 11220
   atgggggagg ctaactgaaa cacggaagga gacaataccg gaaggaaccc gcgctatgac 11280
   ggcaataaaa agacagaata aaacgcacgg gtgttgggtc gtttgttcat aaacgcgggg 11340
   ttcggtccca gggctggcac tctgtcgata ccccaccgag accccattgg ggccaatacg 11400
   cccgcgtttc ttccttttcc ccaccccacc ccccaagttc gggtgaaggc ccagggctcg 11460
   cagccaacgt cggggcggca ggccctgcca tagccactgg ccccgtgggt tagggacggg 11520
   gtcccccatg gggaatggtt tatggttcgt gggggttatt attttgggcg ttgcgtgggg 11580
   tcaggtccac gactggactg agcagacaga cccatggttt ttggatggcc tgggcatgga 11640
   ccgcatgtac tggcgcgaca cgaacaccgg gcgtctgtgg ctgccaaaca cccccgaccc 11700
   ccaaaaacca ccgcgcggat ttctggcgtg ccaagctagt cgaccaattc tcatgtttga 11760
   cagcttatca tcgcagatcc gggcaacgtt gttgccattg ctgcaggcgc agaactggta 11820
   ggtatggaag atctatacat tgaatcaata ttggcaatta gccatattag tcattggtta 11880
   tatagcataa atcaatattg gctattggcc attgcatacg ttgtatctat atcataatat 11940
   gtacatttat attggctcat gtccaatatg accgccat                         11978
<210> 64
   <211> 696
   <212> DNA
   <213> Homo sapiens
<400> 64
   gaaccgaaaa gctgcgataa aacccatacc tgcccgccgt gcccggcgcc ggaactgctg 60
   ggcggcccga gcgtgtttct gtttccgccg aaaccgaaag ataccctgat gattagccgc 120
   accccggaag tgacctgcgt ggtggtggat gtgagccatg aagatccgga agtgaaattt 180
   aactggtatg tggatggcgt ggaagtgcat aacgcgaaaa ccaaaccgcg cgaagaacag 240
   tataacagca cctatcgcgt ggtgagcgtg ctgaccgtgc tgcatcagga ttggctgaac 300
   ggcaaagaat ataaatgcaa agtgagcaac aaagcgctgc cggcgccgat tgaaaaaacc 360
   attagcaaag cgaaaggcca gccgcgcgaa ccgcaggtgt ataccctgcc gccgagccgc 420
   gatgaactga ccaaaaacca ggtgagcctg acctgcctgg tgaaaggctt ttatccgagc 480
   gatattgcgg tggaatggga aagcaacggc cagccggaaa acaactataa aaccaccccg 540
   ccggtgctgg atagcgatgg cagctttttt ctgtatagca aactgaccgt ggataaaagc 600
   cgctggcagc agggcaacgt gtttagctgc agcgtgatgc atgaagcgct gcataaccat 660
   tatacccaga aaagcctgag cctgagcccg ggcaaa                           696
<210> 65
   <211> 232
   <212> PRT
   <213> Homo sapiens
<400> 65
   Glu Pro Lys Ser Cys Asp Lys Thr His Thr Cys Pro Pro Cys Pro Ala
    1               5                  10                  15
   Pro Glu Leu Leu Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Asn Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro Gly Lys
   225                 230
<210> 66
   <211> 45
   <212> DNA
   <213> Homo sapiens
<400> 66
   atgattagcc gcaccccgac cgtgctgcat cagcataacc attat 45
<210> 67
   <211> 15
   <212> PRT
   <213> Homo sapiens
<220>
   <223> In vitro synthesized DNA with expression vector functions that encodes an alpha-MSH mimetibody
<400> 67
   Met Ile Ser Arg Thr Pro Thr Val Leu His Gln His Asn His Tyr
    1               5                  10                  15
<210> 68
   <211> 57
   <212> DNA
   <213> Homo sapiens
<400> 68
   atggcttggg tgtggacctt gctattcctg atggcggccg cccaaagtat acaggcc 57
<210> 69
   <211> 19
   <212> PRT
   <213> Homo sapiens
<400> 69
   Met Ala Trp Val Trp Thr Leu Leu Phe Leu Met Ala Ala Ala Gln Ser
    1               5                  10                  15
   Ile Gln Ala
<210> 70
   <211> 951
   <212> DNA
   <213> Homo sapiens
<400> 70
   atggctgtgc agggatccca gagaagactt ctgggctccc tcaactccac ccccacagcc 60
   atcccccagc tggggctggc tgccaaccag acaggagccc ggtgcctgga ggtgtccatc 120
   tctgacgggc tcttcctcag cctggggctg gtgagcttgg tggagaacgc gctggtggtg 180
   gccaccatcg ccaagaaccg gaacctgcac tcacccatgt actgcttcat ctgctgcctg 240
   gccttgtcgg acctgctggt gagcgggagc aacgtgctgg agacggccgt catcctcctg 300
   ctggaggccg gtgcactggt ggcccgggct gcggtgctgc agcagctgga caatgtcatt 360
   gacgtgatca cctgcagctc catgctgtcc agcctctgct tcctgggcgc catcgccgtg 420
   gaccgctaca tctccatctt ctacgcactg cgctaccaca gcaccgtgac cctgccgcgg 480
   gcgcggcgag ccgttgcggc catctgggtg gccagtgtcg tcttcagcac gctcttcatc 540
   gcctactacg accacgtggc cgtcctgctg tgcctcgtgg tcttcttcct ggctatgctg 600
   gtgctcatgg ccgtgctgta cgtccacatg ctggcccggg cctgccagca cgcccagggc 660
   atcgcccggc tccacaagag gcagcgcccg gtccaccagg gctttggcct taaaggcgct 720
   gtcaccctca ccatcctgct gggcattttc ttcctctgct ggggcccctt cttcctgcat 780
   ctcacactca tcgtcctctg ccccgagcac cccacgtgcg gctgcatctt caagaacttc 840
   aacctctttc tcgccctcat catctgcaat gccatcatcg accccctcat ctacgccttc 900
   cacagccagg agctccgcag gacgctcaag gaggtgctga cgtgctcctg g          951
<210> 71
   <211> 317
   <212> PRT
   <213> Homo sapiens
<400> 71
   Met Ala Val Gln Gly Ser Gln Arg Arg Leu Leu Gly Ser Leu Asn Ser
    1               5                  10                  15
   Thr Pro Thr Ala Ile Pro Gln Leu Gly Leu Ala Ala Asn Gln Thr Gly
               20                  25                  30
   Ala Arg Cys Leu Glu Val Ser Ile Ser Asp Gly Leu Phe Leu Ser Leu
           35                  40                  45
   Gly Leu Val Ser Leu Val Glu Asn Ala Leu Val Val Ala Thr Ile Ala
       50                  55                  60
   Lys Asn Arg Asn Leu His Ser Pro Met Tyr Cys Phe Ile Cys Cys Leu
   65                  70                  75                  80
   Ala Leu Ser Asp Leu Leu Val Ser Gly Ser Asn Val Leu Glu Thr Ala
                   85                  90                  95
   Val Ile Leu Leu Leu Glu Ala Gly Ala Leu Val Ala Arg Ala Ala Val
               100                 105                 110
   Leu Gln Gln Leu Asp Asn Val Ile Asp Val Ile Thr Cys Ser Ser Met
           115                 120                 125
   Leu Ser Ser Leu Cys Phe Leu Gly Ala Ile Ala Val Asp Arg Tyr Ile
       130                 135                 140
   Ser Ile Phe Tyr Ala Leu Arg Tyr His Ser Thr Val Thr Leu Pro Arg
   145                 150                 155                 160
   Ala Arg Arg Ala Val Ala Ala Ile Trp Val Ala Ser Val Val Phe Ser
                   165                 170                 175
   Thr Leu Phe Ile Ala Tyr Tyr Asp His Val Ala Val Leu Leu Cys Leu
               180                 185                 190
   Val Val Phe Phe Leu Ala Met Leu Val Leu Met Ala Val Leu Tyr Val
           195                 200                 205
   His Met Leu Ala Arg Ala Cys Gln His Ala Gln Gly Ile Ala Arg Leu
       210                 215                 220
   His Lys Arg Gln Arg Pro Val His Gln Gly Phe Gly Leu Lys Gly Ala
   225                 230                 235                 240
   Val Thr Leu Thr Ile Leu Leu Gly Ile Phe Phe Leu Cys Trp Gly Pro
                   245                 250                 255
   Phe Phe Leu His Leu Thr Leu Ile Val Leu Cys Pro Glu His Pro Thr
               260                 265                 270
   Cys Gly Cys Ile Phe Lys Asn Phe Asn Leu Phe Leu Ala Leu Ile Ile
           275                 280                 285
   Cys Asn Ala Ile Ile Asp Pro Leu Ile Tyr Ala Phe His Ser Gln Glu
       290                 295                 300
   Leu Arg Arg Thr Leu Lys Glu Val Leu Thr Cys Ser Trp
   305                 310                 315
<210> 72
   <211> 891
   <212> DNA
   <213> Homo sapiens
<400> 72
   atgaagcaca ttatcaactc gtatgaaaac atcaacaaca cagcaagaaa taattccgac 60
   tgtcctcgtg tggttttgcc ggaggagata tttttcacaa tttccattgt tggagttttg 120
   gagaatctga tcgtcctgct ggctgtgttc aagaataaga atctccaggc acccatgtac 180
   tttttcatct gtagcttggc catatctgat atgctgggca gcctatataa gatcttggaa 240
   aatatcctga tcatattgag aaacatgggc tatctcaagc cacgtggcag ttttgaaacc 300
   acagccgatg acatcatcga ctccctgttt gtcctctccc tgcttggctc catcttcagc 360
   ctgtctgtga ttgctgcgga ccgctacatc accatcttcc acgcactgcg gtaccacagc 420
   atcgtgacca tgcgccgcac tgtggtggtg cttacggtca tctggacgtt ctgcacgggg 480
   actggcatca ccatggtgat cttctcccat catgtgccca cagtgatcac cttcacgtcg 540
   ctgttcccgc tgatgctggt cttcatcctg tgcctctatg tgcacatgtt cctgctggct 600
   cgatcccaca ccaggaagat ctccaccctc cccagagcca acatgaaagg ggccatcaca 660
   ctgaccatcc tgctcggggt cttcatcttc tgctgggccc cctttgtgct tcatgtcctc 720
   ttgatgacat tctgcccaag taacccctac tgcgcctgct acatgtctct cttccaggtg 780
   aacggcatgt tgatcatgtg caatgccgtc attgacccct tcatatatgc cttccggagc 840
   ccagagctca gggacgcatt caaaaagatg atcttctgca gcaggtactg g          891
<210> 73
   <211> 297
   <212> PRT
   <213> Homo sapiens
<400> 73
<210> 74
   <211> 1080
   <212> DNA
   <213> Homo sapiens
<400> 74
   atgagcatcc aaaagacgta tctggaggga gattttgtct ttcctgtgag cagcagcagc 60
   ttcctacgga ccctgctgga gccccagctc ggatcagccc ttctgacagc aatgaatgct 120
   tcgtgctgcc tgccctctgt tcagccaaca ctgcctaatg gctcggagca cctccaagcc 180
   cctttcttca gcaaccagag cagcagcgcc ttctgtgagc aggtcttcat caagcccgag 240
   gttttcctgt ctctgggcat cgtcagtctg ctggaaaaca tcctggttat cctggccgtg 300
   gtcaggaacg gcaacctgca ctccccgatg tacttctttc tctgcagcct ggcggtggcc 360
   gacatgctgg taagtgtgtc caatgccctg gagaccatca tgatcgccat cgtccacagc 420
   gactacctga ccttcgagga ccagtttatc cagcacatgg acaacatctt cgactccatg 480
   atctgcatct ccctggtggc ctccatctgc aacctcctgg ccatcgccgt cgacaggtac 540
   gtcaccatct tttacgcgct ccgctaccac agcatcatga ccgtgaggaa ggccctcacc 600
   ttgatcgtgg ccatctgggt ctgctgcggc gtctgtggcg tggtgttcat cgtctactcg 660
   gagagcaaaa tggtcattgt gtgcctcatc accatgttct tcgccatgat gctcctcatg 720
   ggcaccctct acgtgcacat gttcctcttt gcgcggctgc acgtcaagcg catagcagca 780
   ctgccacctg ccgacggggt ggccccacag caacactcat gcatgaaggg ggcagtcacc 840
   atcaccattc tcctgggcgt gttcatcttc tgctgggccc ccttcttcct ccacctggtc 900
   ctcatcatca cctgccccac caacccctac tgcatctgct acactgccca cttcaacacc 960
   tacctggtcc tcatcatgtg caactccgtc atcgacccac tcatctacgc tttccggagc 1020
   ctggaattgc gcaacacctt tagggagatt ctctgtggct gcaacggcat gaacttggga 1080
<210> 75
   <211> 360
   <212> PRT
   <213> Homo sapiens
<400> 75
   Met Ser Ile Gln Lys Thr Tyr Leu Glu Gly Asp Phe Val Phe Pro Val
    1               5                  10                  15
   Ser Ser Ser Ser Phe Leu Arg Thr Leu Leu Glu Pro Gln Leu Gly Ser
               20                  25                  30
   Ala Leu Leu Thr Ala Met Asn Ala Ser Cys Cys Leu Pro Ser Val Gln
           35                  40                  45
   Pro Thr Leu Pro Asn Gly Ser Glu His Leu Gln Ala Pro Phe Phe Ser
       50                  55                  60
   Asn Gln Ser Ser Ser Ala Phe Cys Glu Gln Val Phe Ile Lys Pro Glu
   65                  70                  75                  80
   Val Phe Leu Ser Leu Gly Ile Val Ser Leu Leu Glu Asn Ile Leu Val
                   85                  90                  95
   Ile Leu Ala Val Val Arg Asn Gly Asn Leu His Ser Pro Met Tyr Phe
               100                 105                 110
   Phe Leu Cys Ser Leu Ala Val Ala Asp Met Leu Val Ser Val Ser Asn
           115                 120                 125
   Ala Leu Glu Thr Ile Met Ile Ala Ile Val His Ser Asp Tyr Leu Thr
       130                 135                 140
   Phe Glu Asp Gln Phe Ile Gln His Met Asp Asn Ile Phe Asp Ser Met
   145                 150                 155                 160
   Ile Cys Ile Ser Leu Val Ala Ser Ile Cys Asn Leu Leu Ala Ile Ala
                   165                 170                 175
   Val Asp Arg Tyr Val Thr Ile Phe Tyr Ala Leu Arg Tyr His Ser Ile
               180                 185                 190
   Met Thr Val Arg Lys Ala Leu Thr Leu Ile Val Ala Ile Trp Val Cys
           195                 200                 205
   Cys Gly Val Cys Gly Val Val Phe Ile Val Tyr Ser Glu Ser Lys Met
       210                 215                 220
   Val Ile Val Cys Leu Ile Thr Met Phe Phe Ala Met Met Leu Leu Met
   225                 230                 235                 240
   Gly Thr Leu Tyr Val His Met Phe Leu Phe Ala Arg Leu His Val Lys
                   245                 250                 255
   Arg Ile Ala Ala Leu Pro Pro Ala Asp Gly Val Ala Pro Gln Gln His
               260                 265                 270
   Ser Cys Met Lys Gly Ala Val Thr Ile Thr Ile Leu Leu Gly Val Phe
           275                 280                 285
   Ile Phe Cys Trp Ala Pro Phe Phe Leu His Leu Val Leu Ile Ile Thr
       290                 295                 300
   Cys Pro Thr Asn Pro Tyr Cys Ile Cys Tyr Thr Ala His Phe Asn Thr
   305                 310                 315                 320
   Tyr Leu Val Leu Ile Met Cys Asn Ser Val Ile Asp Pro Leu Ile Tyr
                   325                 330                 335
   Ala Phe Arg Ser Leu Glu Leu Arg Asn Thr Phe Arg Glu Ile Leu Cys
               340                 345                 350
   Gly Cys Asn Gly Met Asn Leu Gly
           355                 360
<210> 76
   <211> 999
   <212> DNA
   <213> Homo sapiens
<400> 76
   atggtgaact ccacccaccg tgggatgcac acttctctgc acctctggaa ccgcagcagt 60
   tacagactgc acagcaatgc cagtgagtcc cttggaaaag gctactctga tggagggtgc 120
   tacgagcaac tttttgtctc tcctgaggtg tttgtgactc tgggtgtcat cagcttgttg 180
   gagaatatct tagtgattgt ggcaatagcc aagaacaaga atctgcattc acccatgtac 240
   tttttcatct gcagcttggc tgtggctgat atgctggtga gcgtttcaaa tggatcagaa 300
   accattatca tcaccctatt aaacagtaca gatacggatg cacagagttt cacagtgaat 360
   attgataatg tcattgactc ggtgatctgt agctccttgc ttgcatccat ttgcagcctg 420
   ctttcaattg cagtggacag gtactttact atcttctatg ctctccagta ccataacatt 480
   atgacagtta agcgggttgg gatcatcata agttgtatct gggcagcttg cacggtttca 540
   ggcattttgt tcatcattta ctcagatagt agtgctgtca tcatctgcct catcaccatg 600
   ttcttcacca tgctggctct catggcttct ctctatgtcc acatgttcct gatggccagg 660
   cttcacatta agaggattgc tgtcctcccc ggcactggtg ccatccgcca aggtgccaat 720
   atgaagggag cgattacctt gaccatcctg attggcgtct ttgttgtctg ctgggcccca 780
   ttcttcctcc acttaatatt ctacatctct tgtcctcaga atccatattg tgtgtgcttc 840
   atgtctcact ttaacttgta tctcatactg atcatgtgta attcaatcat cgatcctctg 900
   atttatgcac tccggagtca agaactgagg aaaaccttca aagagatcat ctgttgctat 960
   cccctgggag gcctttgtga cttgtctagc agatattaa                        999
<210> 77
   <211> 332
   <212> PRT
   <213> Homo sapiens
<400> 77
   Met Val Asn Ser Thr His Arg Gly Met His Thr Ser Leu His Leu Trp
    1               5                  10                  15
   Asn Arg Ser Ser Tyr Arg Leu His Ser Asn Ala Ser Glu Ser Leu Gly
               20                  25                  30
   Lys Gly Tyr Ser Asp Gly Gly Cys Tyr Glu Gln Leu Phe Val Ser Pro
           35                  40                  45
   Glu Val Phe Val Thr Leu Gly Val Ile Ser Leu Leu Glu Asn Ile Leu
       50                  55                  60
   Val Ile Val Ala Ile Ala Lys Asn Lys Asn Leu His Ser Pro Met Tyr
   65                  70                  75                  80
   Phe Phe Ile Cys Ser Leu Ala Val Ala Asp Met Leu Val Ser Val Ser
                   85                  90                  95
   Asn Gly Ser Glu Thr Ile Ile Ile Thr Leu Leu Asn Ser Thr Asp Thr
               100                 105                 110
   Asp Ala Gln Ser Phe Thr Val Asn Ile Asp Asn Val Ile Asp Ser Val
           115                 120                 125
   Ile Cys Ser Ser Leu Leu Ala Ser Ile Cys Ser Leu Leu Ser Ile Ala
       130                 135                 140
   Val Asp Arg Tyr Phe Thr Ile Phe Tyr Ala Leu Gln Tyr His Asn Ile
   145                 150                 155                 160
   Met Thr Val Lys Arg Val Gly Ile Ile Ile Ser Cys Ile Trp Ala Ala
                   165                 170                 175
   Cys Thr Val Ser Gly Ile Leu Phe Ile Ile Tyr Ser Asp Ser Ser Ala
               180                 185                 190
   Val Ile Ile Cys Leu Ile Thr Met Phe Phe Thr Met Leu Ala Leu Met
           195                 200                 205
   Ala Ser Leu Tyr Val His Met Phe Leu Met Ala Arg Leu His Ile Lys
       210                 215                 220
   Arg Ile Ala Val Leu Pro Gly Thr Gly Ala Ile Arg Gln Gly Ala Asn
   225                 230                 235                 240
   Met Lys Gly Ala Ile Thr Leu Thr Ile Leu Ile Gly Val Phe Val Val
                   245                 250                 255
   Cys Trp Ala Pro Phe Phe Leu His Leu Ile Phe Tyr Ile Ser Cys Pro
               260                 265                 270
   Gln Asn Pro Tyr Cys Val Cys Phe Met Ser His Phe Asn Leu Tyr Leu
           275                 280                 285
   Ile Leu Ile Met Cys Asn Ser Ile Ile Asp Pro Leu Ile Tyr Ala Leu
       290                 295                 300
   Arg Ser Gln Glu Leu Arg Lys Thr Phe Lys Glu Ile Ile Cys Cys Tyr
   305                 310                 315                 320
   Pro Leu Gly Gly Leu Cys Asp Leu Ser Ser Arg Tyr
                   325                 330
<210> 78
   <211> 975
   <212> DNA
   <213> Homo sapiens
<400> 78
   atgaattcct catttcacct gcatttcttg gatctcaacc tgaatgccac agagggcaac 60
   ctttcaggac ccaatgtcaa aaacaagtct tcaccatgtg aagacatggg cattgctgtg 120
   gaggtgtttc tcactctggg tgtcatcagc ctcttggaga acatcttggt cataggggcc 180
   atagtgaaga acaaaaacct gcactccccc atgtacttct tcgtgtgcag cctggcagtg 240
   gcggacatgc tggtgagcat gtccagtgcc tgggagacca tcaccatcta cctactcaac 300
   aacaagcacc tagtgatagc agacgccttt gtgcgccaca ttgacaatgt gtttgactcc 360
   atgatctgca tttccgtggt ggcatccatg tgcagcttac tggccattgc agtggatagg 420
   tacgtcacca tcttctacgc cctgcgctac caccacatca tgacggcgag gcgctcaggg 480
   gccatcatcg ccggcatctg ggctttctgc acgggctgcg gcattgtctt catcctgtac 540
   tcagaatcca cctacgtcat cctgtgcctc atctccatgt tcttcgctat gctgttcctc 600
   ctggtgtctc tgtacataca catgttcctc ctggcgcgga ctcacgtcaa gcggatcgcg 660
   gctctgcccg gggccagctc tgcgcggcag aggaccagca tgcagggcgc ggtcaccgtc 720
   accatgctgc tgggcgtgtt taccgtgtgc tgggccccgt tcttccttca tctcacttta 780
   atgctttctt gccctcagaa cctctactgc tctcgcttca tgtctcactt caatatgtac 840
   ctcatactca tcatgtgtaa ttccgtgatg gaccctctca tatatgcctt ccgcagccaa 900
   gagatgcgga agacctttaa ggagattatt tgctgccgtg gtttcaggat cgcctgcagc 960
   tttcccagaa gggat                                                  975
<210> 79
   <211> 325
   <212> PRT
   <213> Homo sapiens
<400> 79
   Met Asn Ser Ser Phe His Leu His Phe Leu Asp Leu Asn Leu Asn Ala
   1               5                   10                  15
   Thr Glu Gly Asn Leu Ser Gly Pro Asn Val Lys Asn Lys Ser Ser Pro
               20                  25                  30
   Cys Glu Asp Met Gly Ile Ala Val Glu Val Phe Leu Thr Leu Gly Val
           35                  40                  45
   Ile Ser Leu Leu Glu Asn Ile Leu Val Ile Gly Ala Ile Val Lys Asn
       50                  55                  60
   Lys Asn Leu His Ser Pro Met Tyr Phe Phe Val Cys Ser Leu Ala Val
   65                  70                  75                  80
   Ala Asp Met Leu Val Ser Met Ser Ser Ala Trp Glu Thr Ile Thr Ile
                   85                  90                  95
   Tyr Leu Leu Asn Asn Lys His Leu Val Ile Ala Asp Ala Phe Val Arg
               100                 105                 110
   His Ile Asp Asn Val Phe Asp Ser Met Ile Cys Ile Ser Val Val Ala
           115                 120                 125
   Ser Met Cys Ser Leu Leu Ala Ile Ala Val Asp Arg Tyr Val Thr Ile
       130                 135                 140
   Phe Tyr Ala Leu Arg Tyr His His Ile Met Thr Ala Arg Arg Ser Gly
   145                 150                 155                 160
   Ala Ile Ile Ala Gly Ile Trp Ala Phe Cys Thr Gly Cys Gly Ile Val
                   165                 170                 175
   Phe Ile Leu Tyr Ser Glu Ser Thr Tyr Val Ile Leu Cys Leu Ile Ser
               180                 185                 190
   Met Phe Phe Ala Met Leu Phe Leu Leu Val Ser Leu Tyr Ile His Met
           195                 200                 205
   Phe Leu Leu Ala Arg Thr His Val Lys Arg Ile Ala Ala Leu Pro Gly
       210                 215                 220
   Ala Ser Ser Ala Arg Gln Arg Thr Ser Met Gln Gly Ala Val Thr Val
   225                 230                 235                 240
   Thr Met Leu Leu Gly Val Phe Thr Val Cys Trp Ala Pro Phe Phe Leu
                   245                 250                 255
   His Leu Thr Leu Met Leu Ser Cys Pro Gln Asn Leu Tyr Cys Ser Arg
               260                 265                 270
   Phe Met Ser His Phe Asn Met Tyr Leu Ile Leu Ile Met Cys Asn Ser
           275                 280                 285
   Val Met Asp Pro Leu Ile Tyr Ala Phe Arg Ser Gln Glu Met Arg Lys
       290                 295                 300
   Thr Phe Lys Glu Ile Ile Cys Cys Arg Gly Phe Arg Ile Ala Cys Ser
   305                 310                 315                 320
   Phe Pro Arg Arg Asp
                   325
<210> 80
   <211> 12
   <212> DNA
   <213> Homo sapiens
<400> 80
   cattttcgct gg 12
<210> 81
   <211> 4
   <212> PRT
   <213> Homo sapiens
<400> 81
   His Phe Arg Trp
   1
<210> 82
   <211> 12
   <212> DNA
   <213> Artificial Sequence
<220>
   <223> In Vitro synthesized DNA encoding a peptide that is a modification of alpha-MSH HFRW core amino acid sequence
<400> 82
   tttcattgga tg 12
<210> 83
   <211> 4
   <212> PRT
   <213> Artificial Sequence
<220>
   <223> A peptide that is a modification of alpha-MSH HFRW core amino acid sequence and is encoded by an in vitro synthesized DNA
<400> 83
   Phe His Trp Met
   1
<210> 84
   <211> 12
   <212> DNA
   <213> Artificial Sequence
<220>
   <223> In vitro synthesized DNA encoding a flexible peptide sequence
<400> 84
   ggcggcggca gc 12
<210> 85
   <211> 4
   <212> PRT
   <213> Artificial Sequence
<220>
   <223> Flexible peptide encoded by an in vitro synthesized DNA
<400> 85
   Gly Gly Gly Ser
   1
<210> 86
   <211> 39
   <212> DNA
   <213> Artificial Sequence
<220>
   <223> In vitro synthesized DNA encoding a constrained peptide sequence
<400> 86
   tcctactgcg gcgagcactt ccgctggggc tgcccggtg 39
<210> 87
   <211> 13
   <212> PRT
   <213> Artificial Sequence
<220>
   <223> Constrained peptide encoded by an in vitro synthesized DNA.
<400> 87
   Ser Tyr Cys Gly Glu His Phe Arg Trp Gly Cys Pro Val
    1               5                  10
<210> 88
   <211> 39
   <212> DNA
   <213> Artificial Sequence
<220>
   <223> In vitro synthesized DNA encoding a constrained peptide sequence.
<400> 88
   tcctactcct gcgagcactt ccgctggggc aagccgtgc 39
<210> 89
   <211> 13
   <212> PRT
   <213> Artificial Sequence
<220>
   <223> Constrained peptide encoded by an in vitro synthesized DNA.
<400> 89
   Ser Tyr Ser Cys Glu His Phe Arg Trp Gly Lys Pro Cys
    1               5                  10
<210> 90
   <211> 39
   <212> DNA
   <213> Artificial Sequence
<220>
   <223> In vitro synthesized DNA encoding a constrained peptide sequence.
<400> 90
   tcctactgca tggagcactt ccgctggggc tgcccggtg 39
<210> 91
   <211> 13
   <212> PRT
   <213> Artificial Sequence
<220>
   <223> Constrained peptide encoded by an in vitro synthesized DNA.
<400> 91
   Ser Tyr Cys Met Glu His Phe Arg Trp Gly Cys Pro Val
    1               5                   10
<210> 92
   <211> 39
   <212> DNA
   <213> Artificial Sequence
<220>
   <223> In vitro synthesized DNA encoding a constrained peptide sequence.
<400> 92
   tcctgctcca tggagcactt ccgctggggc tgcccggtg 39
<210> 93
   <211> 13
   <212> PRT
   <213> Artificial Sequence
<220>
   <223> Constrained peptide encoded by an in vitro synthesized DNA.
<400> 93
   Ser Cys Ser Met Glu His Phe Arg Trp Gly Cys Pro Val
    1               5                   10
<210> 94
   <211> 39
   <212> DNA
   <213> Artificial Sequence
<220>
   <223> In vitro synthesized DNA encoding a constrained peptide sequence.
<400> 94
   tcctactcct gcgagcactt ccgctggggc tgcccggtg 39
<210> 95
   <211> 13
   <212> PRT
   <213> Artificial Sequence
<220>
   <223> Constrained peptide encoded by an in vitro synthesized DNA.
<400> 95
   Ser Tyr Ser Cys Glu His Phe Arg Trp Gly Cys Pro Val
    1               5                   10
<210> 96
   <211> 39
   <212> DNA
   <213> Artificial Sequence
<220>
   <223> In vitro synthesized DNA encoding a constrained peptide sequence.
<400> 96
   tcctactcct gcgagcactt ccgctggggc aagtgcgtg 39
<210> 97
   <211> 13
   <212> PRT
   <213> Artificial Sequence
<220>
   <223> Constrained peptide encoded by an in vitro synthesized DNA.
<400> 97
   Ser Tyr Ser Cys Glu His Phe Arg Trp Gly Lys Cys Val
   1               5                   10
<210> 98
   <211> 15
   <212> DNA
   <213> Artificial Sequence
<220>
   <223> In vitro synthesized DNA encoding a flexible peptide sequence.
<400> 98
   ggaggaggcg gatcc 15
<210> 99
   <211> 5
   <212> PRT
   <213> Artificial Sequence
<220>
   <223> Flexible peptide encoded by an in vitro synthesized DNA.
<400> 99
   Gly Gly Gly Gly Ser
   1               5
<210> 100
   <211> 30
   <212> DNA
   <213> Artificial Sequence
<220>
   <223> In vitro synthesized DNA encoding a flexible peptide sequence.
<400> 100
   ggaggcggag gaagcggcgg aggcggatcc 30
<210> 101
   <211> 10
   <212> PRT
   <213> Artificial Sequence
<220>
   <223> Flexible peptide encoded by an in vitro synthesized DNA.
<400> 101
   Gly Gly Gly Gly Ser Gly Gly Gly Gly Ser
    1               5                  10
<210> 102
   <211> 45
   <212> DNA
   <213> Artificial Sequence
<220>
   <223> In vitro synthesized DNA encoding a flexible peptide sequence.
<400> 102
   gggggcggag gaagcggcgg aggcgggagc ggcggcggag gatcc 45
<210> 103
   <211> 15
   <212> PRT
   <213> Artificial Sequence
<220>
   <223> Flexible peptide encoded by an in vitro synthesized DNA.
<400> 103
   Gly Gly Gly Gly Ser Gly Gly Gly Gly Ser Gly Gly Gly Gly Ser
    1               5                  10                  15
<210> 104
   <211> 60
   <212> DNA
   <213> Artificial Sequence
<220>
   <223> In vitro synthesized DNA encoding a flexible peptide sequence.
<400> 104
   gggggcggag gaagcggcgg aggcgggagc ggcggcggag gctccggtgg cggaggatcc 60
<210> 105
   <211> 20
   <212> PRT
   <213> Artificial Sequence
<220>
   <223> Flexible peptide encoded by an in vitro synthesized DNA.
<400> 105
   Gly Gly Gly Gly Ser Gly Gly Gly Gly Ser Gly Gly Gly Gly Ser Gly
    1               5                  10                  15
   Gly Gly Gly Ser
               20
<210> 106
   <211> 45
   <212> DNA
   <213> Artificial Sequence
<220>
   <223> In vitro mutagenized Homo sapien DNA encoding a Hg peptide sequence comprising a T to A substitution to limit O-linked glycosylation.
<400> 106
   gagcccaaat cttgtgacaa aactcacgcc tgcccaccgt gccca 45
<210> 107
   <211> 15
   <212> PRT
   <213> Artificial Sequence
<220>
   <223> Mutagenized Homo sapien Hg peptide sequence comprising a T to A substitution to limit O-linked glycosylation this is encoded by an in vitro mutagenized Homo sapien DNA.
<400> 107
   Glu Pro Lys Ser Cys Asp Lys Thr His Ala Cys Pro Pro Cys Pro
    1               5                  10                  15
<210> 108
   <211> 45
   <212> DNA
   <213> Artificial Sequence
<220>
   <223> In vitro mutagenized Homo sapien DNA encoding a Hg peptide sequence comprising a C to A substitution to limit aggregation or multimerization.
<400> 108
   gagcccaaat ctgccgacaa aactcacacg tgcccaccgt gccca 45
<210> 109
   <211> 15
   <212> PRT
   <213> Artificial Sequence
<220>
   <223> Mutagenized Homo sapien Hg peptide sequence comprising a C to A substitution to limit aggregation or multimerization that is encoded by an in vitro mutagenized Homo sapien DNA.
<400> 109
   Glu Pro Lys Ser Ala Asp Lys Thr His Thr Cys Pro Pro Cys Pro
    1               5                  10                  15
<210> 110
   <211> 45
   <212> DNA
   <213> Artificial Sequence
<220>
   <223> In vitro mutagenized Homo sapien DNA encoding a Hg peptide sequence comprising a C to A substitution to limit aggregation or multimerization and a T to A substitution to limit O-linked glycosylation.
<400> 110
   gagcccaaat ctgccgacaa aactcacgcc tgcccaccgt gccca 45
<210> 111
   <211> 15
   <212> PRT
   <213> Artificial Sequence
<220>
   <223> Mutagenized Homo sapien Hg peptide sequence comprising a C to A substitution to limit aggregation or multimerization and a T to A substitution to limit O-linked glycosylation. The peptide is encoded by an in vitro mutagenized Homo sapien DNA.
<400> 111
   Glu Pro Lys Ser Ala Asp Lys Thr His Ala Cys Pro Pro Cys Pro
    1               5                  10                  15
<210> 112
   <211> 15
   <212> DNA
   <213> Artificial Sequence
<220>
   <223> In vitro synthesized DNA encoding an amino terminal portion of an immunoglobulin variable region that has been modified for increased flexibility by the addition of G residues.
<400> 112
   cagatccagg gaggc 15
<210> 113
   <211> 5
   <212> PRT
   <213> Artificial Sequence
<220>
   <223> Amino terminal portion of an immunoglobulin variable region peptide that has been modified for increased flexibility by the addition of G residues. The peptide is encoded by an in vitro synthesized DNA.
<400> 113
   Gln Ile Gln Gly Gly
    1               5
<210> 114
   <211> 21
   <212> DNA
   <213> Artificial Sequence
<220>
   <223> In vitro synthesized DNA encoding an amino terminal portion of an immunoglobulin variable region that has been modified for increased flexibility by the addition of G residues.
<400> 114
   cagatccagg gaggcggagg c 21
<210> 115
   <211> 7
   <212> PRT
   <213> Artificial Sequence
<220>
   <223> Amino terminal portion of an immunoglobulin variable region peptide that has been modified for increased flexibility by the addition of G residues. The peptide is encoded by an in vitro synthesized DNA.
<400> 115
   Gln Ile Gln Gly Gly Gly Gly
    1               5
<210> 116
   <211> 330
   <212> DNA
   <213> Homo sapiens
<400> 116
   gcgcctgagg ccgccggggg accatcagtc ttcctgttcc ccccaaaacc caaggacact 60
   ctcatgatct cccggacccc tgaggtcacg tgcgtggtgg tggacgtgag ccaggaagac 120
   cccgaggtcc agttcaactg gtacgtggat ggcgtggagg tgcataatgc caagacaaag 180
   ccgcgggagg agcagttcaa cagcacgtac cgtgtggtca gcgtcctcac cgtcctgcac 240
   caggactggc tgaacggcaa ggagtacaag tgcaaggtct ccaacaaagg cctcccgtcc 300
   tccatcgaga aaaccatctc caaagccaaa                                  330
<210> 117
   <211> 110
   <212> PRT
   <213> Homo sapiens
<400> 117
   Ala Pro Glu Ala Ala Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser Gln Glu Asp Pro Glu Val Gln Phe Asn Trp Tyr
           35                  40                  45
   Val Asp Gly Val Glu Val His Asn Ala Lys Thr Lys Pro Arg Glu Glu
       50                  55                  60
   Gln Phe Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Gly Leu Pro Ser Ser Ile Glu Lys Thr Ile Ser Lys Ala Lys
               100                 105                 110
<210> 118
   <211> 321
   <212> DNA
   <213> Homo sapiens
<400> 118
   gggcagcctc gagagccaca ggtgtacacc ctgcccccat cccaggagga gatgaccaag 60
   aaccaggtca gcctgacctg cctggtcaaa ggcttctacc ccagcgacat cgccgtggag 120
   tgggagagca atgggcagcc ggagaacaac tacaagacca cgcctcccgt gctggactcc 180
   gacggctcct tcttcctcta cagcaggcta accgtggaca agagcaggtg gcaggagggg 240
   aatgtcttct catgctccgt gatgcatgag gctctgcaca accactacac acagaaaagc 300
   ttgtccctgt ctctgggtaa a                                           321
<210> 119
   <211> 107
   <212> PRT
   <213> Homo sapiens
<400> 119
   Gly Gln Pro Arg Glu Pro Gln Val Tyr Thr Leu Pro Pro Ser Gln Glu
    1               5                  10                  15
   Glu Met Thr Lys Asn Gln Val Ser Leu Thr Cys Leu Val Lys Gly Phe
               20                  25                  30
   Tyr Pro Ser Asp Ile Ala Val Glu Trp Glu Ser Asn Gly Gln Pro Glu
           35                  40                  45
   Asn Asn Tyr Lys Thr Thr Pro Pro Val Leu Asp Ser Asp Gly Ser Phe
       50                  55                  60
   Phe Leu Tyr Ser Arg Leu Thr Val Asp Lys Ser Arg Trp Gln Glu Gly
   65                  70                  75                  80
   Asn Val Phe Ser Cys Ser Val Met His Glu Ala Leu His Asn His Tyr
                   85                  90                  95
   Thr Gln Lys Ser Leu Ser Leu Ser Leu Gly Lys
               100                 105
<210> 120
   <211> 777
   <212> DNA
   <213> Artificial Sequence
<220>
   <223> In vitro synthesized DNA encoding a melanocortin receptor binding alpha-MSH mimetibody without secretory signal (p3309).
<400> 120
   tcctactcca tggagcactt ccgctggggc aagccggtgg gatccggtgg aggctccggt 60
   accttagtcg ccgtctcctc agagcccaaa tctgccgaca aaactcacgc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 121
   <211> 259
   <212> PRT
   <213> Artificial Sequence
<220>
   <223> Melanocortin receptor binding alpha-MSH mimetibody without secretory signal encoded by an in vitro synthesized DNA (p3309).
<400> 121
   Ser Tyr Ser Met Glu His Phe Arg Trp Gly Lys Pro Val Gly Ser Gly
    1               5                  10                  15
   Gly Gly Ser Gly Thr Leu Val Ala Val Ser Ser Glu Pro Lys Ser Ala
               20                  25                  30
   Asp Lys Thr His Ala Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 122
   <211> 834
   <212> DNA
   <213> Artificial Sequence
<220>
   <223> In vitro synthesized DNA encoding a melanocortin receptor binding alpha-MSH mimetibody with secretory signal (p3309).
<400> 122
   atggcttggg tgtggacctt gctattcctg atggcggccg cccaaagtat acaggcctcc 60
   tactccatgg agcacttccg ctggggcaag ccggtgggat ccggtggagg ctccggtacc 120
   ttagtcgccg tctcctcaga gcccaaatct gccgacaaaa ctcacgcctg cccaccgtgc 180
   ccagcacctg aactcctggg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 123
   <211> 278
   <212> PRT
   <213> Artificial Sequence
<220>
   <223> Melanocortin receptor binding alpha-MSH mimetibody with secretory signal encoded by an in vitro synthesized DNA (p3309).
<400> 123
   Met Ala Trp Val Trp Thr Leu Leu Phe Leu Met Ala Ala Ala Gln Ser
    1               5                  10                  15
   Ile Gln Ala Ser Tyr Ser Met Glu His Phe Arg Trp Gly Lys Pro Val
               20                  25                  30
   Gly Ser Gly Gly Gly Ser Gly Thr Leu Val Ala Val Ser Ser Glu Pro
           35                  40                  45
   Lys Ser Ala Asp Lys Thr His Ala Cys Pro Pro Cys Pro Ala Pro Glu
       50                  55                  60
   Leu Leu Gly Gly Pro Ser Val Phe Leu Phe Pro Pro Lys Pro Lys Asp
   65                  70                  75                  80
   Thr Leu Met Ile Ser Arg Thr Pro Glu Val Thr Cys Val Val Val Asp
                   85                  90                  95
   Val Ser His Glu Asp Pro Glu Val Lys Phe Asn Trp Tyr Val Asp Gly
               100                 105                 110
   Val Glu Val His Asn Ala Lys Thr Lys Pro Arg Glu Glu Gln Tyr Asn
           115                 120                 125
   Ser Thr Tyr Arg Val Val Ser Val Leu Thr Val Leu His Gln Asp Trp
       130                 135                 140
   Leu Asn Gly Lys Glu Tyr Lys Cys Lys Val Ser Asn Lys Ala Leu Pro
   145                 150                 155                 160
   Ala Pro Ile Glu Lys Thr Ile Ser Lys Ala Lys Gly Gln Pro Arg Glu
                   165                 170                 175
   Pro Gln Val Tyr Thr Leu Pro Pro Ser Arg Asp Glu Leu Thr Lys Asn
               180                 185                 190
   Gln Val Ser Leu Thr Cys Leu Val Lys Gly Phe Tyr Pro Ser Asp Ile
           195                 200                 205
   Ala Val Glu Trp Glu Ser Asn Gly Gln Pro Glu Asn Asn Tyr Lys Thr
       210                 215                 220
   Thr Pro Pro Val Leu Asp Ser Asp Gly Ser Phe Phe Leu Tyr Ser Lys
   225                 230                 235                 240
   Leu Thr Val Asp Lys Ser Arg Trp Gln Gln Gly Asn Val Phe Ser Cys
                   245                 250                 255
   Ser Val Met His Glu Ala Leu His Asn His Tyr Thr Gln Lys Ser Leu
               260                 265                 270
   Ser Leu Ser Pro Gly Lys
           275
<210> 124
   <211> 24
   <212> DNA
   <213> Homo sapiens
<400> 124
   aaaaaaaaaa aaaaaaaaaa aaaa 24
<210> 125
   <211> 11969
   <212> DNA
   <213> Artificial Sequence
<220>
   <223> In vitro synthesized DNA with expression vector functions that encodes an alpha-MSH mimetibody (p3309).
<400> 125
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactccatgg agcacttccg 1680
   ctggggcaag ccggtgggat ccggtggagg ctccggtacc ttagtcgccg tctcctcaga 1740
   gcccaaatct gccgacaaaa ctcacgcctg cccaccgtgc ccagcacctg aactcctggg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaagggggc gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gagggggcct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat 11969
<210> 126
   <211> 720
   <212> DNA
   <213> Artificial Sequence
<220>
   <223> In vitro synthesized DNA encoding a melanocortin receptor binding alpha-MSH mimetibody without secretory signal (p3167).
<400> 126
   tcctactcca tggagcactt ccgctggggc aagccggtgg gaggaggcgg atcctgccca 60
   ccgtgcccag cacctgaact cctgggggga ccgtcagtct tcctcttccc cccaaaaccc 120
   aaggacaccc tcatgatctc ccggacccct gaggtcacat gcgtggtggt ggacgtgagc 180
   cacgaagacc ctgaggtcaa gttcaactgg tacgtggacg gcgtggaggt gcataatgcc 240
   aagacaaagc cgcgggagga gcagtacaac agcacgtacc gtgtggtcag cgtcctcacc 300
   gtcctgcacc aggactggct gaatggcaag gagtacaagt gcaaggtctc caacaaagcc 360
   ctcccagccc ccatcgagaa aaccatctcc aaagccaaag ggcagccccg agaaccacag 420
   gtgtacaccc tgcccccatc ccgggatgag ctgaccaaga accaggtcag cctgacctgc 480
   ctggtcaaag gcttctatcc cagcgacatc gccgtggagt gggagagcaa tgggcagccg 540
   gagaacaact acaagaccac gcctcccgtg ctggactccg acggctcctt cttcctctac 600
   agcaagctca ccgtggacaa gagcaggtgg cagcagggga acgtcttctc atgctccgtg 660
   atgcatgagg ctctgcacaa ccactacacg cagaagagcc tctccctgtc tctgggtaaa 720
<210> 127
   <211> 240
   <212> PRT
   <213> Artificial Sequence
<220>
   <223> Melanocortin receptor binding alpha-MSH mimetibody without secretory signal encoded by an in vitro synthesized DNA (p3167).
<400> 127
   Ser Tyr Ser Met Glu His Phe Arg Trp Gly Lys Pro Val Gly Gly Gly
    1               5                  10                  15
   Gly Ser Cys Pro Pro Cys Pro Ala Pro Glu Leu Leu Gly Gly Pro Ser
               20                  25                  30
   Val Phe Leu Phe Pro Pro Lys Pro Lys Asp Thr Leu Met Ile Ser Arg
           35                  40                  45
   Thr Pro Glu Val Thr Cys Val Val Val Asp Val Ser His Glu Asp Pro
       50                  55                  60
   Glu Val Lys Phe Asn Trp Tyr Val Asp Gly Val Glu Val His Asn Ala
   65                  70                  75                  80
   Lys Thr Lys Pro Arg Glu Glu Gln Tyr Asn Ser Thr Tyr Arg Val Val
                   85                  90                  95
   Ser Val Leu Thr Val Leu His Gln Asp Trp Leu Asn Gly Lys Glu Tyr
               100                 105                 110
   Lys Cys Lys Val Ser Asn Lys Ala Leu Pro Ala Pro Ile Glu Lys Thr
           115                 120                 125
   Ile Ser Lys Ala Lys Gly Gln Pro Arg Glu Pro Gln Val Tyr Thr Leu
       130                 135                 140
   Pro Pro Ser Arg Asp Glu Leu Thr Lys Asn Gln Val Ser Leu Thr Cys
   145                 150                 155                 160
   Leu Val Lys Gly Phe Tyr Pro Ser Asp Ile Ala Val Glu Trp Glu Ser
                   165                 170                 175
   Asn Gly Gln Pro Glu Asn Asn Tyr Lys Thr Thr Pro Pro Val Leu Asp
               180                 185                 190
   Ser Asp Gly Ser Phe Phe Leu Tyr Ser Lys Leu Thr Val Asp Lys Ser
           195                 200                 205
   Arg Trp Gln Gln Gly Asn Val Phe Ser Cys Ser Val Met His Glu Ala
       210                 215                 220
   Leu His Asn His Tyr Thr Gln Lys Ser Leu Ser Leu Ser Leu Gly Lys
   225                 230                 235                 240
<210> 128
   <211> 777
   <212> DNA
   <213> Artificial Sequence
<220>
   <223> In vitro synthesized DNA encoding a melanocortin receptor binding alpha-MSH mimetibody with secretory signal (p3167).
<400> 128
   atggcttggg tgtggacctt gctattcctg atggcggccg cccaaagtat acaggcctcc 60
   tactccatgg agcacttccg ctggggcaag ccggtgggag gaggcggatc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctct gggtaaa    777
<210> 129
   <211> 259
   <212> PRT
   <213> Artificial Sequence
<220>
   <223> Melanocortin receptor binding alpha-MSH mimetibody with secretory signal encoded by an in vitro synthesized DNA (p3167).
<400> 129
   Met Ala Trp Val Trp Thr Leu Leu Phe Leu Met Ala Ala Ala Gln Ser
    1               5                   10                  15
   Ile Gln Ala Ser Tyr Ser Met Glu His Phe Arg Trp Gly Lys Pro Val
               20                  25                  30
   Gly Gly Gly Gly Ser Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Leu Gly Lys
<210> 130
   <211> 11912
   <212> DNA
   <213> Artificial Sequence
<220>
   <223> In vitro synthesized DNA with expression vector functions that encodes an alpha-MSH mimetibody (p3167).
<400> 130
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactccatgg agcacttccg 1680
   ctggggcaag ccggtgggag gaggcggatc ctgcccaccg tgcccagcac ctgaactcct 1740
   ggggggaccg tcagtcttcc tcttcccccc aaaacccaag gacaccctca tgatctcccg 1800
   gacccctgag gtcacatgcg tggtggtgga cgtgagccac gaagaccctg aggtcaagtt 1860
   caactggtac gtggacggcg tggaggtgca taatgccaag acaaagccgc gggaggagca 1920
   gtacaacagc acgtaccgtg tggtcagcgt cctcaccgtc ctgcaccagg actggctgaa 1980
   tggcaaggag tacaagtgca aggtctccaa caaagccctc ccagccccca tcgagaaaac 2040
   catctccaaa gccaaagggc agccccgaga accacaggtg tacaccctgc ccccatcccg 2100
   ggatgagctg accaagaacc aggtcagcct gacctgcctg gtcaaaggct tctatcccag 2160
   cgacatcgcc gtggagtggg agagcaatgg gcagccggag aacaactaca agaccacgcc 2220
   tcccgtgctg gactccgacg gctccttctt cctctacagc aagctcaccg tggacaagag 2280
   caggtggcag caggggaacg tcttctcatg ctccgtgatg catgaggctc tgcacaacca 2340
   ctacacgcag aagagcctct ccctgtctct gggtaaatga gggcccgttt aaacagatcc 2400
   agacatgata agatacattg atgagtttgg acaaaccaca actagaatgc agtgaaaaaa 2460
   atgctttatt tgtgaaattt gtgatgctat tgctttattt gtaaccatta taagctgcaa 2520
   taaacaagtt aacaacaaca attgcattca ttttatgttt caggttcagg gggaggtgtg 2580
   ggaggttttt taaagcaagt aaaacctcta caaatgtggt atggctgatt atgatccggc 2640
   tgcctcgcgc gtttcggtga tgacggtgaa aacctctgac acatgcagct cccggagacg 2700
   gtcacagctt gtctgtaagc ggatgccggg agcagacaag cccgtcaggc gtcagcgggt 2760
   gttggcgggt gtcggggcgc agccatgagg tcgactctag aggatcgatg ccccgccccg 2820
   gacgaactaa acctgactac gacatctctg ccccttcttc gcggggcagt gcatgtaatc 2880
   ccttcagttg gttggtacaa cttgccaact gggccctgtt ccacatgtga cacggggggg 2940
   gaccaaacac aaaggggttc tctgactgta gttgacatcc ttataaatgg atgtgcacat 3000
   ttgccaacac tgagtggctt tcatcctgga gcagactttg cagtctgtgg actgcaacac 3060
   aacattgcct ttatgtgtaa ctcttggctg aagctcttac accaatgctg ggggacatgt 3120
   acctcccagg ggcccaggaa gactacggga ggctacacca acgtcaatca gaggggcctg 3180
   tgtagctacc gataagcgga ccctcaagag ggcattagca atagtgttta taaggccccc 3240
   ttgttaaccc taaacgggta gcatatgctt cccgggtagt agtatatact atccagacta 3300
   accctaattc aatagcatat gttacccaac gggaagcata tgctatcgaa ttagggttag 3360
   taaaagggtc ctaaggaaca gcgatatctc ccaccccatg agctgtcacg gttttattta 3420
   catggggtca ggattccacg agggtagtga accattttag tcacaagggc agtggctgaa 3480
   gatcaaggag cgggcagtga actctcctga atcttcgcct gcttcttcat tctccttcgt 3540
   ttagctaata gaataactgc tgagttgtga acagtaaggt gtatgtgagg tgctcgaaaa 3600
   caaggtttca ggtgacgccc ccagaataaa atttggacgg ggggttcagt ggtggcattg 3660
   tgctatgaca ccaatataac cctcacaaac cccttgggca ataaatacta gtgtaggaat 3720
   gaaacattct gaatatcttt aacaatagaa atccatgggg tggggacaag ccgtaaagac 3780
   tggatgtcca tctcacacga atttatggct atgggcaaca cataatccta gtgcaatatg 3840
   atactggggt tattaagatg tgtcccaggc agggaccaag acaggtgaac catgttgtta 3900
   cactctattt gtaacaaggg gaaagagagt ggacgccgac agcagcggac tccactggtt 3960
   gtctctaaca cccccgaaaa ttaaacgggg ctccacgcca atggggccca taaacaaaga 4020
   caagtggcca ctcttttttt tgaaattgtg gagtgggggc acgcgtcagc ccccacacgc 4080
   cgccctgcgg ttttggactg taaaataagg gtgtaataac ttggctgatt gtaaccccgc 4140
   taaccactgc ggtcaaacca cttgcccaca aaaccactaa tggcaccccg gggaatacct 4200
   gcataagtag gtgggcgggc caagataggg gcgcgattgc tgcgatctgg aggacaaatt 4260
   acacacactt gcgcctgagc gccaagcaca gggttgttgg tcctcatatt cacgaggtcg 4320
   ctgagagcac ggtgggctaa tgttgccatg ggtagcatat actacccaaa tatctggata 4380
   gcatatgcta tcctaatcta tatctgggta gcataggcta tcctaatcta tatctgggta 4440
   gcatatgcta tcctaatcta tatctgggta gtatatgcta tcctaattta tatctgggta 4500
   gcataggcta tcctaatcta tatctgggta gcatatgcta tcctaatcta tatctgggta 4560
   gtatatgcta tcctaatctg tatccgggta gcatatgcta tcctaataga gattagggta 4620
   gtatatgcta tcctaattta tatctgggta gcatatacta cccaaatatc tggatagcat 4680
   atgctatcct aatctatatc tgggtagcat atgctatcct aatctatatc tgggtagcat 4740
   aggctatcct aatctatatc tgggtagcat atgctatcct aatctatatc tgggtagtat 4800
   atgctatcct aatttatatc tgggtagcat aggctatcct aatctatatc tgggtagcat 4860
   atgctatcct aatctatatc tgggtagtat atgctatcct aatctgtatc cgggtagcat 4920
   atgctatcct catgcatata cagtcagcat atgataccca gtagtagagt gggagtgcta 4980
   tcctttgcat atgccgccac ctcccaaggg ggcgtgaatt ttcgctgctt gtccttttcc 5040
   tgctggttgc tcccattctt aggtgaattt aaggaggcca ggctaaagcc gtcgcatgtc 5100
   tgattgctca ccaggtaaat gtcgctaatg ttttccaacg cgagaaggtg ttgagcgcgg 5160
   agctgagtga cgtgacaaca tgggtatgcc caattgcccc atgttgggag gacgaaaatg 5220
   gtgacaagac agatggccag aaatacacca acagcacgca tgatgtctac tggggattta 5280
   ttctttagtg cgggggaata cacggctttt aatacgattg agggcgtctc ctaacaagtt 5340
   acatcactcc tgcccttcct caccctcatc tccatcacct ccttcatctc cgtcatctcc 5400
   gtcatcaccc tccgcggcag ccccttccac cataggtgga aaccagggag gcaaatctac 5460
   tccatcgtca aagctgcaca cagtcaccct gatattgcag gtaggagcgg gctttgtcat 5520
   aacaaggtcc ttaatcgcat ccttcaaaac ctcagcaaat atatgagttt gtaaaaagac 5580
   catgaaataa cagacaatgg actcccttag cgggccaggt tgtgggccgg gtccaggggc 5640
   cattccaaag gggagacgac tcaatggtgt aagacgacat tgtggaatag caagggcagt 5700
   tcctcgcctt aggttgtaaa gggaggtctt actacctcca tatacgaaca caccggcgac 5760
   ccaagttcct tcgtcggtag tcctttctac gtgactccta gccaggagag ctcttaaacc 5820
   ttctgcaatg ttctcaaatt tcgggttgga acctccttga ccacgatgct tttccaaacc 5880
   accctccttt tttgcgccct gcctccatca ccctgacccc ggggtccagt gcttgggcct 5940
   tctcctgggt catctgcggg gccctgctct atcgctcccg ggggcacgtc aggctcacca 6000
   tctgggccac cttcttggtg gtattcaaaa taatcggctt cccctacagg gtggaaaaat 6060
   ggccttctac ctggaggggg cctgcgcggt ggagacccgg atgatgatga ctgactactg 6120
   ggactcctgg gcctcttttc tccacgtcca cgacctctcc ccctggctct ttcacgactt 6180
   ccccccctgg ctctttcacg tcctctaccc cggcggcctc cactacctcc tcgaccccgg 6240
   cctccactac ctcctcgacc ccggcctcca ctgcctcctc gaccccggcc tccacctcct 6300
   gctcctgccc ctcctgctcc tgcccctcct cctgctcctg cccctcctgc ccctcctgct 6360
   cctgcccctc ctgcccctcc tgctcctgcc cctcctgccc ctcctgctcc tgcccctcct 6420
   gcccctcctc ctgctcctgc ccctcctgcc cctcctcctg ctcctgcccc tcctgcccct 6480
   cctgctcctg cccctcctgc ccctcctgct cctgcccctc ctgcccctcc tgctcctgcc 6540
   cctcctgctc ctgcccctcc tgctcctgcc cctcctgctc ctgcccctcc tgcccctcct 6600
   gcccctcctc ctgctcctgc ccctcctgct cctgcccctc ctgcccctcc tgcccctcct 6660
   gctcctgccc ctcctcctgc tcctgcccct cctgcccctc ctgcccctcc tcctgctcct 6720
   gcccctcctg cccctcctcc tgctcctgcc cctcctcctg ctcctgcccc tcctgcccct 6780
   cctgcccctc ctcctgctcc tgcccctcct gcccctcctc ctgctcctgc ccctcctcct 6840
   gctcctgccc ctcctgcccc tcctgcccct cctcctgctc ctgcccctcc tcctgctcct 6900
   gcccctcctg cccctcctgc ccctcctgcc cctcctcctg ctcctgcccc tcctcctgct 6960
   cctgcccctc ctgctcctgc ccctcccgct cctgctcctg ctcctgttcc accgtgggtc 7020
   cctttgcagc caatgcaact tggacgtttt tggggtctcc ggacaccatc tctatgtctt 7080
   ggccctgatc ctgagccgcc cggggctcct ggtcttccgc ctcctcgtcc tcgtcctctt 7140
   ccccgtcctc gtccatggtt atcaccccct cttctttgag gtccactgcc gccggagcct 7200
   tctggtccag atgtgtctcc cttctctcct aggccatttc caggtcctgt acctggcccc 7260
   tcgtcagaca tgattcacac taaaagagat caatagacat ctttattaga cgacgctcag 7320
   tgaatacagg gagtgcagac tcctgccccc tccaacagcc cccccaccct catccccttc 7380
   atggtcgctg tcagacagat ccaggtctga aaattcccca tcctccgaac catcctcgtc 7440
   ctcatcacca attactcgca gcccggaaaa ctcccgctga acatcctcaa gatttgcgtc 7500
   ctgagcctca agccaggcct caaattcctc gtcccccttt ttgctggacg gtagggatgg 7560
   ggattctcgg gacccctcct cttcctcttc aaggtcacca gacagagatg ctactggggc 7620
   aacggaagaa aagctgggtg cggcctgtga ggatcagctt atcgatgata agctgtcaaa 7680
   catgagaatt cttgaagacg aaagggcctc gtgatacgcc tatttttata ggttaatgtc 7740
   atgataataa tggtttctta gacgtcaggt ggcacttttc ggggaaatgt gcgcggaacc 7800
   cctatttgtt tatttttcta aatacattca aatatgtatc cgctcatgag acaataaccc 7860
   tgataaatgc ttcaataata ttgaaaaagg aagagtatga gtattcaaca tttccgtgtc 7920
   gcccttattc ccttttttgc ggcattttgc cttcctgttt ttgctcaccc agaaacgctg 7980
   gtgaaagtaa aagatgctga agatcagttg ggtgcacgag tgggttacat cgaactggat 8040
   ctcaacagcg gtaagatcct tgagagtttt cgccccgaag aacgttttcc aatgatgagc 8100
   acttttaaag ttctgctatg tggcgcggta ttatcccgtg ttgacgccgg gcaagagcaa 8160
   ctcggtcgcc gcatacacta ttctcagaat gacttggttg agtactcacc agtcacagaa 8220
   aagcatctta cggatggcat gacagtaaga gaattatgca gtgctgccat aaccatgagt 8280
   gataacactg cggccaactt acttctgaca acgatcggag gaccgaagga gctaaccgct 8340
   tttttgcaca acatggggga tcatgtaact cgccttgatc gttgggaacc ggagctgaat 8400
   gaagccatac caaacgacga gcgtgacacc acgatgcctg cagcaatggc aacaacgttg 8460
   cgcaaactat taactggcga actacttact ctagcttccc ggcaacaatt aatagactgg 8520
   atggaggcgg ataaagttgc aggaccactt ctgcgctcgg cccttccggc tggctggttt 8580
   attgctgata aatctggagc cggtgagcgt gggtctcgcg gtatcattgc agcactgggg 8640
   ccagatggta agccctcccg tatcgtagtt atctacacga cggggagtca ggcaactatg 8700
   gatgaacgaa atagacagat cgctgagata ggtgcctcac tgattaagca ttggtaactg 8760
   tcagaccaag tttactcata tatactttag attgatttaa aacttcattt ttaatttaaa 8820
   aggatctagg tgaagatcct ttttgataat ctcatgacca aaatccctta acgtgagttt 8880
   tcgttccact gagcgtcaga ccccgtagaa aagatcaaag gatcttcttg agatcctttt 8940
   tttctgcgcg taatctgctg cttgcaaaca aaaaaaccac cgctaccagc ggtggtttgt 9000
   ttgccggatc aagagctacc aactcttttt ccgaaggtaa ctggcttcag cagagcgcag 9060
   ataccaaata ctgtccttct agtgtagccg tagttaggcc accacttcaa gaactctgta 9120
   gcaccgccta catacctcgc tctgctaatc ctgttaccag tggctgctgc cagtggcgat 9180
   aagtcgtgtc ttaccgggtt ggactcaaga cgatagttac cggataaggc gcagcggtcg 9240
   ggctgaacgg ggggttcgtg cacacagccc agcttggagc gaacgaccta caccgaactg 9300
   agatacctac agcgtgagct atgagaaagc gccacgcttc ccgaagggag aaaggcggac 9360
   aggtatccgg taagcggcag ggtcggaaca ggagagcgca cgagggagct tccaggggga 9420
   aacgcctggt atctttatag tcctgtcggg tttcgccacc tctgacttga gcgtcgattt 9480
   ttgtgatgct cgtcaggggg gcggagccta tggaaaaacg ccagcaacgc ggccttttta 9540
   cggttcctgg ccttttgctg gccttgaagc tgtccctgat ggtcgtcatc tacctgcctg 9600
   gacagcatgg cctgcaacgc gggcatcccg atgccgccgg aagcgagaag aatcataatg 9660
   gggaaggcca tccagcctcg cgtcgcgaac gccagcaaga cgtagcccag cgcgtcggcc 9720
   ccgagatgcg ccgcgtgcgg ctgctggaga tggcggacgc gatggatatg ttctgccaag 9780
   ggttggtttg cgcattcaca gttctccgca agaattgatt ggctccaatt cttggagtgg 9840
   tgaatccgtt agcgaggtgc cgccctgctt catccccgtg gcccgttgct cgcgtttgct 9900
   ggcggtgtcc ccggaagaaa tatatttgca tgtctttagt tctatgatga cacaaacccc 9960
   gcccagcgtc ttgtcattgg cgaattcgaa cacgcagatg cagtcggggc ggcgcggtcc 10020
   gaggtccact tcgcatatta aggtgacgcg tgtggcctcg aacaccgagc gaccctgcag 10080
   cgacccgctt aacagcgtca acagcgtgcc gcagatcccg gggggcaatg agatatgaaa 10140
   aagcctgaac tcaccgcgac gtctgtcgag aagtttctga tcgaaaagtt cgacagcgtc 10200
   tccgacctga tgcagctctc ggagggcgaa gaatctcgtg ctttcagctt cgatgtagga 10260
   gggcgtggat atgtcctgcg ggtaaatagc tgcgccgatg gtttctacaa agatcgttat 10320
   gtttatcggc actttgcatc ggccgcgctc ccgattccgg aagtgcttga cattggggaa 10380
   ttcagcgaga gcctgaccta ttgcatctcc cgccgtgcac agggtgtcac gttgcaagac 10440
   ctgcctgaaa ccgaactgcc cgctgttctg cagccggtcg cggaggccat ggatgcgatc 10500
   gctgcggccg atcttagcca gacgagcggg ttcggcccat tcggaccgca aggaatcggt 10560
   caatacacta catggcgtga tttcatatgc gcgattgctg atccccatgt gtatcactgg 10620
   caaactgtga tggacgacac cgtcagtgcg tccgtcgcgc aggctctcga tgagctgatg 10680
   ctttgggccg aggactgccc cgaagtccgg cacctcgtgc acgcggattt cggctccaac 10740
   aatgtcctga cggacaatgg ccgcataaca gcggtcattg actggagcga ggcgatgttc 10800
   ggggattccc aatacgaggt cgccaacatc ttcttctgga ggccgtggtt ggcttgtatg 10860
   gagcagcaga cgcgctactt cgagcggagg catccggagc ttgcaggatc gccgcggctc 10920
   cgggcgtata tgctccgcat tggtcttgac caactctatc agagcttggt tgacggcaat 10980
   ttcgatgatg cagcttgggc gcagggtcga tgcgacgcaa tcgtccgatc cggagccggg 11040
   actgtcgggc gtacacaaat cgcccgcaga agcgcggccg tctggaccga tggctgtgta 11100
   gaagtactcg ccgatagtgg aaaccgacgc cccagcactc gtccggatcg ggagatgggg 11160
   gaggctaact gaaacacgga aggagacaat accggaagga acccgcgcta tgacggcaat 11220
   aaaaagacag aataaaacgc acgggtgttg ggtcgtttgt tcataaacgc ggggttcggt 11280
   cccagggctg gcactctgtc gataccccac cgagacccca ttggggccaa tacgcccgcg 11340
   tttcttcctt ttccccaccc caccccccaa gttcgggtga aggcccaggg ctcgcagcca 11400
   acgtcggggc ggcaggccct gccatagcca ctggccccgt gggttaggga cggggtcccc 11460
   catggggaat ggtttatggt tcgtgggggt tattattttg ggcgttgcgt ggggtcaggt 11520
   ccacgactgg actgagcaga cagacccatg gtttttggat ggcctgggca tggaccgcat 11580
   gtactggcgc gacacgaaca ccgggcgtct gtggctgcca aacacccccg acccccaaaa 11640
   accaccgcgc ggatttctgg cgtgccaagc tagtcgacca attctcatgt ttgacagctt 11700
   atcatcgcag atccgggcaa cgttgttgcc attgctgcag gcgcagaact ggtaggtatg 11760
   gaagatctat acattgaatc aatattggca attagccata ttagtcattg gttatatagc 11820
   ataaatcaat attggctatt ggccattgca tacgttgtat ctatatcata atatgtacat 11880
   ttatattggc tcatgtccaa tatgaccgcc at                               11912
<210> 131
   <211> 735
   <212> DNA
   <213> Artificial Sequence
<220>
   <223> In vitro synthesized DNA encoding a melanocortin receptor binding alpha-MSH mimetibody without secretory signal (p3168).
<400> 131
   tcctactcca tggagcactt ccgctggggc aagccggtgg ggggcggagg aagcggcgga 60
   ggcggatcct gcccaccgtg cccagcacct gaactcctgg ggggaccgtc agtcttcctc 120
   ttccccccaa aacccaagga caccctcatg atctcccgga cccctgaggt cacatgcgtg 180
   gtggtggacg tgagccacga agaccctgag gtcaagttca actggtacgt ggacggcgtg 240
   gaggtgcata atgccaagac aaagccgcgg gaggagcagt acaacagcac gtaccgtgtg 300
   gtcagcgtcc tcaccgtcct gcaccaggac tggctgaatg gcaaggagta caagtgcaag 360
   gtctccaaca aagccctccc agcccccatc gagaaaacca tctccaaagc caaagggcag 420
   ccccgagaac cacaggtgta caccctgccc ccatcccggg atgagctgac caagaaccag 480
   gtcagcctga cctgcctggt caaaggcttc tatcccagcg acatcgccgt ggagtgggag 540
   agcaatgggc agccggagaa caactacaag accacgcctc ccgtgctgga ctccgacggc 600
   tccttcttcc tctacagcaa gctcaccgtg gacaagagca ggtggcagca ggggaacgtc 660
   ttctcatgct ccgtgatgca tgaggctctg cacaaccact acacgcagaa gagcctctcc 720
   ctgtctctgg gtaaa                                                  735
<210> 132
   <211> 245
   <212> PRT
   <213> Artificial Sequence
<220>
   <223> Melanocortin receptor binding alpha-MSH mimetibody without secretory signal encoded by an in vitro synthesized DNA (p3168).
<400> 132
   Ser Tyr Ser Met Glu His Phe Arg Trp Gly Lys Pro Val Gly Gly Gly
    1               5                  10                  15
   Gly Ser Gly Gly Gly Gly Ser Cys Pro Pro Cys Pro Ala Pro Glu Leu
               20                  25                  30
   Leu Gly Gly Pro Ser Val Phe Leu Phe Pro Pro Lys Pro Lys Asp Thr
           35                  40                  45
   Leu Met Ile Ser Arg Thr Pro Glu Val Thr Cys Val Val Val Asp Val
       50                  55                  60
   Ser His Glu Asp Pro Glu Val Lys Phe Asn Trp Tyr Val Asp Gly Val
   65                  70                  75                  80
   Glu Val His Asn Ala Lys Thr Lys Pro Arg Glu Glu Gln Tyr Asn Ser
                   85                  90                  95
   Thr Tyr Arg Val Val Ser Val Leu Thr Val Leu His Gln Asp Trp Leu
               100                 105                 110
   Asn Gly Lys Glu Tyr Lys Cys Lys Val Ser Asn Lys Ala Leu Pro Ala
           115                 120                 125
   Pro Ile Glu Lys Thr Ile Ser Lys Ala Lys Gly Gln Pro Arg Glu Pro
       130                 135                 140
   Gln Val Tyr Thr Leu Pro Pro Ser Arg Asp Glu Leu Thr Lys Asn Gln
   145                 150                 155                 160
   Val Ser Leu Thr Cys Leu Val Lys Gly Phe Tyr Pro Ser Asp Ile Ala
                   165                 170                 175
   Val Glu Trp Glu Ser Asn Gly Gln Pro Glu Asn Asn Tyr Lys Thr Thr
               180                 185                 190
   Pro Pro Val Leu Asp Ser Asp Gly Ser Phe Phe Leu Tyr Ser Lys Leu
           195                 200                 205
   Thr Val Asp Lys Ser Arg Trp Gln Gln Gly Asn Val Phe Ser Cys Ser
       210                 215                 220
   Val Met His Glu Ala Leu His Asn His Tyr Thr Gln Lys Ser Leu Ser
   225                 230                 235                 240
   Leu Ser Leu Gly Lys
                   245
<210> 133
   <211> 792
   <212> DNA
   <213> Artificial Sequence
<220>
   <223> In vitro synthesized DNA encoding a melanocortin receptor binding alpha-MSH mimetibody with secretory signal (p3168).
<400> 133
   atggcttggg tgtggacctt gctattcctg atggcggccg cccaaagtat acaggcctcc 60
   tactccatgg agcacttccg ctggggcaag ccggtggggg gcggaggaag cggcggaggc 120
   ggatcctgcc caccgtgccc agcacctgaa ctcctggggg gaccgtcagt cttcctcttc 180
   cccccaaaac ccaaggacac cctcatgatc tcccggaccc ctgaggtcac atgcgtggtg 240
   gtggacgtga gccacgaaga ccctgaggtc aagttcaact ggtacgtgga cggcgtggag 300
   gtgcataatg ccaagacaaa gccgcgggag gagcagtaca acagcacgta ccgtgtggtc 360
   agcgtcctca ccgtcctgca ccaggactgg ctgaatggca aggagtacaa gtgcaaggtc 420
   tccaacaaag ccctcccagc ccccatcgag aaaaccatct ccaaagccaa agggcagccc 480
   cgagaaccac aggtgtacac cctgccccca tcccgggatg agctgaccaa gaaccaggtc 540
   agcctgacct gcctggtcaa aggcttctat cccagcgaca tcgccgtgga gtgggagagc 600
   aatgggcagc cggagaacaa ctacaagacc acgcctcccg tgctggactc cgacggctcc 660
   ttcttcctct acagcaagct caccgtggac aagagcaggt ggcagcaggg gaacgtcttc 720
   tcatgctccg tgatgcatga ggctctgcac aaccactaca cgcagaagag cctctccctg 780
   tctctgggta aa                                                     792
<210> 134
   <211> 264
   <212> PRT
   <213> Artificial Sequence
<220>
   <223> Melanocortin receptor binding alpha-MSH mimetibody with secretory signal encoded by an in vitro synthesized DNA (p3168).
<400> 134
   Met Ala Trp Val Trp Thr Leu Leu Phe Leu Met Ala Ala Ala Gln Ser
    1               5                  10                  15
   Ile Gln Ala Ser Tyr Ser Met Glu His Phe Arg Trp Gly Lys Pro Val
               20                  25                  30
   Gly Gly Gly Gly Ser Gly Gly Gly Gly Ser Cys Pro Pro Cys Pro Ala
           35                  40                  45
   Pro Glu Leu Leu Gly Gly Pro Ser Val Phe Leu Phe Pro Pro Lys Pro
       50                  55                  60
   Lys Asp Thr Leu Met Ile Ser Arg Thr Pro Glu Val Thr Cys Val Val
   65                  70                  75                  80
   Val Asp Val Ser His Glu Asp Pro Glu Val Lys Phe Asn Trp Tyr Val
                   85                  90                  95
   Asp Gly Val Glu Val His Asn Ala Lys Thr Lys Pro Arg Glu Glu Gln
               100                 105                 110
   Tyr Asn Ser Thr Tyr Arg Val Val Ser Val Leu Thr Val Leu His Gln
           115                 120                 125
   Asp Trp Leu Asn Gly Lys Glu Tyr Lys Cys Lys Val Ser Asn Lys Ala
       130                 135                 140
   Leu Pro Ala Pro Ile Glu Lys Thr Ile Ser Lys Ala Lys Gly Gln Pro
   145                 150                 155                 160
   Arg Glu Pro Gln Val Tyr Thr Leu Pro Pro Ser Arg Asp Glu Leu Thr
                   165                 170                 175
   Lys Asn Gln Val Ser Leu Thr Cys Leu Val Lys Gly Phe Tyr Pro Ser
               180                 185                 190
   Asp Ile Ala Val Glu Trp Glu Ser Asn Gly Gln Pro Glu Asn Asn Tyr
           195                 200                 205
   Lys Thr Thr Pro Pro Val Leu Asp Ser Asp Gly Ser Phe Phe Leu Tyr
       210                 215                 220
   Ser Lys Leu Thr Val Asp Lys Ser Arg Trp Gln Gln Gly Asn Val Phe
   225                 230                 235                 240
   Ser Cys Ser Val Met His Glu Ala Leu His Asn His Tyr Thr Gln Lys
                   245                 250                 255
   Ser Leu Ser Leu Ser Leu Gly Lys
               260
<210> 135
   <211> 11927
   <212> DNA
   <213> Artificial Sequence
<220>
   <223> In vitro synthesized DNA with expression vector functions that encodes an alpha-MSH mimetibody (p3168).
<400> 135
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactccatgg agcacttccg 1680
   ctggggcaag ccggtggggg gcggaggaag cggcggaggc ggatcctgcc caccgtgccc 1740
   agcacctgaa ctcctggggg gaccgtcagt cttcctcttc cccccaaaac ccaaggacac 1800
   cctcatgatc tcccggaccc ctgaggtcac atgcgtggtg gtggacgtga gccacgaaga 1860
   ccctgaggtc aagttcaact ggtacgtgga cggcgtggag gtgcataatg ccaagacaaa 1920
   gccgcgggag gagcagtaca acagcacgta ccgtgtggtc agcgtcctca ccgtcctgca 1980
   ccaggactgg ctgaatggca aggagtacaa gtgcaaggtc tccaacaaag ccctcccagc 2040
   ccccatcgag aaaaccatct ccaaagccaa agggcagccc cgagaaccac aggtgtacac 2100
   cctgccccca tcccgggatg agctgaccaa gaaccaggtc agcctgacct gcctggtcaa 2160
   aggcttctat cccagcgaca tcgccgtgga gtgggagagc aatgggcagc cggagaacaa 2220
   ctacaagacc acgcctcccg tgctggactc cgacggctcc ttcttcctct acagcaagct 2280
   caccgtggac aagagcaggt ggcagcaggg gaacgtcttc tcatgctccg tgatgcatga 2340
   ggctctgcac aaccactaca cgcagaagag cctctccctg tctctgggta aatgagggcc 2400
   cgtttaaaca gatccagaca tgataagata cattgatgag tttggacaaa ccacaactag 2460
   aatgcagtga aaaaaatgct ttatttgtga aatttgtgat gctattgctt tatttgtaac 2520
   cattataagc tgcaataaac aagttaacaa caacaattgc attcatttta tgtttcaggt 2580
   tcagggggag gtgtgggagg ttttttaaag caagtaaaac ctctacaaat gtggtatggc 2640
   tgattatgat ccggctgcct cgcgcgtttc ggtgatgacg gtgaaaacct ctgacacatg 2700
   cagctcccgg agacggtcac agcttgtctg taagcggatg ccgggagcag acaagcccgt 2760
   caggcgtcag cgggtgttgg cgggtgtcgg ggcgcagcca tgaggtcgac tctagaggat 2820
   cgatgccccg ccccggacga actaaacctg actacgacat ctctgcccct tcttcgcggg 2880
   gcagtgcatg taatcccttc agttggttgg tacaacttgc caactgggcc ctgttccaca 2940
   tgtgacacgg ggggggacca aacacaaagg ggttctctga ctgtagttga catccttata 3000
   aatggatgtg cacatttgcc aacactgagt ggctttcatc ctggagcaga ctttgcagtc 3060
   tgtggactgc aacacaacat tgcctttatg tgtaactctt ggctgaagct cttacaccaa 3120
   tgctggggga catgtacctc ccaggggccc aggaagacta cgggaggcta caccaacgtc 3180
   aatcagaggg gcctgtgtag ctaccgataa gcggaccctc aagagggcat tagcaatagt 3240
   gtttataagg cccccttgtt aaccctaaac gggtagcata tgcttcccgg gtagtagtat 3300
   atactatcca gactaaccct aattcaatag catatgttac ccaacgggaa gcatatgcta 3360
   tcgaattagg gttagtaaaa gggtcctaag gaacagcgat atctcccacc ccatgagctg 3420
   tcacggtttt atttacatgg ggtcaggatt ccacgagggt agtgaaccat tttagtcaca 3480
   agggcagtgg ctgaagatca aggagcgggc agtgaactct cctgaatctt cgcctgcttc 3540
   ttcattctcc ttcgtttagc taatagaata actgctgagt tgtgaacagt aaggtgtatg 3600
   tgaggtgctc gaaaacaagg tttcaggtga cgcccccaga ataaaatttg gacggggggt 3660
   tcagtggtgg cattgtgcta tgacaccaat ataaccctca caaacccctt gggcaataaa 3720
   tactagtgta ggaatgaaac attctgaata tctttaacaa tagaaatcca tggggtgggg 3780
   acaagccgta aagactggat gtccatctca cacgaattta tggctatggg caacacataa 3840
   tcctagtgca atatgatact ggggttatta agatgtgtcc caggcaggga ccaagacagg 3900
   tgaaccatgt tgttacactc tatttgtaac aaggggaaag agagtggacg ccgacagcag 3960
   cggactccac tggttgtctc taacaccccc gaaaattaaa cggggctcca cgccaatggg 4020
   gcccataaac aaagacaagt ggccactctt ttttttgaaa ttgtggagtg ggggcacgcg 4080
   tcagccccca cacgccgccc tgcggttttg gactgtaaaa taagggtgta ataacttggc 4140
   tgattgtaac cccgctaacc actgcggtca aaccacttgc ccacaaaacc actaatggca 4200
   ccccggggaa tacctgcata agtaggtggg cgggccaaga taggggcgcg attgctgcga 4260
   tctggaggac aaattacaca cacttgcgcc tgagcgccaa gcacagggtt gttggtcctc 4320
   atattcacga ggtcgctgag agcacggtgg gctaatgttg ccatgggtag catatactac 4380
   ccaaatatct ggatagcata tgctatccta atctatatct gggtagcata ggctatccta 4440
   atctatatct gggtagcata tgctatccta atctatatct gggtagtata tgctatccta 4500
   atttatatct gggtagcata ggctatccta atctatatct gggtagcata tgctatccta 4560
   atctatatct gggtagtata tgctatccta atctgtatcc gggtagcata tgctatccta 4620
   atagagatta gggtagtata tgctatccta atttatatct gggtagcata tactacccaa 4680
   atatctggat agcatatgct atcctaatct atatctgggt agcatatgct atcctaatct 4740
   atatctgggt agcataggct atcctaatct atatctgggt agcatatgct atcctaatct 4800
   atatctgggt agtatatgct atcctaattt atatctgggt agcataggct atcctaatct 4860
   atatctgggt agcatatgct atcctaatct atatctgggt agtatatgct atcctaatct 4920
   gtatccgggt agcatatgct atcctcatgc atatacagtc agcatatgat acccagtagt 4980
   agagtgggag tgctatcctt tgcatatgcc gccacctccc aagggggcgt gaattttcgc 5040
   tgcttgtcct tttcctgctg gttgctccca ttcttaggtg aatttaagga ggccaggcta 5100
   aagccgtcgc atgtctgatt gctcaccagg taaatgtcgc taatgttttc caacgcgaga 5160
   aggtgttgag cgcggagctg agtgacgtga caacatgggt atgcccaatt gccccatgtt 5220
   gggaggacga aaatggtgac aagacagatg gccagaaata caccaacagc acgcatgatg 5280
   tctactgggg atttattctt tagtgcgggg gaatacacgg cttttaatac gattgagggc 5340
   gtctcctaac aagttacatc actcctgccc ttcctcaccc tcatctccat cacctccttc 5400
   atctccgtca tctccgtcat caccctccgc ggcagcccct tccaccatag gtggaaacca 5460
   gggaggcaaa tctactccat cgtcaaagct gcacacagtc accctgatat tgcaggtagg 5520
   agcgggcttt gtcataacaa ggtccttaat cgcatccttc aaaacctcag caaatatatg 5580
   agtttgtaaa aagaccatga aataacagac aatggactcc cttagcgggc caggttgtgg 5640
   gccgggtcca ggggccattc caaaggggag acgactcaat ggtgtaagac gacattgtgg 5700
   aatagcaagg gcagttcctc gccttaggtt gtaaagggag gtcttactac ctccatatac 5760
   gaacacaccg gcgacccaag ttccttcgtc ggtagtcctt tctacgtgac tcctagccag 5820
   gagagctctt aaaccttctg caatgttctc aaatttcggg ttggaacctc cttgaccacg 5880
   atgcttttcc aaaccaccct ccttttttgc gccctgcctc catcaccctg accccggggt 5940
   ccagtgcttg ggccttctcc tgggtcatct gcggggccct gctctatcgc tcccgggggc 6000
   acgtcaggct caccatctgg gccaccttct tggtggtatt caaaataatc ggcttcccct 6060
   acagggtgga aaaatggcct tctacctgga gggggcctgc gcggtggaga cccggatgat 6120
   gatgactgac tactgggact cctgggcctc ttttctccac gtccacgacc tctccccctg 6180
   gctctttcac gacttccccc cctggctctt tcacgtcctc taccccggcg gcctccacta 6240
   cctcctcgac cccggcctcc actacctcct cgaccccggc ctccactgcc tcctcgaccc 6300
   cggcctccac ctcctgctcc tgcccctcct gctcctgccc ctcctcctgc tcctgcccct 6360
   cctgcccctc ctgctcctgc ccctcctgcc cctcctgctc ctgcccctcc tgcccctcct 6420
   gctcctgccc ctcctgcccc tcctcctgct cctgcccctc ctgcccctcc tcctgctcct 6480
   gcccctcctg cccctcctgc tcctgcccct cctgcccctc ctgctcctgc ccctcctgcc 6540
   cctcctgctc ctgcccctcc tgctcctgcc cctcctgctc ctgcccctcc tgctcctgcc 6600
   cctcctgccc ctcctgcccc tcctcctgct cctgcccctc ctgctcctgc ccctcctgcc 6660
   cctcctgccc ctcctgctcc tgcccctcct cctgctcctg cccctcctgc ccctcctgcc 6720
   cctcctcctg ctcctgcccc tcctgcccct cctcctgctc ctgcccctcc tcctgctcct 6780
   gcccctcctg cccctcctgc ccctcctcct gctcctgccc ctcctgcccc tcctcctgct 6840
   cctgcccctc ctcctgctcc tgcccctcct gcccctcctg cccctcctcc tgctcctgcc 6900
   cctcctcctg ctcctgcccc tcctgcccct cctgcccctc ctgcccctcc tcctgctcct 6960
   gcccctcctc ctgctcctgc ccctcctgct cctgcccctc ccgctcctgc tcctgctcct 7020
   gttccaccgt gggtcccttt gcagccaatg caacttggac gtttttgggg tctccggaca 7080
   ccatctctat gtcttggccc tgatcctgag ccgcccgggg ctcctggtct tccgcctcct 7140
   cgtcctcgtc ctcttccccg tcctcgtcca tggttatcac cccctcttct ttgaggtcca 7200
   ctgccgccgg agccttctgg tccagatgtg tctcccttct ctcctaggcc atttccaggt 7260
   cctgtacctg gcccctcgtc agacatgatt cacactaaaa gagatcaata gacatcttta 7320
   ttagacgacg ctcagtgaat acagggagtg cagactcctg ccccctccaa cagccccccc 7380
   accctcatcc ccttcatggt cgctgtcaga cagatccagg tctgaaaatt ccccatcctc 7440
   cgaaccatcc tcgtcctcat caccaattac tcgcagcccg gaaaactccc gctgaacatc 7500
   ctcaagattt gcgtcctgag cctcaagcca ggcctcaaat tcctcgtccc cctttttgct 7560
   ggacggtagg gatggggatt ctcgggaccc ctcctcttcc tcttcaaggt caccagacag 7620
   agatgctact ggggcaacgg aagaaaagct gggtgcggcc tgtgaggatc agcttatcga 7680
   tgataagctg tcaaacatga gaattcttga agacgaaagg gcctcgtgat acgcctattt 7740
   ttataggtta atgtcatgat aataatggtt tcttagacgt caggtggcac ttttcgggga 7800
   aatgtgcgcg gaacccctat ttgtttattt ttctaaatac attcaaatat gtatccgctc 7860
   atgagacaat aaccctgata aatgcttcaa taatattgaa aaaggaagag tatgagtatt 7920
   caacatttcc gtgtcgccct tattcccttt tttgcggcat tttgccttcc tgtttttgct 7980
   cacccagaaa cgctggtgaa agtaaaagat gctgaagatc agttgggtgc acgagtgggt 8040
   tacatcgaac tggatctcaa cagcggtaag atccttgaga gttttcgccc cgaagaacgt 8100
   tttccaatga tgagcacttt taaagttctg ctatgtggcg cggtattatc ccgtgttgac 8160
   gccgggcaag agcaactcgg tcgccgcata cactattctc agaatgactt ggttgagtac 8220
   tcaccagtca cagaaaagca tcttacggat ggcatgacag taagagaatt atgcagtgct 8280
   gccataacca tgagtgataa cactgcggcc aacttacttc tgacaacgat cggaggaccg 8340
   aaggagctaa ccgctttttt gcacaacatg ggggatcatg taactcgcct tgatcgttgg 8400
   gaaccggagc tgaatgaagc cataccaaac gacgagcgtg acaccacgat gcctgcagca 8460
   atggcaacaa cgttgcgcaa actattaact ggcgaactac ttactctagc ttcccggcaa 8520
   caattaatag actggatgga ggcggataaa gttgcaggac cacttctgcg ctcggccctt 8580
   ccggctggct ggtttattgc tgataaatct ggagccggtg agcgtgggtc tcgcggtatc 8640
   attgcagcac tggggccaga tggtaagccc tcccgtatcg tagttatcta cacgacgggg 8700
   agtcaggcaa ctatggatga acgaaataga cagatcgctg agataggtgc ctcactgatt 8760
   aagcattggt aactgtcaga ccaagtttac tcatatatac tttagattga tttaaaactt 8820
   catttttaat ttaaaaggat ctaggtgaag atcctttttg ataatctcat gaccaaaatc 8880
   ccttaacgtg agttttcgtt ccactgagcg tcagaccccg tagaaaagat caaaggatct 8940
   tcttgagatc ctttttttct gcgcgtaatc tgctgcttgc aaacaaaaaa accaccgcta 9000
   ccagcggtgg tttgtttgcc ggatcaagag ctaccaactc tttttccgaa ggtaactggc 9060
   ttcagcagag cgcagatacc aaatactgtc cttctagtgt agccgtagtt aggccaccac 9120
   ttcaagaact ctgtagcacc gcctacatac ctcgctctgc taatcctgtt accagtggct 9180
   gctgccagtg gcgataagtc gtgtcttacc gggttggact caagacgata gttaccggat 9240
   aaggcgcagc ggtcgggctg aacggggggt tcgtgcacac agcccagctt ggagcgaacg 9300
   acctacaccg aactgagata cctacagcgt gagctatgag aaagcgccac gcttcccgaa 9360
   gggagaaagg cggacaggta tccggtaagc ggcagggtcg gaacaggaga gcgcacgagg 9420
   gagcttccag ggggaaacgc ctggtatctt tatagtcctg tcgggtttcg ccacctctga 9480
   cttgagcgtc gatttttgtg atgctcgtca ggggggcgga gcctatggaa aaacgccagc 9540
   aacgcggcct ttttacggtt cctggccttt tgctggcctt gaagctgtcc ctgatggtcg 9600
   tcatctacct gcctggacag catggcctgc aacgcgggca tcccgatgcc gccggaagcg 9660
   agaagaatca taatggggaa ggccatccag cctcgcgtcg cgaacgccag caagacgtag 9720
   cccagcgcgt cggccccgag atgcgccgcg tgcggctgct ggagatggcg gacgcgatgg 9780
   atatgttctg ccaagggttg gtttgcgcat tcacagttct ccgcaagaat tgattggctc 9840
   caattcttgg agtggtgaat ccgttagcga ggtgccgccc tgcttcatcc ccgtggcccg 9900
   ttgctcgcgt ttgctggcgg tgtccccgga agaaatatat ttgcatgtct ttagttctat 9960
   gatgacacaa accccgccca gcgtcttgtc attggcgaat tcgaacacgc agatgcagtc 10020
   ggggcggcgc ggtccgaggt ccacttcgca tattaaggtg acgcgtgtgg cctcgaacac 10080
   cgagcgaccc tgcagcgacc cgcttaacag cgtcaacagc gtgccgcaga tcccgggggg 10140
   caatgagata tgaaaaagcc tgaactcacc gcgacgtctg tcgagaagtt tctgatcgaa 10200
   aagttcgaca gcgtctccga cctgatgcag ctctcggagg gcgaagaatc tcgtgctttc 10260
   agcttcgatg taggagggcg tggatatgtc ctgcgggtaa atagctgcgc cgatggtttc 10320
   tacaaagatc gttatgttta tcggcacttt gcatcggccg cgctcccgat tccggaagtg 10380
   cttgacattg gggaattcag cgagagcctg acctattgca tctcccgccg tgcacagggt 10440
   gtcacgttgc aagacctgcc tgaaaccgaa ctgcccgctg ttctgcagcc ggtcgcggag 10500
   gccatggatg cgatcgctgc ggccgatctt agccagacga gcgggttcgg cccattcgga 10560
   ccgcaaggaa tcggtcaata cactacatgg cgtgatttca tatgcgcgat tgctgatccc 10620
   catgtgtatc actggcaaac tgtgatggac gacaccgtca gtgcgtccgt cgcgcaggct 10680
   ctcgatgagc tgatgctttg ggccgaggac tgccccgaag tccggcacct cgtgcacgcg 10740
   gatttcggct ccaacaatgt cctgacggac aatggccgca taacagcggt cattgactgg 10800
   agcgaggcga tgttcgggga ttcccaatac gaggtcgcca acatcttctt ctggaggccg 10860
   tggttggctt gtatggagca gcagacgcgc tacttcgagc ggaggcatcc ggagcttgca 10920
   ggatcgccgc ggctccgggc gtatatgctc cgcattggtc ttgaccaact ctatcagagc 10980
   ttggttgacg gcaatttcga tgatgcagct tgggcgcagg gtcgatgcga cgcaatcgtc 11040
   cgatccggag ccgggactgt cgggcgtaca caaatcgccc gcagaagcgc ggccgtctgg 11100
   accgatggct gtgtagaagt actcgccgat agtggaaacc gacgccccag cactcgtccg 11160
   gatcgggaga tgggggaggc taactgaaac acggaaggag acaataccgg aaggaacccg 11220
   cgctatgacg gcaataaaaa gacagaataa aacgcacggg tgttgggtcg tttgttcata 11280
   aacgcggggt tcggtcccag ggctggcact ctgtcgatac cccaccgaga ccccattggg 11340
   gccaatacgc ccgcgtttct tccttttccc caccccaccc cccaagttcg ggtgaaggcc 11400
   cagggctcgc agccaacgtc ggggcggcag gccctgccat agccactggc cccgtgggtt 11460
   agggacgggg tcccccatgg ggaatggttt atggttcgtg ggggttatta ttttgggcgt 11520
   tgcgtggggt caggtccacg actggactga gcagacagac ccatggtttt tggatggcct 11580
   gggcatggac cgcatgtact ggcgcgacac gaacaccggg cgtctgtggc tgccaaacac 11640
   ccccgacccc caaaaaccac cgcgcggatt tctggcgtgc caagctagtc gaccaattct 11700
   catgtttgac agcttatcat cgcagatccg ggcaacgttg ttgccattgc tgcaggcgca 11760
   gaactggtag gtatggaaga tctatacatt gaatcaatat tggcaattag ccatattagt 11820
   cattggttat atagcataaa tcaatattgg ctattggcca ttgcatacgt tgtatctata 11880
   tcataatatg tacatttata ttggctcatg tccaatatga ccgccat 11927
<210> 136
   <211> 750
   <212> DNA
   <213> Artificial Sequence
<220>
   <223> In vitro synthesized DNA encoding a melanocortin receptor binding alpha-MSH mimetibody without secretory signal (p3169).
<400> 136
   tcctactcca tggagcactt ccgctggggc aagccggtgg ggggcggagg aagcggcgga 60
   ggcgggagcg gcggcggagg atcctgccca ccgtgcccag cacctgaact cctgggggga 120
   ccgtcagtct tcctcttccc cccaaaaccc aaggacaccc tcatgatctc ccggacccct 180
   gaggtcacat gcgtggtggt ggacgtgagc cacgaagacc ctgaggtcaa gttcaactgg 240
   tacgtggacg gcgtggaggt gcataatgcc aagacaaagc cgcgggagga gcagtacaac 300
   agcacgtacc gtgtggtcag cgtcctcacc gtcctgcacc aggactggct gaatggcaag 360
   gagtacaagt gcaaggtctc caacaaagcc ctcccagccc ccatcgagaa aaccatctcc 420
   aaagccaaag ggcagccccg agaaccacag gtgtacaccc tgcccccatc ccgggatgag 480
   ctgaccaaga accaggtcag cctgacctgc ctggtcaaag gcttctatcc cagcgacatc 540
   gccgtggagt gggagagcaa tgggcagccg gagaacaact acaagaccac gcctcccgtg 600
   ctggactccg acggctcctt cttcctctac agcaagctca ccgtggacaa gagcaggtgg 660
   cagcagggga acgtcttctc atgctccgtg atgcatgagg ctctgcacaa ccactacacg 720
   cagaagagcc tctccctgtc tctgggtaaa                                  750
<210> 137
   <211> 250
   <212> PRT
   <213> Artificial Sequence
<220>
   <223> Melanocortin receptor binding alpha-MSH mimetibody without secretory signal encoded by an in vitro synthesized DNA (p3169).
<400> 137
   Ser Tyr Ser Met Glu His Phe Arg Trp Gly Lys Pro Val Gly Gly Gly
    1               5                  10                  15
   Gly Ser Gly Gly Gly Gly Ser Gly Gly Gly Gly Ser Cys Pro Pro Cys
               20                  25                  30
   Pro Ala Pro Glu Leu Leu Gly Gly Pro Ser Val Phe Leu Phe Pro Pro
           35                  40                  45
   Lys Pro Lys Asp Thr Leu Met Ile Ser Arg Thr Pro Glu Val Thr Cys
       50                  55                  60
   Val Val Val Asp Val Ser His Glu Asp Pro Glu Val Lys Phe Asn Trp
   65                  70                  75                  80
   Tyr Val Asp Gly Val Glu Val His Asn Ala Lys Thr Lys Pro Arg Glu
                   85                  90                  95
   Glu Gln Tyr Asn Ser Thr Tyr Arg Val Val Ser Val Leu Thr Val Leu
               100                 105                 110
   His Gln Asp Trp Leu Asn Gly Lys Glu Tyr Lys Cys Lys Val Ser Asn
           115                 120                 125
   Lys Ala Leu Pro Ala Pro Ile Glu Lys Thr Ile Ser Lys Ala Lys Gly
       130                 135                 140
   Gln Pro Arg Glu Pro Gln Val Tyr Thr Leu Pro Pro Ser Arg Asp Glu
   145                 150                 155                 160
   Leu Thr Lys Asn Gln Val Ser Leu Thr Cys Leu Val Lys Gly Phe Tyr
                   165                 170                 175
   Pro Ser Asp Ile Ala Val Glu Trp Glu Ser Asn Gly Gln Pro Glu Asn
               180                 185                 190
   Asn Tyr Lys Thr Thr Pro Pro Val Leu Asp Ser Asp Gly Ser Phe Phe
           195                 200                 205
   Leu Tyr Ser Lys Leu Thr Val Asp Lys Ser Arg Trp Gln Gln Gly Asn
       210                 215                 220
   Val Phe Ser Cys Ser Val Met His Glu Ala Leu His Asn His Tyr Thr
   225                 230                 235                 240
   Gln Lys Ser Leu Ser Leu Ser Leu Gly Lys
                   245                 250
<210> 138
   <211> 807
   <212> DNA
   <213> Artificial Sequence
<220>
   <223> In vitro synthesized DNA encoding a melanocortin receptor binding alpha-MSH mimetibody with secretory signal (p3169).
<400> 138
   atggcttggg tgtggacctt gctattcctg atggcggccg cccaaagtat acaggcctcc 60
   tactccatgg agcacttccg ctggggcaag ccggtggggg gcggaggaag cggcggaggc 120
   gggagcggcg gcggaggatc ctgcccaccg tgcccagcac ctgaactcct ggggggaccg 180
   tcagtcttcc tcttcccccc aaaacccaag gacaccctca tgatctcccg gacccctgag 240
   gtcacatgcg tggtggtgga cgtgagccac gaagaccctg aggtcaagtt caactggtac 300
   gtggacggcg tggaggtgca taatgccaag acaaagccgc gggaggagca gtacaacagc 360
   acgtaccgtg tggtcagcgt cctcaccgtc ctgcaccagg actggctgaa tggcaaggag 420
   tacaagtgca aggtctccaa caaagccctc ccagccccca tcgagaaaac catctccaaa 480
   gccaaagggc agccccgaga accacaggtg tacaccctgc ccccatcccg ggatgagctg 540
   accaagaacc aggtcagcct gacctgcctg gtcaaaggct tctatcccag cgacatcgcc 600
   gtggagtggg agagcaatgg gcagccggag aacaactaca agaccacgcc tcccgtgctg 660
   gactccgacg gctccttctt cctctacagc aagctcaccg tggacaagag caggtggcag 720
   caggggaacg tcttctcatg ctccgtgatg catgaggctc tgcacaacca ctacacgcag 780
   aagagcctct ccctgtctct gggtaaa                                     807
<210> 139
   <211> 269
   <212> PRT
   <213> Artificial Sequence
<220>
   <223> Melanocortin receptor binding alpha-MSH mimetibody with secretory signal encoded by an in vitro synthesized DNA (p3169).
<400> 139
   Met Ala Trp Val Trp Thr Leu Leu Phe Leu Met Ala Ala Ala Gln Ser
    1               5                  10                  15
   Ile Gln Ala Ser Tyr Ser Met Glu His Phe Arg Trp Gly Lys Pro Val
               20                  25                  30
   Gly Gly Gly Gly Ser Gly Gly Gly Gly Ser Gly Gly Gly Gly Ser Cys
           35                  40                  45
   Pro Pro Cys Pro Ala Pro Glu Leu Leu Gly Gly Pro Ser Val Phe Leu
       50                  55                  60
   Phe Pro Pro Lys Pro Lys Asp Thr Leu Met Ile Ser Arg Thr Pro Glu
   65                  70                  75                  80
   Val Thr Cys Val Val Val Asp Val Ser His Glu Asp Pro Glu Val Lys
                   85                  90                  95
   Phe Asn Trp Tyr Val Asp Gly Val Glu Val His Asn Ala Lys Thr Lys
               100                 105                 110
   Pro Arg Glu Glu Gln Tyr Asn Ser Thr Tyr Arg Val Val Ser Val Leu
           115                 120                 125
   Thr Val Leu His Gln Asp Trp Leu Asn Gly Lys Glu Tyr Lys Cys Lys
       130                 135                 140
   Val Ser Asn Lys Ala Leu Pro Ala Pro Ile Glu Lys Thr Ile Ser Lys
   145                 150                 155                 160
   Ala Lys Gly Gln Pro Arg Glu Pro Gln Val Tyr Thr Leu Pro Pro Ser
                   165                 170                 175
   Arg Asp Glu Leu Thr Lys Asn Gln Val Ser Leu Thr Cys Leu Val Lys
               180                 185                 190
   Gly Phe Tyr Pro Ser Asp Ile Ala Val Glu Trp Glu Ser Asn Gly Gln
           195                 200                 205
   Pro Glu Asn Asn Tyr Lys Thr Thr Pro Pro Val Leu Asp Ser Asp Gly
       210                 215                 220
   Ser Phe Phe Leu Tyr Ser Lys Leu Thr Val Asp Lys Ser Arg Trp Gln
   225                 230                 235                 240
   Gln Gly Asn Val Phe Ser Cys Ser Val Met His Glu Ala Leu His Asn
                   245                 250                 255
   His Tyr Thr Gln Lys Ser Leu Ser Leu Ser Leu Gly Lys
               260                 265
<210> 140
   <211> 11942
   <212> DNA
   <213> Artificial Sequence
<220>
   <223> In vitro synthesized DNA with expression vector functions that encodes an alpha-MSH mimetibody (p3169).
<400> 140
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactccatgg agcacttccg 1680
   ctggggcaag ccggtggggg gcggaggaag cggcggaggc gggagcggcg gcggaggatc 1740
   ctgcccaccg tgcccagcac ctgaactcct ggggggaccg tcagtcttcc tcttcccccc 1800
   aaaacccaag gacaccctca tgatctcccg gacccctgag gtcacatgcg tggtggtgga 1860
   cgtgagccac gaagaccctg aggtcaagtt caactggtac gtggacggcg tggaggtgca 1920
   taatgccaag acaaagccgc gggaggagca gtacaacagc acgtaccgtg tggtcagcgt 1980
   cctcaccgtc ctgcaccagg actggctgaa tggcaaggag tacaagtgca aggtctccaa 2040
   caaagccctc ccagccccca tcgagaaaac catctccaaa gccaaagggc agccccgaga 2100
   accacaggtg tacaccctgc ccccatcccg ggatgagctg accaagaacc aggtcagcct 2160
   gacctgcctg gtcaaaggct tctatcccag cgacatcgcc gtggagtggg agagcaatgg 2220
   gcagccggag aacaactaca agaccacgcc tcccgtgctg gactccgacg gctccttctt 2280
   cctctacagc aagctcaccg tggacaagag caggtggcag caggggaacg tcttctcatg 2340
   ctccgtgatg catgaggctc tgcacaacca ctacacgcag aagagcctct ccctgtctct 2400
   gggtaaatga gggcccgttt aaacagatcc agacatgata agatacattg atgagtttgg 2460
   acaaaccaca actagaatgc agtgaaaaaa atgctttatt tgtgaaattt gtgatgctat 2520
   tgctttattt gtaaccatta taagctgcaa taaacaagtt aacaacaaca attgcattca 2580
   ttttatgttt caggttcagg gggaggtgtg ggaggttttt taaagcaagt aaaacctcta 2640
   caaatgtggt atggctgatt atgatccggc tgcctcgcgc gtttcggtga tgacggtgaa 2700
   aacctctgac acatgcagct cccggagacg gtcacagctt gtctgtaagc ggatgccggg 2760
   agcagacaag cccgtcaggc gtcagcgggt gttggcgggt gtcggggcgc agccatgagg 2820
   tcgactctag aggatcgatg ccccgccccg gacgaactaa acctgactac gacatctctg 2880
   ccccttcttc gcggggcagt gcatgtaatc ccttcagttg gttggtacaa cttgccaact 2940
   gggccctgtt ccacatgtga cacggggggg gaccaaacac aaaggggttc tctgactgta 3000
   gttgacatcc ttataaatgg atgtgcacat ttgccaacac tgagtggctt tcatcctgga 3060
   gcagactttg cagtctgtgg actgcaacac aacattgcct ttatgtgtaa ctcttggctg 3120
   aagctcttac accaatgctg ggggacatgt acctcccagg ggcccaggaa gactacggga 3180
   ggctacacca acgtcaatca gaggggcctg tgtagctacc gataagcgga ccctcaagag 3240
   ggcattagca atagtgttta taaggccccc ttgttaaccc taaacgggta gcatatgctt 3300
   cccgggtagt agtatatact atccagacta accctaattc aatagcatat gttacccaac 3360
   gggaagcata tgctatcgaa ttagggttag taaaagggtc ctaaggaaca gcgatatctc 3420
   ccaccccatg agctgtcacg gttttattta catggggtca ggattccacg agggtagtga 3480
   accattttag tcacaagggc agtggctgaa gatcaaggag cgggcagtga actctcctga 3540
   atcttcgcct gcttcttcat tctccttcgt ttagctaata gaataactgc tgagttgtga 3600
   acagtaaggt gtatgtgagg tgctcgaaaa caaggtttca ggtgacgccc ccagaataaa 3660
   atttggacgg ggggttcagt ggtggcattg tgctatgaca ccaatataac cctcacaaac 3720
   cccttgggca ataaatacta gtgtaggaat gaaacattct gaatatcttt aacaatagaa 3780
   atccatgggg tggggacaag ccgtaaagac tggatgtcca tctcacacga atttatggct 3840
   atgggcaaca cataatccta gtgcaatatg atactggggt tattaagatg tgtcccaggc 3900
   agggaccaag acaggtgaac catgttgtta cactctattt gtaacaaggg gaaagagagt 3960
   ggacgccgac agcagcggac tccactggtt gtctctaaca cccccgaaaa ttaaacgggg 4020
   ctccacgcca atggggccca taaacaaaga caagtggcca ctcttttttt tgaaattgtg 4080
   gagtgggggc acgcgtcagc ccccacacgc cgccctgcgg ttttggactg taaaataagg 4140
   gtgtaataac ttggctgatt gtaaccccgc taaccactgc ggtcaaacca cttgcccaca 4200
   aaaccactaa tggcaccccg gggaatacct gcataagtag gtgggcgggc caagataggg 4260
   gcgcgattgc tgcgatctgg aggacaaatt acacacactt gcgcctgagc gccaagcaca 4320
   gggttgttgg tcctcatatt cacgaggtcg ctgagagcac ggtgggctaa tgttgccatg 4380
   ggtagcatat actacccaaa tatctggata gcatatgcta tcctaatcta tatctgggta 4440
   gcataggcta tcctaatcta tatctgggta gcatatgcta tcctaatcta tatctgggta 4500
   gtatatgcta tcctaattta tatctgggta gcataggcta tcctaatcta tatctgggta 4560
   gcatatgcta tcctaatcta tatctgggta gtatatgcta tcctaatctg tatccgggta 4620
   gcatatgcta tcctaataga gattagggta gtatatgcta tcctaattta tatctgggta 4680
   gcatatacta cccaaatatc tggatagcat atgctatcct aatctatatc tgggtagcat 4740
   atgctatcct aatctatatc tgggtagcat aggctatcct aatctatatc tgggtagcat 4800
   atgctatcct aatctatatc tgggtagtat atgctatcct aatttatatc tgggtagcat 4860
   aggctatcct aatctatatc tgggtagcat atgctatcct aatctatatc tgggtagtat 4920
   atgctatcct aatctgtatc cgggtagcat atgctatcct catgcatata cagtcagcat 4980
   atgataccca gtagtagagt gggagtgcta tcctttgcat atgccgccac ctcccaaggg 5040
   ggcgtgaatt ttcgctgctt gtccttttcc tgctggttgc tcccattctt aggtgaattt 5100
   aaggaggcca ggctaaagcc gtcgcatgtc tgattgctca ccaggtaaat gtcgctaatg 5160
   ttttccaacg cgagaaggtg ttgagcgcgg agctgagtga cgtgacaaca tgggtatgcc 5220
   caattgcccc atgttgggag gacgaaaatg gtgacaagac agatggccag aaatacacca 5280
   acagcacgca tgatgtctac tggggattta ttctttagtg cgggggaata cacggctttt 5340
   aatacgattg agggcgtctc ctaacaagtt acatcactcc tgcccttcct caccctcatc 5400
   tccatcacct ccttcatctc cgtcatctcc gtcatcaccc tccgcggcag ccccttccac 5460
   cataggtgga aaccagggag gcaaatctac tccatcgtca aagctgcaca cagtcaccct 5520
   gatattgcag gtaggagcgg gctttgtcat aacaaggtcc ttaatcgcat ccttcaaaac 5580
   ctcagcaaat atatgagttt gtaaaaagac catgaaataa cagacaatgg actcccttag 5640
   cgggccaggt tgtgggccgg gtccaggggc cattccaaag gggagacgac tcaatggtgt 5700
   aagacgacat tgtggaatag caagggcagt tcctcgcctt aggttgtaaa gggaggtctt 5760
   actacctcca tatacgaaca caccggcgac ccaagttcct tcgtcggtag tcctttctac 5820
   gtgactccta gccaggagag ctcttaaacc ttctgcaatg ttctcaaatt tcgggttgga 5880
   acctccttga ccacgatgct tttccaaacc accctccttt tttgcgccct gcctccatca 5940
   ccctgacccc ggggtccagt gcttgggcct tctcctgggt catctgcggg gccctgctct 6000
   atcgctcccg ggggcacgtc aggctcacca tctgggccac cttcttggtg gtattcaaaa 6060
   taatcggctt cccctacagg gtggaaaaat ggccttctac ctggaggggg cctgcgcggt 6120
   ggagacccgg atgatgatga ctgactactg ggactcctgg gcctcttttc tccacgtcca 6180
   cgacctctcc ccctggctct ttcacgactt ccccccctgg ctctttcacg tcctctaccc 6240
   cggcggcctc cactacctcc tcgaccccgg cctccactac ctcctcgacc ccggcctcca 6300
   ctgcctcctc gaccccggcc tccacctcct gctcctgccc ctcctgctcc tgcccctcct 6360
   cctgctcctg cccctcctgc ccctcctgct cctgcccctc ctgcccctcc tgctcctgcc 6420
   cctcctgccc ctcctgctcc tgcccctcct gcccctcctc ctgctcctgc ccctcctgcc 6480
   cctcctcctg ctcctgcccc tcctgcccct cctgctcctg cccctcctgc ccctcctgct 6540
   cctgcccctc ctgcccctcc tgctcctgcc cctcctgctc ctgcccctcc tgctcctgcc 6600
   cctcctgctc ctgcccctcc tgcccctcct gcccctcctc ctgctcctgc ccctcctgct 6660
   cctgcccctc ctgcccctcc tgcccctcct gctcctgccc ctcctcctgc tcctgcccct 6720
   cctgcccctc ctgcccctcc tcctgctcct gcccctcctg cccctcctcc tgctcctgcc 6780
   cctcctcctg ctcctgcccc tcctgcccct cctgcccctc ctcctgctcc tgcccctcct 6840
   gcccctcctc ctgctcctgc ccctcctcct gctcctgccc ctcctgcccc tcctgcccct 6900
   cctcctgctc ctgcccctcc tcctgctcct gcccctcctg cccctcctgc ccctcctgcc 6960
   cctcctcctg ctcctgcccc tcctcctgct cctgcccctc ctgctcctgc ccctcccgct 7020
   cctgctcctg ctcctgttcc accgtgggtc cctttgcagc caatgcaact tggacgtttt 7080
   tggggtctcc ggacaccatc tctatgtctt ggccctgatc ctgagccgcc cggggctcct 7140
   ggtcttccgc ctcctcgtcc tcgtcctctt ccccgtcctc gtccatggtt atcaccccct 7200
   cttctttgag gtccactgcc gccggagcct tctggtccag atgtgtctcc cttctctcct 7260
   aggccatttc caggtcctgt acctggcccc tcgtcagaca tgattcacac taaaagagat 7320
   caatagacat ctttattaga cgacgctcag tgaatacagg gagtgcagac tcctgccccc 7380
   tccaacagcc cccccaccct catccccttc atggtcgctg tcagacagat ccaggtctga 7440
   aaattcccca tcctccgaac catcctcgtc ctcatcacca attactcgca gcccggaaaa 7500
   ctcccgctga acatcctcaa gatttgcgtc ctgagcctca agccaggcct caaattcctc 7560
   gtcccccttt ttgctggacg gtagggatgg ggattctcgg gacccctcct cttcctcttc 7620
   aaggtcacca gacagagatg ctactggggc aacggaagaa aagctgggtg cggcctgtga 7680
   ggatcagctt atcgatgata agctgtcaaa catgagaatt cttgaagacg aaagggcctc 7740
   gtgatacgcc tatttttata ggttaatgtc atgataataa tggtttctta gacgtcaggt 7800
   ggcacttttc ggggaaatgt gcgcggaacc cctatttgtt tatttttcta aatacattca 7860
   aatatgtatc cgctcatgag acaataaccc tgataaatgc ttcaataata ttgaaaaagg 7920
   aagagtatga gtattcaaca tttccgtgtc gcccttattc ccttttttgc ggcattttgc 7980
   cttcctgttt ttgctcaccc agaaacgctg gtgaaagtaa aagatgctga agatcagttg 8040
   ggtgcacgag tgggttacat cgaactggat ctcaacagcg gtaagatcct tgagagtttt 8100
   cgccccgaag aacgttttcc aatgatgagc acttttaaag ttctgctatg tggcgcggta 8160
   ttatcccgtg ttgacgccgg gcaagagcaa ctcggtcgcc gcatacacta ttctcagaat 8220
   gacttggttg agtactcacc agtcacagaa aagcatctta cggatggcat gacagtaaga 8280
   gaattatgca gtgctgccat aaccatgagt gataacactg cggccaactt acttctgaca 8340
   acgatcggag gaccgaagga gctaaccgct tttttgcaca acatggggga tcatgtaact 8400
   cgccttgatc gttgggaacc ggagctgaat gaagccatac caaacgacga gcgtgacacc 8460
   acgatgcctg cagcaatggc aacaacgttg cgcaaactat taactggcga actacttact 8520
   ctagcttccc ggcaacaatt aatagactgg atggaggcgg ataaagttgc aggaccactt 8580
   ctgcgctcgg cccttccggc tggctggttt attgctgata aatctggagc cggtgagcgt 8640
   gggtctcgcg gtatcattgc agcactgggg ccagatggta agccctcccg tatcgtagtt 8700
   atctacacga cggggagtca ggcaactatg gatgaacgaa atagacagat cgctgagata 8760
   ggtgcctcac tgattaagca ttggtaactg tcagaccaag tttactcata tatactttag 8820
   attgatttaa aacttcattt ttaatttaaa aggatctagg tgaagatcct ttttgataat 8880
   ctcatgacca aaatccctta acgtgagttt tcgttccact gagcgtcaga ccccgtagaa 8940
   aagatcaaag gatcttcttg agatcctttt tttctgcgcg taatctgctg cttgcaaaca 9000
   aaaaaaccac cgctaccagc ggtggtttgt ttgccggatc aagagctacc aactcttttt 9060
   ccgaaggtaa ctggcttcag cagagcgcag ataccaaata ctgtccttct agtgtagccg 9120
   tagttaggcc accacttcaa gaactctgta gcaccgccta catacctcgc tctgctaatc 9180
   ctgttaccag tggctgctgc cagtggcgat aagtcgtgtc ttaccgggtt ggactcaaga 9240
   cgatagttac cggataaggc gcagcggtcg ggctgaacgg ggggttcgtg cacacagccc 9300
   agcttggagc gaacgaccta caccgaactg agatacctac agcgtgagct atgagaaagc 9360
   gccacgcttc ccgaagggag aaaggcggac aggtatccgg taagcggcag ggtcggaaca 9420
   ggagagcgca cgagggagct tccaggggga aacgcctggt atctttatag tcctgtcggg 9480
   tttcgccacc tctgacttga gcgtcgattt ttgtgatgct cgtcaggggg gcggagccta 9540
   tggaaaaacg ccagcaacgc ggccttttta cggttcctgg ccttttgctg gccttgaagc 9600
   tgtccctgat ggtcgtcatc tacctgcctg gacagcatgg cctgcaacgc gggcatcccg 9660
   atgccgccgg aagcgagaag aatcataatg gggaaggcca tccagcctcg cgtcgcgaac 9720
   gccagcaaga cgtagcccag cgcgtcggcc ccgagatgcg ccgcgtgcgg ctgctggaga 9780
   tggcggacgc gatggatatg ttctgccaag ggttggtttg cgcattcaca gttctccgca 9840
   agaattgatt ggctccaatt cttggagtgg tgaatccgtt agcgaggtgc cgccctgctt 9900
   catccccgtg gcccgttgct cgcgtttgct ggcggtgtcc ccggaagaaa tatatttgca 9960
   tgtctttagt tctatgatga cacaaacccc gcccagcgtc ttgtcattgg cgaattcgaa 10020
   cacgcagatg cagtcggggc ggcgcggtcc gaggtccact tcgcatatta aggtgacgcg 10080
   tgtggcctcg aacaccgagc gaccctgcag cgacccgctt aacagcgtca acagcgtgcc 10140
   gcagatcccg gggggcaatg agatatgaaa aagcctgaac tcaccgcgac gtctgtcgag 10200
   aagtttctga tcgaaaagtt cgacagcgtc tccgacctga tgcagctctc ggagggcgaa 10260
   gaatctcgtg ctttcagctt cgatgtagga gggcgtggat atgtcctgcg ggtaaatagc 10320
   tgcgccgatg gtttctacaa agatcgttat gtttatcggc actttgcatc ggccgcgctc 10380
   ccgattccgg aagtgcttga cattggggaa ttcagcgaga gcctgaccta ttgcatctcc 10440
   cgccgtgcac agggtgtcac gttgcaagac ctgcctgaaa ccgaactgcc cgctgttctg 10500
   cagccggtcg cggaggccat ggatgcgatc gctgcggccg atcttagcca gacgagcggg 10560
   ttcggcccat tcggaccgca aggaatcggt caatacacta catggcgtga tttcatatgc 10620
   gcgattgctg atccccatgt gtatcactgg caaactgtga tggacgacac cgtcagtgcg 10680
   tccgtcgcgc aggctctcga tgagctgatg ctttgggccg aggactgccc cgaagtccgg 10740
   cacctcgtgc acgcggattt cggctccaac aatgtcctga cggacaatgg ccgcataaca 10800
   gcggtcattg actggagcga ggcgatgttc ggggattccc aatacgaggt cgccaacatc 10860
   ttcttctgga ggccgtggtt ggcttgtatg gagcagcaga cgcgctactt cgagcggagg 10920
   catccggagc ttgcaggatc gccgcggctc cgggcgtata tgctccgcat tggtcttgac 10980
   caactctatc agagcttggt tgacggcaat ttcgatgatg cagcttgggc gcagggtcga 11040
   tgcgacgcaa tcgtccgatc cggagccggg actgtcgggc gtacacaaat cgcccgcaga 11100
   agcgcggccg tctggaccga tggctgtgta gaagtactcg ccgatagtgg aaaccgacgc 11160
   cccagcactc gtccggatcg ggagatgggg gaggctaact gaaacacgga aggagacaat 11220
   accggaagga acccgcgcta tgacggcaat aaaaagacag aataaaacgc acgggtgttg 11280
   ggtcgtttgt tcataaacgc ggggttcggt cccagggctg gcactctgtc gataccccac 11340
   cgagacccca ttggggccaa tacgcccgcg tttcttcctt ttccccaccc caccccccaa 11400
   gttcgggtga aggcccaggg ctcgcagcca acgtcggggc ggcaggccct gccatagcca 11460
   ctggccccgt gggttaggga cggggtcccc catggggaat ggtttatggt tcgtgggggt 11520
   tattattttg ggcgttgcgt ggggtcaggt ccacgactgg actgagcaga cagacccatg 11580
   gtttttggat ggcctgggca tggaccgcat gtactggcgc gacacgaaca ccgggcgtct 11640
   gtggctgcca aacacccccg acccccaaaa accaccgcgc ggatttctgg cgtgccaagc 11700
   tagtcgacca attctcatgt ttgacagctt atcatcgcag atccgggcaa cgttgttgcc 11760
   attgctgcag gcgcagaact ggtaggtatg gaagatctat acattgaatc aatattggca 11820
   attagccata ttagtcattg gttatatagc ataaatcaat attggctatt ggccattgca 11880
   tacgttgtat ctatatcata atatgtacat ttatattggc tcatgtccaa tatgaccgcc 11940
   at                                                                11942
<210> 141
   <211> 765
   <212> DNA
   <213> Artificial Sequence
<220>
   <223> In vitro synthesized DNA encoding a melanocortin receptor binding alpha-MSH mimetibody without secretory signal (p3170).
<400> 141
   tcctactcca tggagcactt ccgctggggc aagccggtgg ggggcggagg aagcggcgga 60
   ggcgggagcg gcggcggagg ctccggtggc ggaggatcct gcccaccgtg cccagcacct 120
   gaactcctgg ggggaccgtc agtcttcctc ttccccccaa aacccaagga caccctcatg 180
   atctcccgga cccctgaggt cacatgcgtg gtggtggacg tgagccacga agaccctgag 240
   gtcaagttca actggtacgt ggacggcgtg gaggtgcata atgccaagac aaagccgcgg 300
   gaggagcagt acaacagcac gtaccgtgtg gtcagcgtcc tcaccgtcct gcaccaggac 360
   tggctgaatg gcaaggagta caagtgcaag gtctccaaca aagccctccc agcccccatc 420
   gagaaaacca tctccaaagc caaagggcag ccccgagaac cacaggtgta caccctgccc 480
   ccatcccggg atgagctgac caagaaccag gtcagcctga cctgcctggt caaaggcttc 540
   tatcccagcg acatcgccgt ggagtgggag agcaatgggc agccggagaa caactacaag 600
   accacgcctc ccgtgctgga ctccgacggc tccttcttcc tctacagcaa gctcaccgtg 660
   gacaagagca ggtggcagca ggggaacgtc ttctcatgct ccgtgatgca tgaggctctg 720
   cacaaccact acacgcagaa gagcctctcc ctgtctctgg gtaaa                765
<210> 142
   <211> 255
   <212> PRT
   <213> Artificial Sequence
<220>
   <223> Melanocortin receptor binding alpha-MSH mimetibody without secretory signal encoded by an in vitro synthesized DNA (p3170).
<400> 142
   Ser Tyr Ser Met Glu His Phe Arg Trp Gly Lys Pro Val Gly Gly Gly
    1               5                  10                  15
   Gly Ser Gly Gly Gly Gly Ser Gly Gly Gly Gly Ser Gly Gly Gly Gly
               20                  25                  30
   Ser Cys Pro Pro Cys Pro Ala Pro Glu Leu Leu Gly Gly Pro Ser Val
           35                  40                  45
   Phe Leu Phe Pro Pro Lys Pro Lys Asp Thr Leu Met Ile Ser Arg Thr
       50                  55                  60
   Pro Glu Val Thr Cys Val Val Val Asp Val Ser His Glu Asp Pro Glu
   65                  70                  75                  80
   Val Lys Phe Asn Trp Tyr Val Asp Gly Val Glu Val His Asn Ala Lys
                   85                  90                  95
   Thr Lys Pro Arg Glu Glu Gln Tyr Asn Ser Thr Tyr Arg Val Val Ser
               100                 105                 110
   Val Leu Thr Val Leu His Gln Asp Trp Leu Asn Gly Lys Glu Tyr Lys
           115                 120                 125
   Cys Lys Val Ser Asn Lys Ala Leu Pro Ala Pro Ile Glu Lys Thr Ile
       130                 135                 140
   Ser Lys Ala Lys Gly Gln Pro Arg Glu Pro Gln Val Tyr Thr Leu Pro
   145                 150                 155                 160
   Pro Ser Arg Asp Glu Leu Thr Lys Asn Gln Val Ser Leu Thr Cys Leu
                   165                 170                 175
   Val Lys Gly Phe Tyr Pro Ser Asp Ile Ala Val Glu Trp Glu Ser Asn
               180                 185                 190
   Gly Gln Pro Glu Asn Asn Tyr Lys Thr Thr Pro Pro Val Leu Asp Ser
           195                 200                 205
   Asp Gly Ser Phe Phe Leu Tyr Ser Lys Leu Thr Val Asp Lys Ser Arg
       210                 215                 220
   Trp Gln Gln Gly Asn Val Phe Ser Cys Ser Val Met His Glu Ala Leu
   225                 230                 235                 240
   His Asn His Tyr Thr Gln Lys Ser Leu Ser Leu Ser Leu Gly Lys
                   245                 250                 255
<210> 143
   <211> 822
   <212> DNA
   <213> Artificial Sequence
<220>
   <223> In vitro synthesized DNA encoding a melanocortin receptor binding alpha-MSH mimetibody with secretory signal (p3170).
<400> 143
   atggcttggg tgtggacctt gctattcctg atggcggccg cccaaagtat acaggcctcc 60
   tactccatgg agcacttccg ctggggcaag ccggtggggg gcggaggaag cggcggaggc 120
   gggagcggcg gcggaggctc cggtggcgga ggatcctgcc caccgtgccc agcacctgaa 180
   ctcctggggg gaccgtcagt cttcctcttc cccccaaaac ccaaggacac cctcatgatc 240
   tcccggaccc ctgaggtcac atgcgtggtg gtggacgtga gccacgaaga ccctgaggtc 300
   aagttcaact ggtacgtgga cggcgtggag gtgcataatg ccaagacaaa gccgcgggag 360
   gagcagtaca acagcacgta ccgtgtggtc agcgtcctca ccgtcctgca ccaggactgg 420
   ctgaatggca aggagtacaa gtgcaaggtc tccaacaaag ccctcccagc ccccatcgag 480
   aaaaccatct ccaaagccaa agggcagccc cgagaaccac aggtgtacac cctgccccca 540
   tcccgggatg agctgaccaa gaaccaggtc agcctgacct gcctggtcaa aggcttctat 600
   cccagcgaca tcgccgtgga gtgggagagc aatgggcagc cggagaacaa ctacaagacc 660
   acgcctcccg tgctggactc cgacggctcc ttcttcctct acagcaagct caccgtggac 720
   aagagcaggt ggcagcaggg gaacgtcttc tcatgctccg tgatgcatga ggctctgcac 780
   aaccactaca cgcagaagag cctctccctg tctctgggta aa                    822
<210> 144
   <211> 274
   <212> PRT
   <213> Artificial Sequence
<220>
   <223> Melanocortin receptor binding alpha-MSH mimetibody with secretory signal encoded by an in vitro synthesized DNA (p3170).
<400> 144
   Met Ala Trp Val Trp Thr Leu Leu Phe Leu Met Ala Ala Ala Gln Ser
    1               5                  10                  15
   Ile Gln Ala Ser Tyr Ser Met Glu His Phe Arg Trp Gly Lys Pro Val
               20                  25                  30
   Gly Gly Gly Gly Ser Gly Gly Gly Gly Ser Gly Gly Gly Gly Ser Gly
           35                  40                  45
   Gly Gly Gly Ser Cys Pro Pro Cys Pro Ala Pro Glu Leu Leu Gly Gly
       50                  55                  60
   Pro Ser Val Phe Leu Phe Pro Pro Lys Pro Lys Asp Thr Leu Met Ile
   65                  70                  75                  80
   Ser Arg Thr Pro Glu Val Thr Cys Val Val Val Asp Val Ser His Glu
                   85                  90                  95
   Asp Pro Glu Val Lys Phe Asn Trp Tyr Val Asp Gly Val Glu Val His
               100                 105                 110
   Asn Ala Lys Thr Lys Pro Arg Glu Glu Gln Tyr Asn Ser Thr Tyr Arg
           115                 120                 125
   Val Val Ser Val Leu Thr Val Leu His Gln Asp Trp Leu Asn Gly Lys
       130                 135                 140
   Glu Tyr Lys Cys Lys Val Ser Asn Lys Ala Leu Pro Ala Pro Ile Glu
   145                 150                 155                 160
   Lys Thr Ile Ser Lys Ala Lys Gly Gln Pro Arg Glu Pro Gln Val Tyr
                   165                 170                 175
   Thr Leu Pro Pro Ser Arg Asp Glu Leu Thr Lys Asn Gln Val Ser Leu
               180                 185                 190
   Thr Cys Leu Val Lys Gly Phe Tyr Pro Ser Asp Ile Ala Val Glu Trp
           195                 200                 205
   Glu Ser Asn Gly Gln Pro Glu Asn Asn Tyr Lys Thr Thr Pro Pro Val
       210                 215                 220
   Leu Asp Ser Asp Gly Ser Phe Phe Leu Tyr Ser Lys Leu Thr Val Asp
   225                 230                 235                 240
   Lys Ser Arg Trp Gln Gln Gly Asn Val Phe Ser Cys Ser Val Met His
                   245                 250                 255
   Glu Ala Leu His Asn His Tyr Thr Gln Lys Ser Leu Ser Leu Ser Leu
               260                 265                 270
   Gly Lys
<210> 145
   <211> 11957
   <212> DNA
   <213> Artificial Sequence
<220>
   <223> In vitro synthesized DNA with expression vector functions that encodes an alpha-MSH mimetibody (p3170).
<400> 145
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactccatgg agcacttccg 1680
   ctggggcaag ccggtggggg gcggaggaag cggcggaggc gggagcggcg gcggaggctc 1740
   cggtggcgga ggatcctgcc caccgtgccc agcacctgaa ctcctggggg gaccgtcagt 1800
   cttcctcttc cccccaaaac ccaaggacac cctcatgatc tcccggaccc ctgaggtcac 1860
   atgcgtggtg gtggacgtga gccacgaaga ccctgaggtc aagttcaact ggtacgtgga 1920
   cggcgtggag gtgcataatg ccaagacaaa gccgcgggag gagcagtaca acagcacgta 1980
   ccgtgtggtc agcgtcctca ccgtcctgca ccaggactgg ctgaatggca aggagtacaa 2040
   gtgcaaggtc tccaacaaag ccctcccagc ccccatcgag aaaaccatct ccaaagccaa 2100
   agggcagccc cgagaaccac aggtgtacac cctgccccca tcccgggatg agctgaccaa 2160
   gaaccaggtc agcctgacct gcctggtcaa aggcttctat cccagcgaca tcgccgtgga 2220
   gtgggagagc aatgggcagc cggagaacaa ctacaagacc acgcctcccg tgctggactc 2280
   cgacggctcc ttcttcctct acagcaagct caccgtggac aagagcaggt ggcagcaggg 2340
   gaacgtcttc tcatgctccg tgatgcatga ggctctgcac aaccactaca cgcagaagag 2400
   cctctccctg tctctgggta aatgagggcc cgtttaaaca gatccagaca tgataagata 2460
   cattgatgag tttggacaaa ccacaactag aatgcagtga aaaaaatgct ttatttgtga 2520
   aatttgtgat gctattgctt tatttgtaac cattataagc tgcaataaac aagttaacaa 2580
   caacaattgc attcatttta tgtttcaggt tcagggggag gtgtgggagg ttttttaaag 2640
   caagtaaaac ctctacaaat gtggtatggc tgattatgat ccggctgcct cgcgcgtttc 2700
   ggtgatgacg gtgaaaacct ctgacacatg cagctcccgg agacggtcac agcttgtctg 2760
   taagcggatg ccgggagcag acaagcccgt caggcgtcag cgggtgttgg cgggtgtcgg 2820
   ggcgcagcca tgaggtcgac tctagaggat cgatgccccg ccccggacga actaaacctg 2880
   actacgacat ctctgcccct tcttcgcggg gcagtgcatg taatcccttc agttggttgg 2940
   tacaacttgc caactgggcc ctgttccaca tgtgacacgg ggggggacca aacacaaagg 3000
   ggttctctga ctgtagttga catccttata aatggatgtg cacatttgcc aacactgagt 3060
   ggctttcatc ctggagcaga ctttgcagtc tgtggactgc aacacaacat tgcctttatg 3120
   tgtaactctt ggctgaagct cttacaccaa tgctggggga catgtacctc ccaggggccc 3180
   aggaagacta cgggaggcta caccaacgtc aatcagaggg gcctgtgtag ctaccgataa 3240
   gcggaccctc aagagggcat tagcaatagt gtttataagg cccccttgtt aaccctaaac 3300
   gggtagcata tgcttcccgg gtagtagtat atactatcca gactaaccct aattcaatag 3360
   catatgttac ccaacgggaa gcatatgcta tcgaattagg gttagtaaaa gggtcctaag 3420
   gaacagcgat atctcccacc ccatgagctg tcacggtttt atttacatgg ggtcaggatt 3480
   ccacgagggt agtgaaccat tttagtcaca agggcagtgg ctgaagatca aggagcgggc 3540
   agtgaactct cctgaatctt cgcctgcttc ttcattctcc ttcgtttagc taatagaata 3600
   actgctgagt tgtgaacagt aaggtgtatg tgaggtgctc gaaaacaagg tttcaggtga 3660
   cgcccccaga ataaaatttg gacggggggt tcagtggtgg cattgtgcta tgacaccaat 3720
   ataaccctca caaacccctt gggcaataaa tactagtgta ggaatgaaac attctgaata 3780
   tctttaacaa tagaaatcca tggggtgggg acaagccgta aagactggat gtccatctca 3840
   cacgaattta tggctatggg caacacataa tcctagtgca atatgatact ggggttatta 3900
   agatgtgtcc caggcaggga ccaagacagg tgaaccatgt tgttacactc tatttgtaac 3960
   aaggggaaag agagtggacg ccgacagcag cggactccac tggttgtctc taacaccccc 4020
   gaaaattaaa cggggctcca cgccaatggg gcccataaac aaagacaagt ggccactctt 4080
   ttttttgaaa ttgtggagtg ggggcacgcg tcagccccca cacgccgccc tgcggttttg 4140
   gactgtaaaa taagggtgta ataacttggc tgattgtaac cccgctaacc actgcggtca 4200
   aaccacttgc ccacaaaacc actaatggca ccccggggaa tacctgcata agtaggtggg 4260
   cgggccaaga taggggcgcg attgctgcga tctggaggac aaattacaca cacttgcgcc 4320
   tgagcgccaa gcacagggtt gttggtcctc atattcacga ggtcgctgag agcacggtgg 4380
   gctaatgttg ccatgggtag catatactac ccaaatatct ggatagcata tgctatccta 4440
   atctatatct gggtagcata ggctatccta atctatatct gggtagcata tgctatccta 4500
   atctatatct gggtagtata tgctatccta atttatatct gggtagcata ggctatccta 4560
   atctatatct gggtagcata tgctatccta atctatatct gggtagtata tgctatccta 4620
   atctgtatcc gggtagcata tgctatccta atagagatta gggtagtata tgctatccta 4680
   atttatatct gggtagcata tactacccaa atatctggat agcatatgct atcctaatct 4740
   atatctgggt agcatatgct atcctaatct atatctgggt agcataggct atcctaatct 4800
   atatctgggt agcatatgct atcctaatct atatctgggt agtatatgct atcctaattt 4860
   atatctgggt agcataggct atcctaatct atatctgggt agcatatgct atcctaatct 4920
   atatctgggt agtatatgct atcctaatct gtatccgggt agcatatgct atcctcatgc 4980
   atatacagtc agcatatgat acccagtagt agagtgggag tgctatcctt tgcatatgcc 5040
   gccacctccc aagggggcgt gaattttcgc tgcttgtcct tttcctgctg gttgctccca 5100
   ttcttaggtg aatttaagga ggccaggcta aagccgtcgc atgtctgatt gctcaccagg 5160
   taaatgtcgc taatgttttc caacgcgaga aggtgttgag cgcggagctg agtgacgtga 5220
   caacatgggt atgcccaatt gccccatgtt gggaggacga aaatggtgac aagacagatg 5280
   gccagaaata caccaacagc acgcatgatg tctactgggg atttattctt tagtgcgggg 5340
   gaatacacgg cttttaatac gattgagggc gtctcctaac aagttacatc actcctgccc 5400
   ttcctcaccc tcatctccat cacctccttc atctccgtca tctccgtcat caccctccgc 5460
   ggcagcccct tccaccatag gtggaaacca gggaggcaaa tctactccat cgtcaaagct 5520
   gcacacagtc accctgatat tgcaggtagg agcgggcttt gtcataacaa ggtccttaat 5580
   cgcatccttc aaaacctcag caaatatatg agtttgtaaa aagaccatga aataacagac 5640
   aatggactcc cttagcgggc caggttgtgg gccgggtcca ggggccattc caaaggggag 5700
   acgactcaat ggtgtaagac gacattgtgg aatagcaagg gcagttcctc gccttaggtt 5760
   gtaaagggag gtcttactac ctccatatac gaacacaccg gcgacccaag ttccttcgtc 5820
   ggtagtcctt tctacgtgac tcctagccag gagagctctt aaaccttctg caatgttctc 5880
   aaatttcggg ttggaacctc cttgaccacg atgcttttcc aaaccaccct ccttttttgc 5940
   gccctgcctc catcaccctg accccggggt ccagtgcttg ggccttctcc tgggtcatct 6000
   gcggggccct gctctatcgc tcccgggggc acgtcaggct caccatctgg gccaccttct 6060
   tggtggtatt caaaataatc ggcttcccct acagggtgga aaaatggcct tctacctgga 6120
   gggggcctgc gcggtggaga cccggatgat gatgactgac tactgggact cctgggcctc 6180
   ttttctccac gtccacgacc tctccccctg gctctttcac gacttccccc cctggctctt 6240
   tcacgtcctc taccccggcg gcctccacta cctcctcgac cccggcctcc actacctcct 6300
   cgaccccggc ctccactgcc tcctcgaccc cggcctccac ctcctgctcc tgcccctcct 6360
   gctcctgccc ctcctcctgc tcctgcccct cctgcccctc ctgctcctgc ccctcctgcc 6420
   cctcctgctc ctgcccctcc tgcccctcct gctcctgccc ctcctgcccc tcctcctgct 6480
   cctgcccctc ctgcccctcc tcctgctcct gcccctcctg cccctcctgc tcctgcccct 6540
   cctgcccctc ctgctcctgc ccctcctgcc cctcctgctc ctgcccctcc tgctcctgcc 6600
   cctcctgctc ctgcccctcc tgctcctgcc cctcctgccc ctcctgcccc tcctcctgct 6660
   cctgcccctc ctgctcctgc ccctcctgcc cctcctgccc ctcctgctcc tgcccctcct 6720
   cctgctcctg cccctcctgc ccctcctgcc cctcctcctg ctcctgcccc tcctgcccct 6780
   cctcctgctc ctgcccctcc tcctgctcct gcccctcctg cccctcctgc ccctcctcct 6840
   gctcctgccc ctcctgcccc tcctcctgct cctgcccctc ctcctgctcc tgcccctcct 6900
   gcccctcctg cccctcctcc tgctcctgcc cctcctcctg ctcctgcccc tcctgcccct 6960
   cctgcccctc ctgcccctcc tcctgctcct gcccctcctc ctgctcctgc ccctcctgct 7020
   cctgcccctc ccgctcctgc tcctgctcct gttccaccgt gggtcccttt gcagccaatg 7080
   caacttggac gtttttgggg tctccggaca ccatctctat gtcttggccc tgatcctgag 7140
   ccgcccgggg ctcctggtct tccgcctcct cgtcctcgtc ctcttccccg tcctcgtcca 7200
   tggttatcac cccctcttct ttgaggtcca ctgccgccgg agccttctgg tccagatgtg 7260
   tctcccttct ctcctaggcc atttccaggt cctgtacctg gcccctcgtc agacatgatt 7320
   cacactaaaa gagatcaata gacatcttta ttagacgacg ctcagtgaat acagggagtg 7380
   cagactcctg ccccctccaa cagccccccc accctcatcc ccttcatggt cgctgtcaga 7440
   cagatccagg tctgaaaatt ccccatcctc cgaaccatcc tcgtcctcat caccaattac 7500
   tcgcagcccg gaaaactccc gctgaacatc ctcaagattt gcgtcctgag cctcaagcca 7560
   ggcctcaaat tcctcgtccc cctttttgct ggacggtagg gatggggatt ctcgggaccc 7620
   ctcctcttcc tcttcaaggt caccagacag agatgctact ggggcaacgg aagaaaagct 7680
   gggtgcggcc tgtgaggatc agcttatcga tgataagctg tcaaacatga gaattcttga 7740
   agacgaaagg gcctcgtgat acgcctattt ttataggtta atgtcatgat aataatggtt 7800
   tcttagacgt caggtggcac ttttcgggga aatgtgcgcg gaacccctat ttgtttattt 7860
   ttctaaatac attcaaatat gtatccgctc atgagacaat aaccctgata aatgcttcaa 7920
   taatattgaa aaaggaagag tatgagtatt caacatttcc gtgtcgccct tattcccttt 7980
   tttgcggcat tttgccttcc tgtttttgct cacccagaaa cgctggtgaa agtaaaagat 8040
   gctgaagatc agttgggtgc acgagtgggt tacatcgaac tggatctcaa cagcggtaag 8100
   atccttgaga gttttcgccc cgaagaacgt tttccaatga tgagcacttt taaagttctg 8160
   ctatgtggcg cggtattatc ccgtgttgac gccgggcaag agcaactcgg tcgccgcata 8220
   cactattctc agaatgactt ggttgagtac tcaccagtca cagaaaagca tcttacggat 8280
   ggcatgacag taagagaatt atgcagtgct gccataacca tgagtgataa cactgcggcc 8340
   aacttacttc tgacaacgat cggaggaccg aaggagctaa ccgctttttt gcacaacatg 8400
   ggggatcatg taactcgcct tgatcgttgg gaaccggagc tgaatgaagc cataccaaac 8460
   gacgagcgtg acaccacgat gcctgcagca atggcaacaa cgttgcgcaa actattaact 8520
   ggcgaactac ttactctagc ttcccggcaa caattaatag actggatgga ggcggataaa 8580
   gttgcaggac cacttctgcg ctcggccctt ccggctggct ggtttattgc tgataaatct 8640
   ggagccggtg agcgtgggtc tcgcggtatc attgcagcac tggggccaga tggtaagccc 8700
   tcccgtatcg tagttatcta cacgacgggg agtcaggcaa ctatggatga acgaaataga 8760
   cagatcgctg agataggtgc ctcactgatt aagcattggt aactgtcaga ccaagtttac 8820
   tcatatatac tttagattga tttaaaactt catttttaat ttaaaaggat ctaggtgaag 8880
   atcctttttg ataatctcat gaccaaaatc ccttaacgtg agttttcgtt ccactgagcg 8940
   tcagaccccg tagaaaagat caaaggatct tcttgagatc ctttttttct gcgcgtaatc 9000
   tgctgcttgc aaacaaaaaa accaccgcta ccagcggtgg tttgtttgcc ggatcaagag 9060
   ctaccaactc tttttccgaa ggtaactggc ttcagcagag cgcagatacc aaatactgtc 9120
   cttctagtgt agccgtagtt aggccaccac ttcaagaact ctgtagcacc gcctacatac 9180
   ctcgctctgc taatcctgtt accagtggct gctgccagtg gcgataagtc gtgtcttacc 9240
   gggttggact caagacgata gttaccggat aaggcgcagc ggtcgggctg aacggggggt 9300
   tcgtgcacac agcccagctt ggagcgaacg acctacaccg aactgagata cctacagcgt 9360
   gagctatgag aaagcgccac gcttcccgaa gggagaaagg cggacaggta tccggtaagc 9420
   ggcagggtcg gaacaggaga gcgcacgagg gagcttccag ggggaaacgc ctggtatctt 9480
   tatagtcctg tcgggtttcg ccacctctga cttgagcgtc gatttttgtg atgctcgtca 9540
   ggggggcgga gcctatggaa aaacgccagc aacgcggcct ttttacggtt cctggccttt 9600
   tgctggcctt gaagctgtcc ctgatggtcg tcatctacct gcctggacag catggcctgc 9660
   aacgcgggca tcccgatgcc gccggaagcg agaagaatca taatggggaa ggccatccag 9720
   cctcgcgtcg cgaacgccag caagacgtag cccagcgcgt cggccccgag atgcgccgcg 9780
   tgcggctgct ggagatggcg gacgcgatgg atatgttctg ccaagggttg gtttgcgcat 9840
   tcacagttct ccgcaagaat tgattggctc caattcttgg agtggtgaat ccgttagcga 9900
   ggtgccgccc tgcttcatcc ccgtggcccg ttgctcgcgt ttgctggcgg tgtccccgga 9960
   agaaatatat ttgcatgtct ttagttctat gatgacacaa accccgccca gcgtcttgtc 10020
   attggcgaat tcgaacacgc agatgcagtc ggggcggcgc ggtccgaggt ccacttcgca 10080
   tattaaggtg acgcgtgtgg cctcgaacac cgagcgaccc tgcagcgacc cgcttaacag 10140
   cgtcaacagc gtgccgcaga tcccgggggg caatgagata tgaaaaagcc tgaactcacc 10200
   gcgacgtctg tcgagaagtt tctgatcgaa aagttcgaca gcgtctccga cctgatgcag 10260
   ctctcggagg gcgaagaatc tcgtgctttc agcttcgatg taggagggcg tggatatgtc 10320
   ctgcgggtaa atagctgcgc cgatggtttc tacaaagatc gttatgttta tcggcacttt 10380
   gcatcggccg cgctcccgat tccggaagtg cttgacattg gggaattcag cgagagcctg 10440
   acctattgca tctcccgccg tgcacagggt gtcacgttgc aagacctgcc tgaaaccgaa 10500
   ctgcccgctg ttctgcagcc ggtcgcggag gccatggatg cgatcgctgc ggccgatctt 10560
   agccagacga gcgggttcgg cccattcgga ccgcaaggaa tcggtcaata cactacatgg 10620
   cgtgatttca tatgcgcgat tgctgatccc catgtgtatc actggcaaac tgtgatggac 10680
   gacaccgtca gtgcgtccgt cgcgcaggct ctcgatgagc tgatgctttg ggccgaggac 10740
   tgccccgaag tccggcacct cgtgcacgcg gatttcggct ccaacaatgt cctgacggac 10800
   aatggccgca taacagcggt cattgactgg agcgaggcga tgttcgggga ttcccaatac 10860
   gaggtcgcca acatcttctt ctggaggccg tggttggctt gtatggagca gcagacgcgc 10920
   tacttcgagc ggaggcatcc ggagcttgca ggatcgccgc ggctccgggc gtatatgctc 10980
   cgcattggtc ttgaccaact ctatcagagc ttggttgacg gcaatttcga tgatgcagct 11040
   tgggcgcagg gtcgatgcga cgcaatcgtc cgatccggag ccgggactgt cgggcgtaca 11100
   caaatcgccc gcagaagcgc ggccgtctgg accgatggct gtgtagaagt actcgccgat 11160
   agtggaaacc gacgccccag cactcgtccg gatcgggaga tgggggaggc taactgaaac 11220
   acggaaggag acaataccgg aaggaacccg cgctatgacg gcaataaaaa gacagaataa 11280
   aacgcacggg tgttgggtcg tttgttcata aacgcggggt tcggtcccag ggctggcact 11340
   ctgtcgatac cccaccgaga ccccattggg gccaatacgc ccgcgtttct tccttttccc 11400
   caccccaccc cccaagttcg ggtgaaggcc cagggctcgc agccaacgtc ggggcggcag 11460
   gccctgccat agccactggc cccgtgggtt agggacgggg tcccccatgg ggaatggttt 11520
   atggttcgtg ggggttatta ttttgggcgt tgcgtggggt caggtccacg actggactga 11580
   gcagacagac ccatggtttt tggatggcct gggcatggac cgcatgtact ggcgcgacac 11640
   gaacaccggg cgtctgtggc tgccaaacac ccccgacccc caaaaaccac cgcgcggatt 11700
   tctggcgtgc caagctagtc gaccaattct catgtttgac agcttatcat cgcagatccg 11760
   ggcaacgttg ttgccattgc tgcaggcgca gaactggtag gtatggaaga tctatacatt 11820
   gaatcaatat tggcaattag ccatattagt cattggttat atagcataaa tcaatattgg 11880
   ctattggcca ttgcatacgt tgtatctata tcataatatg tacatttata ttggctcatg 11940
   tccaatatga ccgccat                                               11957
<210> 146
   <211> 777
   <212> DNA
   <213> Artificial Sequence
<220>
   <223> In vitro synthesized DNA encoding a melanocortin receptor binding alpha-MSH mimetibody without secretory signal (p3159).
<400> 146
   tcctactcct gcgagcactt ccgctggtgc aagccggtgg gatccggtgg aggctccggt 60
   accttagtcg ccgtctcctc agagcccaaa tctgccgaca aaactcacgc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 147
   <211> 259
   <212> PRT
   <213> Artificial Sequence
<220>
   <223> Melanocortin receptor binding alpha-MSH mimetibody without secretory signal encoded by an in vitro synthesized DNA (p3159).
<400> 147
   Ser Tyr Ser Cys Glu His Phe Arg Trp Cys Lys Pro Val Gly Ser Gly
    1               5                  10                  15
   Gly Gly Ser Gly Thr Leu Val Ala Val Ser Ser Glu Pro Lys Ser Ala
               20                  25                  30
   Asp Lys Thr His Ala Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 148
   <211> 834
   <212> DNA
   <213> Artificial Sequence
<220>
   <223> In vitro synthesized DNA encoding a melanocortin receptor binding alpha-MSH mimetibody with secretory signal (p3159).
<400> 148
   atggcttggg tgtggacctt gctattcctg atggcggccg cccaaagtat acaggcctcc 60
   tactcctgcg agcacttccg ctggtgcaag ccggtgggat ccggtggagg ctccggtacc 120
   ttagtcgccg tctcctcaga gcccaaatct gccgacaaaa ctcacgcctg cccaccgtgc 180
   ccagcacctg aactcctggg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 149
   <211> 278
   <212> PRT
   <213> Artificial Sequence
<220>
   <223> Melanocortin receptor binding alpha-MSH mimetibody with secretory signal encoded by an in vitro synthesized DNA (p3159).
<400> 149
   Met Ala Trp Val Trp Thr Leu Leu Phe Leu Met Ala Ala Ala Gln Ser
    1               5                  10                  15
   Ile Gln Ala Ser Tyr Ser Cys Glu His Phe Arg Trp Cys Lys Pro Val
               20                  25                  30
   Gly Ser Gly Gly Gly Ser Gly Thr Leu Val Ala Val Ser Ser Glu Pro
           35                  40                  45
   Lys Ser Ala Asp Lys Thr His Ala Cys Pro Pro Cys Pro Ala Pro Glu
       50                  55                  60
   Leu Leu Gly Gly Pro Ser Val Phe Leu Phe Pro Pro Lys Pro Lys Asp
   65                  70                  75                  80
   Thr Leu Met Ile Ser Arg Thr Pro Glu Val Thr Cys Val Val Val Asp
                   85                  90                  95
   Val Ser His Glu Asp Pro Glu Val Lys Phe Asn Trp Tyr Val Asp Gly
               100                 105                 110
   Val Glu Val His Asn Ala Lys Thr Lys Pro Arg Glu Glu Gln Tyr Asn
           115                 120                 125
   Ser Thr Tyr Arg Val Val Ser Val Leu Thr Val Leu His Gln Asp Trp
       130                 135                 140
   Leu Asn Gly Lys Glu Tyr Lys Cys Lys Val Ser Asn Lys Ala Leu Pro
   145                 150                 155                 160
   Ala Pro Ile Glu Lys Thr Ile Ser Lys Ala Lys Gly Gln Pro Arg Glu
                   165                 170                 175
   Pro Gln Val Tyr Thr Leu Pro Pro Ser Arg Asp Glu Leu Thr Lys Asn
               180                 185                 190
   Gln Val Ser Leu Thr Cys Leu Val Lys Gly Phe Tyr Pro Ser Asp Ile
           195                 200                 205
   Ala Val Glu Trp Glu Ser Asn Gly Gln Pro Glu Asn Asn Tyr Lys Thr
       210                 215                 220
   Thr Pro Pro Val Leu Asp Ser Asp Gly Ser Phe Phe Leu Tyr Ser Lys
   225                 230                 235                 240
   Leu Thr Val Asp Lys Ser Arg Trp Gln Gln Gly Asn Val Phe Ser Cys
                   245                 250                 255
   Ser Val Met His Glu Ala Leu His Asn His Tyr Thr Gln Lys Ser Leu
               260                 265                 270
   Ser Leu Ser Pro Gly Lys
           275
<210> 150
   <211> 11969
   <212> DNA
   <213> Artificial Sequence
<220>
   <223> In vitro synthesized DNA with expression vector functions that encodes an alpha-MSH mimetibody (p3159).
<400> 150
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactcctgcg agcacttccg 1680
   ctggtgcaag ccggtgggat ccggtggagg ctccggtacc ttagtcgccg tctcctcaga 1740
   gcccaaatct gccgacaaaa ctcacgcctg cccaccgtgc ccagcacctg aactcctggg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaagggggc gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gagggggcct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat                                   11969
<210> 151
   <211> 768
   <212> DNA
   <213> Artificial Sequence
<220>
   <223> In vitro synthesized DNA encoding a melanocortin receptor binding alpha-MSH mimetibody without secretory signal (p3652).
<400> 151
   tcctactcct gcgagcactt ccgctggtgc aagccggtgg gatccggtgg aggctccggt 60
   accttagtca ccgtctcctc agagtccaaa tatggtcccc catgcccacc atgcccggcg 120
   cctgaggccg ccgggggacc atcagtcttc ctgttccccc caaaacccaa ggacactctc 180
   atgatctccc ggacccctga ggtcacgtgc gtggtggtgg acgtgagcca ggaagacccc 240
   gaggtccagt tcaactggta cgtggatggc gtggaggtgc ataatgccaa gacaaagccg 300
   cgggaggagc agttcaacag cacgtaccgt gtggtcagcg tcctcaccgt cctgcaccag 360
   gactggctga acggcaagga gtacaagtgc aaggtctcca acaaaggcct cccgtcctcc 420
   atcgagaaaa ccatctccaa agccaaaggg cagcctcgag agccacaggt gtacaccctg 480
   cccccatccc aggaggagat gaccaagaac caggtcagcc tgacctgcct ggtcaaaggc 540
   ttctacccca gcgacatcgc cgtggagtgg gagagcaatg ggcagccgga gaacaactac 600
   aagaccacgc ctcccgtgct ggactccgac ggctccttct tcctctacag caggctaacc 660
   gtggacaaga gcaggtggca ggaggggaat gtcttctcat gctccgtgat gcatgaggct 720
   ctgcacaacc actacacaca gaaaagcttg tccctgtctc tgggtaaa              768
<210> 152
   <211> 256
   <212> PRT
   <213> Artificial Sequence
<220>
   <223> Melanocortin receptor binding alpha-MSH mimetibody without secretory signal encoded by an in vitro synthesized DNA (p3652).
<400> 152
   Ser Tyr Ser Cys Glu His Phe Arg Trp Cys Lys Pro Val Gly Ser Gly
    1               5                  10                  15
   Gly Gly Ser Gly Thr Leu Val Thr Val Ser Ser Glu Ser Lys Tyr Gly
               20                  25                  30
   Pro Pro Cys Pro Pro Cys Pro Ala Pro Glu Ala Ala Gly Gly Pro Ser
           35                  40                  45
   Val Phe Leu Phe Pro Pro Lys Pro Lys Asp Thr Leu Met Ile Ser Arg
       50                  55                  60
   Thr Pro Glu Val Thr Cys Val Val Val Asp Val Ser Gln Glu Asp Pro
   65                  70                  75                  80
   Glu Val Gln Phe Asn Trp Tyr Val Asp Gly Val Glu Val His Asn Ala
                   85                  90                  95
   Lys Thr Lys Pro Arg Glu Glu Gln Phe Asn Ser Thr Tyr Arg Val Val
               100                 105                 110
   Ser Val Leu Thr Val Leu His Gln Asp Trp Leu Asn Gly Lys Glu Tyr
           115                 120                 125
   Lys Cys Lys Val Ser Asn Lys Gly Leu Pro Ser Ser Ile Glu Lys Thr
       130                 135                 140
   Ile Ser Lys Ala Lys Gly Gln Pro Arg Glu Pro Gln Val Tyr Thr Leu
   145                 150                 155                 160
   Pro Pro Ser Gln Glu Glu Met Thr Lys Asn Gln Val Ser Leu Thr Cys
                   165                 170                 175
   Leu Val Lys Gly Phe Tyr Pro Ser Asp Ile Ala Val Glu Trp Glu Ser
               180                 185                 190
   Asn Gly Gln Pro Glu Asn Asn Tyr Lys Thr Thr Pro Pro Val Leu Asp
           195                 200                 205
   Ser Asp Gly Ser Phe Phe Leu Tyr Ser Arg Leu Thr Val Asp Lys Ser
       210                 215                 220
   Arg Trp Gln Glu Gly Asn Val Phe Ser Cys Ser Val Met His Glu Ala
   225                 230                 235                 240
   Leu His Asn His Tyr Thr Gln Lys Ser Leu Ser Leu Ser Leu Gly Lys
                   245                 250                 255
<210> 153
   <211> 825
   <212> DNA
   <213> Artificial Sequence
<220>
   <223> In vitro synthesized DNA encoding a melanocortin receptor binding alpha-MSH mimetibody with secretory signal (p3652).
<400> 153
   atggcttggg tgtggacctt gctattcctg atggcggccg cccaaagtat acaggcctcc 60
   tactcctgcg agcacttccg ctggtgcaag ccggtgggat ccggtggagg ctccggtacc 120
   ttagtcaccg tctcctcaga gtccaaatat ggtcccccat gcccaccatg cccggcgcct 180
   gaggccgccg ggggaccatc agtcttcctg ttccccccaa aacccaagga cactctcatg 240
   atctcccgga cccctgaggt cacgtgcgtg gtggtggacg tgagccagga agaccccgag 300
   gtccagttca actggtacgt ggatggcgtg gaggtgcata atgccaagac aaagccgcgg 360
   gaggagcagt tcaacagcac gtaccgtgtg gtcagcgtcc tcaccgtcct gcaccaggac 420
   tggctgaacg gcaaggagta caagtgcaag gtctccaaca aaggcctccc gtcctccatc 480
   gagaaaacca tctccaaagc caaagggcag cctcgagagc cacaggtgta caccctgccc 540
   ccatcccagg aggagatgac caagaaccag gtcagcctga cctgcctggt caaaggcttc 600
   taccccagcg acatcgccgt ggagtgggag agcaatgggc agccggagaa caactacaag 660
   accacgcctc ccgtgctgga ctccgacggc tccttcttcc tctacagcag gctaaccgtg 720
   gacaagagca ggtggcagga ggggaatgtc ttctcatgct ccgtgatgca tgaggctctg 780
   cacaaccact acacacagaa aagcttgtcc ctgtctctgg gtaaa                825
<210> 154
   <211> 275
   <212> PRT
   <213> Artificial Sequence
<220>
   <223> Melanocortin receptor binding alpha-MSH mimetibody with secretory signal encoded by an in vitro synthesized DNA (p3652).
<400> 154
   Met Ala Trp Val Trp Thr Leu Leu Phe Leu Met Ala Ala Ala Gln Ser
    1               5                  10                  15
   Ile Gln Ala Ser Tyr Ser Cys Glu His Phe Arg Trp Cys Lys Pro Val
               20                  25                  30
   Gly Ser Gly Gly Gly Ser Gly Thr Leu Val Thr Val Ser Ser Glu Ser
           35                  40                  45
   Lys Tyr Gly Pro Pro Cys Pro Pro Cys Pro Ala Pro Glu Ala Ala Gly
       50                  55                  60
   Gly Pro Ser Val Phe Leu Phe Pro Pro Lys Pro Lys Asp Thr Leu Met
   65                  70                  75                  80
   Ile Ser Arg Thr Pro Glu Val Thr Cys Val Val Val Asp Val Ser Gln
                   85                  90                  95
   Glu Asp Pro Glu Val Gln Phe Asn Trp Tyr Val Asp Gly Val Glu Val
               100                 105                 110
   His Asn Ala Lys Thr Lys Pro Arg Glu Glu Gln Phe Asn Ser Thr Tyr
           115                 120                 125
   Arg Val Val Ser Val Leu Thr Val Leu His Gln Asp Trp Leu Asn Gly
       130                 135                 140
   Lys Glu Tyr Lys Cys Lys Val Ser Asn Lys Gly Leu Pro Ser Ser Ile
   145                 150                 155                 160
   Glu Lys Thr Ile Ser Lys Ala Lys Gly Gln Pro Arg Glu Pro Gln Val
                   165                 170                 175
   Tyr Thr Leu Pro Pro Ser Gln Glu Glu Met Thr Lys Asn Gln Val Ser
               180                 185                 190
   Leu Thr Cys Leu Val Lys Gly Phe Tyr Pro Ser Asp Ile Ala Val Glu
           195                 200                 205
   Trp Glu Ser Asn Gly Gln Pro Glu Asn Asn Tyr Lys Thr Thr Pro Pro
       210                 215                 220
   Val Leu Asp Ser Asp Gly Ser Phe Phe Leu Tyr Ser Arg Leu Thr Val
   225                 230                 235                 240
   Asp Lys Ser Arg Trp Gln Glu Gly Asn Val Phe Ser Cys Ser Val Met
                   245                 250                 255
   His Glu Ala Leu His Asn His Tyr Thr Gln Lys Ser Leu Ser Leu Ser
               260                 265                 270
   Leu Gly Lys
           275
<210> 155
   <211> 11960
   <212> DNA
   <213> Artificial Sequence
<220>
   <223> In vitro synthesized DNA with expression vector functions that encodes an alpha-MSH mimetibody (p3652).
<400> 155
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactcctgcg agcacttccg 1680
   ctggtgcaag ccggtgggat ccggtggagg ctccggtacc ttagtcaccg tctcctcaga 1740
   gtccaaatat ggtcccccat gcccaccatg cccggcgcct gaggccgccg ggggaccatc 1800
   agtcttcctg ttccccccaa aacccaagga cactctcatg atctcccgga cccctgaggt 1860
   cacgtgcgtg gtggtggacg tgagccagga agaccccgag gtccagttca actggtacgt 1920
   ggatggcgtg gaggtgcata atgccaagac aaagccgcgg gaggagcagt tcaacagcac 1980
   gtaccgtgtg gtcagcgtcc tcaccgtcct gcaccaggac tggctgaacg gcaaggagta 2040
   caagtgcaag gtctccaaca aaggcctccc gtcctccatc gagaaaacca tctccaaagc 2100
   caaagggcag cctcgagagc cacaggtgta caccctgccc ccatcccagg aggagatgac 2160
   caagaaccag gtcagcctga cctgcctggt caaaggcttc taccccagcg acatcgccgt 2220
   ggagtgggag agcaatgggc agccggagaa caactacaag accacgcctc ccgtgctgga 2280
   ctccgacggc tccttcttcc tctacagcag gctaaccgtg gacaagagca ggtggcagga 2340
   ggggaatgtc ttctcatgct ccgtgatgca tgaggctctg cacaaccact acacacagaa 2400
   aagcttgtcc ctgtctctgg gtaaatgagg gcccgtttaa acagatccag acatgataag 2460
   atacattgat gagtttggac aaaccacaac tagaatgcag tgaaaaaaat gctttatttg 2520
   tgaaatttgt gatgctattg ctttatttgt aaccattata agctgcaata aacaagttaa 2580
   caacaacaat tgcattcatt ttatgtttca ggttcagggg gaggtgtggg aggtttttta 2640
   aagcaagtaa aacctctaca aatgtggtat ggctgattat gatccggctg cctcgcgcgt 2700
   ttcggtgatg acggtgaaaa cctctgacac atgcagctcc cggagacggt cacagcttgt 2760
   ctgtaagcgg atgccgggag cagacaagcc cgtcaggcgt cagcgggtgt tggcgggtgt 2820
   cggggcgcag ccatgaggtc gactctagag gatcgatgcc ccgccccgga cgaactaaac 2880
   ctgactacga catctctgcc ccttcttcgc ggggcagtgc atgtaatccc ttcagttggt 2940
   tggtacaact tgccaactgg gccctgttcc acatgtgaca cgggggggga ccaaacacaa 3000
   aggggttctc tgactgtagt tgacatcctt ataaatggat gtgcacattt gccaacactg 3060
   agtggctttc atcctggagc agactttgca gtctgtggac tgcaacacaa cattgccttt 3120
   atgtgtaact cttggctgaa gctcttacac caatgctggg ggacatgtac ctcccagggg 3180
   cccaggaaga ctacgggagg ctacaccaac gtcaatcaga ggggcctgtg tagctaccga 3240
   taagcggacc ctcaagaggg cattagcaat agtgtttata aggccccctt gttaacccta 3300
   aacgggtagc atatgcttcc cgggtagtag tatatactat ccagactaac cctaattcaa 3360
   tagcatatgt tacccaacgg gaagcatatg ctatcgaatt agggttagta aaagggtcct 3420
   aaggaacagc gatatctccc accccatgag ctgtcacggt tttatttaca tggggtcagg 3480
   attccacgag ggtagtgaac cattttagtc acaagggcag tggctgaaga tcaaggagcg 3540
   ggcagtgaac tctcctgaat cttcgcctgc ttcttcattc tccttcgttt agctaataga 3600
   ataactgctg agttgtgaac agtaaggtgt atgtgaggtg ctcgaaaaca aggtttcagg 3660
   tgacgccccc agaataaaat ttggacgggg ggttcagtgg tggcattgtg ctatgacacc 3720
   aatataaccc tcacaaaccc cttgggcaat aaatactagt gtaggaatga aacattctga 3780
   atatctttaa caatagaaat ccatggggtg gggacaagcc gtaaagactg gatgtccatc 3840
   tcacacgaat ttatggctat gggcaacaca taatcctagt gcaatatgat actggggtta 3900
   ttaagatgtg tcccaggcag ggaccaagac aggtgaacca tgttgttaca ctctatttgt 3960
   aacaagggga aagagagtgg acgccgacag cagcggactc cactggttgt ctctaacacc 4020
   cccgaaaatt aaacggggct ccacgccaat ggggcccata aacaaagaca agtggccact 4080
   cttttttttg aaattgtgga gtgggggcac gcgtcagccc ccacacgccg ccctgcggtt 4140
   ttggactgta aaataagggt gtaataactt ggctgattgt aaccccgcta accactgcgg 4200
   tcaaaccact tgcccacaaa accactaatg gcaccccggg gaatacctgc ataagtaggt 4260
   gggcgggcca agataggggc gcgattgctg cgatctggag gacaaattac acacacttgc 4320
   gcctgagcgc caagcacagg gttgttggtc ctcatattca cgaggtcgct gagagcacgg 4380
   tgggctaatg ttgccatggg tagcatatac tacccaaata tctggatagc atatgctatc 4440
   ctaatctata tctgggtagc ataggctatc ctaatctata tctgggtagc atatgctatc 4500
   ctaatctata tctgggtagt atatgctatc ctaatttata tctgggtagc ataggctatc 4560
   ctaatctata tctgggtagc atatgctatc ctaatctata tctgggtagt atatgctatc 4620
   ctaatctgta tccgggtagc atatgctatc ctaatagaga ttagggtagt atatgctatc 4680
   ctaatttata tctgggtagc atatactacc caaatatctg gatagcatat gctatcctaa 4740
   tctatatctg ggtagcatat gctatcctaa tctatatctg ggtagcatag gctatcctaa 4800
   tctatatctg ggtagcatat gctatcctaa tctatatctg ggtagtatat gctatcctaa 4860
   tttatatctg ggtagcatag gctatcctaa tctatatctg ggtagcatat gctatcctaa 4920
   tctatatctg ggtagtatat gctatcctaa tctgtatccg ggtagcatat gctatcctca 4980
   tgcatataca gtcagcatat gatacccagt agtagagtgg gagtgctatc ctttgcatat 5040
   gccgccacct cccaaggggg cgtgaatttt cgctgcttgt ccttttcctg ctggttgctc 5100
   ccattcttag gtgaatttaa ggaggccagg ctaaagccgt cgcatgtctg attgctcacc 5160
   aggtaaatgt cgctaatgtt ttccaacgcg agaaggtgtt gagcgcggag ctgagtgacg 5220
   tgacaacatg ggtatgccca attgccccat gttgggagga cgaaaatggt gacaagacag 5280
   atggccagaa atacaccaac agcacgcatg atgtctactg gggatttatt ctttagtgcg 5340
   ggggaataca cggcttttaa tacgattgag ggcgtctcct aacaagttac atcactcctg 5400
   cccttcctca ccctcatctc catcacctcc ttcatctccg tcatctccgt catcaccctc 5460
   cgcggcagcc ccttccacca taggtggaaa ccagggaggc aaatctactc catcgtcaaa 5520
   gctgcacaca gtcaccctga tattgcaggt aggagcgggc tttgtcataa caaggtcctt 5580
   aatcgcatcc ttcaaaacct cagcaaatat atgagtttgt aaaaagacca tgaaataaca 5640
   gacaatggac tcccttagcg ggccaggttg tgggccgggt ccaggggcca ttccaaaggg 5700
   gagacgactc aatggtgtaa gacgacattg tggaatagca agggcagttc ctcgccttag 5760
   gttgtaaagg gaggtcttac tacctccata tacgaacaca ccggcgaccc aagttccttc 5820
   gtcggtagtc ctttctacgt gactcctagc caggagagct cttaaacctt ctgcaatgtt 5880
   ctcaaatttc gggttggaac ctccttgacc acgatgcttt tccaaaccac cctccttttt 5940
   tgcgccctgc ctccatcacc ctgaccccgg ggtccagtgc ttgggccttc tcctgggtca 6000
   tctgcggggc cctgctctat cgctcccggg ggcacgtcag gctcaccatc tgggccacct 6060
   tcttggtggt attcaaaata atcggcttcc cctacagggt ggaaaaatgg ccttctacct 6120
   ggagggggcc tgcgcggtgg agacccggat gatgatgact gactactggg actcctgggc 6180
   ctcttttctc cacgtccacg acctctcccc ctggctcttt cacgacttcc ccccctggct 6240
   ctttcacgtc ctctaccccg gcggcctcca ctacctcctc gaccccggcc tccactacct 6300
   cctcgacccc ggcctccact gcctcctcga ccccggcctc cacctcctgc tcctgcccct 6360
   cctgctcctg cccctcctcc tgctcctgcc cctcctgccc ctcctgctcc tgcccctcct 6420
   gcccctcctg ctcctgcccc tcctgcccct cctgctcctg cccctcctgc ccctcctcct 6480
   gctcctgccc ctcctgcccc tcctcctgct cctgcccctc ctgcccctcc tgctcctgcc 6540
   cctcctgccc ctcctgctcc tgcccctcct gcccctcctg ctcctgcccc tcctgctcct 6600
   gcccctcctg ctcctgcccc tcctgctcct gcccctcctg cccctcctgc ccctcctcct 6660
   gctcctgccc ctcctgctcc tgcccctcct gcccctcctg cccctcctgc tcctgcccct 6720
   cctcctgctc ctgcccctcc tgcccctcct gcccctcctc ctgctcctgc ccctcctgcc 6780
   cctcctcctg ctcctgcccc tcctcctgct cctgcccctc ctgcccctcc tgcccctcct 6840
   cctgctcctg cccctcctgc ccctcctcct gctcctgccc ctcctcctgc tcctgcccct 6900
   cctgcccctc ctgcccctcc tcctgctcct gcccctcctc ctgctcctgc ccctcctgcc 6960
   cctcctgccc ctcctgcccc tcctcctgct cctgcccctc ctcctgctcc tgcccctcct 7020
   gctcctgccc ctcccgctcc tgctcctgct cctgttccac cgtgggtccc tttgcagcca 7080
   atgcaacttg gacgtttttg gggtctccgg acaccatctc tatgtcttgg ccctgatcct 7140
   gagccgcccg gggctcctgg tcttccgcct cctcgtcctc gtcctcttcc ccgtcctcgt 7200
   ccatggttat caccccctct tctttgaggt ccactgccgc cggagccttc tggtccagat 7260
   gtgtctccct tctctcctag gccatttcca ggtcctgtac ctggcccctc gtcagacatg 7320
   attcacacta aaagagatca atagacatct ttattagacg acgctcagtg aatacaggga 7380
   gtgcagactc ctgccccctc caacagcccc cccaccctca tccccttcat ggtcgctgtc 7440
   agacagatcc aggtctgaaa attccccatc ctccgaacca tcctcgtcct catcaccaat 7500
   tactcgcagc ccggaaaact cccgctgaac atcctcaaga tttgcgtcct gagcctcaag 7560
   ccaggcctca aattcctcgt cccccttttt gctggacggt agggatgggg attctcggga 7620
   cccctcctct tcctcttcaa ggtcaccaga cagagatgct actggggcaa cggaagaaaa 7680
   gctgggtgcg gcctgtgagg atcagcttat cgatgataag ctgtcaaaca tgagaattct 7740
   tgaagacgaa agggcctcgt gatacgccta tttttatagg ttaatgtcat gataataatg 7800
   gtttcttaga cgtcaggtgg cacttttcgg ggaaatgtgc gcggaacccc tatttgttta 7860
   tttttctaaa tacattcaaa tatgtatccg ctcatgagac aataaccctg ataaatgctt 7920
   caataatatt gaaaaaggaa gagtatgagt attcaacatt tccgtgtcgc ccttattccc 7980
   ttttttgcgg cattttgcct tcctgttttt gctcacccag aaacgctggt gaaagtaaaa 8040
   gatgctgaag atcagttggg tgcacgagtg ggttacatcg aactggatct caacagcggt 8100
   aagatccttg agagttttcg ccccgaagaa cgttttccaa tgatgagcac ttttaaagtt 8160
   ctgctatgtg gcgcggtatt atcccgtgtt gacgccgggc aagagcaact cggtcgccgc 8220
   atacactatt ctcagaatga cttggttgag tactcaccag tcacagaaaa gcatcttacg 8280
   gatggcatga cagtaagaga attatgcagt gctgccataa ccatgagtga taacactgcg 8340
   gccaacttac ttctgacaac gatcggagga ccgaaggagc taaccgcttt tttgcacaac 8400
   atgggggatc atgtaactcg ccttgatcgt tgggaaccgg agctgaatga agccatacca 8460
   aacgacgagc gtgacaccac gatgcctgca gcaatggcaa caacgttgcg caaactatta 8520
   actggcgaac tacttactct agcttcccgg caacaattaa tagactggat ggaggcggat 8580
   aaagttgcag gaccacttct gcgctcggcc cttccggctg gctggtttat tgctgataaa 8640
   tctggagccg gtgagcgtgg gtctcgcggt atcattgcag cactggggcc agatggtaag 8700
   ccctcccgta tcgtagttat ctacacgacg gggagtcagg caactatgga tgaacgaaat 8760
   agacagatcg ctgagatagg tgcctcactg attaagcatt ggtaactgtc agaccaagtt 8820
   tactcatata tactttagat tgatttaaaa cttcattttt aatttaaaag gatctaggtg 8880
   aagatccttt ttgataatct catgaccaaa atcccttaac gtgagttttc gttccactga 8940
   gcgtcagacc ccgtagaaaa gatcaaagga tcttcttgag atcctttttt tctgcgcgta 9000
   atctgctgct tgcaaacaaa aaaaccaccg ctaccagcgg tggtttgttt gccggatcaa 9060
   gagctaccaa ctctttttcc gaaggtaact ggcttcagca gagcgcagat accaaatact 9120
   gtccttctag tgtagccgta gttaggccac cacttcaaga actctgtagc accgcctaca 9180
   tacctcgctc tgctaatcct gttaccagtg gctgctgcca gtggcgataa gtcgtgtctt 9240
   accgggttgg actcaagacg atagttaccg gataaggcgc agcggtcggg ctgaacgggg 9300
   ggttcgtgca cacagcccag cttggagcga acgacctaca ccgaactgag atacctacag 9360
   cgtgagctat gagaaagcgc cacgcttccc gaagggagaa aggcggacag gtatccggta 9420
   agcggcaggg tcggaacagg agagcgcacg agggagcttc cagggggaaa cgcctggtat 9480
   ctttatagtc ctgtcgggtt tcgccacctc tgacttgagc gtcgattttt gtgatgctcg 9540
   tcaggggggc ggagcctatg gaaaaacgcc agcaacgcgg cctttttacg gttcctggcc 9600
   ttttgctggc cttgaagctg tccctgatgg tcgtcatcta cctgcctgga cagcatggcc 9660
   tgcaacgcgg gcatcccgat gccgccggaa gcgagaagaa tcataatggg gaaggccatc 9720
   cagcctcgcg tcgcgaacgc cagcaagacg tagcccagcg cgtcggcccc gagatgcgcc 9780
   gcgtgcggct gctggagatg gcggacgcga tggatatgtt ctgccaaggg ttggtttgcg 9840
   cattcacagt tctccgcaag aattgattgg ctccaattct tggagtggtg aatccgttag 9900
   cgaggtgccg ccctgcttca tccccgtggc ccgttgctcg cgtttgctgg cggtgtcccc 9960
   ggaagaaata tatttgcatg tctttagttc tatgatgaca caaaccccgc ccagcgtctt 10020
   gtcattggcg aattcgaaca cgcagatgca gtcggggcgg cgcggtccga ggtccacttc 10080
   gcatattaag gtgacgcgtg tggcctcgaa caccgagcga ccctgcagcg acccgcttaa 10140
   cagcgtcaac agcgtgccgc agatcccggg gggcaatgag atatgaaaaa gcctgaactc 10200
   accgcgacgt ctgtcgagaa gtttctgatc gaaaagttcg acagcgtctc cgacctgatg 10260
   cagctctcgg agggcgaaga atctcgtgct ttcagcttcg atgtaggagg gcgtggatat 10320
   gtcctgcggg taaatagctg cgccgatggt ttctacaaag atcgttatgt ttatcggcac 10380
   tttgcatcgg ccgcgctccc gattccggaa gtgcttgaca ttggggaatt cagcgagagc 10440
   ctgacctatt gcatctcccg ccgtgcacag ggtgtcacgt tgcaagacct gcctgaaacc 10500
   gaactgcccg ctgttctgca gccggtcgcg gaggccatgg atgcgatcgc tgcggccgat 10560
   cttagccaga cgagcgggtt cggcccattc ggaccgcaag gaatcggtca atacactaca 10620
   tggcgtgatt tcatatgcgc gattgctgat ccccatgtgt atcactggca aactgtgatg 10680
   gacgacaccg tcagtgcgtc cgtcgcgcag gctctcgatg agctgatgct ttgggccgag 10740
   gactgccccg aagtccggca cctcgtgcac gcggatttcg gctccaacaa tgtcctgacg 10800
   gacaatggcc gcataacagc ggtcattgac tggagcgagg cgatgttcgg ggattcccaa 10860
   tacgaggtcg ccaacatctt cttctggagg ccgtggttgg cttgtatgga gcagcagacg 10920
   cgctacttcg agcggaggca tccggagctt gcaggatcgc cgcggctccg ggcgtatatg 10980
   ctccgcattg gtcttgacca actctatcag agcttggttg acggcaattt cgatgatgca 11040
   gcttgggcgc agggtcgatg cgacgcaatc gtccgatccg gagccgggac tgtcgggcgt 11100
   acacaaatcg cccgcagaag cgcggccgtc tggaccgatg gctgtgtaga agtactcgcc 11160
   gatagtggaa accgacgccc cagcactcgt ccggatcggg agatggggga ggctaactga 11220
   aacacggaag gagacaatac cggaaggaac ccgcgctatg acggcaataa aaagacagaa 11280
   taaaacgcac gggtgttggg tcgtttgttc ataaacgcgg ggttcggtcc cagggctggc 11340
   actctgtcga taccccaccg agaccccatt ggggccaata cgcccgcgtt tcttcctttt 11400
   ccccacccca ccccccaagt tcgggtgaag gcccagggct cgcagccaac gtcggggcgg 11460
   caggccctgc catagccact ggccccgtgg gttagggacg gggtccccca tggggaatgg 11520
   tttatggttc gtgggggtta ttattttggg cgttgcgtgg ggtcaggtcc acgactggac 11580
   tgagcagaca gacccatggt ttttggatgg cctgggcatg gaccgcatgt actggcgcga 11640
   cacgaacacc gggcgtctgt ggctgccaaa cacccccgac cccaaaaaac caccgcgcgg 11700
   atttctggcg tgccaagcta gtcgaccaat tctcatgttt gacagcttat catcgcagat 11760
   ccgggcaacg ttgttgccat tgctgcaggc gcagaactgg taggtatgga agatctatac 11820
   attgaatcaa tattggcaat tagccatatt agtcattggt tatatagcat aaatcaatat 11880
   tggctattgg ccattgcata cgttgtatct atatcataat atgtacattt atattggctc 11940
   atgtccaata tgaccgccat                                             11960
<210> 156
   <211> 777
   <212> DNA
   <213> Artificial Sequence
<220>
   <223> In vitro synthesized DNA encoding a melanocortin receptor binding alpha-MSH mimetibody without secretory signal (p3677).
<400> 156
   tcctactcct gcgagcactt ccgctggtgc aagccggtgg gatccggggg aggctccggt 60
   accttagtca ccgtctcctc agagcccaaa tctgctgaca aaactcacac atgcccaccg 120
   tgcccagcac ctgaagccgc cgggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 157
   <211> 259
   <212> PRT
   <213> Artificial Sequence
<220>
   <223> Melanocortin receptor binding alpha-MSH mimetibody without secretory signal encoded by an in vitro synthesized DNA (p3677).
<400> 157
   Ser Tyr Ser Cys Glu His Phe Arg Trp Cys Lys Pro Val Gly Ser Gly
    1              5                   10                  15
   Gly Gly Ser Gly Thr Leu Val Thr Val Ser Ser Glu Pro Lys Ser Ala
               20                  25                  30
   Asp Lys Thr His Thr Cys Pro Pro Cys Pro Ala Pro Glu Ala Ala Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 158
   <211> 834
   <212> DNA
   <213> Artificial Sequence
<220>
   <223> In vitro synthesized DNA encoding a melanocortin receptor binding alpha-MSH mimetibody with secretory signal (p3677).
<400> 158
   atggcttggg tgtggacctt gctattcctg atggcggccg cccaaagtat acaggcctcc 60
   tactcctgcg agcacttccg ctggtgcaag ccggtgggat ccgggggagg ctccggtacc 120
   ttagtcaccg tctcctcaga gcccaaatct gctgacaaaa ctcacacatg cccaccgtgc 180
   ccagcacctg aagccgccgg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 159
   <211> 278
   <212> PRT
   <213> Artificial Sequence
<220>
   <223> Melanocortin receptor binding alpha-MSH mimetibody with secretory signal encoded by an in vitro synthesized DNA (p3677).
<400> 159
   Met Ala Trp Val Trp Thr Leu Leu Phe Leu Met Ala Ala Ala Gln Ser
   1               5                   10                  15
   Ile Gln Ala Ser Tyr Ser Cys Glu His Phe Arg Trp Cys Lys Pro Val
               20                  25                  30
   Gly Ser Gly Gly Gly Ser Gly Thr Leu Val Thr Val Ser Ser Glu Pro
           35                  40                  45
   Lys Ser Ala Asp Lys Thr His Thr Cys Pro Pro Cys Pro Ala Pro Glu
       50                  55                  60
   Ala Ala Gly Gly Pro Ser Val Phe Leu Phe Pro Pro Lys Pro Lys Asp
   65                  70                  75                  80
   Thr Leu Met Ile Ser Arg Thr Pro Glu Val Thr Cys Val Val Val Asp
                   85                  90                  95
   Val Ser His Glu Asp Pro Glu Val Lys Phe Asn Trp Tyr Val Asp Gly
               100                 105                 110
   Val Glu Val His Asn Ala Lys Thr Lys Pro Arg Glu Glu Gln Tyr Asn
           115                 120                 125
   Ser Thr Tyr Arg Val Val Ser Val Leu Thr Val Leu His Gln Asp Trp
       130                 135                 140
   Leu Asn Gly Lys Glu Tyr Lys Cys Lys Val Ser Asn Lys Ala Leu Pro
   145                 150                 155                 160
   Ala Pro Ile Glu Lys Thr Ile Ser Lys Ala Lys Gly Gln Pro Arg Glu
                   165                 170                 175
   Pro Gln Val Tyr Thr Leu Pro Pro Ser Arg Asp Glu Leu Thr Lys Asn
               180                 185                 190
   Gln Val Ser Leu Thr Cys Leu Val Lys Gly Phe Tyr Pro Ser Asp Ile
           195                 200                 205
   Ala Val Glu Trp Glu Ser Asn Gly Gln Pro Glu Asn Asn Tyr Lys Thr
       210                 215                 220
   Thr Pro Pro Val Leu Asp Ser Asp Gly Ser Phe Phe Leu Tyr Ser Lys
   225                 230                 235                 240
   Leu Thr Val Asp Lys Ser Arg Trp Gln Gln Gly Asn Val Phe Ser Cys
                   245                 250                 255
   Ser Val Met His Glu Ala Leu His Asn His Tyr Thr Gln Lys Ser Leu
               260                 265                 270
   Ser Leu Ser Pro Gly Lys
           275
<210> 160
   <211> 11969
   <212> DNA
   <213> Artificial Sequence
<220>
   <223> In vitro synthesized DNA with expression vector functions that encodes an alpha-MSH mimetibody (p3677).
<400> 160
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactcctgcg agcacttccg 1680
   ctggtgcaag ccggtgggat ccgggggagg ctccggtacc ttagtcaccg tctcctcaga 1740
   gcccaaatct gctgacaaaa ctcacacatg cccaccgtgc ccagcacctg aagccgccgg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaagggggc gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gagggggcct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat 11969
<210> 161
   <211> 777
   <212> DNA
   <213> Artificial Sequence
<220>
   <223> In vitro synthesized DNA encoding a melanocortin receptor binding alpha-MSH mimetibody without secretory signal (p3160).
<400> 161
   tcctactgca tggagcactt ccgctggtgc aagccggtgg gatccggtgg aggctccggt 60
   accttagtcg ccgtctcctc agagcccaaa tctgccgaca aaactcacgc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa 777
<210> 162
   <211> 259
   <212> PRT
   <213> Artificial Sequence
<220>
   <223> Melanocortin receptor binding alpha-MSH mimetibody without secretory signal encoded by an in vitro synthesized DNA (p3160).
<400> 162
   Ser Tyr Cys Met Glu His Phe Arg Trp Cys Lys Pro Val Gly Ser Gly
    1               5                  10                  15
   Gly Gly Ser Gly Thr Leu Val Ala Val Ser Ser Glu Pro Lys Ser Ala
               20                  25                  30
   Asp Lys Thr His Ala Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 163
   <211> 834
   <212> DNA
   <213> Artificial Sequence
<220>
   <223> In vitro synthesized DNA encoding a melanocortin receptor binding alpha-MSH mimetibody with secretory signal (p3160).
<400> 163
   atggcttggg tgtggacctt gctattcctg atggcggccg cccaaagtat acaggcctcc 60
   tactgcatgg agcacttccg ctggtgcaag ccggtgggat ccggtggagg ctccggtacc 120
   ttagtcgccg tctcctcaga gcccaaatct gccgacaaaa ctcacgcctg cccaccgtgc 180
   ccagcacctg aactcctggg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 164
   <211> 278
   <212> PRT
   <213> Artificial Sequence
<220>
   <223> Melanocortin receptor binding alpha-MSH mimetibody with secretory signal encoded by an in vitro synthesized DNA (p3160).
<400> 164
   Met Ala Trp Val Trp Thr Leu Leu Phe Leu Met Ala Ala Ala Gln Ser
    1               5                   10                  15
   Ile Gln Ala Ser Tyr Cys Met Glu His Phe Arg Trp Cys Lys Pro Val
               20                  25                  30
   Gly Ser Gly Gly Gly Ser Gly Thr Leu Val Ala Val Ser Ser Glu Pro
           35                  40                  45
   Lys Ser Ala Asp Lys Thr His Ala Cys Pro Pro Cys Pro Ala Pro Glu
       50                  55                  60
   Leu Leu Gly Gly Pro Ser Val Phe Leu Phe Pro Pro Lys Pro Lys Asp
   65                  70                  75                  80
   Thr Leu Met Ile Ser Arg Thr Pro Glu Val Thr Cys Val Val Val Asp
                   85                  90                  95
   Val Ser His Glu Asp Pro Glu Val Lys Phe Asn Trp Tyr Val Asp Gly
               100                 105                 110
   Val Glu Val His Asn Ala Lys Thr Lys Pro Arg Glu Glu Gln Tyr Asn
           115                 120                 125
   Ser Thr Tyr Arg Val Val Ser Val Leu Thr Val Leu His Gln Asp Trp
       130                 135                 140
   Leu Asn Gly Lys Glu Tyr Lys Cys Lys Val Ser Asn Lys Ala Leu Pro
   145                 150                 155                 160
   Ala Pro Ile Glu Lys Thr Ile Ser Lys Ala Lys Gly Gln Pro Arg Glu
                   165                 170                 175
   Pro Gln Val Tyr Thr Leu Pro Pro Ser Arg Asp Glu Leu Thr Lys Asn
               180                 185                 190
   Gln Val Ser Leu Thr Cys Leu Val Lys Gly Phe Tyr Pro Ser Asp Ile
           195                 200                 205
   Ala Val Glu Trp Glu Ser Asn Gly Gln Pro Glu Asn Asn Tyr Lys Thr
       210                 215                 220
   Thr Pro Pro Val Leu Asp Ser Asp Gly Ser Phe Phe Leu Tyr Ser Lys
   225                 230                 235                 240
   Leu Thr Val Asp Lys Ser Arg Trp Gln Gln Gly Asn Val Phe Ser Cys
                   245                 250                 255
   Ser Val Met His Glu Ala Leu His Asn His Tyr Thr Gln Lys Ser Leu
               260                 265                 270
   Ser Leu Ser Pro Gly Lys
           275
<210> 165
   <211> 11969
   <212> DNA
   <213> Artificial Sequence
<220>
   <223> In vitro synthesized DNA with expression vector functions that encodes an alpha-MSH mimetibody (p3160).
<400> 165
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactgcatgg agcacttccg 1680
   ctggtgcaag ccggtgggat ccggtggagg ctccggtacc ttagtcgccg tctcctcaga 1740
   gcccaaatct gccgacaaaa ctcacgcctg cccaccgtgc ccagcacctg aactcctggg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaagggggc gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gagggggcct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat 11969
<210> 166
   <211> 777
   <212> DNA
   <213> Artificial Sequence
<220>
   <223> In vitro synthesized DNA encoding a melanocortin receptor binding alpha-MSH mimetibody without secretory signal (p3161).
<400> 166
   tcctgctcca tggagcactt ccgctggtgc aagccggtgg gatccggtgg aggctccggt 60
   accttagtcg ccgtctcctc agagcccaaa tctgccgaca aaactcacgc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 167
   <211> 259
   <212> PRT
   <213> Artificial Sequence
<220>
   <223> Melanocortin receptor binding alpha-MSH mimetibody without secretory signal encoded by an in vitro synthesized DNA (p3161).
<400> 167
   Ser Cys Ser Met Glu His Phe Arg Trp Cys Lys Pro Val Gly Ser Gly
    1               5                  10                  15
   Gly Gly Ser Gly Thr Leu Val Ala Val Ser Ser Glu Pro Lys Ser Ala
               20                  25                  30
   Asp Lys Thr His Ala Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 168
   <211> 834
   <212> DNA
   <213> Artificial Sequence
<220>
   <223> In vitro synthesized DNA encoding a melanocortin receptor binding alpha-MSH mimetibody with secretory signal (p3161).
<400> 168
   atggcttggg tgtggacctt gctattcctg atggcggccg cccaaagtat acaggcctcc 60
   tgctccatgg agcacttccg ctggtgcaag ccggtgggat ccggtggagg ctccggtacc 120
   ttagtcgccg tctcctcaga gcccaaatct gccgacaaaa ctcacgcctg cccaccgtgc 180
   ccagcacctg aactcctggg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 169
   <211> 278
   <212> PRT
   <213> Artificial Sequence
<220>
   <223> Melanocortin receptor binding alpha-MSH mimetibody with secretory signal encoded by an in vitro synthesized DNA (p3161).
<400> 169
   Met Ala Trp Val Trp Thr Leu Leu Phe Leu Met Ala Ala Ala Gln Ser
    1               5                  10                  15
   Ile Gln Ala Ser Cys Ser Met Glu His Phe Arg Trp Cys Lys Pro Val
               20                  25                  30
   Gly Ser Gly Gly Gly Ser Gly Thr Leu Val Ala Val Ser Ser Glu Pro
           35                  40                  45
   Lys Ser Ala Asp Lys Thr His Ala Cys Pro Pro Cys Pro Ala Pro Glu
       50                  55                  60
   Leu Leu Gly Gly Pro Ser Val Phe Leu Phe Pro Pro Lys Pro Lys Asp
   65                  70                  75                  80
   Thr Leu Met Ile Ser Arg Thr Pro Glu Val Thr Cys Val Val Val Asp
                   85                  90                  95
   Val Ser His Glu Asp Pro Glu Val Lys Phe Asn Trp Tyr Val Asp Gly
               100                 105                 110
   Val Glu Val His Asn Ala Lys Thr Lys Pro Arg Glu Glu Gln Tyr Asn
           115                 120                 125
   Ser Thr Tyr Arg Val Val Ser Val Leu Thr Val Leu His Gln Asp Trp
       130                 135                 140
   Leu Asn Gly Lys Glu Tyr Lys Cys Lys Val Ser Asn Lys Ala Leu Pro
   145                 150                 155                 160
   Ala Pro Ile Glu Lys Thr Ile Ser Lys Ala Lys Gly Gln Pro Arg Glu
                   165                 170                 175
   Pro Gln Val Tyr Thr Leu Pro Pro Ser Arg Asp Glu Leu Thr Lys Asn
               180                 185                 190
   Gln Val Ser Leu Thr Cys Leu Val Lys Gly Phe Tyr Pro Ser Asp Ile
           195                 200                 205
   Ala Val Glu Trp Glu Ser Asn Gly Gln Pro Glu Asn Asn Tyr Lys Thr
       210                 215                 220
   Thr Pro Pro Val Leu Asp Ser Asp Gly Ser Phe Phe Leu Tyr Ser Lys
   225                 230                 235                 240
   Leu Thr Val Asp Lys Ser Arg Trp Gln Gln Gly Asn Val Phe Ser Cys
                   245                 250                 255
   Ser Val Met His Glu Ala Leu His Asn His Tyr Thr Gln Lys Ser Leu
               260                 265                 270
   Ser Leu Ser Pro Gly Lys
           275
<210> 170
   <211> 11969
   <212> DNA
   <213> Artificial Sequence
<220>
   <223> In vitro synthesized DNA with expression vector functions that encodes an alpha-MSH mimetibody (p3161).
<400> 170
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tgctccatgg agcacttccg 1680
   ctggtgcaag ccggtgggat ccggtggagg ctccggtacc ttagtcgccg tctcctcaga 1740
   gcccaaatct gccgacaaaa ctcacgcctg cccaccgtgc ccagcacctg aactcctggg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaagggggc gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gagggggcct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat 11969
<210> 171
   <211> 777
   <212> DNA
   <213> Artificial Sequence
<220>
   <223> In vitro synthesized DNA encoding a melanocortin receptor binding alpha-MSH mimetibody without secretory signal (p3215).
<400> 171
   tcctactcct gcgagcactt ccgctggggc tgcccggtgg gatccggtgg aggctccggt 60
   accttagtcg ccgtctcctc agagcccaaa tctgccgaca aaactcacgc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 172
   <211> 259
   <212> PRT
   <213> Artificial Sequence
<220>
   <223> Melanocortin receptor binding alpha-MSH mimetibody without secretory signal encoded by an in vitro synthesized DNA (p3215).
<400> 172
   Ser Tyr Ser Cys Glu His Phe Arg Trp Gly Cys Pro Val Gly Ser Gly
    1               5                  10                  15
   Gly Gly Ser Gly Thr Leu Val Ala Val Ser Ser Glu Pro Lys Ser Ala
               20                  25                  30
   Asp Lys Thr His Ala Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 173
   <211> 834
   <212> DNA
   <213> Artificial Sequence
<220>
   <223> In vitro synthesized DNA encoding a melanocortin receptor binding alpha-MSH mimetibody with secretory signal (p3215).
<400> 173
   atggcttggg tgtggacctt gctattcctg atggcggccg cccaaagtat acaggcctcc 60
   tactcctgcg agcacttccg ctggggctgc ccggtgggat ccggtggagg ctccggtacc 120
   ttagtcgccg tctcctcaga gcccaaatct gccgacaaaa ctcacgcctg cccaccgtgc 180
   ccagcacctg aactcctggg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 174
   <211> 278
   <212> PRT
   <213> Artificial Sequence
<220>
   <223> Melanocortin receptor binding alpha-MSH mimetibody with secretory signal encoded by an in vitro synthesized DNA (p3215).
<400> 174
   Met Ala Trp Val Trp Thr Leu Leu Phe Leu Met Ala Ala Ala Gln Ser
    1               5                  10                  15
   Ile Gln Ala Ser Tyr Ser Cys Glu His Phe Arg Trp Gly Cys Pro Val
               20                  25                  30
   Gly Ser Gly Gly Gly Ser Gly Thr Leu Val Ala Val Ser Ser Glu Pro
           35                  40                  45
   Lys Ser Ala Asp Lys Thr His Ala Cys Pro Pro Cys Pro Ala Pro Glu
       50                  55                  60
   Leu Leu Gly Gly Pro Ser Val Phe Leu Phe Pro Pro Lys Pro Lys Asp
   65                  70                  75                  80
   Thr Leu Met Ile Ser Arg Thr Pro Glu Val Thr Cys Val Val Val Asp
                   85                  90                  95
   Val Ser His Glu Asp Pro Glu Val Lys Phe Asn Trp Tyr Val Asp Gly
                   100                 105                 110
   Val Glu Val His Asn Ala Lys Thr Lys Pro Arg Glu Glu Gln Tyr Asn
               115                 120                 125
   Ser Thr Tyr Arg Val Val Ser Val Leu Thr Val Leu His Gln Asp Trp
           130                 135                 140
   Leu Asn Gly Lys Glu Tyr Lys Cys Lys Val Ser Asn Lys Ala Leu Pro
   145                 150                 155                 160
   Ala Pro Ile Glu Lys Thr Ile Ser Lys Ala Lys Gly Gln Pro Arg Glu
                   165                 170                 175
   Pro Gln Val Tyr Thr Leu Pro Pro Ser Arg Asp Glu Leu Thr Lys Asn
               180                 185                 190
   Gln Val Ser Leu Thr Cys Leu Val Lys Gly Phe Tyr Pro Ser Asp Ile
           195                 200                 205
   Ala Val Glu Trp Glu Ser Asn Gly Gln Pro Glu Asn Asn Tyr Lys Thr
       210                 215                 220
   Thr Pro Pro Val Leu Asp Ser Asp Gly Ser Phe Phe Leu Tyr Ser Lys
   225                 230                 235                 240
   Leu Thr Val Asp Lys Ser Arg Trp Gln Gln Gly Asn Val Phe Ser Cys
                   245                 250                 255
   Ser Val Met His Glu Ala Leu His Asn His Tyr Thr Gln Lys Ser Leu
               260                 265                 270
   Ser Leu Ser Pro Gly Lys
           275
<210> 175
   <211> 11969
   <212> DNA
   <213> Artificial Sequence
<220>
   <223> In vitro synthesized DNA with expression vector functions that encodes an alpha-MSH mimetibody (p3215).
<400> 175
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactcctgcg agcacttccg 1680
   ctggggctgc ccggtgggat ccggtggagg ctccggtacc ttagtcgccg tctcctcaga 1740
   gcccaaatct gccgacaaaa ctcacgcctg cccaccgtgc ccagcacctg aactcctggg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaagggggc gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gagggggcct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat                                   11969
<210> 176
   <211> 777
   <212> DNA
   <213> Artificial Sequence
<220>
   <223> In vitro synthesized DNA encoding a melanocortin receptor binding alpha-MSH mimetibody without secretory signal (p3216).
<400> 176
   tcctactcct gcgagcactt ccgctggggc aagtgcgtgg gatccggtgg aggctccggt 60
   accttagtcg ccgtctcctc agagcccaaa tctgccgaca aaactcacgc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 177
   <211> 259
   <212> PRT
   <213> Artificial Sequence
<220>
   <223> Melanocortin receptor binding alpha-MSH mimetibody without secretory signal encoded by an in vitro synthesized DNA (p3216).
<400> 177
   Ser Tyr Ser Cys Glu His Phe Arg Trp Gly Lys Cys Val Gly Ser Gly
    1               5                  10                  15
   Gly Gly Ser Gly Thr Leu Val Ala Val Ser Ser Glu Pro Lys Ser Ala
               20                  25                  30
   Asp Lys Thr His Ala Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 178
   <211> 834
   <212> DNA
   <213> Artificial Sequence
<220>
   <223> In vitro synthesized DNA encoding a melanocortin receptor binding alpha-MSH mimetibody with secretory signal (p3216).
<400> 178
   atggcttggg tgtggacctt gctattcctg atggcggccg cccaaagtat acaggcctcc 60
   tactcctgcg agcacttccg ctggggcaag tgcgtgggat ccggtggagg ctccggtacc 120
   ttagtcgccg tctcctcaga gcccaaatct gccgacaaaa ctcacgcctg cccaccgtgc 180
   ccagcacctg aactcctggg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 179
   <211> 278
   <212> PRT
   <213> Artificial Sequence
<220>
   <223> Melanocortin receptor binding alpha-MSH mimetibody with secretory signal encoded by an in vitro synthesized DNA (p3216).
<400> 179
   Met Ala Trp Val Trp Thr Leu Leu Phe Leu Met Ala Ala Ala Gln Ser
    1               5                  10                  15
   Ile Gln Ala Ser Tyr Ser Cys Glu His Phe Arg Trp Gly Lys Cys Val
               20                  25                  30
   Gly Ser Gly Gly Gly Ser Gly Thr Leu Val Ala Val Ser Ser Glu Pro
           35                  40                  45
   Lys Ser Ala Asp Lys Thr His Ala Cys Pro Pro Cys Pro Ala Pro Glu
       50                  55                  60
   Leu Leu Gly Gly Pro Ser Val Phe Leu Phe Pro Pro Lys Pro Lys Asp
   65                  70                  75                  80
   Thr Leu Met Ile Ser Arg Thr Pro Glu Val Thr Cys Val Val Val Asp
                   85                  90                  95
   Val Ser His Glu Asp Pro Glu Val Lys Phe Asn Trp Tyr Val Asp Gly
               100                 105                 110
   Val Glu Val His Asn Ala Lys Thr Lys Pro Arg Glu Glu Gln Tyr Asn
           115                 120                 125
   Ser Thr Tyr Arg Val Val Ser Val Leu Thr Val Leu His Gln Asp Trp
       130                 135                 140
   Leu Asn Gly Lys Glu Tyr Lys Cys Lys Val Ser Asn Lys Ala Leu Pro
   145                 150                 155                 160
   Ala Pro Ile Glu Lys Thr Ile Ser Lys Ala Lys Gly Gln Pro Arg Glu
                   165                 170                 175
   Pro Gln Val Tyr Thr Leu Pro Pro Ser Arg Asp Glu Leu Thr Lys Asn
               180                 185                 190
   Gln Val Ser Leu Thr Cys Leu Val Lys Gly Phe Tyr Pro Ser Asp Ile
           195                 200                 205
   Ala Val Glu Trp Glu Ser Asn Gly Gln Pro Glu Asn Asn Tyr Lys Thr
       210                 215                 220
   Thr Pro Pro Val Leu Asp Ser Asp Gly Ser Phe Phe Leu Tyr Ser Lys
   225                 230                 235                 240
   Leu Thr Val Asp Lys Ser Arg Trp Gln Gln Gly Asn Val Phe Ser Cys
                   245                 250                 255
   Ser Val Met His Glu Ala Leu His Asn His Tyr Thr Gln Lys Ser Leu
               260                 265                 270
   Ser Leu Ser Pro Gly Lys
           275
<210> 180
   <211> 11969
   <212> DNA
   <213> Artificial Sequence
<220>
   <223> In vitro synthesized DNA with expression vector functions that encodes an alpha-MSH mimetibody (p3216).
<400> 180
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactcctgcg agcacttccg 1680
   ctggggcaag tgcgtgggat ccggtggagg ctccggtacc ttagtcgccg tctcctcaga 1740
   gcccaaatct gccgacaaaa ctcacgcctg cccaccgtgc ccagcacctg aactcctggg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaagggggc gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gaggggggct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat 11969
<210> 181
   <211> 777
   <212> DNA
   <213> Artificial Sequence
<220>
   <223> In vitro synthesized DNA encoding a melanocortin receptor binding alpha-MSH mimetibody without secretory signal (p3217).
<400> 181
   tcctactcct gcgagcactt ccgctggggc aagccgtgcg gatccggtgg aggctccggt 60
   accttagtcg ccgtctcctc agagcccaaa tctgccgaca aaactcacgc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 182
   <211> 259
   <212> PRT
   <213> Artificial Sequence
<220>
   <223> Melanocortin receptor binding alpha-MSH mimetibody without secretory signal encoded by an in vitro synthesized DNA (p3217).
<400> 182
   Ser Tyr Ser Cys Glu His Phe Arg Trp Gly Lys Pro Cys Gly Ser Gly
    1               5                  10                  15
   Gly Gly Ser Gly Thr Leu Val Ala Val Ser Ser Glu Pro Lys Ser Ala
               20                  25                  30
   Asp Lys Thr His Ala Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 183
   <211> 834
   <212> DNA
   <213> Artificial Sequence
<220>
   <223> In vitro synthesized DNA encoding a melanocortin receptor binding alpha-MSH mimetibody with secretory signal (p3217).
<400> 183
   atggcttggg tgtggacctt gctattcctg atggcggccg cccaaagtat acaggcctcc 60
   tactcctgcg agcacttccg ctggggcaag ccgtgcggat ccggtggagg ctccggtacc 120
   ttagtcgccg tctcctcaga gcccaaatct gccgacaaaa ctcacgcctg cccaccgtgc 180
   ccagcacctg aactcctggg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 184
   <211> 278
   <212> PRT
   <213> Artificial Sequence
<220>
   <223> Melanocortin receptor binding alpha-MSH mimetibody with secretory signal encoded by an in vitro synthesized DNA (p3217).
<400> 184
   Met Ala Trp Val Trp Thr Leu Leu Phe Leu Met Ala Ala Ala Gln Ser
    1               5                  10                  15
   Ile Gln Ala Ser Tyr Ser Cys Glu His Phe Arg Trp Gly Lys Pro Cys
               20                  25                  30
   Gly Ser Gly Gly Gly Ser Gly Thr Leu Val Ala Val Ser Ser Glu Pro
           35                  40                  45
   Lys Ser Ala Asp Lys Thr His Ala Cys Pro Pro Cys Pro Ala Pro Glu
       50                  55                  60
   Leu Leu Gly Gly Pro Ser Val Phe Leu Phe Pro Pro Lys Pro Lys Asp
   65                  70                  75                  80
   Thr Leu Met Ile Ser Arg Thr Pro Glu Val Thr Cys Val Val Val Asp
                   85                  90                  95
   Val Ser His Glu Asp Pro Glu Val Lys Phe Asn Trp Tyr Val Asp Gly
               100                 105                 110
   Val Glu Val His Asn Ala Lys Thr Lys Pro Arg Glu Glu Gln Tyr Asn
           115                 120                 125
   Ser Thr Tyr Arg Val Val Ser Val Leu Thr Val Leu His Gln Asp Trp
       130                 135                 140
   Leu Asn Gly Lys Glu Tyr Lys Cys Lys Val Ser Asn Lys Ala Leu Pro
   145                 150                 155                 160
   Ala Pro Ile Glu Lys Thr Ile Ser Lys Ala Lys Gly Gln Pro Arg Glu
                   165                 170                 175
   Pro Gln Val Tyr Thr Leu Pro Pro Ser Arg Asp Glu Leu Thr Lys Asn
               180                 185                 190
   Gln Val Ser Leu Thr Cys Leu Val Lys Gly Phe Tyr Pro Ser Asp Ile
           195                 200                 205
   Ala Val Glu Trp Glu Ser Asn Gly Gln Pro Glu Asn Asn Tyr Lys Thr
       210                 215                 220
   Thr Pro Pro Val Leu Asp Ser Asp Gly Ser Phe Phe Leu Tyr Ser Lys
   225                 230                 235                 240
   Leu Thr Val Asp Lys Ser Arg Trp Gln Gln Gly Asn Val Phe Ser Cys
                   245                 250                 255
   Ser Val Met His Glu Ala Leu His Asn His Tyr Thr Gln Lys Ser Leu
               260                 265                 270
   Ser Leu Ser Pro Gly Lys
           275
<210> 185
   <211> 11969
   <212> DNA
   <213> Artificial Sequence
<220>
   <223> In vitro synthesized DNA with expression vector functions that encodes an alpha-MSH mimetibody (p3217).
<400> 185
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactcctgcg agcacttccg 1680
   ctggggcaag ccgtgcggat ccggtggagg ctccggtacc ttagtcgccg tctcctcaga 1740
   gcccaaatct gccgacaaaa ctcacgcctg cccaccgtgc ccagcacctg aactcctggg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaaggggcg gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gagggggcct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat                                   11969
<210> 186
   <211> 777
   <212> DNA
   <213> Artificial Sequence
<220>
   <223> In vitro synthesized DNA encoding a melanocortin receptor binding alpha-MSH mimetibody without secretory signal (p3218).
<400> 186
   tcctactgca tggagcactt ccgctggggc tgcccggtgg gatccggtgg aggctccggt 60
   accttagtcg ccgtctcctc agagcccaaa tctgccgaca aaactcacgc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 187
   <211> 259
   <212> PRT
   <213> Artificial Sequence
<220>
   <223> Melanocortin receptor binding alpha-MSH mimetibody without secretory signal encoded by an in vitro synthesized DNA (p3218).
<400> 187
   Ser Tyr Cys Met Glu His Phe Arg Trp Gly Cys Pro Val Gly Ser Gly
    1               5                  10                  15
   Gly Gly Ser Gly Thr Leu Val Ala Val Ser Ser Glu Pro Lys Ser Ala
               20                  25                  30
   Asp Lys Thr His Ala Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 188
   <211> 834
   <212> DNA
   <213> Artificial Sequence
<220>
   <223> In vitro synthesized DNA encoding a melanocortin receptor binding alpha-MSH mimetibody with secretory signal (p3218).
<400> 188
   atggcttggg tgtggacctt gctattcctg atggcggccg cccaaagtat acaggcctcc 60
   tactgcatgg agcacttccg ctggggctgc ccggtgggat ccggtggagg ctccggtacc 120
   ttagtcgccg tctcctcaga gcccaaatct gccgacaaaa ctcacgcctg cccaccgtgc 180
   ccagcacctg aactcctggg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 189
   <211> 278
   <212> PRT
   <213> Artificial Sequence
<220>
   <223> Melanocortin receptor binding alpha-MSH mimetibody with secretory signal encoded by an in vitro synthesized DNA (p3218).
<400> 189
   Met Ala Trp Val Trp Thr Leu Leu Phe Leu Met Ala Ala Ala Gln Ser
    1               5                  10                  15
   Ile Gln Ala Ser Tyr Cys Met Glu His Phe Arg Trp Gly Cys Pro Val
               20                  25                  30
   Gly Ser Gly Gly Gly Ser Gly Thr Leu Val Ala Val Ser Ser Glu Pro
           35                  40                  45
   Lys Ser Ala Asp Lys Thr His Ala Cys Pro Pro Cys Pro Ala Pro Glu
       50                  55                  60
   Leu Leu Gly Gly Pro Ser Val Phe Leu Phe Pro Pro Lys Pro Lys Asp
   65                  70                  75                  80
   Thr Leu Met Ile Ser Arg Thr Pro Glu Val Thr Cys Val Val Val Asp
                   85                  90                  95
   Val Ser His Glu Asp Pro Glu Val Lys Phe Asn Trp Tyr Val Asp Gly
               100                 105                 110
   Val Glu Val His Asn Ala Lys Thr Lys Pro Arg Glu Glu Gln Tyr Asn
           115                 120                 125
   Ser Thr Tyr Arg Val Val Ser Val Leu Thr Val Leu His Gln Asp Trp
       130                 135                 140
   Leu Asn Gly Lys Glu Tyr Lys Cys Lys Val Ser Asn Lys Ala Leu Pro
   145                 150                 155                 160
   Ala Pro Ile Glu Lys Thr Ile Ser Lys Ala Lys Gly Gln Pro Arg Glu
                   165                 170                 175
   Pro Gln Val Tyr Thr Leu Pro Pro Ser Arg Asp Glu Leu Thr Lys Asn
               180                 185                 190
   Gln Val Ser Leu Thr Cys Leu Val Lys Gly Phe Tyr Pro Ser Asp Ile
           195                 200                 205
   Ala Val Glu Trp Glu Ser Asn Gly Gln Pro Glu Asn Asn Tyr Lys Thr
       210                 215                 220
   Thr Pro Pro Val Leu Asp Ser Asp Gly Ser Phe Phe Leu Tyr Ser Lys
   225                 230                 235                 240
   Leu Thr Val Asp Lys Ser Arg Trp Gln Gln Gly Asn Val Phe Ser Cys
                   245                 250                 255
   Ser Val Met His Glu Ala Leu His Asn His Tyr Thr Gln Lys Ser Leu
               260                 265                 270
   Ser Leu Ser Pro Gly Lys
           275
<210> 190
   <211> 11969
   <212> DNA
   <213> Artificial Sequence
<220>
   <223> In vitro synthesized DNA with expression vector functions that encodes an alpha-MSH mimetibody (p3218).
<400> 190
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactgcatgg agcacttccg 1680
   ctggggctgc ccggtgggat ccggtggagg ctccggtacc ttagtcgccg tctcctcaga 1740
   gcccaaatct gccgacaaaa ctcacgcctg cccaccgtgc ccagcacctg aactcctggg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaagggggc gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gagggggcct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat                                  11969
<210> 191
   <211> 777
   <212> DNA
   <213> Artificial Sequence
<220>
   <223> In vitro synthesized DNA encoding a melanocortin receptor binding alpha-MSH mimetibody without secretory signal (p3219).
<400> 191
   tcctgctcca tggagcactt ccgctggggc tgcccggtgg gatccggtgg aggctccggt 60
   accttagtcg ccgtctcctc agagcccaaa tctgccgaca aaactcacgc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 192
   <211> 259
   <212> PRT
   <213> Artificial Sequence
<220>
   <223> Melanocortin receptor binding alpha-MSH mimetibody without secretory signal encoded by an in vitro synthesized DNA (p3219).
<400> 192
   Ser Cys Ser Met Glu His Phe Arg Trp Gly Cys Pro Val Gly Ser Gly
    1               5                  10                  15
   Gly Gly Ser Gly Thr Leu Val Ala Val Ser Ser Glu Pro Lys Ser Ala
               20                  25                  30
   Asp Lys Thr His Ala Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 193
   <211> 834
   <212> DNA
   <213> Artificial Sequence
<220>
   <223> In vitro synthesized DNA encoding a melanocortin receptor binding alpha-MSH mimetibody with secretory signal (p3219).
<400> 193
   atggcttggg tgtggacctt gctattcctg atggcggccg cccaaagtat acaggcctcc 60
   tgctccatgg agcacttccg ctggggctgc ccggtgggat ccggtggagg ctccggtacc 120
   ttagtcgccg tctcctcaga gcccaaatct gccgacaaaa ctcacgcctg cccaccgtgc 180
   ccagcacctg aactcctggg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 194
   <211> 278
   <212> PRT
   <213> Artificial Sequence
<220>
   <223> Melanocortin receptor binding alpha-MSH mimetibody with secretory signal encoded by an in vitro synthesized DNA (p3219).
<400> 194
   Met Ala Trp Val Trp Thr Leu Leu Phe Leu Met Ala Ala Ala Gln Ser
    1               5                  10                  15
   Ile Gln Ala Ser Cys Ser Met Glu His Phe Arg Trp Gly Cys Pro Val
               20                  25                  30
   Gly Ser Gly Gly Gly Ser Gly Thr Leu Val Ala Val Ser Ser Glu Pro
           35                  40                  45
   Lys Ser Ala Asp Lys Thr His Ala Cys Pro Pro Cys Pro Ala Pro Glu
       50                  55                  60
   Leu Leu Gly Gly Pro Ser Val Phe Leu Phe Pro Pro Lys Pro Lys Asp
   65                  70                  75                  80
   Thr Leu Met Ile Ser Arg Thr Pro Glu Val Thr Cys Val Val Val Asp
                   85                  90                  95
   Val Ser His Glu Asp Pro Glu Val Lys Phe Asn Trp Tyr Val Asp Gly
               100                 105                 110
   Val Glu Val His Asn Ala Lys Thr Lys Pro Arg Glu Glu Gln Tyr Asn
           115                 120                 125
   Ser Thr Tyr Arg Val Val Ser Val Leu Thr Val Leu His Gln Asp Trp
       130                 135                 140
   Leu Asn Gly Lys Glu Tyr Lys Cys Lys Val Ser Asn Lys Ala Leu Pro
   145                 150                 155                 160
   Ala Pro Ile Glu Lys Thr Ile Ser Lys Ala Lys Gly Gln Pro Arg Glu
                   165                 170                 175
   Pro Gln Val Tyr Thr Leu Pro Pro Ser Arg Asp Glu Leu Thr Lys Asn
               180                 185                 190
   Gln Val Ser Leu Thr Cys Leu Val Lys Gly Phe Tyr Pro Ser Asp Ile
           195                 200                 205
   Ala Val Glu Trp Glu Ser Asn Gly Gln Pro Glu Asn Asn Tyr Lys Thr
       210                 215                 220
   Thr Pro Pro Val Leu Asp Ser Asp Gly Ser Phe Phe Leu Tyr Ser Lys
   225                 230                 235                 240
   Leu Thr Val Asp Lys Ser Arg Trp Gln Gln Gly Asn Val Phe Ser Cys
                   245                 250                 255
   Ser Val Met His Glu Ala Leu His Asn His Tyr Thr Gln Lys Ser Leu
               260                 265                 270
   Ser Leu Ser Pro Gly Lys
           275
<210> 195
   <211> 11969
   <212> DNA
   <213> Artificial Sequence
<220>
   <223> In vitro synthesized DNA with expression vector functions that encodes an alpha-MSH mimetibody (p3219).
<400> 195
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tgctccatgg agcacttccg 1680
   ctggggctgc ccggtgggat ccggtggagg ctccggtacc ttagtcgccg tctcctcaga 1740
   gcccaaatct gccgacaaaa ctcacgcctg cccaccgtgc ccagcacctg aactcctggg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaagggggc gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gagggggcct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat                                  11969
<210> 196
   <211> 777
   <212> DNA
   <213> Artificial Sequence
<220>
   <223> In vitro synthesized DNA encoding a melanocortin receptor binding alpha-MSH mimetibody without secretory signal (p3162).
<400> 196
   tgctactcca tggagcactt ccgctggggc tgcccggtgg gatccggtgg aggctccggt 60
   accttagtcg ccgtctcctc agagcccaaa tctgccgaca aaactcacgc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 197
   <211> 259
   <212> PRT
   <213> Artificial Sequence
<220>
   <223> Melanocortin receptor binding alpha-MSH mimetibody without secretory signal encoded by an in vitro synthesized DNA (p3162).
<400> 197
   Cys Tyr Ser Met Glu His Phe Arg Trp Gly Cys Pro Val Gly Ser Gly
    1               5                  10                  15
   Gly Gly Ser Gly Thr Leu Val Ala Val Ser Ser Glu Pro Lys Ser Ala
               20                  25                  30
   Asp Lys Thr His Ala Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 198
   <211> 834
   <212> DNA
   <213> Artificial Sequence
<220>
   <223> In vitro synthesized DNA encoding a melanocortin receptor binding alpha-MSH mimetibody with secretory signal (p3162).
<400> 198
   atggcttggg tgtggacctt gctattcctg atggcggccg cccaaagtat acaggcctgc 60
   tactccatgg agcacttccg ctggggctgc ccggtgggat ccggtggagg ctccggtacc 120
   ttagtcgccg tctcctcaga gcccaaatct gccgacaaaa ctcacgcctg cccaccgtgc 180
   ccagcacctg aactcctggg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 199
   <211> 278
   <212> PRT
   <213> Artificial Sequence
<220>
   <223> Melanocortin receptor binding alpha-MSH mimetibody with secretory signal encoded by an in vitro synthesized DNA (p3162).
<400> 199
<210> 200
   <211> 11969
   <212> DNA
   <213> Artificial Sequence
<220>
   <223> In vitro synthesized DNA with expression vector functions that encodes an alpha-MSH mimetibody (p3162).
<400> 200
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctgc tactccatgg agcacttccg 1680
   ctggggctgc ccggtgggat ccggtggagg ctccggtacc ttagtcgccg tctcctcaga 1740
   gcccaaatct gccgacaaaa ctcacgcctg cccaccgtgc ccagcacctg aactcctggg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaagggggc gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gagggggcct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat                                  11969
<210> 201
   <211> 720
   <212> DNA
   <213> Artificial Sequence
<220>
   <223> In vitro synthesized DNA encoding a melanocortin receptor binding alpha-MSH mimetibody without secretory signal (p3250).
<400> 201
   tcctactcct gcgagcactt ccgctggtgc aagccggtgg gaggaggcgg atcctgccca 60
   ccgtgcccag cacctgaact cctgggggga ccgtcagtct tcctcttccc cccaaaaccc 120
   aaggacaccc tcatgatctc ccggacccct gaggtcacat gcgtggtggt ggacgtgagc 180
   cacgaagacc ctgaggtcaa gttcaactgg tacgtggacg gcgtggaggt gcataatgcc 240
   aagacaaagc cgcgggagga gcagtacaac agcacgtacc gtgtggtcag cgtcctcacc 300
   gtcctgcacc aggactggct gaatggcaag gagtacaagt gcaaggtctc caacaaagcc 360
   ctcccagccc ccatcgagaa aaccatctcc aaagccaaag ggcagccccg agaaccacag 420
   gtgtacaccc tgcccccatc ccgggatgag ctgaccaaga accaggtcag cctgacctgc 480
   ctggtcaaag gcttctatcc cagcgacatc gccgtggagt gggagagcaa tgggcagccg 540
   gagaacaact acaagaccac gcctcccgtg ctggactccg acggctcctt cttcctctac 600
   agcaagctca ccgtggacaa gagcaggtgg cagcagggga acgtcttctc atgctccgtg 660
   atgcatgagg ctctgcacaa ccactacacg cagaagagcc tctccctgtc tctgggtaaa 720
<210> 202
   <211> 240
   <212> PRT
   <213> Artificial Sequence
<220>
   <223> Melanocortin receptor binding alpha-MSH mimetibody without secretory signal encoded by an in vitro synthesized DNA (p3250).
<400> 202
   Ser Tyr Ser Cys Glu His Phe Arg Trp Cys Lys Pro Val Gly Gly Gly
    1               5                  10                  15
   Gly Ser Cys Pro Pro Cys Pro Ala Pro Glu Leu Leu Gly Gly Pro Ser
               20                  25                  30
   Val Phe Leu Phe Pro Pro Lys Pro Lys Asp Thr Leu Met Ile Ser Arg
           35                  40                  45
   Thr Pro Glu Val Thr Cys Val Val Val Asp Val Ser His Glu Asp Pro
       50                  55                  60
   Glu Val Lys Phe Asn Trp Tyr Val Asp Gly Val Glu Val His Asn Ala
   65                  70                  75                  80
   Lys Thr Lys Pro Arg Glu Glu Gln Tyr Asn Ser Thr Tyr Arg Val Val
                   85                  90                  95
   Ser Val Leu Thr Val Leu His Gln Asp Trp Leu Asn Gly Lys Glu Tyr
               100                 105                 110
   Lys Cys Lys Val Ser Asn Lys Ala Leu Pro Ala Pro Ile Glu Lys Thr
           115                 120                 125
   Ile Ser Lys Ala Lys Gly Gln Pro Arg Glu Pro Gln Val Tyr Thr Leu
       130                 135                 140
   Pro Pro Ser Arg Asp Glu Leu Thr Lys Asn Gln Val Ser Leu Thr Cys
   145                 150                 155                 160
   Leu Val Lys Gly Phe Tyr Pro Ser Asp Ile Ala Val Glu Trp Glu Ser
                   165                 170                 175
   Asn Gly Gln Pro Glu Asn Asn Tyr Lys Thr Thr Pro Pro Val Leu Asp
               180                 185                 190
   Ser Asp Gly Ser Phe Phe Leu Tyr Ser Lys Leu Thr Val Asp Lys Ser
           195                 200                 205
   Arg Trp Gln Gln Gly Asn Val Phe Ser Cys Ser Val Met His Glu Ala
       210                 215                 220
   Leu His Asn His Tyr Thr Gln Lys Ser Leu Ser Leu Ser Leu Gly Lys
   225                 230                 235                 240
<210> 203
   <211> 777
   <212> DNA
   <213> Artificial Sequence
<220>
   <223> In vitro synthesized DNA encoding a melanocortin receptor binding alpha-MSH mimetibody with secretory signal (p3250).
<400> 203
   atggcttggg tgtggacctt gctattcctg atggcggccg cccaaagtat acaggcctcc 60
   tactcctgcg agcacttccg ctggtgcaag ccggtgggag gaggcggatc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctct gggtaaa    777
<210> 204
   <211> 259
   <212> PRT
   <213> Artificial Sequence
<220>
   <223> Melanocortin receptor binding alpha-MSH mimetibody with secretory signal encoded by an in vitro synthesized DNA (p3250).
<400> 204
   Met Ala Trp Val Trp Thr Leu Leu Phe Leu Met Ala Ala Ala Gln Ser
    1              5                   10                  15
   Ile Gln Ala Ser Tyr Ser Cys Glu His Phe Arg Trp Cys Lys Pro Val
               20                  25                  30
   Gly Gly Gly Gly Ser Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Leu Gly Lys
<210> 205
   <211> 11912
   <212> DNA
   <213> Artificial Sequence
<220>
   <223> In vitro synthesized DNA with expression vector functions that encodes an alpha-MSH mimetibody (p3250).
<400> 205
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactcctgcg agcacttccg 1680
   ctggtgcaag ccggtgggag gaggcggatc ctgcccaccg tgcccagcac ctgaactcct 1740
   ggggggaccg tcagtcttcc tcttcccccc aaaacccaag gacaccctca tgatctcccg 1800
   gacccctgag gtcacatgcg tggtggtgga cgtgagccac gaagaccctg aggtcaagtt 1860
   caactggtac gtggacggcg tggaggtgca taatgccaag acaaagccgc gggaggagca 1920
   gtacaacagc acgtaccgtg tggtcagcgt cctcaccgtc ctgcaccagg actggctgaa 1980
   tggcaaggag tacaagtgca aggtctccaa caaagccctc ccagccccca tcgagaaaac 2040
   catctccaaa gccaaagggc agccccgaga accacaggtg tacaccctgc ccccatcccg 2100
   ggatgagctg accaagaacc aggtcagcct gacctgcctg gtcaaaggct tctatcccag 2160
   cgacatcgcc gtggagtggg agagcaatgg gcagccggag aacaactaca agaccacgcc 2220
   tcccgtgctg gactccgacg gctccttctt cctctacagc aagctcaccg tggacaagag 2280
   caggtggcag caggggaacg tcttctcatg ctccgtgatg catgaggctc tgcacaacca 2340
   ctacacgcag aagagcctct ccctgtctct gggtaaatga gggcccgttt aaacagatcc 2400
   agacatgata agatacattg atgagtttgg acaaaccaca actagaatgc agtgaaaaaa 2460
   atgctttatt tgtgaaattt gtgatgctat tgctttattt gtaaccatta taagctgcaa 2520
   taaacaagtt aacaacaaca attgcattca ttttatgttt caggttcagg gggaggtgtg 2580
   ggaggttttt taaagcaagt aaaacctcta caaatgtggt atggctgatt atgatccggc 2640
   tgcctcgcgc gtttcggtga tgacggtgaa aacctctgac acatgcagct cccggagacg 2700
   gtcacagctt gtctgtaagc ggatgccggg agcagacaag cccgtcaggc gtcagcgggt 2760
   gttggcgggt gtcggggcgc agccatgagg tcgactctag aggatcgatg ccccgccccg 2820
   gacgaactaa acctgactac gacatctctg ccccttcttc gcggggcagt gcatgtaatc 2880
   ccttcagttg gttggtacaa cttgccaact gggccctgtt ccacatgtga cacggggggg 2940
   gaccaaacac aaaggggttc tctgactgta gttgacatcc ttataaatgg atgtgcacat 3000
   ttgccaacac tgagtggctt tcatcctgga gcagactttg cagtctgtgg actgcaacac 3060
   aacattgcct ttatgtgtaa ctcttggctg aagctcttac accaatgctg ggggacatgt 3120
   acctcccagg ggcccaggaa gactacggga ggctacacca acgtcaatca gaggggcctg 3180
   tgtagctacc gataagcgga ccctcaagag ggcattagca atagtgttta taaggccccc 3240
   ttgttaaccc taaacgggta gcatatgctt cccgggtagt agtatatact atccagacta 3300
   accctaattc aatagcatat gttacccaac gggaagcata tgctatcgaa ttagggttag 3360
   taaaagggtc ctaaggaaca gcgatatctc ccaccccatg agctgtcacg gttttattta 3420
   catggggtca ggattccacg agggtagtga accattttag tcacaagggc agtggctgaa 3480
   gatcaaggag cgggcagtga actctcctga atcttcgcct gcttcttcat tctccttcgt 3540
   ttagctaata gaataactgc tgagttgtga acagtaaggt gtatgtgagg tgctcgaaaa 3600
   caaggtttca ggtgacgccc ccagaataaa atttggacgg ggggttcagt ggtggcattg 3660
   tgctatgaca ccaatataac cctcacaaac cccttgggca ataaatacta gtgtaggaat 3720
   gaaacattct gaatatcttt aacaatagaa atccatgggg tggggacaag ccgtaaagac 3780
   tggatgtcca tctcacacga atttatggct atgggcaaca cataatccta gtgcaatatg 3840
   atactggggt tattaagatg tgtcccaggc agggaccaag acaggtgaac catgttgtta 3900
   cactctattt gtaacaaggg gaaagagagt ggacgccgac agcagcggac tccactggtt 3960
   gtctctaaca cccccgaaaa ttaaacgggg ctccacgcca atggggccca taaacaaaga 4020
   caagtggcca ctcttttttt tgaaattgtg gagtgggggc acgcgtcagc ccccacacgc 4080
   cgccctgcgg ttttggactg taaaataagg gtgtaataac ttggctgatt gtaaccccgc 4140
   taaccactgc ggtcaaacca cttgcccaca aaaccactaa tggcaccccg gggaatacct 4200
   gcataagtag gtgggcgggc caagataggg gcgcgattgc tgcgatctgg aggacaaatt 4260
   acacacactt gcgcctgagc gccaagcaca gggttgttgg tcctcatatt cacgaggtcg 4320
   ctgagagcac ggtgggctaa tgttgccatg ggtagcatat actacccaaa tatctggata 4380
   gcatatgcta tcctaatcta tatctgggta gcataggcta tcctaatcta tatctgggta 4440
   gcatatgcta tcctaatcta tatctgggta gtatatgcta tcctaattta tatctgggta 4500
   gcataggcta tcctaatcta tatctgggta gcatatgcta tcctaatcta tatctgggta 4560
   gtatatgcta tcctaatctg tatccgggta gcatatgcta tcctaataga gattagggta 4620
   gtatatgcta tcctaattta tatctgggta gcatatacta cccaaatatc tggatagcat 4680
   atgctatcct aatctatatc tgggtagcat atgctatcct aatctatatc tgggtagcat 4740
   aggctatcct aatctatatc tgggtagcat atgctatcct aatctatatc tgggtagtat 4800
   atgctatcct aatttatatc tgggtagcat aggctatcct aatctatatc tgggtagcat 4860
   atgctatcct aatctatatc tgggtagtat atgctatcct aatctgtatc cgggtagcat 4920
   atgctatcct catgcatata cagtcagcat atgataccca gtagtagagt gggagtgcta 4980
   tcctttgcat atgccgccac ctcccaaggg ggcgtgaatt ttcgctgctt gtccttttcc 5040
   tgctggttgc tcccattctt aggtgaattt aaggaggcca ggctaaagcc gtcgcatgtc 5100
   tgattgctca ccaggtaaat gtcgctaatg ttttccaacg cgagaaggtg ttgagcgcgg 5160
   agctgagtga cgtgacaaca tgggtatgcc caattgcccc atgttgggag gacgaaaatg 5220
   gtgacaagac agatggccag aaatacacca acagcacgca tgatgtctac tggggattta 5280
   ttctttagtg cgggggaata cacggctttt aatacgattg agggcgtctc ctaacaagtt 5340
   acatcactcc tgcccttcct caccctcatc tccatcacct ccttcatctc cgtcatctcc 5400
   gtcatcaccc tccgcggcag ccccttccac cataggtgga aaccagggag gcaaatctac 5460
   tccatcgtca aagctgcaca cagtcaccct gatattgcag gtaggagcgg gctttgtcat 5520
   aacaaggtcc ttaatcgcat ccttcaaaac ctcagcaaat atatgagttt gtaaaaagac 5580
   catgaaataa cagacaatgg actcccttag cgggccaggt tgtgggccgg gtccaggggc 5640
   cattccaaag gggagacgac tcaatggtgt aagacgacat tgtggaatag caagggcagt 5700
   tcctcgcctt aggttgtaaa gggaggtctt actacctcca tatacgaaca caccggcgac 5760
   ccaagttcct tcgtcggtag tcctttctac gtgactccta gccaggagag ctcttaaacc 5820
   ttctgcaatg ttctcaaatt tcgggttgga acctccttga ccacgatgct tttccaaacc 5880
   accctccttt tttgcgccct gcctccatca ccctgacccc ggggtccagt gcttgggcct 5940
   tctcctgggt catctgcggg gccctgctct atcgctcccg ggggcacgtc aggctcacca 6000
   tctgggccac cttcttggtg gtattcaaaa taatcggctt cccctacagg gtggaaaaat 6060
   ggccttctac ctggaggggg cctgcgcggt ggagacccgg atgatgatga ctgactactg 6120
   ggactcctgg gcctcttttc tccacgtcca cgacctctcc ccctggctct ttcacgactt 6180
   ccccccctgg ctctttcacg tcctctaccc cggcggcctc cactacctcc tcgaccccgg 6240
   cctccactac ctcctcgacc ccggcctcca ctgcctcctc gaccccggcc tccacctcct 6300
   gctcctgccc ctcctgctcc tgcccctcct cctgctcctg cccctcctgc ccctcctgct 6360
   cctgcccctc ctgcccctcc tgctcctgcc cctcctgccc ctcctgctcc tgcccctcct 6420
   gcccctcctc ctgctcctgc ccctcctgcc cctcctcctg ctcctgcccc tcctgcccct 6480
   cctgctcctg cccctcctgc ccctcctgct cctgcccctc ctgcccctcc tgctcctgcc 6540
   cctcctgctc ctgcccctcc tgctcctgcc cctcctgctc ctgcccctcc tgcccctcct 6600
   gcccctcctc ctgctcctgc ccctcctgct cctgcccctc ctgcccctcc tgcccctcct 6660
   gctcctgccc ctcctcctgc tcctgcccct cctgcccctc ctgcccctcc tcctgctcct 6720
   gcccctcctg cccctcctcc tgctcctgcc cctcctcctg ctcctgcccc tcctgcccct 6780
   cctgcccctc ctcctgctcc tgcccctcct gcccctcctc ctgctcctgc ccctcctcct 6840
   gctcctgccc ctcctgcccc tcctgcccct cctcctgctc ctgcccctcc tcctgctcct 6900
   gcccctcctg cccctcctgc ccctcctgcc cctcctcctg ctcctgcccc tcctcctgct 6960
   cctgcccctc ctgctcctgc ccctcccgct cctgctcctg ctcctgttcc accgtgggtc 7020
   cctttgcagc caatgcaact tggacgtttt tggggtctcc ggacaccatc tctatgtctt 7080
   ggccctgatc ctgagccgcc cggggctcct ggtcttccgc ctcctcgtcc tcgtcctctt 7140
   ccccgtcctc gtccatggtt atcaccccct cttctttgag gtccactgcc gccggagcct 7200
   tctggtccag atgtgtctcc cttctctcct aggccatttc caggtcctgt acctggcccc 7260
   tcgtcagaca tgattcacac taaaagagat caatagacat ctttattaga cgacgctcag 7320
   tgaatacagg gagtgcagac tcctgccccc tccaacagcc cccccaccct catccccttc 7380
   atggtcgctg tcagacagat ccaggtctga aaattcccca tcctccgaac catcctcgtc 7440
   ctcatcacca attactcgca gcccggaaaa ctcccgctga acatcctcaa gatttgcgtc 7500
   ctgagcctca agccaggcct caaattcctc gtcccccttt ttgctggacg gtagggatgg 7560
   ggattctcgg gacccctcct cttcctcttc aaggtcacca gacagagatg ctactggggc 7620
   aacggaagaa aagctgggtg cggcctgtga ggatcagctt atcgatgata agctgtcaaa 7680
   catgagaatt cttgaagacg aaagggcctc gtgatacgcc tatttttata ggttaatgtc 7740
   atgataataa tggtttctta gacgtcaggt ggcacttttc ggggaaatgt gcgcggaacc 7800
   cctatttgtt tatttttcta aatacattca aatatgtatc cgctcatgag acaataaccc 7860
   tgataaatgc ttcaataata ttgaaaaagg aagagtatga gtattcaaca tttccgtgtc 7920
   gcccttattc ccttttttgc ggcattttgc cttcctgttt ttgctcaccc agaaacgctg 7980
   gtgaaagtaa aagatgctga agatcagttg ggtgcacgag tgggttacat cgaactggat 8040
   ctcaacagcg gtaagatcct tgagagtttt cgccccgaag aacgttttcc aatgatgagc 8100
   acttttaaag ttctgctatg tggcgcggta ttatcccgtg ttgacgccgg gcaagagcaa 8160
   ctcggtcgcc gcatacacta ttctcagaat gacttggttg agtactcacc agtcacagaa 8220
   aagcatctta cggatggcat gacagtaaga gaattatgca gtgctgccat aaccatgagt 8280
   gataacactg cggccaactt acttctgaca acgatcggag gaccgaagga gctaaccgct 8340
   tttttgcaca acatggggga tcatgtaact cgccttgatc gttgggaacc ggagctgaat 8400
   gaagccatac caaacgacga gcgtgacacc acgatgcctg cagcaatggc aacaacgttg 8460
   cgcaaactat taactggcga actacttact ctagcttccc ggcaacaatt aatagactgg 8520
   atggaggcgg ataaagttgc aggaccactt ctgcgctcgg cccttccggc tggctggttt 8580
   attgctgata aatctggagc cggtgagcgt gggtctcgcg gtatcattgc agcactgggg 8640
   ccagatggta agccctcccg tatcgtagtt atctacacga cggggagtca ggcaactatg 8700
   gatgaacgaa atagacagat cgctgagata ggtgcctcac tgattaagca ttggtaactg 8760
   tcagaccaag tttactcata tatactttag attgatttaa aacttcattt ttaatttaaa 8820
   aggatctagg tgaagatcct ttttgataat ctcatgacca aaatccctta acgtgagttt 8880
   tcgttccact gagcgtcaga ccccgtagaa aagatcaaag gatcttcttg agatcctttt 8940
   tttctgcgcg taatctgctg cttgcaaaca aaaaaaccac cgctaccagc ggtggtttgt 9000
   ttgccggatc aagagctacc aactcttttt ccgaaggtaa ctggcttcag cagagcgcag 9060
   ataccaaata ctgtccttct agtgtagccg tagttaggcc accacttcaa gaactctgta 9120
   gcaccgccta catacctcgc tctgctaatc ctgttaccag tggctgctgc cagtggcgat 9180
   aagtcgtgtc ttaccgggtt ggactcaaga cgatagttac cggataaggc gcagcggtcg 9240
   ggctgaacgg ggggttcgtg cacacagccc agcttggagc gaacgaccta caccgaactg 9300
   agatacctac agcgtgagct atgagaaagc gccacgcttc ccgaagggag aaaggcggac 9360
   aggtatccgg taagcggcag ggtcggaaca ggagagcgca cgagggagct tccaggggga 9420
   aacgcctggt atctttatag tcctgtcggg tttcgccacc tctgacttga gcgtcgattt 9480
   ttgtgatgct cgtcaggggg gcggagccta tggaaaaacg ccagcaacgc ggccttttta 9540
   cggttcctgg ccttttgctg gccttgaagc tgtccctgat ggtcgtcatc tacctgcctg 9600
   gacagcatgg cctgcaacgc gggcatcccg atgccgccgg aagcgagaag aatcataatg 9660
   gggaaggcca tccagcctcg cgtcgcgaac gccagcaaga cgtagcccag cgcgtcggcc 9720
   ccgagatgcg ccgcgtgcgg ctgctggaga tggcggacgc gatggatatg ttctgccaag 9780
   ggttggtttg cgcattcaca gttctccgca agaattgatt ggctccaatt cttggagtgg 9840
   tgaatccgtt agcgaggtgc cgccctgctt catccccgtg gcccgttgct cgcgtttgct 9900
   ggcggtgtcc ccggaagaaa tatatttgca tgtctttagt tctatgatga cacaaacccc 9960
   gcccagcgtc ttgtcattgg cgaattcgaa cacgcagatg cagtcggggc ggcgcggtcc 10020
   gaggtccact tcgcatatta aggtgacgcg tgtggcctcg aacaccgagc gaccctgcag 10080
   cgacccgctt aacagcgtca acagcgtgcc gcagatcccg gggggcaatg agatatgaaa 10140
   aagcctgaac tcaccgcgac gtctgtcgag aagtttctga tcgaaaagtt cgacagcgtc 10200
   tccgacctga tgcagctctc ggagggcgaa gaatctcgtg ctttcagctt cgatgtagga 10260
   gggcgtggat atgtcctgcg ggtaaatagc tgcgccgatg gtttctacaa agatcgttat 10320
   gtttatcggc actttgcatc ggccgcgctc ccgattccgg aagtgcttga cattggggaa 10380
   ttcagcgaga gcctgaccta ttgcatctcc cgccgtgcac agggtgtcac gttgcaagac 10440
   ctgcctgaaa ccgaactgcc cgctgttctg cagccggtcg cggaggccat ggatgcgatc 10500
   gctgcggccg atcttagcca gacgagcggg ttcggcccat tcggaccgca aggaatcggt 10560
   caatacacta catggcgtga tttcatatgc gcgattgctg atccccatgt gtatcactgg 10620
   caaactgtga tggacgacac cgtcagtgcg tccgtcgcgc aggctctcga tgagctgatg 10680
   ctttgggccg aggactgccc cgaagtccgg cacctcgtgc acgcggattt cggctccaac 10740
   aatgtcctga cggacaatgg ccgcataaca gcggtcattg actggagcga ggcgatgttc 10800
   ggggattccc aatacgaggt cgccaacatc ttcttctgga ggccgtggtt ggcttgtatg 10860
   gagcagcaga cgcgctactt cgagcggagg catccggagc ttgcaggatc gccgcggctc 10920
   cgggcgtata tgctccgcat tggtcttgac caactctatc agagcttggt tgacggcaat 10980
   ttcgatgatg cagcttgggc gcagggtcga tgcgacgcaa tcgtccgatc cggagccggg 11040
   actgtcgggc gtacacaaat cgcccgcaga agcgcggccg tctggaccga tggctgtgta 11100
   gaagtactcg ccgatagtgg aaaccgacgc cccagcactc gtccggatcg ggagatgggg 11160
   gaggctaact gaaacacgga aggagacaat accggaagga acccgcgcta tgacggcaat 11220
   aaaaagacag aataaaacgc acgggtgttg ggtcgtttgt tcataaacgc ggggttcggt 11280
   cccagggctg gcactctgtc gataccccac cgagacccca ttggggccaa tacgcccgcg 11340
   tttcttcctt ttccccaccc caccccccaa gttcgggtga aggcccaggg ctcgcagcca 11400
   acgtcggggc ggcaggccct gccatagcca ctggccccgt gggttaggga cggggtcccc 11460
   catggggaat ggtttatggt tcgtgggggt tattattttg ggcgttgcgt ggggtcaggt 11520
   ccacgactgg actgagcaga cagacccatg gtttttggat ggcctgggca tggaccgcat 11580
   gtactggcgc gacacgaaca ccgggcgtct gtggctgcca aacacccccg acccccaaaa 11640
   accaccgcgc ggatttctgg cgtgccaagc tagtcgacca attctcatgt ttgacagctt 11700
   atcatcgcag atccgggcaa cgttgttgcc attgctgcag gcgcagaact ggtaggtatg 11760
   gaagatctat acattgaatc aatattggca attagccata ttagtcattg gttatatagc 11820
   ataaatcaat attggctatt ggccattgca tacgttgtat ctatatcata atatgtacat 11880
   ttatattggc tcatgtccaa tatgaccgcc at                               11912
<210> 206
   <211> 735
   <212> DNA
   <213> Artificial Sequence
<220>
   <223> In vitro synthesized DNA encoding a melanocortin receptor binding alpha-MSH mimetibody without secretory signal (p3251).
<400> 206
   tcctactcct gcgagcactt ccgctggtgc aagccggtgg gaggcggagg aagcggcgga 60
   ggcggatcct gcccaccgtg cccagcacct gaactcctgg ggggaccgtc agtcttcctc 120
   ttccccccaa aacccaagga caccctcatg atctcccgga cccctgaggt cacatgcgtg 180
   gtggtggacg tgagccacga agaccctgag gtcaagttca actggtacgt ggacggcgtg 240
   gaggtgcata atgccaagac aaagccgcgg gaggagcagt acaacagcac gtaccgtgtg 300
   gtcagcgtcc tcaccgtcct gcaccaggac tggctgaatg gcaaggagta caagtgcaag 360
   gtctccaaca aagccctccc agcccccatc gagaaaacca tctccaaagc caaagggcag 420
   ccccgagaac cacaggtgta caccctgccc ccatcccggg atgagctgac caagaaccag 480
   gtcagcctga cctgcctggt caaaggcttc tatcccagcg acatcgccgt ggagtgggag 540
   agcaatgggc agccggagaa caactacaag accacgcctc ccgtgctgga ctccgacggc 600
   tccttcttcc tctacagcaa gctcaccgtg gacaagagca ggtggcagca ggggaacgtc 660
   ttctcatgct ccgtgatgca tgaggctctg cacaaccact acacgcagaa gagcctctcc 720
   ctgtctctgg gtaaa                                                  735
<210> 207
   <211> 245
   <212> PRT
   <213> Artificial Sequence
<220>
   <223> Melanocortin receptor binding alpha-MSH mimetibody without secretory signal encoded by an in vitro synthesized DNA (p3251).
<400> 207
   Ser Tyr Ser Cys Glu His Phe Arg Trp Cys Lys Pro Val Gly Gly Gly
    1               5                  10                  15
   Gly Ser Gly Gly Gly Gly Ser Cys Pro Pro Cys Pro Ala Pro Glu Leu
               20                  25                  30
   Leu Gly Gly Pro Ser Val Phe Leu Phe Pro Pro Lys Pro Lys Asp Thr
           35                  40                  45
   Leu Met Ile Ser Arg Thr Pro Glu Val Thr Cys Val Val Val Asp Val
       50                  55                  60
   Ser His Glu Asp Pro Glu Val Lys Phe Asn Trp Tyr Val Asp Gly Val
   65                  70                  75                  80
   Glu Val His Asn Ala Lys Thr Lys Pro Arg Glu Glu Gln Tyr Asn Ser
                   85                  90                  95
   Thr Tyr Arg Val Val Ser Val Leu Thr Val Leu His Gln Asp Trp Leu
               100                 105                 110
   Asn Gly Lys Glu Tyr Lys Cys Lys Val Ser Asn Lys Ala Leu Pro Ala
           115                 120                 125
   Pro Ile Glu Lys Thr Ile Ser Lys Ala Lys Gly Gln Pro Arg Glu Pro
       130                 135                 140
   Gln Val Tyr Thr Leu Pro Pro Ser Arg Asp Glu Leu Thr Lys Asn Gln
   145                 150                 155                 160
   Val Ser Leu Thr Cys Leu Val Lys Gly Phe Tyr Pro Ser Asp Ile Ala
                   165                 170                 175
   Val Glu Trp Glu Ser Asn Gly Gln Pro Glu Asn Asn Tyr Lys Thr Thr
               180                 185                 190
   Pro Pro Val Leu Asp Ser Asp Gly Ser Phe Phe Leu Tyr Ser Lys Leu
           195                 200                 205
   Thr Val Asp Lys Ser Arg Trp Gln Gln Gly Asn Val Phe Ser Cys Ser
       210                 215                 220
   Val Met His Glu Ala Leu His Asn His Tyr Thr Gln Lys Ser Leu Ser
   225                 230                 235                 240
   Leu Ser Leu Gly Lys
                   245
<210> 208
   <211> 792
   <212> DNA
   <213> Artificial Sequence
<220>
   <223> In vitro synthesized DNA encoding a melanocortin receptor binding alpha-MSH mimetibody with secretory signal (p3251).
<400> 208
   atggcttggg tgtggacctt gctattcctg atggcggccg cccaaagtat acaggcctcc 60
   tactcctgcg agcacttccg ctggtgcaag ccggtgggag gcggaggaag cggcggaggc 120
   ggatcctgcc caccgtgccc agcacctgaa ctcctggggg gaccgtcagt cttcctcttc 180
   cccccaaaac ccaaggacac cctcatgatc tcccggaccc ctgaggtcac atgcgtggtg 240
   gtggacgtga gccacgaaga ccctgaggtc aagttcaact ggtacgtgga cggcgtggag 300
   gtgcataatg ccaagacaaa gccgcgggag gagcagtaca acagcacgta ccgtgtggtc 360
   agcgtcctca ccgtcctgca ccaggactgg ctgaatggca aggagtacaa gtgcaaggtc 420
   tccaacaaag ccctcccagc ccccatcgag aaaaccatct ccaaagccaa agggcagccc 480
   cgagaaccac aggtgtacac cctgccccca tcccgggatg agctgaccaa gaaccaggtc 540
   agcctgacct gcctggtcaa aggcttctat cccagcgaca tcgccgtgga gtgggagagc 600
   aatgggcagc cggagaacaa ctacaagacc acgcctcccg tgctggactc cgacggctcc 660
   ttcttcctct acagcaagct caccgtggac aagagcaggt ggcagcaggg gaacgtcttc 720
   tcatgctccg tgatgcatga ggctctgcac aaccactaca cgcagaagag cctctccctg 780
   tctctgggta aa                                                     792
<210> 209
   <211> 264
   <212> PRT
   <213> Artificial Sequence
<220>
   <223> Melanocortin receptor binding alpha-MSH mimetibody with secretory signal encoded by an in vitro synthesized DNA (p3251).
<400> 209
   Met Ala Trp Val Trp Thr Leu Leu Phe Leu Met Ala Ala Ala Gln Ser
    1               5                  10                  15
   Ile Gln Ala Ser Tyr Ser Cys Glu His Phe Arg Trp Cys Lys Pro Val
               20                  25                  30
   Gly Gly Gly Gly Ser Gly Gly Gly Gly Ser Cys Pro Pro Cys Pro Ala
           35                  40                  45
   Pro Glu Leu Leu Gly Gly Pro Ser Val Phe Leu Phe Pro Pro Lys Pro
       50                  55                  60
   Lys Asp Thr Leu Met Ile Ser Arg Thr Pro Glu Val Thr Cys Val Val
   65                  70                  75                  80
   Val Asp Val Ser His Glu Asp Pro Glu Val Lys Phe Asn Trp Tyr Val
                   85                  90                  95
   Asp Gly Val Glu Val His Asn Ala Lys Thr Lys Pro Arg Glu Glu Gln
               100                 105                 110
   Tyr Asn Ser Thr Tyr Arg Val Val Ser Val Leu Thr Val Leu His Gln
           115                 120                 125
   Asp Trp Leu Asn Gly Lys Glu Tyr Lys Cys Lys Val Ser Asn Lys Ala
       130                 135                 140
   Leu Pro Ala Pro Ile Glu Lys Thr Ile Ser Lys Ala Lys Gly Gln Pro
   145                 150                 155                 160
   Arg Glu Pro Gln Val Tyr Thr Leu Pro Pro Ser Arg Asp Glu Leu Thr
                   165                 170                 175
   Lys Asn Gln Val Ser Leu Thr Cys Leu Val Lys Gly Phe Tyr Pro Ser
               180                 185                 190
   Asp Ile Ala Val Glu Trp Glu Ser Asn Gly Gln Pro Glu Asn Asn Tyr
           195                 200                 205
   Lys Thr Thr Pro Pro Val Leu Asp Ser Asp Gly Ser Phe Phe Leu Tyr
       210                 215                 220
   Ser Lys Leu Thr Val Asp Lys Ser Arg Trp Gln Gln Gly Asn Val Phe
   225                 230                 235                 240
   Ser Cys Ser Val Met His Glu Ala Leu His Asn His Tyr Thr Gln Lys
                   245                 250                 255
   Ser Leu Ser Leu Ser Leu Gly Lys
               260
<210> 210
   <211> 11927
   <212> DNA
   <213> Artificial Sequence
<220>
   <223> In vitro synthesized DNA with expression vector functions that encodes an alpha-MSH mimetibody (p3251).
<400> 210
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactcctgcg agcacttccg 1680
   ctggtgcaag ccggtgggag gcggaggaag cggcggaggc ggatcctgcc caccgtgccc 1740
   agcacctgaa ctcctggggg gaccgtcagt cttcctcttc cccccaaaac ccaaggacac 1800
   cctcatgatc tcccggaccc ctgaggtcac atgcgtggtg gtggacgtga gccacgaaga 1860
   ccctgaggtc aagttcaact ggtacgtgga cggcgtggag gtgcataatg ccaagacaaa 1920
   gccgcgggag gagcagtaca acagcacgta ccgtgtggtc agcgtcctca ccgtcctgca 1980
   ccaggactgg ctgaatggca aggagtacaa gtgcaaggtc tccaacaaag ccctcccagc 2040
   ccccatcgag aaaaccatct ccaaagccaa agggcagccc cgagaaccac aggtgtacac 2100
   cctgccccca tcccgggatg agctgaccaa gaaccaggtc agcctgacct gcctggtcaa 2160
   aggcttctat cccagcgaca tcgccgtgga gtgggagagc aatgggcagc cggagaacaa 2220
   ctacaagacc acgcctcccg tgctggactc cgacggctcc ttcttcctct acagcaagct 2280
   caccgtggac aagagcaggt ggcagcaggg gaacgtcttc tcatgctccg tgatgcatga 2340
   ggctctgcac aaccactaca cgcagaagag cctctccctg tctctgggta aatgagggcc 2400
   cgtttaaaca gatccagaca tgataagata cattgatgag tttggacaaa ccacaactag 2460
   aatgcagtga aaaaaatgct ttatttgtga aatttgtgat gctattgctt tatttgtaac 2520
   cattataagc tgcaataaac aagttaacaa caacaattgc attcatttta tgtttcaggt 2580
   tcagggggag gtgtgggagg ttttttaaag caagtaaaac ctctacaaat gtggtatggc 2640
   tgattatgat ccggctgcct cgcgcgtttc ggtgatgacg gtgaaaacct ctgacacatg 2700
   cagctcccgg agacggtcac agcttgtctg taagcggatg ccgggagcag acaagcccgt 2760
   caggcgtcag cgggtgttgg cgggtgtcgg ggcgcagcca tgaggtcgac tctagaggat 2820
   cgatgccccg ccccggacga actaaacctg actacgacat ctctgcccct tcttcgcggg 2880
   gcagtgcatg taatcccttc agttggttgg tacaacttgc caactgggcc ctgttccaca 2940
   tgtgacacgg ggggggacca aacacaaagg ggttctctga ctgtagttga catccttata 3000
   aatggatgtg cacatttgcc aacactgagt ggctttcatc ctggagcaga ctttgcagtc 3060
   tgtggactgc aacacaacat tgcctttatg tgtaactctt ggctgaagct cttacaccaa 3120
   tgctggggga catgtacctc ccaggggccc aggaagacta cgggaggcta caccaacgtc 3180
   aatcagaggg gcctgtgtag ctaccgataa gcggaccctc aagagggcat tagcaatagt 3240
   gtttataagg cccccttgtt aaccctaaac gggtagcata tgcttcccgg gtagtagtat 3300
   atactatcca gactaaccct aattcaatag catatgttac ccaacgggaa gcatatgcta 3360
   tcgaattagg gttagtaaaa gggtcctaag gaacagcgat atctcccacc ccatgagctg 3420
   tcacggtttt atttacatgg ggtcaggatt ccacgagggt agtgaaccat tttagtcaca 3480
   agggcagtgg ctgaagatca aggagcgggc agtgaactct cctgaatctt cgcctgcttc 3540
   ttcattctcc ttcgtttagc taatagaata actgctgagt tgtgaacagt aaggtgtatg 3600
   tgaggtgctc gaaaacaagg tttcaggtga cgcccccaga ataaaatttg gacggggggt 3660
   tcagtggtgg cattgtgcta tgacaccaat ataaccctca caaacccctt gggcaataaa 3720
   tactagtgta ggaatgaaac attctgaata tctttaacaa tagaaatcca tggggtgggg 3780
   acaagccgta aagactggat gtccatctca cacgaattta tggctatggg caacacataa 3840
   tcctagtgca atatgatact ggggttatta agatgtgtcc caggcaggga ccaagacagg 3900
   tgaaccatgt tgttacactc tatttgtaac aaggggaaag agagtggacg ccgacagcag 3960
   cggactccac tggttgtctc taacaccccc gaaaattaaa cggggctcca cgccaatggg 4020
   gcccataaac aaagacaagt ggccactctt ttttttgaaa ttgtggagtg ggggcacgcg 4080
   tcagccccca cacgccgccc tgcggttttg gactgtaaaa taagggtgta ataacttggc 4140
   tgattgtaac cccgctaacc actgcggtca aaccacttgc ccacaaaacc actaatggca 4200
   ccccggggaa tacctgcata agtaggtggg cgggccaaga taggggcgcg attgctgcga 4260
   tctggaggac aaattacaca cacttgcgcc tgagcgccaa gcacagggtt gttggtcctc 4320
   atattcacga ggtcgctgag agcacggtgg gctaatgttg ccatgggtag catatactac 4380
   ccaaatatct ggatagcata tgctatccta atctatatct gggtagcata ggctatccta 4440
   atctatatct gggtagcata tgctatccta atctatatct gggtagtata tgctatccta 4500
   atttatatct gggtagcata ggctatccta atctatatct gggtagcata tgctatccta 4560
   atctatatct gggtagtata tgctatccta atctgtatcc gggtagcata tgctatccta 4620
   atagagatta gggtagtata tgctatccta atttatatct gggtagcata tactacccaa 4680
   atatctggat agcatatgct atcctaatct atatctgggt agcatatgct atcctaatct 4740
   atatctgggt agcataggct atcctaatct atatctgggt agcatatgct atcctaatct 4800
   atatctgggt agtatatgct atcctaattt atatctgggt agcataggct atcctaatct 4860
   atatctgggt agcatatgct atcctaatct atatctgggt agtatatgct atcctaatct 4920
   gtatccgggt agcatatgct atcctcatgc atatacagtc agcatatgat acccagtagt 4980
   agagtgggag tgctatcctt tgcatatgcc gccacctccc aagggggcgt gaattttcgc 5040
   tgcttgtcct tttcctgctg gttgctccca ttcttaggtg aatttaagga ggccaggcta 5100
   aagccgtcgc atgtctgatt gctcaccagg taaatgtcgc taatgttttc caacgcgaga 5160
   aggtgttgag cgcggagctg agtgacgtga caacatgggt atgcccaatt gccccatgtt 5220
   gggaggacga aaatggtgac aagacagatg gccagaaata caccaacagc acgcatgatg 5280
   tctactgggg atttattctt tagtgcgggg gaatacacgg cttttaatac gattgagggc 5340
   gtctcctaac aagttacatc actcctgccc ttcctcaccc tcatctccat cacctccttc 5400
   atctccgtca tctccgtcat caccctccgc ggcagcccct tccaccatag gtggaaacca 5460
   gggaggcaaa tctactccat cgtcaaagct gcacacagtc accctgatat tgcaggtagg 5520
   agcgggcttt gtcataacaa ggtccttaat cgcatccttc aaaacctcag caaatatatg 5580
   agtttgtaaa aagaccatga aataacagac aatggactcc cttagcgggc caggttgtgg 5640
   gccgggtcca ggggccattc caaaggggag acgactcaat ggtgtaagac gacattgtgg 5700
   aatagcaagg gcagttcctc gccttaggtt gtaaagggag gtcttactac ctccatatac 5760
   gaacacaccg gcgacccaag ttccttcgtc ggtagtcctt tctacgtgac tcctagccag 5820
   gagagctctt aaaccttctg caatgttctc aaatttcggg ttggaacctc cttgaccacg 5880
   atgcttttcc aaaccaccct ccttttttgc gccctgcctc catcaccctg accccggggt 5940
   ccagtgcttg ggccttctcc tgggtcatct gcggggccct gctctatcgc tcccgggggc 6000
   acgtcaggct caccatctgg gccaccttct tggtggtatt caaaataatc ggcttcccct 6060
   acagggtgga aaaatggcct tctacctgga gggggcctgc gcggtggaga cccggatgat 6120
   gatgactgac tactgggact cctgggcctc ttttctccac gtccacgacc tctccccctg 6180
   gctctttcac gacttccccc cctggctctt tcacgtcctc taccccggcg gcctccacta 6240
   cctcctcgac cccggcctcc actacctcct cgaccccggc ctccactgcc tcctcgaccc 6300
   cggcctccac ctcctgctcc tgcccctcct gctcctgccc ctcctcctgc tcctgcccct 6360
   cctgcccctc ctgctcctgc ccctcctgcc cctcctgctc ctgcccctcc tgcccctcct 6420
   gctcctgccc ctcctgcccc tcctcctgct cctgcccctc ctgcccctcc tcctgctcct 6480
   gcccctcctg cccctcctgc tcctgcccct cctgcccctc ctgctcctgc ccctcctgcc 6540
   cctcctgctc ctgcccctcc tgctcctgcc cctcctgctc ctgcccctcc tgctcctgcc 6600
   cctcctgccc ctcctgcccc tcctcctgct cctgcccctc ctgctcctgc ccctcctgcc 6660
   cctcctgccc ctcctgctcc tgcccctcct cctgctcctg cccctcctgc ccctcctgcc 6720
   cctcctcctg ctcctgcccc tcctgcccct cctcctgctc ctgcccctcc tcctgctcct 6780
   gcccctcctg cccctcctgc ccctcctcct gctcctgccc ctcctgcccc tcctcctgct 6840
   cctgcccctc ctcctgctcc tgcccctcct gcccctcctg cccctcctcc tgctcctgcc 6900
   cctcctcctg ctcctgcccc tcctgcccct cctgcccctc ctgcccctcc tcctgctcct 6960
   gcccctcctc ctgctcctgc ccctcctgct cctgcccctc ccgctcctgc tcctgctcct 7020
   gttccaccgt gggtcccttt gcagccaatg caacttggac gtttttgggg tctccggaca 7080
   ccatctctat gtcttggccc tgatcctgag ccgcccgggg ctcctggtct tccgcctcct 7140
   cgtcctcgtc ctcttccccg tcctcgtcca tggttatcac cccctcttct ttgaggtcca 7200
   ctgccgccgg agccttctgg tccagatgtg tctcccttct ctcctaggcc atttccaggt 7260
   cctgtacctg gcccctcgtc agacatgatt cacactaaaa gagatcaata gacatcttta 7320
   ttagacgacg ctcagtgaat acagggagtg cagactcctg ccccctccaa cagccccccc 7380
   accctcatcc ccttcatggt cgctgtcaga cagatccagg tctgaaaatt ccccatcctc 7440
   cgaaccatcc tcgtcctcat caccaattac tcgcagcccg gaaaactccc gctgaacatc 7500
   ctcaagattt gcgtcctgag cctcaagcca ggcctcaaat tcctcgtccc cctttttgct 7560
   ggacggtagg gatggggatt ctcgggaccc ctcctcttcc tcttcaaggt caccagacag 7620
   agatgctact ggggcaacgg aagaaaagct gggtgcggcc tgtgaggatc agcttatcga 7680
   tgataagctg tcaaacatga gaattcttga agacgaaagg gcctcgtgat acgcctattt 7740
   ttataggtta atgtcatgat aataatggtt tcttagacgt caggtggcac ttttcgggga 7800
   aatgtgcgcg gaacccctat ttgtttattt ttctaaatac attcaaatat gtatccgctc 7860
   atgagacaat aaccctgata aatgcttcaa taatattgaa aaaggaagag tatgagtatt 7920
   caacatttcc gtgtcgccct tattcccttt tttgcggcat tttgccttcc tgtttttgct 7980
   cacccagaaa cgctggtgaa agtaaaagat gctgaagatc agttgggtgc acgagtgggt 8040
   tacatcgaac tggatctcaa cagcggtaag atccttgaga gttttcgccc cgaagaacgt 8100
   tttccaatga tgagcacttt taaagttctg ctatgtggcg cggtattatc ccgtgttgac 8160
   gccgggcaag agcaactcgg tcgccgcata cactattctc agaatgactt ggttgagtac 8220
   tcaccagtca cagaaaagca tcttacggat ggcatgacag taagagaatt atgcagtgct 8280
   gccataacca tgagtgataa cactgcggcc aacttacttc tgacaacgat cggaggaccg 8340
   aaggagctaa ccgctttttt gcacaacatg ggggatcatg taactcgcct tgatcgttgg 8400
   gaaccggagc tgaatgaagc cataccaaac gacgagcgtg acaccacgat gcctgcagca 8460
   atggcaacaa cgttgcgcaa actattaact ggcgaactac ttactctagc ttcccggcaa 8520
   caattaatag actggatgga ggcggataaa gttgcaggac cacttctgcg ctcggccctt 8580
   ccggctggct ggtttattgc tgataaatct ggagccggtg agcgtgggtc tcgcggtatc 8640
   attgcagcac tggggccaga tggtaagccc tcccgtatcg tagttatcta cacgacgggg 8700
   agtcaggcaa ctatggatga acgaaataga cagatcgctg agataggtgc ctcactgatt 8760
   aagcattggt aactgtcaga ccaagtttac tcatatatac tttagattga tttaaaactt 8820
   catttttaat ttaaaaggat ctaggtgaag atcctttttg ataatctcat gaccaaaatc 8880
   ccttaacgtg agttttcgtt ccactgagcg tcagaccccg tagaaaagat caaaggatct 8940
   tcttgagatc ctttttttct gcgcgtaatc tgctgcttgc aaacaaaaaa accaccgcta 9000
   ccagcggtgg tttgtttgcc ggatcaagag ctaccaactc tttttccgaa ggtaactggc 9060
   ttcagcagag cgcagatacc aaatactgtc cttctagtgt agccgtagtt aggccaccac 9120
   ttcaagaact ctgtagcacc gcctacatac ctcgctctgc taatcctgtt accagtggct 9180
   gctgccagtg gcgataagtc gtgtcttacc gggttggact caagacgata gttaccggat 9240
   aaggcgcagc ggtcgggctg aacggggggt tcgtgcacac agcccagctt ggagcgaacg 9300
   acctacaccg aactgagata cctacagcgt gagctatgag aaagcgccac gcttcccgaa 9360
   gggagaaagg cggacaggta tccggtaagc ggcagggtcg gaacaggaga gcgcacgagg 9420
   gagcttccag ggggaaacgc ctggtatctt tatagtcctg tcgggtttcg ccacctctga 9480
   cttgagcgtc gatttttgtg atgctcgtca ggggggcgga gcctatggaa aaacgccagc 9540
   aacgcggcct ttttacggtt cctggccttt tgctggcctt gaagctgtcc ctgatggtcg 9600
   tcatctacct gcctggacag catggcctgc aacgcgggca tcccgatgcc gccggaagcg 9660
   agaagaatca taatggggaa ggccatccag cctcgcgtcg cgaacgccag caagacgtag 9720
   cccagcgcgt cggccccgag atgcgccgcg tgcggctgct ggagatggcg gacgcgatgg 9780
   atatgttctg ccaagggttg gtttgcgcat tcacagttct ccgcaagaat tgattggctc 9840
   caattcttgg agtggtgaat ccgttagcga ggtgccgccc tgcttcatcc ccgtggcccg 9900
   ttgctcgcgt ttgctggcgg tgtccccgga agaaatatat ttgcatgtct ttagttctat 9960
   gatgacacaa accccgccca gcgtcttgtc attggcgaat tcgaacacgc agatgcagtc 10020
   ggggcggcgc ggtccgaggt ccacttcgca tattaaggtg acgcgtgtgg cctcgaacac 10080
   cgagcgaccc tgcagcgacc cgcttaacag cgtcaacagc gtgccgcaga tcccgggggg 10140
   caatgagata tgaaaaagcc tgaactcacc gcgacgtctg tcgagaagtt tctgatcgaa 10200
   aagttcgaca gcgtctccga cctgatgcag ctctcggagg gcgaagaatc tcgtgctttc 10260
   agcttcgatg taggagggcg tggatatgtc ctgcgggtaa atagctgcgc cgatggtttc 10320
   tacaaagatc gttatgttta tcggcacttt gcatcggccg cgctcccgat tccggaagtg 10380
   cttgacattg gggaattcag cgagagcctg acctattgca tctcccgccg tgcacagggt 10440
   gtcacgttgc aagacctgcc tgaaaccgaa ctgcccgctg ttctgcagcc ggtcgcggag 10500
   gccatggatg cgatcgctgc ggccgatctt agccagacga gcgggttcgg cccattcgga 10560
   ccgcaaggaa tcggtcaata cactacatgg cgtgatttca tatgcgcgat tgctgatccc 10620
   catgtgtatc actggcaaac tgtgatggac gacaccgtca gtgcgtccgt cgcgcaggct 10680
   ctcgatgagc tgatgctttg ggccgaggac tgccccgaag tccggcacct cgtgcacgcg 10740
   gatttcggct ccaacaatgt cctgacggac aatggccgca taacagcggt cattgactgg 10800
   agcgaggcga tgttcgggga ttcccaatac gaggtcgcca acatcttctt ctggaggccg 10860
   tggttggctt gtatggagca gcagacgcgc tacttcgagc ggaggcatcc ggagcttgca 10920
   ggatcgccgc ggctccgggc gtatatgctc cgcattggtc ttgaccaact ctatcagagc 10980
   ttggttgacg gcaatttcga tgatgcagct tgggcgcagg gtcgatgcga cgcaatcgtc 11040
   cgatccggag ccgggactgt cgggcgtaca caaatcgccc gcagaagcgc ggccgtctgg 11100
   accgatggct gtgtagaagt actcgccgat agtggaaacc gacgccccag cactcgtccg 11160
   gatcgggaga tgggggaggc taactgaaac acggaaggag acaataccgg aaggaacccg 11220
   cgctatgacg gcaataaaaa gacagaataa aacgcacggg tgttgggtcg tttgttcata 11280
   aacgcggggt tcggtcccag ggctggcact ctgtcgatac cccaccgaga ccccattggg 11340
   gccaatacgc ccgcgtttct tccttttccc caccccaccc cccaagttcg ggtgaaggcc 11400
   cagggctcgc agccaacgtc ggggcggcag gccctgccat agccactggc cccgtgggtt 11460
   agggacgggg tcccccatgg ggaatggttt atggttcgtg ggggttatta ttttgggcgt 11520
   tgcgtggggt caggtccacg actggactga gcagacagac ccatggtttt tggatggcct 11580
   gggcatggac cgcatgtact ggcgcgacac gaacaccggg cgtctgtggc tgccaaacac 11640
   ccccgacccc caaaaaccac cgcgcggatt tctggcgtgc caagctagtc gaccaattct 11700
   catgtttgac agcttatcat cgcagatccg ggcaacgttg ttgccattgc tgcaggcgca 11760
   gaactggtag gtatggaaga tctatacatt gaatcaatat tggcaattag ccatattagt 11820
   cattggttat atagcataaa tcaatattgg ctattggcca ttgcatacgt tgtatctata 11880
   tcataatatg tacatttata ttggctcatg tccaatatga ccgccat              11927
<210> 211
   <211> 735
   <212> DNA
   <213> Artificial Sequence
<220>
   <223> In vitro synthesized DNA encoding a melanocortin receptor binding alpha-MSH mimetibody without secretory signal (p3650).
<400> 211
   tcctactcct gcgagcactt ccgctggtgc aagccggtgg gaggcggagg aagcggcgga 60
   ggcggatcct gcccaccatg cccggcgcct gaggccgccg ggggaccatc agtcttcctg 120
   ttccccccaa aacccaagga cactctcatg atctcccgga cccctgaggt cacgtgcgtg 180
   gtggtggacg tgagccagga agaccccgag gtccagttca actggtacgt ggatggcgtg 240
   gaggtgcata atgccaagac aaagccgcgg gaggagcagt tcaacagcac gtaccgtgtg 300
   gtcagcgtcc tcaccgtcct gcaccaggac tggctgaacg gcaaggagta caagtgcaag 360
   gtctccaaca aaggcctccc gtcctccatc gagaaaacca tctccaaagc caaagggcag 420
   cctcgagagc cacaggtgta caccctgccc ccatcccagg aggagatgac caagaaccag 480
   gtcagcctga cctgcctggt caaaggcttc taccccagcg acatcgccgt ggagtgggag 540
   agcaatgggc agccggagaa caactacaag accacgcctc ccgtgctgga ctccgacggc 600
   tccttcttcc tctacagcag gctaaccgtg gacaagagca ggtggcagga ggggaatgtc 660
   ttctcatgct ccgtgatgca tgaggctctg cacaaccact acacacagaa aagcttgtcc 720
   ctgtctctgg gtaaa                                                  735
<210> 212
   <211> 245
   <212> PRT
   <213> Artificial Sequence
<220>
   <223> Melanocortin receptor binding alpha-MSH mimetibody without secretory signal encoded by an in vitro synthesized DNA (p3650).
<400> 212
   Ser Tyr Ser Cys Glu His Phe Arg Trp Cys Lys Pro Val Gly Gly Gly
    1               5                  10                  15
   Gly Ser Gly Gly Gly Gly Ser Cys Pro Pro Cys Pro Ala Pro Glu Ala
               20                  25                  30
   Ala Gly Gly Pro Ser Val Phe Leu Phe Pro Pro Lys Pro Lys Asp Thr
           35                  40                  45
   Leu Met Ile Ser Arg Thr Pro Glu Val Thr Cys Val Val Val Asp Val
       50                  55                  60
   Ser Gln Glu Asp Pro Glu Val Gln Phe Asn Trp Tyr Val Asp Gly Val
   65                  70                  75                  80
   Glu Val His Asn Ala Lys Thr Lys Pro Arg Glu Glu Gln Phe Asn Ser
                   85                  90                  95
   Thr Tyr Arg Val Val Ser Val Leu Thr Val Leu His Gln Asp Trp Leu
               100                 105                 110
   Asn Gly Lys Glu Tyr Lys Cys Lys Val Ser Asn Lys Gly Leu Pro Ser
           115                 120                 125
   Ser Ile Glu Lys Thr Ile Ser Lys Ala Lys Gly Gln Pro Arg Glu Pro
       130                 135                 140
   Gln Val Tyr Thr Leu Pro Pro Ser Gln Glu Glu Met Thr Lys Asn Gln
   145                 150                 155                 160
   Val Ser Leu Thr Cys Leu Val Lys Gly Phe Tyr Pro Ser Asp Ile Ala
                   165                 170                 175
   Val Glu Trp Glu Ser Asn Gly Gln Pro Glu Asn Asn Tyr Lys Thr Thr
               180                 185                 190
   Pro Pro Val Leu Asp Ser Asp Gly Ser Phe Phe Leu Tyr Ser Arg Leu
           195                 200                 205
   Thr Val Asp Lys Ser Arg Trp Gln Glu Gly Asn Val Phe Ser Cys Ser
       210                 215                 220
   Val Met His Glu Ala Leu His Asn His Tyr Thr Gln Lys Ser Leu Ser
   225                 230                 235                 240
   Leu Ser Leu Gly Lys
                   245
<210> 213
   <211> 792
   <212> DNA
   <213> Artificial Sequence
<220>
   <223> In vitro synthesized DNA encoding a melanocortin receptor binding alpha-MSH mimetibody with secretory signal (p3650).
<400> 213
   atggcttggg tgtggacctt gctattcctg atggcggccg cccaaagtat acaggcctcc 60
   tactcctgcg agcacttccg ctggtgcaag ccggtgggag gcggaggaag cggcggaggc 120
   ggatcctgcc caccatgccc ggcgcctgag gccgccgggg gaccatcagt cttcctgttc 180
   cccccaaaac ccaaggacac tctcatgatc tcccggaccc ctgaggtcac gtgcgtggtg 240
   gtggacgtga gccaggaaga ccccgaggtc cagttcaact ggtacgtgga tggcgtggag 300
   gtgcataatg ccaagacaaa gccgcgggag gagcagttca acagcacgta ccgtgtggtc 360
   agcgtcctca ccgtcctgca ccaggactgg ctgaacggca aggagtacaa gtgcaaggtc 420
   tccaacaaag gcctcccgtc ctccatcgag aaaaccatct ccaaagccaa agggcagcct 480
   cgagagccac aggtgtacac cctgccccca tcccaggagg agatgaccaa gaaccaggtc 540
   agcctgacct gcctggtcaa aggcttctac cccagcgaca tcgccgtgga gtgggagagc 600
   aatgggcagc cggagaacaa ctacaagacc acgcctcccg tgctggactc cgacggctcc 660
   ttcttcctct acagcaggct aaccgtggac aagagcaggt ggcaggaggg gaatgtcttc 720
   tcatgctccg tgatgcatga ggctctgcac aaccactaca cacagaaaag cttgtccctg 780
   tctctgggta aa                                                     792
<210> 214
   <211> 264
   <212> PRT
   <213> Artificial Sequence
<220>
   <223> Melanocortin receptor binding alpha-MSH mimetibody with secretory signal encoded by an in vitro synthesized DNA (p3650).
<400> 214
   Met Ala Trp Val Trp Thr Leu Leu Phe Leu Met Ala Ala Ala Gln Ser
    1               5                  10                  15
   Ile Gln Ala Ser Tyr Ser Cys Glu His Phe Arg Trp Cys Lys Pro Val
               20                  25                  30
   Gly Gly Gly Gly Ser Gly Gly Gly Gly Ser Cys Pro Pro Cys Pro Ala
           35                  40                  45
   Pro Glu Ala Ala Gly Gly Pro Ser Val Phe Leu Phe Pro Pro Lys Pro
       50                  55                  60
   Lys Asp Thr Leu Met Ile Ser Arg Thr Pro Glu Val Thr Cys Val Val
   65                  70                  75                  80
   Val Asp Val Ser Gln Glu Asp Pro Glu Val Gln Phe Asn Trp Tyr Val
                   85                  90                  95
   Asp Gly Val Glu Val His Asn Ala Lys Thr Lys Pro Arg Glu Glu Gln
               100                 105                 110
   Phe Asn Ser Thr Tyr Arg Val Val Ser Val Leu Thr Val Leu His Gln
           115                 120                 125
   Asp Trp Leu Asn Gly Lys Glu Tyr Lys Cys Lys Val Ser Asn Lys Gly
       130                 135                 140
   Leu Pro Ser Ser Ile Glu Lys Thr Ile Ser Lys Ala Lys Gly Gln Pro
   145                 150                 155                 160
   Arg Glu Pro Gln Val Tyr Thr Leu Pro Pro Ser Gln Glu Glu Met Thr
                   165                 170                 175
   Lys Asn Gln Val Ser Leu Thr Cys Leu Val Lys Gly Phe Tyr Pro Ser
               180                 185                 190
   Asp Ile Ala Val Glu Trp Glu Ser Asn Gly Gln Pro Glu Asn Asn Tyr
           195                 200                 205
   Lys Thr Thr Pro Pro Val Leu Asp Ser Asp Gly Ser Phe Phe Leu Tyr
       210                 215                 220
   Ser Arg Leu Thr Val Asp Lys Ser Arg Trp Gln Glu Gly Asn Val Phe
   225                 230                 235                 240
   Ser Cys Ser Val Met His Glu Ala Leu His Asn His Tyr Thr Gln Lys
                   245                 250                 255
   Ser Leu Ser Leu Ser Leu Gly Lys
               260
<210> 215
   <211> 11927
   <212> DNA
   <213> Artificial Sequence
<220>
   <223> Melanocortin receptor binding alpha-MSH mimetibody with secretory signal encoded by an in vitro synthesized DNA (p3650).
<400> 215
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactcctgcg agcacttccg 1680
   ctggtgcaag ccggtgggag gcggaggaag cggcggaggc ggatcctgcc caccatgccc 1740
   ggcgcctgag gccgccgggg gaccatcagt cttcctgttc cccccaaaac ccaaggacac 1800
   tctcatgatc tcccggaccc ctgaggtcac gtgcgtggtg gtggacgtga gccaggaaga 1860
   ccccgaggtc cagttcaact ggtacgtgga tggcgtggag gtgcataatg ccaagacaaa 1920
   gccgcgggag gagcagttca acagcacgta ccgtgtggtc agcgtcctca ccgtcctgca 1980
   ccaggactgg ctgaacggca aggagtacaa gtgcaaggtc tccaacaaag gcctcccgtc 2040
   ctccatcgag aaaaccatct ccaaagccaa agggcagcct cgagagccac aggtgtacac 2100
   cctgccccca tcccaggagg agatgaccaa gaaccaggtc agcctgacct gcctggtcaa 2160
   aggcttctac cccagcgaca tcgccgtgga gtgggagagc aatgggcagc cggagaacaa 2220
   ctacaagacc acgcctcccg tgctggactc cgacggctcc ttcttcctct acagcaggct 2280
   aaccgtggac aagagcaggt ggcaggaggg gaatgtcttc tcatgctccg tgatgcatga 2340
   ggctctgcac aaccactaca cacagaaaag cttgtccctg tctctgggta aatgagggcc 2400
   cgtttaaaca gatccagaca tgataagata cattgatgag tttggacaaa ccacaactag 2460
   aatgcagtga aaaaaatgct ttatttgtga aatttgtgat gctattgctt tatttgtaac 2520
   cattataagc tgcaataaac aagttaacaa caacaattgc attcatttta tgtttcaggt 2580
   tcagggggag gtgtgggagg ttttttaaag caagtaaaac ctctacaaat gtggtatggc 2640
   tgattatgat ccggctgcct cgcgcgtttc ggtgatgacg gtgaaaacct ctgacacatg 2700
   cagctcccgg agacggtcac agcttgtctg taagcggatg ccgggagcag acaagcccgt 2760
   caggcgtcag cgggtgttgg cgggtgtcgg ggcgcagcca tgaggtcgac tctagaggat 2820
   cgatgccccg ccccggacga actaaacctg actacgacat ctctgcccct tcttcgcggg 2880
   gcagtgcatg taatcccttc agttggttgg tacaacttgc caactgggcc ctgttccaca 2940
   tgtgacacgg ggggggacca aacacaaagg ggttctctga ctgtagttga catccttata 3000
   aatggatgtg cacatttgcc aacactgagt ggctttcatc ctggagcaga ctttgcagtc 3060
   tgtggactgc aacacaacat tgcctttatg tgtaactctt ggctgaagct cttacaccaa 3120
   tgctggggga catgtacctc ccaggggccc aggaagacta cgggaggcta caccaacgtc 3180
   aatcagaggg gcctgtgtag ctaccgataa gcggaccctc aagagggcat tagcaatagt 3240
   gtttataagg cccccttgtt aaccctaaac gggtagcata tgcttcccgg gtagtagtat 3300
   atactatcca gactaaccct aattcaatag catatgttac ccaacgggaa gcatatgcta 3360
   tcgaattagg gttagtaaaa gggtcctaag gaacagcgat atctcccacc ccatgagctg 3420
   tcacggtttt atttacatgg ggtcaggatt ccacgagggt agtgaaccat tttagtcaca 3480
   agggcagtgg ctgaagatca aggagcgggc agtgaactct cctgaatctt cgcctgcttc 3540
   ttcattctcc ttcgtttagc taatagaata actgctgagt tgtgaacagt aaggtgtatg 3600
   tgaggtgctc gaaaacaagg tttcaggtga cgcccccaga ataaaatttg gacggggggt 3660
   tcagtggtgg cattgtgcta tgacaccaat ataaccctca caaacccctt gggcaataaa 3720
   tactagtgta ggaatgaaac attctgaata tctttaacaa tagaaatcca tggggtgggg 3780
   acaagccgta aagactggat gtccatctca cacgaattta tggctatggg caacacataa 3840
   tcctagtgca atatgatact ggggttatta agatgtgtcc caggcaggga ccaagacagg 3900
   tgaaccatgt tgttacactc tatttgtaac aaggggaaag agagtggacg ccgacagcag 3960
   cggactccac tggttgtctc taacaccccc gaaaattaaa cggggctcca cgccaatggg 4020
   gcccataaac aaagacaagt ggccactctt ttttttgaaa ttgtggagtg ggggcacgcg 4080
   tcagccccca cacgccgccc tgcggttttg gactgtaaaa taagggtgta ataacttggc 4140
   tgattgtaac cccgctaacc actgcggtca aaccacttgc ccacaaaacc actaatggca 4200
   ccccggggaa tacctgcata agtaggtggg cgggccaaga taggggcgcg attgctgcga 4260
   tctggaggac aaattacaca cacttgcgcc tgagcgccaa gcacagggtt gttggtcctc 4320
   atattcacga ggtcgctgag agcacggtgg gctaatgttg ccatgggtag catatactac 4380
   ccaaatatct ggatagcata tgctatccta atctatatct gggtagcata ggctatccta 4440
   atctatatct gggtagcata tgctatccta atctatatct gggtagtata tgctatccta 4500
   atttatatct gggtagcata ggctatccta atctatatct gggtagcata tgctatccta 4560
   atctatatct gggtagtata tgctatccta atctgtatcc gggtagcata tgctatccta 4620
   atagagatta gggtagtata tgctatccta atttatatct gggtagcata tactacccaa 4680
   atatctggat agcatatgct atcctaatct atatctgggt agcatatgct atcctaatct 4740
   atatctgggt agcataggct atcctaatct atatctgggt agcatatgct atcctaatct 4800
   atatctgggt agtatatgct atcctaattt atatctgggt agcataggct atcctaatct 4860
   atatctgggt agcatatgct atcctaatct atatctgggt agtatatgct atcctaatct 4920
   gtatccgggt agcatatgct atcctcatgc atatacagtc agcatatgat acccagtagt 4980
   agagtgggag tgctatcctt tgcatatgcc gccacctccc aagggggcgt gaattttcgc 5040
   tgcttgtcct tttcctgctg gttgctccca ttcttaggtg aatttaagga ggccaggcta 5100
   aagccgtcgc atgtctgatt gctcaccagg taaatgtcgc taatgttttc caacgcgaga 5160
   aggtgttgag cgcggagctg agtgacgtga caacatgggt atgcccaatt gccccatgtt 5220
   gggaggacga aaatggtgac aagacagatg gccagaaata caccaacagc acgcatgatg 5280
   tctactgggg atttattctt tagtgcgggg gaatacacgg cttttaatac gattgagggc 5340
   gtctcctaac aagttacatc actcctgccc ttcctcaccc tcatctccat cacctccttc 5400
   atctccgtca tctccgtcat caccctccgc ggcagcccct tccaccatag gtggaaacca 5460
   gggaggcaaa tctactccat cgtcaaagct gcacacagtc accctgatat tgcaggtagg 5520
   agcgggcttt gtcataacaa ggtccttaat cgcatccttc aaaacctcag caaatatatg 5580
   agtttgtaaa aagaccatga aataacagac aatggactcc cttagcgggc caggttgtgg 5640
   gccgggtcca ggggccattc caaaggggag acgactcaat ggtgtaagac gacattgtgg 5700
   aatagcaagg gcagttcctc gccttaggtt gtaaagggag gtcttactac ctccatatac 5760
   gaacacaccg gcgacccaag ttccttcgtc ggtagtcctt tctacgtgac tcctagccag 5820
   gagagctctt aaaccttctg caatgttctc aaatttcggg ttggaacctc cttgaccacg 5880
   atgcttttcc aaaccaccct ccttttttgc gccctgcctc catcaccctg accccggggt 5940
   ccagtgcttg ggccttctcc tgggtcatct gcggggccct gctctatcgc tcccgggggc 6000
   acgtcaggct caccatctgg gccaccttct tggtggtatt caaaataatc ggcttcccct 6060
   acagggtgga aaaatggcct tctacctgga gggggcctgc gcggtggaga cccggatgat 6120
   gatgactgac tactgggact cctgggcctc ttttctccac gtccacgacc tctccccctg 6180
   gctctttcac gacttccccc cctggctctt tcacgtcctc taccccggcg gcctccacta 6240
   cctcctcgac cccggcctcc actacctcct cgaccccggc ctccactgcc tcctcgaccc 6300
   cggcctccac ctcctgctcc tgcccctcct gctcctgccc ctcctcctgc tcctgcccct 6360
   cctgcccctc ctgctcctgc ccctcctgcc cctcctgctc ctgcccctcc tgcccctcct 6420
   gctcctgccc ctcctgcccc tcctcctgct cctgcccctc ctgcccctcc tcctgctcct 6480
   gcccctcctg cccctcctgc tcctgcccct cctgcccctc ctgctcctgc ccctcctgcc 6540
   cctcctgctc ctgcccctcc tgctcctgcc cctcctgctc ctgcccctcc tgctcctgcc 6600
   cctcctgccc ctcctgcccc tcctcctgct cctgcccctc ctgctcctgc ccctcctgcc 6660
   cctcctgccc ctcctgctcc tgcccctcct cctgctcctg cccctcctgc ccctcctgcc 6720
   cctcctcctg ctcctgcccc tcctgcccct cctcctgctc ctgcccctcc tcctgctcct 6780
   gcccctcctg cccctcctgc ccctcctcct gctcctgccc ctcctgcccc tcctcctgct 6840
   cctgcccctc ctcctgctcc tgcccctcct gcccctcctg cccctcctcc tgctcctgcc 6900
   cctcctcctg ctcctgcccc tcctgcccct cctgcccctc ctgcccctcc tcctgctcct 6960
   gcccctcctc ctgctcctgc ccctcctgct cctgcccctc ccgctcctgc tcctgctcct 7020
   gttccaccgt gggtcccttt gcagccaatg caacttggac gtttttgggg tctccggaca 7080
   ccatctctat gtcttggccc tgatcctgag ccgccccggg ctcctggtct tccgcctcct 7140
   cgtcctcgtc ctcttccccg tcctcgtcca tggttatcac cccctcttct ttgaggtcca 7200
   ctgccgccgg agccttctgg tccagatgtg tctcccttct ctcctaggcc atttccaggt 7260
   cctgtacctg gcccctcgtc agacatgatt cacactaaaa gagatcaata gacatcttta 7320
   ttagacgacg ctcagtgaat acagggagtg cagactcctg ccccctccaa cagccccccc 7380
   accctcatcc ccttcatggt cgctgtcaga cagatccagg tctgaaaatt ccccatcctc 7440
   cgaaccatcc tcgtcctcat caccaattac tcgcagcccg gaaaactccc gctgaacatc 7500
   ctcaagattt gcgtcctgag cctcaagcca ggcctcaaat tcctcgtccc cctttttgct 7560
   ggacggtagg gatggggatt ctcgggaccc ctcctcttcc tcttcaaggt caccagacag 7620
   agatgctact ggggcaacgg aagaaaagct gggtgcggcc tgtgaggatc agcttatcga 7680
   tgataagctg tcaaacatga gaattcttga agacgaaagg gcctcgtgat acgcctattt 7740
   ttataggtta atgtcatgat aataatggtt tcttagacgt caggtggcac ttttcgggga 7800
   aatgtgcgcg gaacccctat ttgtttattt ttctaaatac attcaaatat gtatccgctc 7860
   atgagacaat aaccctgata aatgcttcaa taatattgaa aaaggaagag tatgagtatt 7920
   caacatttcc gtgtcgccct tattcccttt tttgcggcat tttgccttcc tgtttttgct 7980
   cacccagaaa cgctggtgaa agtaaaagat gctgaagatc agttgggtgc acgagtgggt 8040
   tacatcgaac tggatctcaa cagcggtaag atccttgaga gttttcgccc cgaagaacgt 8100
   tttccaatga tgagcacttt taaagttctg ctatgtggcg cggtattatc ccgtgttgac 8160
   gccgggcaag agcaactcgg tcgccgcata cactattctc agaatgactt ggttgagtac 8220
   tcaccagtca cagaaaagca tcttacggat ggcatgacag taagagaatt atgcagtgct 8280
   gccataacca tgagtgataa cactgcggcc aacttacttc tgacaacgat cggaggaccg 8340
   aaggagctaa ccgctttttt gcacaacatg ggggatcatg taactcgcct tgatcgttgg 8400
   gaaccggagc tgaatgaagc cataccaaac gacgagcgtg acaccacgat gcctgcagca 8460
   atggcaacaa cgttgcgcaa actattaact ggcgaactac ttactctagc ttcccggcaa 8520
   caattaatag actggatgga ggcggataaa gttgcaggac cacttctgcg ctcggccctt 8580
   ccggctggct ggtttattgc tgataaatct ggagccggtg agcgtgggtc tcgcggtatc 8640
   attgcagcac tggggccaga tggtaagccc tcccgtatcg tagttatcta cacgacgggg 8700
   agtcaggcaa ctatggatga acgaaataga cagatcgctg agataggtgc ctcactgatt 8760
   aagcattggt aactgtcaga ccaagtttac tcatatatac tttagattga tttaaaactt 8820
   catttttaat ttaaaaggat ctaggtgaag atcctttttg ataatctcat gaccaaaatc 8880
   ccttaacgtg agttttcgtt ccactgagcg tcagaccccg tagaaaagat caaaggatct 8940
   tcttgagatc ctttttttct gcgcgtaatc tgctgcttgc aaacaaaaaa accaccgcta 9000
   ccagcggtgg tttgtttgcc ggatcaagag ctaccaactc tttttccgaa ggtaactggc 9060
   ttcagcagag cgcagatacc aaatactgtc cttctagtgt agccgtagtt aggccaccac 9120
   ttcaagaact ctgtagcacc gcctacatac ctcgctctgc taatcctgtt accagtggct 9180
   gctgccagtg gcgataagtc gtgtcttacc gggttggact caagacgata gttaccggat 9240
   aaggcgcagc ggtcgggctg aacggggggt tcgtgcacac agcccagctt ggagcgaacg 9300
   acctacaccg aactgagata cctacagcgt gagctatgag aaagcgccac gcttcccgaa 9360
   gggagaaagg cggacaggta tccggtaagc ggcagggtcg gaacaggaga gcgcacgagg 9420
   gagcttccag ggggaaacgc ctggtatctt tatagtcctg tcgggtttcg ccacctctga 9480
   cttgagcgtc gatttttgtg atgctcgtca ggggggcgga gcctatggaa aaacgccagc 9540
   aacgcggcct ttttacggtt cctggccttt tgctggcctt gaagctgtcc ctgatggtcg 9600
   tcatctacct gcctggacag catggcctgc aacgcgggca tcccgatgcc gccggaagcg 9660
   agaagaatca taatggggaa ggccatccag cctcgcgtcg cgaacgccag caagacgtag 9720
   cccagcgcgt cggccccgag atgcgccgcg tgcggctgct ggagatggcg gacgcgatgg 9780
   atatgttctg ccaagggttg gtttgcgcat tcacagttct ccgcaagaat tgattggctc 9840
   caattcttgg agtggtgaat ccgttagcga ggtgccgccc tgcttcatcc ccgtggcccg 9900
   ttgctcgcgt ttgctggcgg tgtccccgga agaaatatat ttgcatgtct ttagttctat 9960
   gatgacacaa accccgccca gcgtcttgtc attggcgaat tcgaacacgc agatgcagtc 10020
   ggggcggcgc ggtccgaggt ccacttcgca tattaaggtg acgcgtgtgg cctcgaacac 10080
   cgagcgaccc tgcagcgacc cgcttaacag cgtcaacagc gtgccgcaga tcccgggggg 10140
   caatgagata tgaaaaagcc tgaactcacc gcgacgtctg tcgagaagtt tctgatcgaa 10200
   aagttcgaca gcgtctccga cctgatgcag ctctcggagg gcgaagaatc tcgtgctttc 10260
   agcttcgatg taggagggcg tggatatgtc ctgcgggtaa atagctgcgc cgatggtttc 10320
   tacaaagatc gttatgttta tcggcacttt gcatcggccg cgctcccgat tccggaagtg 10380
   cttgacattg gggaattcag cgagagcctg acctattgca tctcccgccg tgcacagggt 10440
   gtcacgttgc aagacctgcc tgaaaccgaa ctgcccgctg ttctgcagcc ggtcgcggag 10500
   gccatggatg cgatcgctgc ggccgatctt agccagacga gcgggttcgg cccattcgga 10560
   ccgcaaggaa tcggtcaata cactacatgg cgtgatttca tatgcgcgat tgctgatccc 10620
   catgtgtatc actggcaaac tgtgatggac gacaccgtca gtgcgtccgt cgcgcaggct 10680
   ctcgatgagc tgatgctttg ggccgaggac tgccccgaag tccggcacct cgtgcacgcg 10740
   gatttcggct ccaacaatgt cctgacggac aatggccgca taacagcggt cattgactgg 10800
   agcgaggcga tgttcgggga ttcccaatac gaggtcgcca acatcttctt ctggaggccg 10860
   tggttggctt gtatggagca gcagacgcgc tacttcgagc ggaggcatcc ggagcttgca 10920
   ggatcgccgc ggctccgggc gtatatgctc cgcattggtc ttgaccaact ctatcagagc 10980
   ttggttgacg gcaatttcga tgatgcagct tgggcgcagg gtcgatgcga cgcaatcgtc 11040
   cgatccggag ccgggactgt cgggcgtaca caaatcgccc gcagaagcgc ggccgtctgg 11100
   accgatggct gtgtagaagt actcgccgat agtggaaacc gacgccccag cactcgtccg 11160
   gatcgggaga tgggggaggc taactgaaac acggaaggag acaataccgg aaggaacccg 11220
   cgctatgacg gcaataaaaa gacagaataa aacgcacggg tgttgggtcg tttgttcata 11280
   aacgcggggt tcggtcccag ggctggcact ctgtcgatac cccaccgaga ccccattggg 11340
   gccaatacgc ccgcgtttct tccttttccc caccccaccc cccaagttcg ggtgaaggcc 11400
   cagggctcgc agccaacgtc ggggcggcag gccctgccat agccactggc cccgtgggtt 11460
   agggacgggg tcccccatgg ggaatggttt atggttcgtg ggggttatta ttttgggcgt 11520
   tgcgtggggt caggtccacg actggactga gcagacagac ccatggtttt tggatggcct 11580
   gggcatggac cgcatgtact ggcgcgacac gaacaccggg cgtctgtggc tgccaaacac 11640
   ccccgacccc caaaaaccac cgcgcggatt tctggcgtgc caagctagtc gaccaattct 11700
   catgtttgac agcttatcat cgcagatccg ggcaacgttg ttgccattgc tgcaggcgca 11760
   gaactggtag gtatggaaga tctatacatt gaatcaatat tggcaattag ccatattagt 11820
   cattggttat atagcataaa tcaatattgg ctattggcca ttgcatacgt tgtatctata 11880
   tcataatatg tacatttata ttggctcatg tccaatatga ccgccat           11927
<210> 216
   <211> 735
   <212> DNA
   <213> Artificial Sequence
<220>
   <223> In vitro synthesized DNA encoding a melanocortin receptor binding alpha-MSH mimetibody without secretory signal (p3651).
<400> 216
   tcctactcct gcgagcactt ccgctggtgc aagccggtgg gaggcggagg aagcggcgga 60
   ggcggatcct gcccaccgtg cccagcacct gaagccgctg ggggaccgtc agtcttcctc 120
   ttccccccaa aacccaagga caccctcatg atctcccgga cccctgaggt cacatgcgtg 180
   gtggtggacg tgagccacga agaccctgag gtcaagttca actggtacgt ggacggcgtg 240
   gaggtgcata atgccaagac aaagccgcgg gaggagcagt acaacagcac gtaccgggtg 300
   gtcagcgtcc tcaccgtcct gcaccaggac tggctgaatg gcaaggagta caagtgcaag 360
   gtctccaaca aagccctccc agcccccatc gagaaaacca tctccaaagc caaagggcag 420
   ccccgagaac cacaggtgta caccctgccc ccatcccggg atgagctgac caagaaccag 480
   gtcagcctga cctgcctggt caaaggcttc tatcccagcg acatcgccgt ggagtgggag 540
   agcaatgggc agccggagaa caactacaag accacgcctc ccgtgctgga ctccgacggc 600
   tccttcttcc tctacagcaa gctcaccgtg gacaagagca ggtggcagca ggggaacgtc 660
   ttctcatgct ccgtgatgca tgaggctctg cacaaccact acacgcagaa gagcctctcc 720
   ctgtctccgg gtaaa                                                  735
<210> 217
   <211> 245
   <212> PRT
   <213> Artificial Sequence
<220>
   <223> Melanocortin receptor binding alpha-MSH mimetibody without secretory signal encoded by an in vitro synthesized DNA (p3651).
<400> 217
   Ser Tyr Ser Cys Glu His Phe Arg Trp Cys Lys Pro Val Gly Gly Gly
    1               5                  10                  15
   Gly Ser Gly Gly Gly Gly Ser Cys Pro Pro Cys Pro Ala Pro Glu Ala
               20                  25                  30
   Ala Gly Gly Pro Ser Val Phe Leu Phe Pro Pro Lys Pro Lys Asp Thr
           35                  40                  45
   Leu Met Ile Ser Arg Thr Pro Glu Val Thr Cys Val Val Val Asp Val
       50                  55                  60
   Ser His Glu Asp Pro Glu Val Lys Phe Asn Trp Tyr Val Asp Gly Val
   65                  70                  75                  80
   Glu Val His Asn Ala Lys Thr Lys Pro Arg Glu Glu Gln Tyr Asn Ser
                   85                  90                  95
   Thr Tyr Arg Val Val Ser Val Leu Thr Val Leu His Gln Asp Trp Leu
               100                 105                 110
   Asn Gly Lys Glu Tyr Lys Cys Lys Val Ser Asn Lys Ala Leu Pro Ala
           115                 120                 125
   Pro Ile Glu Lys Thr Ile Ser Lys Ala Lys Gly Gln Pro Arg Glu Pro
       130                 135                 140
   Gln Val Tyr Thr Leu Pro Pro Ser Arg Asp Glu Leu Thr Lys Asn Gln
   145                 150                 155                 160
   Val Ser Leu Thr Cys Leu Val Lys Gly Phe Tyr Pro Ser Asp Ile Ala
                   165                 170                 175
   Val Glu Trp Glu Ser Asn Gly Gln Pro Glu Asn Asn Tyr Lys Thr Thr
               180                 185                 190
   Pro Pro Val Leu Asp Ser Asp Gly Ser Phe Phe Leu Tyr Ser Lys Leu
           195                 200                 205
   Thr Val Asp Lys Ser Arg Trp Gln Gln Gly Asn Val Phe Ser Cys Ser
       210                 215                 220
   Val Met His Glu Ala Leu His Asn His Tyr Thr Gln Lys Ser Leu Ser
   225                 230                 235                 240
   Leu Ser Pro Gly Lys
                   245
<210> 218
   <211> 792
   <212> DNA
   <213> Artificial Sequence
<220>
   <223> In vitro synthesized DNA encoding a melanocortin receptor binding alpha-MSH mimetibody with secretory signal (p3651).
<400> 218
   atggcttggg tgtggacctt gctattcctg atggcggccg cccaaagtat acaggcctcc 60
   tactcctgcg agcacttccg ctggtgcaag ccggtgggag gcggaggaag cggcggaggc 120
   ggatcctgcc caccgtgccc agcacctgaa gccgctgggg gaccgtcagt cttcctcttc 180
   cccccaaaac ccaaggacac cctcatgatc tcccggaccc ctgaggtcac atgcgtggtg 240
   gtggacgtga gccacgaaga ccctgaggtc aagttcaact ggtacgtgga cggcgtggag 300
   gtgcataatg ccaagacaaa gccgcgggag gagcagtaca acagcacgta ccgggtggtc 360
   agcgtcctca ccgtcctgca ccaggactgg ctgaatggca aggagtacaa gtgcaaggtc 420
   tccaacaaag ccctcccagc ccccatcgag aaaaccatct ccaaagccaa agggcagccc 480
   cgagaaccac aggtgtacac cctgccccca tcccgggatg agctgaccaa gaaccaggtc 540
   agcctgacct gcctggtcaa aggcttctat cccagcgaca tcgccgtgga gtgggagagc 600
   aatgggcagc cggagaacaa ctacaagacc acgcctcccg tgctggactc cgacggctcc 660
   ttcttcctct acagcaagct caccgtggac aagagcaggt ggcagcaggg gaacgtcttc 720
   tcatgctccg tgatgcatga ggctctgcac aaccactaca cgcagaagag cctctccctg 780
   tctccgggta aa                                                     792
<210> 219
   <211> 264
   <212> PRT
   <213> Artificial Sequence
<220>
   <223> Melanocortin receptor binding alpha-MSH mimetibody with secretory signal encoded by an in vitro synthesized DNA (p3651).
<400> 219
   Met Ala Trp Val Trp Thr Leu Leu Phe Leu Met Ala Ala Ala Gln Ser
    1               5                  10                  15
   Ile Gln Ala Ser Tyr Ser Cys Glu His Phe Arg Trp Cys Lys Pro Val
               20                  25                  30
   Gly Gly Gly Gly Ser Gly Gly Gly Gly Ser Cys Pro Pro Cys Pro Ala
           35                  40                  45
   Pro Glu Ala Ala Gly Gly Pro Ser Val Phe Leu Phe Pro Pro Lys Pro
       50                  55                  60
   Lys Asp Thr Leu Met Ile Ser Arg Thr Pro Glu Val Thr Cys Val Val
   65                  70                  75                  80
   Val Asp Val Ser His Glu Asp Pro Glu Val Lys Phe Asn Trp Tyr Val
                   85                  90                  95
   Asp Gly Val Glu Val His Asn Ala Lys Thr Lys Pro Arg Glu Glu Gln
               100                 105                 110
   Tyr Asn Ser Thr Tyr Arg Val Val Ser Val Leu Thr Val Leu His Gln
           115                 120                 125
   Asp Trp Leu Asn Gly Lys Glu Tyr Lys Cys Lys Val Ser Asn Lys Ala
       130                 135                 140
   Leu Pro Ala Pro Ile Glu Lys Thr Ile Ser Lys Ala Lys Gly Gln Pro
   145                 150                 155                 160
   Arg Glu Pro Gln Val Tyr Thr Leu Pro Pro Ser Arg Asp Glu Leu Thr
                   165                 170                 175
   Lys Asn Gln Val Ser Leu Thr Cys Leu Val Lys Gly Phe Tyr Pro Ser
               180                 185                 190
   Asp Ile Ala Val Glu Trp Glu Ser Asn Gly Gln Pro Glu Asn Asn Tyr
           195                 200                 205
   Lys Thr Thr Pro Pro Val Leu Asp Ser Asp Gly Ser Phe Phe Leu Tyr
       210                 215                 220
   Ser Lys Leu Thr Val Asp Lys Ser Arg Trp Gln Gln Gly Asn Val Phe
   225                 230                 235                 240
   Ser Cys Ser Val Met His Glu Ala Leu His Asn His Tyr Thr Gln Lys
                   245                 250                 255
   Ser Leu Ser Leu Ser Pro Gly Lys
               260
<210> 220
   <211> 11927
   <212> DNA
   <213> Artificial Sequence
<220>
   <223> In vitro synthesized DNA with expression vector functions that encodes an alpha-MSH mimetibody (p3651).
<400> 220
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactcctgcg agcacttccg 1680
   ctggtgcaag ccggtgggag gcggaggaag cggcggaggc ggatcctgcc caccgtgccc 1740
   agcacctgaa gccgctgggg gaccgtcagt cttcctcttc cccccaaaac ccaaggacac 1800
   cctcatgatc tcccggaccc ctgaggtcac atgcgtggtg gtggacgtga gccacgaaga 1860
   ccctgaggtc aagttcaact ggtacgtgga cggcgtggag gtgcataatg ccaagacaaa 1920
   gccgcgggag gagcagtaca acagcacgta ccgggtggtc agcgtcctca ccgtcctgca 1980
   ccaggactgg ctgaatggca aggagtacaa gtgcaaggtc tccaacaaag ccctcccagc 2040
   ccccatcgag aaaaccatct ccaaagccaa agggcagccc cgagaaccac aggtgtacac 2100
   cctgccccca tcccgggatg agctgaccaa gaaccaggtc agcctgacct gcctggtcaa 2160
   aggcttctat cccagcgaca tcgccgtgga gtgggagagc aatgggcagc cggagaacaa 2220
   ctacaagacc acgcctcccg tgctggactc cgacggctcc ttcttcctct acagcaagct 2280
   caccgtggac aagagcaggt ggcagcaggg gaacgtcttc tcatgctccg tgatgcatga 2340
   ggctctgcac aaccactaca cgcagaagag cctctccctg tctccgggta aatgagggcc 2400
   cgtttaaaca gatccagaca tgataagata cattgatgag tttggacaaa ccacaactag 2460
   aatgcagtga aaaaaatgct ttatttgtga aatttgtgat gctattgctt tatttgtaac 2520
   cattataagc tgcaataaac aagttaacaa caacaattgc attcatttta tgtttcaggt 2580
   tcagggggag gtgtgggagg ttttttaaag caagtaaaac ctctacaaat gtggtatggc 2640
   tgattatgat ccggctgcct cgcgcgtttc ggtgatgacg gtgaaaacct ctgacacatg 2700
   cagctcccgg agacggtcac agcttgtctg taagcggatg ccgggagcag acaagcccgt 2760
   caggcgtcag cgggtgttgg cgggtgtcgg ggcgcagcca tgaggtcgac tctagaggat 2820
   cgatgccccg ccccggacga actaaacctg actacgacat ctctgcccct tcttcgcggg 2880
   gcagtgcatg taatcccttc agttggttgg tacaacttgc caactgggcc ctgttccaca 2940
   tgtgacacgg ggggggacca aacacaaagg ggttctctga ctgtagttga catccttata 3000
   aatggatgtg cacatttgcc aacactgagt ggctttcatc ctggagcaga ctttgcagtc 3060
   tgtggactgc aacacaacat tgcctttatg tgtaactctt ggctgaagct cttacaccaa 3120
   tgctggggga catgtacctc ccaggggccc aggaagacta cgggaggcta caccaacgtc 3180
   aatcagaggg gcctgtgtag ctaccgataa gcggaccctc aagagggcat tagcaatagt 3240
   gtttataagg cccccttgtt aaccctaaac gggtagcata tgcttcccgg gtagtagtat 3300
   atactatcca gactaaccct aattcaatag catatgttac ccaacgggaa gcatatgcta 3360
   tcgaattagg gttagtaaaa gggtcctaag gaacagcgat atctcccacc ccatgagctg 3420
   tcacggtttt atttacatgg ggtcaggatt ccacgagggt agtgaaccat tttagtcaca 3480
   agggcagtgg ctgaagatca aggagcgggc agtgaactct cctgaatctt cgcctgcttc 3540
   ttcattctcc ttcgtttagc taatagaata actgctgagt tgtgaacagt aaggtgtatg 3600
   tgaggtgctc gaaaacaagg tttcaggtga cgcccccaga ataaaatttg gacggggggt 3660
   tcagtggtgg cattgtgcta tgacaccaat ataaccctca caaacccctt gggcaataaa 3720
   tactagtgta ggaatgaaac attctgaata tctttaacaa tagaaatcca tggggtgggg 3780
   acaagccgta aagactggat gtccatctca cacgaattta tggctatggg caacacataa 3840
   tcctagtgca atatgatact ggggttatta agatgtgtcc caggcaggga ccaagacagg 3900
   tgaaccatgt tgttacactc tatttgtaac aaggggaaag agagtggacg ccgacagcag 3960
   cggactccac tggttgtctc taacaccccc gaaaattaaa cggggctcca cgccaatggg 4020
   gcccataaac aaagacaagt ggccactctt ttttttgaaa ttgtggagtg ggggcacgcg 4080
   tcagccccca cacgccgccc tgcggttttg gactgtaaaa taagggtgta ataacttggc 4140
   tgattgtaac cccgctaacc actgcggtca aaccacttgc ccacaaaacc actaatggca 4200
   ccccggggaa tacctgcata agtaggtggg cgggccaaga taggggcgcg attgctgcga 4260
   tctggaggac aaattacaca cacttgcgcc tgagcgccaa gcacagggtt gttggtcctc 4320
   atattcacga ggtcgctgag agcacggtgg gctaatgttg ccatgggtag catatactac 4380
   ccaaatatct ggatagcata tgctatccta atctatatct gggtagcata ggctatccta 4440
   atctatatct gggtagcata tgctatccta atctatatct gggtagtata tgctatccta 4500
   atttatatct gggtagcata ggctatccta atctatatct gggtagcata tgctatccta 4560
   atctatatct gggtagtata tgctatccta atctgtatcc gggtagcata tgctatccta 4620
   atagagatta gggtagtata tgctatccta atttatatct gggtagcata tactacccaa 4680
   atatctggat agcatatgct atcctaatct atatctgggt agcatatgct atcctaatct 4740
   atatctgggt agcataggct atcctaatct atatctgggt agcatatgct atcctaatct 4800
   atatctgggt agtatatgct atcctaattt atatctgggt agcataggct atcctaatct 4860
   atatctgggt agcatatgct atcctaatct atatctgggt agtatatgct atcctaatct 4920
   gtatccgggt agcatatgct atcctcatgc atatacagtc agcatatgat acccagtagt 4980
   agagtgggag tgctatcctt tgcatatgcc gccacctccc aagggggcgt gaattttcgc 5040
   tgcttgtcct tttcctgctg gttgctccca ttcttaggtg aatttaagga ggccaggcta 5100
   aagccgtcgc atgtctgatt gctcaccagg taaatgtcgc taatgttttc caacgcgaga 5160
   aggtgttgag cgcggagctg agtgacgtga caacatgggt atgcccaatt gccccatgtt 5220
   gggaggacga aaatggtgac aagacagatg gccagaaata caccaacagc acgcatgatg 5280
   tctactgggg atttattctt tagtgcgggg gaatacacgg cttttaatac gattgagggc 5340
   gtctcctaac aagttacatc actcctgccc ttcctcaccc tcatctccat cacctccttc 5400
   atctccgtca tctccgtcat caccctccgc ggcagcccct tccaccatag gtggaaacca 5460
   gggaggcaaa tctactccat cgtcaaagct gcacacagtc accctgatat tgcaggtagg 5520
   agcgggcttt gtcataacaa ggtccttaat cgcatccttc aaaacctcag caaatatatg 5580
   agtttgtaaa aagaccatga aataacagac aatggactcc cttagcgggc caggttgtgg 5640
   gccgggtcca ggggccattc caaaggggag acgactcaat ggtgtaagac gacattgtgg 5700
   aatagcaagg gcagttcctc gccttaggtt gtaaagggag gtcttactac ctccatatac 5760
   gaacacaccg gcgacccaag ttccttcgtc ggtagtcctt tctacgtgac tcctagccag 5820
   gagagctctt aaaccttctg caatgttctc aaatttcggg ttggaacctc cttgaccacg 5880
   atgcttttcc aaaccaccct ccttttttgc gccctgcctc catcaccctg accccggggt 5940
   ccagtgcttg ggccttctcc tgggtcatct gcggggccct gctctatcgc tcccgggggc 6000
   acgtcaggct caccatctgg gccaccttct tggtggtatt caaaataatc ggcttcccct 6060
   acagggtgga aaaatggcct tctacctgga gggggcctgc gcggtggaga cccggatgat 6120
   gatgactgac tactgggact cctgggcctc ttttctccac gtccacgacc tctccccctg 6180
   gctctttcac gacttccccc cctggctctt tcacgtcctc taccccggcg gcctccacta 6240
   cctcctcgac cccggcctcc actacctcct cgaccccggc ctccactgcc tcctcgaccc 6300
   cggcctccac ctcctgctcc tgcccctcct gctcctgccc ctcctcctgc tcctgcccct 6360
   cctgcccctc ctgctcctgc ccctcctgcc cctcctgctc ctgcccctcc tgcccctcct 6420
   gctcctgccc ctcctgcccc tcctcctgct cctgcccctc ctgcccctcc tcctgctcct 6480
   gcccctcctg cccctcctgc tcctgcccct cctgcccctc ctgctcctgc ccctcctgcc 6540
   cctcctgctc ctgcccctcc tgctcctgcc cctcctgctc ctgcccctcc tgctcctgcc 6600
   cctcctgccc ctcctgcccc tcctcctgct cctgcccctc ctgctcctgc ccctcctgcc 6660
   cctcctgccc ctcctgctcc tgcccctcct cctgctcctg cccctcctgc ccctcctgcc 6720
   cctcctcctg ctcctgcccc tcctgcccct cctcctgctc ctgcccctcc tcctgctcct 6780
   gcccctcctg cccctcctgc ccctcctcct gctcctgccc ctcctgcccc tcctcctgct 6840
   cctgcccctc ctcctgctcc tgcccctcct gcccctcctg cccctcctcc tgctcctgcc 6900
   cctcctcctg ctcctgcccc tcctgcccct cctgcccctc ctgcccctcc tcctgctcct 6960
   gcccctcctc ctgctcctgc ccctcctgct cctgcccctc ccgctcctgc tcctgctcct 7020
   gttccaccgt gggtcccttt gcagccaatg caacttggac gtttttgggg tctccggaca 7080
   ccatctctat gtcttggccc tgatcctgag ccgcccgggg ctcctggtct tccgcctcct 7140
   cgtcctcgtc ctcttccccg tcctcgtcca tggttatcac cccctcttct ttgaggtcca 7200
   ctgccgccgg agccttctgg tccagatgtg tctcccttct ctcctaggcc atttccaggt 7260
   cctgtacctg gcccctcgtc agacatgatt cacactaaaa gagatcaata gacatcttta 7320
   ttagacgacg ctcagtgaat acagggagtg cagactcctg ccccctccaa cagccccccc 7380
   accctcatcc ccttcatggt cgctgtcaga cagatccagg tctgaaaatt ccccatcctc 7440
   cgaaccatcc tcgtcctcat caccaattac tcgcagcccg gaaaactccc gctgaacatc 7500
   ctcaagattt gcgtcctgag cctcaagcca ggcctcaaat tcctcgtccc cctttttgct 7560
   ggacggtagg gatggggatt ctcgggaccc ctcctcttcc tcttcaaggt caccagacag 7620
   agatgctact ggggcaacgg aagaaaagct gggtgcggcc tgtgaggatc agcttatcga 7680
   tgataagctg tcaaacatga gaattcttga agacgaaagg gcctcgtgat acgcctattt 7740
   ttataggtta atgtcatgat aataatggtt tcttagacgt caggtggcac ttttcgggga 7800
   aatgtgcgcg gaacccctat ttgtttattt ttctaaatac attcaaatat gtatccgctc 7860
   atgagacaat aaccctgata aatgcttcaa taatattgaa aaaggaagag tatgagtatt 7920
   caacatttcc gtgtcgccct tattcccttt tttgcggcat tttgccttcc tgtttttgct 7980
   cacccagaaa cgctggtgaa agtaaaagat gctgaagatc agttgggtgc acgagtgggt 8040
   tacatcgaac tggatctcaa cagcggtaag atccttgaga gttttcgccc cgaagaacgt 8100
   tttccaatga tgagcacttt taaagttctg ctatgtggcg cggtattatc ccgtgttgac 8160
   gccgggcaag agcaactcgg tcgccgcata cactattctc agaatgactt ggttgagtac 8220
   tcaccagtca cagaaaagca tcttacggat ggcatgacag taagagaatt atgcagtgct 8280
   gccataacca tgagtgataa cactgcggcc aacttacttc tgacaacgat cggaggaccg 8340
   aaggagctaa ccgctttttt gcacaacatg ggggatcatg taactcgcct tgatcgttgg 8400
   gaaccggagc tgaatgaagc cataccaaac gacgagcgtg acaccacgat gcctgcagca 8460
   atggcaacaa cgttgcgcaa actattaact ggcgaactac ttactctagc ttcccggcaa 8520
   caattaatag actggatgga ggcggataaa gttgcaggac cacttctgcg ctcggccctt 8580
   ccggctggct ggtttattgc tgataaatct ggagccggtg agcgtgggtc tcgcggtatc 8640
   attgcagcac tggggccaga tggtaagccc tcccgtatcg tagttatcta cacgacgggg 8700
   agtcaggcaa ctatggatga acgaaataga cagatcgctg agataggtgc ctcactgatt 8760
   aagcattggt aactgtcaga ccaagtttac tcatatatac tttagattga tttaaaactt 8820
   catttttaat ttaaaaggat ctaggtgaag atcctttttg ataatctcat gaccaaaatc 8880
   ccttaacgtg agttttcgtt ccactgagcg tcagaccccg tagaaaagat caaaggatct 8940
   tcttgagatc ctttttttct gcgcgtaatc tgctgcttgc aaacaaaaaa accaccgcta 9000
   ccagcggtgg tttgtttgcc ggatcaagag ctaccaactc tttttccgaa ggtaactggc 9060
   ttcagcagag cgcagatacc aaatactgtc cttctagtgt agccgtagtt aggccaccac 9120
   ttcaagaact ctgtagcacc gcctacatac ctcgctctgc taatcctgtt accagtggct 9180
   gctgccagtg gcgataagtc gtgtcttacc gggttggact caagacgata gttaccggat 9240
   aaggcgcagc ggtcgggctg aacggggggt tcgtgcacac agcccagctt ggagcgaacg 9300
   acctacaccg aactgagata cctacagcgt gagctatgag aaagcgccac gcttcccgaa 9360
   gggagaaagg cggacaggta tccggtaagc ggcagggtcg gaacaggaga gcgcacgagg 9420
   gagcttccag ggggaaacgc ctggtatctt tatagtcctg tcgggtttcg ccacctctga 9480
   cttgagcgtc gatttttgtg atgctcgtca ggggggcgga gcctatggaa aaacgccagc 9540
   aacgcggcct ttttacggtt cctggccttt tgctggcctt gaagctgtcc ctgatggtcg 9600
   tcatctacct gcctggacag catggcctgc aacgcgggca tcccgatgcc gccggaagcg 9660
   agaagaatca taatggggaa ggccatccag cctcgcgtcg cgaacgccag caagacgtag 9720
   cccagcgcgt cggccccgag atgcgccgcg tgcggctgct ggagatggcg gacgcgatgg 9780
   atatgttctg ccaagggttg gtttgcgcat tcacagttct ccgcaagaat tgattggctc 9840
   caattcttgg agtggtgaat ccgttagcga ggtgccgccc tgcttcatcc ccgtggcccg 9900
   ttgctcgcgt ttgctggcgg tgtccccgga agaaatatat ttgcatgtct ttagttctat 9960
   gatgacacaa accccgccca gcgtcttgtc attggcgaat tcgaacacgc agatgcagtc 10020
   ggggcggcgc ggtccgaggt ccacttcgca tattaaggtg acgcgtgtgg cctcgaacac 10080
   cgagcgaccc tgcagcgacc cgcttaacag cgtcaacagc gtgccgcaga tcccgggggg 10140
   caatgagata tgaaaaagcc tgaactcacc gcgacgtctg tcgagaagtt tctgatcgaa 10200
   aagttcgaca gcgtctccga cctgatgcag ctctcggagg gcgaagaatc tcgtgctttc 10260
   agcttcgatg taggagggcg tggatatgtc ctgcgggtaa atagctgcgc cgatggtttc 10320
   tacaaagatc gttatgttta tcggcacttt gcatcggccg cgctcccgat tccggaagtg 10380
   cttgacattg gggaattcag cgagagcctg acctattgca tctcccgccg tgcacagggt 10440
   gtcacgttgc aagacctgcc tgaaaccgaa ctgcccgctg ttctgcagcc ggtcgcggag 10500
   gccatggatg cgatcgctgc ggccgatctt agccagacga gcgggttcgg cccattcgga 10560
   ccgcaaggaa tcggtcaata cactacatgg cgtgatttca tatgcgcgat tgctgatccc 10620
   catgtgtatc actggcaaac tgtgatggac gacaccgtca gtgcgtccgt cgcgcaggct 10680
   ctcgatgagc tgatgctttg ggccgaggac tgccccgaag tccggcacct cgtgcacgcg 10740
   gatttcggct ccaacaatgt cctgacggac aatggccgca taacagcggt cattgactgg 10800
   agcgaggcga tgttcgggga ttcccaatac gaggtcgcca acatcttctt ctggaggccg 10860
   tggttggctt gtatggagca gcagacgcgc tacttcgagc ggaggcatcc ggagcttgca 10920
   ggatcgccgc ggctccgggc gtatatgctc cgcattggtc ttgaccaact ctatcagagc 10980
   ttggttgacg gcaatttcga tgatgcagct tgggcgcagg gtcgatgcga cgcaatcgtc 11040
   cgatccggag ccgggactgt cgggcgtaca caaatcgccc gcagaagcgc ggccgtctgg 11100
   accgatggct gtgtagaagt actcgccgat agtggaaacc gacgccccag cactcgtccg 11160
   gatcgggaga tgggggaggc taactgaaac acggaaggag acaataccgg aaggaacccg 11220
   cgctatgacg gcaataaaaa gacagaataa aacgcacggg tgttgggtcg tttgttcata 11280
   aacgcggggt tcggtcccag ggctggcact ctgtcgatac cccaccgaga ccccattggg 11340
   gccaatacgc ccgcgtttct tccttttccc caccccaccc cccaagttcg ggtgaaggcc 11400
   cagggctcgc agccaacgtc ggggcggcag gccctgccat agccactggc cccgtgggtt 11460
   agggacgggg tcccccatgg ggaatggttt atggttcgtg ggggttatta ttttgggcgt 11520
   tgcgtggggt caggtccacg actggactga gcagacagac ccatggtttt tggatggcct 11580
   gggcatggac cgcatgtact ggcgcgacac gaacaccggg cgtctgtggc tgccaaacac 11640
   ccccgacccc caaaaaccac cgcgcggatt tctggcgtgc caagctagtc gaccaattct 11700
   catgtttgac agcttatcat cgcagatccg ggcaacgttg ttgccattgc tgcaggcgca 11760
   gaactggtag gtatggaaga tctatacatt gaatcaatat tggcaattag ccatattagt 11820
   cattggttat atagcataaa tcaatattgg ctattggcca ttgcatacgt tgtatctata 11880
   tcataatatg tacatttata ttggctcatg tccaatatga ccgccat             11927
<210> 221
   <211> 729
   <212> DNA
   <213> Artificial Sequence
<220>
   <223> In vitro synthesized DNA encoding a melanocortin receptor binding alpha-MSH mimetibody without secretory signal (p3390).
<400> 221
   cagatccagt cctactcctg cgagcacttc cgctggtgca agccggtggg aggaggcgga 60
   tcctgcccac cgtgcccagc acctgaactc ctggggggac cgtcagtctt cctcttcccc 120
   ccaaaaccca aggacaccct catgatctcc cggacccctg aggtcacatg cgtggtggtg 180
   gacgtgagcc acgaagaccc tgaggtcaag ttcaactggt acgtggacgg cgtggaggtg 240
   cataatgcca agacaaagcc gcgggaggag cagtacaaca gcacgtaccg tgtggtcagc 300
   gtcctcaccg tcctgcacca ggactggctg aatggcaagg agtacaagtg caaggtctcc 360
   aacaaagccc tcccagcccc catcgagaaa accatctcca aagccaaagg gcagccccga 420
   gaaccacagg tgtacaccct gcccccatcc cgggatgagc tgaccaagaa ccaggtcagc 480
   ctgacctgcc tggtcaaagg cttctatccc agcgacatcg ccgtggagtg ggagagcaat 540
   gggcagccgg agaacaacta caagaccacg cctcccgtgc tggactccga cggctccttc 600
   ttcctctaca gcaagctcac cgtggacaag agcaggtggc agcaggggaa cgtcttctca 660
   tgctccgtga tgcatgaggc tctgcacaac cactacacgc agaagagcct ctccctgtct 720
   ctgggtaaa                                                         729
<210> 222
   <211> 243
   <212> PRT
   <213> Artificial Sequence
<220>
   <223> Melanocortin receptor binding alpha-MSH mimetibody without secretory signal encoded by an in vitro synthesized DNA (p3390).
<400> 222
   Gln Ile Gln Ser Tyr Ser Cys Glu His Phe Arg Trp Cys Lys Pro Val
    1               5                  10                  15
   Gly Gly Gly Gly Ser Cys Pro Pro Cys Pro Ala Pro Glu Leu Leu Gly
               20                  25                  30
   Gly Pro Ser Val Phe Leu Phe Pro Pro Lys Pro Lys Asp Thr Leu Met
           35                  40                  45
   Ile Ser Arg Thr Pro Glu Val Thr Cys Val Val Val Asp Val Ser His
       50                  55                  60
   Glu Asp Pro Glu Val Lys Phe Asn Trp Tyr Val Asp Gly Val Glu Val
   65                  70                  75                  80
   His Asn Ala Lys Thr Lys Pro Arg Glu Glu Gln Tyr Asn Ser Thr Tyr
                   85                  90                  95
   Arg Val Val Ser Val Leu Thr Val Leu His Gln Asp Trp Leu Asn Gly
               100                 105                 110
   Lys Glu Tyr Lys Cys Lys Val Ser Asn Lys Ala Leu Pro Ala Pro Ile
           115                 120                 125
   Glu Lys Thr Ile Ser Lys Ala Lys Gly Gln Pro Arg Glu Pro Gln Val
       130                 135                 140
   Tyr Thr Leu Pro Pro Ser Arg Asp Glu Leu Thr Lys Asn Gln Val Ser
   145                 150                 155                 160
   Leu Thr Cys Leu Val Lys Gly Phe Tyr Pro Ser Asp Ile Ala Val Glu
                   165                 170                 175
   Trp Glu Ser Asn Gly Gln Pro Glu Asn Asn Tyr Lys Thr Thr Pro Pro
               180                 185                 190
   Val Leu Asp Ser Asp Gly Ser Phe Phe Leu Tyr Ser Lys Leu Thr Val
           195                 200                 205
   Asp Lys Ser Arg Trp Gln Gln Gly Asn Val Phe Ser Cys Ser Val Met
       210                 215                 220
   His Glu Ala Leu His Asn His Tyr Thr Gln Lys Ser Leu Ser Leu Ser
   225                 230                 235                 240
   Leu Gly Lys
<210> 223
   <211> 786
   <212> DNA
   <213> Artificial Sequence
<220>
   <223> In vitro synthesized DNA encoding a melanocortin receptor binding alpha-MSH mimetibody with secretory signal and V1 (p3390).
<400> 223
   atggcttggg tgtggacctt gctattcctg atggcggccg cccaaagtat acaggcccag 60
   atccagtcct actcctgcga gcacttccgc tggtgcaagc cggtgggagg aggcggatcc 120
   tgcccaccgt gcccagcacc tgaactcctg gggggaccgt cagtcttcct cttcccccca 180
   aaacccaagg acaccctcat gatctcccgg acccctgagg tcacatgcgt ggtggtggac 240
   gtgagccacg aagaccctga ggtcaagttc aactggtacg tggacggcgt ggaggtgcat 300
   aatgccaaga caaagccgcg ggaggagcag tacaacagca cgtaccgtgt ggtcagcgtc 360
   ctcaccgtcc tgcaccagga ctggctgaat ggcaaggagt acaagtgcaa ggtctccaac 420
   aaagccctcc cagcccccat cgagaaaacc atctccaaag ccaaagggca gccccgagaa 480
   ccacaggtgt acaccctgcc cccatcccgg gatgagctga ccaagaacca ggtcagcctg 540
   acctgcctgg tcaaaggctt ctatcccagc gacatcgccg tggagtggga gagcaatggg 600
   cagccggaga acaactacaa gaccacgcct cccgtgctgg actccgacgg ctccttcttc 660
   ctctacagca agctcaccgt ggacaagagc aggtggcagc aggggaacgt cttctcatgc 720
   tccgtgatgc atgaggctct gcacaaccac tacacgcaga agagcctctc cctgtctctg 780
   ggtaaa                                                            786
<210> 224
   <211> 262
   <212> PRT
   <213> Artificial Sequence
<220>
   <223> Melanocortin receptor binding alpha-MSH mimetibody with secretory signal and V1 encoded by an in vitro synthesized DNA (p3390).
<400> 224
   Met Ala Trp Val Trp Thr Leu Leu Phe Leu Met Ala Ala Ala Gln Ser
    1               5                  10                  15
   Ile Gln Ala Gln Ile Gln Ser Tyr Ser Cys Glu His Phe Arg Trp Cys
               20                  25                  30
   Lys Pro Val Gly Gly Gly Gly Ser Cys Pro Pro Cys Pro Ala Pro Glu
           35                  40                  45
   Leu Leu Gly Gly Pro Ser Val Phe Leu Phe Pro Pro Lys Pro Lys Asp
       50                  55                  60
   Thr Leu Met Ile Ser Arg Thr Pro Glu Val Thr Cys Val Val Val Asp
   65                  70                  75                  80
   Val Ser His Glu Asp Pro Glu Val Lys Phe Asn Trp Tyr Val Asp Gly
                   85                  90                  95
   Val Glu Val His Asn Ala Lys Thr Lys Pro Arg Glu Glu Gln Tyr Asn
               100                 105                 110
   Ser Thr Tyr Arg Val Val Ser Val Leu Thr Val Leu His Gln Asp Trp
           115                 120                 125
   Leu Asn Gly Lys Glu Tyr Lys Cys Lys Val Ser Asn Lys Ala Leu Pro
       130                 135                 140
   Ala Pro Ile Glu Lys Thr Ile Ser Lys Ala Lys Gly Gln Pro Arg Glu
   145                 150                 155                 160
   Pro Gln Val Tyr Thr Leu Pro Pro Ser Arg Asp Glu Leu Thr Lys Asn
                   165                 170                 175
   Gln Val Ser Leu Thr Cys Leu Val Lys Gly Phe Tyr Pro Ser Asp Ile
               180                 185                 190
   Ala Val Glu Trp Glu Ser Asn Gly Gln Pro Glu Asn Asn Tyr Lys Thr
           195                 200                 205
   Thr Pro Pro Val Leu Asp Ser Asp Gly Ser Phe Phe Leu Tyr Ser Lys
       210                 215                 220
   Leu Thr Val Asp Lys Ser Arg Trp Gln Gln Gly Asn Val Phe Ser Cys
   225                 230                 235                 240
   Ser Val Met His Glu Ala Leu His Asn His Tyr Thr Gln Lys Ser Leu
                   245                 250                 255
   Ser Leu Ser Leu Gly Lys
               260
<210> 225
   <211> 11921
   <212> DNA
   <213> Artificial Sequence
<220>
   <223> In vitro synthesized DNA with expression vector functions that encodes an alpha-MSH mimetibody (p3390).
<400> 225
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcccag atccagtcct actcctgcga 1680
   gcacttccgc tggtgcaagc cggtgggagg aggcggatcc tgcccaccgt gcccagcacc 1740
   tgaactcctg gggggaccgt cagtcttcct cttcccccca aaacccaagg acaccctcat 1800
   gatctcccgg acccctgagg tcacatgcgt ggtggtggac gtgagccacg aagaccctga 1860
   ggtcaagttc aactggtacg tggacggcgt ggaggtgcat aatgccaaga caaagccgcg 1920
   ggaggagcag tacaacagca cgtaccgtgt ggtcagcgtc ctcaccgtcc tgcaccagga 1980
   ctggctgaat ggcaaggagt acaagtgcaa ggtctccaac aaagccctcc cagcccccat 2040
   cgagaaaacc atctccaaag ccaaagggca gccccgagaa ccacaggtgt acaccctgcc 2100
   cccatcccgg gatgagctga ccaagaacca ggtcagcctg acctgcctgg tcaaaggctt 2160
   ctatcccagc gacatcgccg tggagtggga gagcaatggg cagccggaga acaactacaa 2220
   gaccacgcct cccgtgctgg actccgacgg ctccttcttc ctctacagca agctcaccgt 2280
   ggacaagagc aggtggcagc aggggaacgt cttctcatgc tccgtgatgc atgaggctct 2340
   gcacaaccac tacacgcaga agagcctctc cctgtctctg ggtaaatgag ggcccgttta 2400
   aacagatcca gacatgataa gatacattga tgagtttgga caaaccacaa ctagaatgca 2460
   gtgaaaaaaa tgctttattt gtgaaatttg tgatgctatt gctttatttg taaccattat 2520
   aagctgcaat aaacaagtta acaacaacaa ttgcattcat tttatgtttc aggttcaggg 2580
   ggaggtgtgg gaggtttttt aaagcaagta aaacctctac aaatgtggta tggctgatta 2640
   tgatccggct gcctcgcgcg tttcggtgat gacggtgaaa acctctgaca catgcagctc 2700
   ccggagacgg tcacagcttg tctgtaagcg gatgccggga gcagacaagc ccgtcaggcg 2760
   tcagcgggtg ttggcgggtg tcggggcgca gccatgaggt cgactctaga ggatcgatgc 2820
   cccgccccgg acgaactaaa cctgactacg acatctctgc cccttcttcg cggggcagtg 2880
   catgtaatcc cttcagttgg ttggtacaac ttgccaactg ggccctgttc cacatgtgac 2940
   acgggggggg accaaacaca aaggggttct ctgactgtag ttgacatcct tataaatgga 3000
   tgtgcacatt tgccaacact gagtggcttt catcctggag cagactttgc agtctgtgga 3060
   ctgcaacaca acattgcctt tatgtgtaac tcttggctga agctcttaca ccaatgctgg 3120
   gggacatgta cctcccaggg gcccaggaag actacgggag gctacaccaa cgtcaatcag 3180
   aggggcctgt gtagctaccg ataagcggac cctcaagagg gcattagcaa tagtgtttat 3240
   aaggccccct tgttaaccct aaacgggtag catatgcttc ccgggtagta gtatatacta 3300
   tccagactaa ccctaattca atagcatatg ttacccaacg ggaagcatat gctatcgaat 3360
   tagggttagt aaaagggtcc taaggaacag cgatatctcc caccccatga gctgtcacgg 3420
   ttttatttac atggggtcag gattccacga gggtagtgaa ccattttagt cacaagggca 3480
   gtggctgaag atcaaggagc gggcagtgaa ctctcctgaa tcttcgcctg cttcttcatt 3540
   ctccttcgtt tagctaatag aataactgct gagttgtgaa cagtaaggtg tatgtgaggt 3600
   gctcgaaaac aaggtttcag gtgacgcccc cagaataaaa tttggacggg gggttcagtg 3660
   gtggcattgt gctatgacac caatataacc ctcacaaacc ccttgggcaa taaatactag 3720
   tgtaggaatg aaacattctg aatatcttta acaatagaaa tccatggggt ggggacaagc 3780
   cgtaaagact ggatgtccat ctcacacgaa tttatggcta tgggcaacac ataatcctag 3840
   tgcaatatga tactggggtt attaagatgt gtcccaggca gggaccaaga caggtgaacc 3900
   atgttgttac actctatttg taacaagggg aaagagagtg gacgccgaca gcagcggact 3960
   ccactggttg tctctaacac ccccgaaaat taaacggggc tccacgccaa tggggcccat 4020
   aaacaaagac aagtggccac tctttttttt gaaattgtgg agtgggggca cgcgtcagcc 4080
   cccacacgcc gccctgcggt tttggactgt aaaataaggg tgtaataact tggctgattg 4140
   taaccccgct aaccactgcg gtcaaaccac ttgcccacaa aaccactaat ggcaccccgg 4200
   ggaatacctg cataagtagg tgggcgggcc aagatagggg cgcgattgct gcgatctgga 4260
   ggacaaatta cacacacttg cgcctgagcg ccaagcacag ggttgttggt cctcatattc 4320
   acgaggtcgc tgagagcacg gtgggctaat gttgccatgg gtagcatata ctacccaaat 4380
   atctggatag catatgctat cctaatctat atctgggtag cataggctat cctaatctat 4440
   atctgggtag catatgctat cctaatctat atctgggtag tatatgctat cctaatttat 4500
   atctgggtag cataggctat cctaatctat atctgggtag catatgctat cctaatctat 4560
   atctgggtag tatatgctat cctaatctgt atccgggtag catatgctat cctaatagag 4620
   attagggtag tatatgctat cctaatttat atctgggtag catatactac ccaaatatct 4680
   ggatagcata tgctatccta atctatatct gggtagcata tgctatccta atctatatct 4740
   gggtagcata ggctatccta atctatatct gggtagcata tgctatccta atctatatct 4800
   gggtagtata tgctatccta atttatatct gggtagcata ggctatccta atctatatct 4860
   gggtagcata tgctatccta atctatatct gggtagtata tgctatccta atctgtatcc 4920
   gggtagcata tgctatcctc atgcatatac agtcagcata tgatacccag tagtagagtg 4980
   ggagtgctat cctttgcata tgccgccacc tcccaagggg gcgtgaattt tcgctgcttg 5040
   tccttttcct gctggttgct cccattctta ggtgaattta aggaggccag gctaaagccg 5100
   tcgcatgtct gattgctcac caggtaaatg tcgctaatgt tttccaacgc gagaaggtgt 5160
   tgagcgcgga gctgagtgac gtgacaacat gggtatgccc aattgcccca tgttgggagg 5220
   acgaaaatgg tgacaagaca gatggccaga aatacaccaa cagcacgcat gatgtctact 5280
   ggggatttat tctttagtgc gggggaatac acggctttta atacgattga gggcgtctcc 5340
   taacaagtta catcactcct gcccttcctc accctcatct ccatcacctc cttcatctcc 5400
   gtcatctccg tcatcaccct ccgcggcagc cccttccacc ataggtggaa accagggagg 5460
   caaatctact ccatcgtcaa agctgcacac agtcaccctg atattgcagg taggagcggg 5520
   ctttgtcata acaaggtcct taatcgcatc cttcaaaacc tcagcaaata tatgagtttg 5580
   taaaaagacc atgaaataac agacaatgga ctcccttagc gggccaggtt gtgggccggg 5640
   tccaggggcc attccaaagg ggagacgact caatggtgta agacgacatt gtggaatagc 5700
   aagggcagtt cctcgcctta ggttgtaaag ggaggtctta ctacctccat atacgaacac 5760
   accggcgacc caagttcctt cgtcggtagt cctttctacg tgactcctag ccaggagagc 5820
   tcttaaacct tctgcaatgt tctcaaattt cgggttggaa cctccttgac cacgatgctt 5880
   ttccaaacca ccctcctttt ttgcgccctg cctccatcac cctgaccccg gggtccagtg 5940
   cttgggcctt ctcctgggtc atctgcgggg ccctgctcta tcgctcccgg gggcacgtca 6000
   ggctcaccat ctgggccacc ttcttggtgg tattcaaaat aatcggcttc ccctacaggg 6060
   tggaaaaatg gccttctacc tggagggggc ctgcgcggtg gagacccgga tgatgatgac 6120
   tgactactgg gactcctggg cctcttttct ccacgtccac gacctctccc cctggctctt 6180
   tcacgacttc cccccctggc tctttcacgt cctctacccc ggcggcctcc actacctcct 6240
   cgaccccggc ctccactacc tcctcgaccc cggcctccac tgcctcctcg accccggcct 6300
   ccacctcctg ctcctgcccc tcctgctcct gcccctcctc ctgctcctgc ccctcctgcc 6360
   cctcctgctc ctgcccctcc tgcccctcct gctcctgccc ctcctgcccc tcctgctcct 6420
   gcccctcctg cccctcctcc tgctcctgcc cctcctgccc ctcctcctgc tcctgcccct 6480
   cctgcccctc ctgctcctgc ccctcctgcc cctcctgctc ctgcccctcc tgcccctcct 6540
   gctcctgccc ctcctgctcc tgcccctcct gctcctgccc ctcctgctcc tgcccctcct 6600
   gcccctcctg cccctcctcc tgctcctgcc cctcctgctc ctgcccctcc tgcccctcct 6660
   gcccctcctg ctcctgcccc tcctcctgct cctgcccctc ctgcccctcc tgcccctcct 6720
   cctgctcctg cccctcctgc ccctcctcct gctcctgccc ctcctcctgc tcctgcccct 6780
   cctgcccctc ctgcccctcc tcctgctcct gcccctcctg cccctcctcc tgctcctgcc 6840
   cctcctcctg ctcctgcccc tcctgcccct cctgcccctc ctcctgctcc tgcccctcct 6900
   cctgctcctg cccctcctgc ccctcctgcc cctcctgccc ctcctcctgc tcctgcccct 6960
   cctcctgctc ctgcccctcc tgctcctgcc cctcccgctc ctgctcctgc tcctgttcca 7020
   ccgtgggtcc ctttgcagcc aatgcaactt ggacgttttt ggggtctccg gacaccatct 7080
   ctatgtcttg gccctgatcc tgagccgccc ggggctcctg gtcttccgcc tcctcgtcct 7140
   cgtcctcttc cccgtcctcg tccatggtta tcaccccctc ttctttgagg tccactgccg 7200
   ccggagcctt ctggtccaga tgtgtctccc ttctctccta ggccatttcc aggtcctgta 7260
   cctggcccct cgtcagacat gattcacact aaaagagatc aatagacatc tttattagac 7320
   gacgctcagt gaatacaggg agtgcagact cctgccccct ccaacagccc ccccaccctc 7380
   atccccttca tggtcgctgt cagacagatc caggtctgaa aattccccat cctccgaacc 7440
   atcctcgtcc tcatcaccaa ttactcgcag cccggaaaac tcccgctgaa catcctcaag 7500
   atttgcgtcc tgagcctcaa gccaggcctc aaattcctcg tccccctttt tgctggacgg 7560
   tagggatggg gattctcggg acccctcctc ttcctcttca aggtcaccag acagagatgc 7620
   tactggggca acggaagaaa agctgggtgc ggcctgtgag gatcagctta tcgatgataa 7680
   gctgtcaaac atgagaattc ttgaagacga aagggcctcg tgatacgcct atttttatag 7740
   gttaatgtca tgataataat ggtttcttag acgtcaggtg gcacttttcg gggaaatgtg 7800
   cgcggaaccc ctatttgttt atttttctaa atacattcaa atatgtatcc gctcatgaga 7860
   caataaccct gataaatgct tcaataatat tgaaaaagga agagtatgag tattcaacat 7920
   ttccgtgtcg cccttattcc cttttttgcg gcattttgcc ttcctgtttt tgctcaccca 7980
   gaaacgctgg tgaaagtaaa agatgctgaa gatcagttgg gtgcacgagt gggttacatc 8040
   gaactggatc tcaacagcgg taagatcctt gagagttttc gccccgaaga acgttttcca 8100
   atgatgagca cttttaaagt tctgctatgt ggcgcggtat tatcccgtgt tgacgccggg 8160
   caagagcaac tcggtcgccg catacactat tctcagaatg acttggttga gtactcacca 8220
   gtcacagaaa agcatcttac ggatggcatg acagtaagag aattatgcag tgctgccata 8280
   accatgagtg ataacactgc ggccaactta cttctgacaa cgatcggagg accgaaggag 8340
   ctaaccgctt ttttgcacaa catgggggat catgtaactc gccttgatcg ttgggaaccg 8400
   gagctgaatg aagccatacc aaacgacgag cgtgacacca cgatgcctgc agcaatggca 8460
   acaacgttgc gcaaactatt aactggcgaa ctacttactc tagcttcccg gcaacaatta 8520
   atagactgga tggaggcgga taaagttgca ggaccacttc tgcgctcggc ccttccggct 8580
   ggctggttta ttgctgataa atctggagcc ggtgagcgtg ggtctcgcgg tatcattgca 8640
   gcactggggc cagatggtaa gccctcccgt atcgtagtta tctacacgac ggggagtcag 8700
   gcaactatgg atgaacgaaa tagacagatc gctgagatag gtgcctcact gattaagcat 8760
   tggtaactgt cagaccaagt ttactcatat atactttaga ttgatttaaa acttcatttt 8820
   taatttaaaa ggatctaggt gaagatcctt tttgataatc tcatgaccaa aatcccttaa 8880
   cgtgagtttt cgttccactg agcgtcagac cccgtagaaa agatcaaagg atcttcttga 8940
   gatccttttt ttctgcgcgt aatctgctgc ttgcaaacaa aaaaaccacc gctaccagcg 9000
   gtggtttgtt tgccggatca agagctacca actctttttc cgaaggtaac tggcttcagc 9060
   agagcgcaga taccaaatac tgtccttcta gtgtagccgt agttaggcca ccacttcaag 9120
   aactctgtag caccgcctac atacctcgct ctgctaatcc tgttaccagt ggctgctgcc 9180
   agtggcgata agtcgtgtct taccgggttg gactcaagac gatagttacc ggataaggcg 9240
   cagcggtcgg gctgaacggg gggttcgtgc acacagccca gcttggagcg aacgacctac 9300
   accgaactga gatacctaca gcgtgagcta tgagaaagcg ccacgcttcc cgaagggaga 9360
   aaggcggaca ggtatccggt aagcggcagg gtcggaacag gagagcgcac gagggagctt 9420
   ccagggggaa acgcctggta tctttatagt cctgtcgggt ttcgccacct ctgacttgag 9480
   cgtcgatttt tgtgatgctc gtcagggggg cggagcctat ggaaaaacgc cagcaacgcg 9540
   gcctttttac ggttcctggc cttttgctgg ccttgaagct gtccctgatg gtcgtcatct 9600
   acctgcctgg acagcatggc ctgcaacgcg ggcatcccga tgccgccgga agcgagaaga 9660
   atcataatgg ggaaggccat ccagcctcgc gtcgcgaacg ccagcaagac gtagcccagc 9720
   gcgtcggccc cgagatgcgc cgcgtgcggc tgctggagat ggcggacgcg atggatatgt 9780
   tctgccaagg gttggtttgc gcattcacag ttctccgcaa gaattgattg gctccaattc 9840
   ttggagtggt gaatccgtta gcgaggtgcc gccctgcttc atccccgtgg cccgttgctc 9900
   gcgtttgctg gcggtgtccc cggaagaaat atatttgcat gtctttagtt ctatgatgac 9960
   acaaaccccg cccagcgtct tgtcattggc gaattcgaac acgcagatgc agtcggggcg 10020
   gcgcggtccg aggtccactt cgcatattaa ggtgacgcgt gtggcctcga acaccgagcg 10080
   accctgcagc gacccgctta acagcgtcaa cagcgtgccg cagatcccgg ggggcaatga 10140
   gatatgaaaa agcctgaact caccgcgacg tctgtcgaga agtttctgat cgaaaagttc 10200
   gacagcgtct ccgacctgat gcagctctcg gagggcgaag aatctcgtgc tttcagcttc 10260
   gatgtaggag ggcgtggata tgtcctgcgg gtaaatagct gcgccgatgg tttctacaaa 10320
   gatcgttatg tttatcggca ctttgcatcg gccgcgctcc cgattccgga agtgcttgac 10380
   attggggaat tcagcgagag cctgacctat tgcatctccc gccgtgcaca gggtgtcacg 10440
   ttgcaagacc tgcctgaaac cgaactgccc gctgttctgc agccggtcgc ggaggccatg 10500
   gatgcgatcg ctgcggccga tcttagccag acgagcgggt tcggcccatt cggaccgcaa 10560
   ggaatcggtc aatacactac atggcgtgat ttcatatgcg cgattgctga tccccatgtg 10620
   tatcactggc aaactgtgat ggacgacacc gtcagtgcgt ccgtcgcgca ggctctcgat 10680
   gagctgatgc tttgggccga ggactgcccc gaagtccggc acctcgtgca cgcggatttc 10740
   ggctccaaca atgtcctgac ggacaatggc cgcataacag cggtcattga ctggagcgag 10800
   gcgatgttcg gggattccca atacgaggtc gccaacatct tcttctggag gccgtggttg 10860
   gcttgtatgg agcagcagac gcgctacttc gagcggaggc atccggagct tgcaggatcg 10920
   ccgcggctcc gggcgtatat gctccgcatt ggtcttgacc aactctatca gagcttggtt 10980
   gacggcaatt tcgatgatgc agcttgggcg cagggtcgat gcgacgcaat cgtccgatcc 11040
   ggagccggga ctgtcgggcg tacacaaatc gcccgcagaa gcgcggccgt ctggaccgat 11100
   ggctgtgtag aagtactcgc cgatagtgga aaccgacgcc ccagcactcg tccggatcgg 11160
   gagatggggg aggctaactg aaacacggaa ggagacaata ccggaaggaa cccgcgctat 11220
   gacggcaata aaaagacaga ataaaacgca cgggtgttgg gtcgtttgtt cataaacgcg 11280
   gggttcggtc ccagggctgg cactctgtcg ataccccacc gagaccccat tggggccaat 11340
   acgcccgcgt ttcttccttt tccccacccc accccccaag ttcgggtgaa ggcccagggc 11400
   tcgcagccaa cgtcggggcg gcaggccctg ccatagccac tggccccgtg ggttagggac 11460
   ggggtccccc atggggaatg gtttatggtt cgtgggggtt attattttgg gcgttgcgtg 11520
   gggtcaggtc cacgactgga ctgagcagac agacccatgg tttttggatg gcctgggcat 11580
   ggaccgcatg tactggcgcg acacgaacac cgggcgtctg tggctgccaa acacccccga 11640
   cccccaaaaa ccaccgcgcg gatttctggc gtgccaagct agtcgaccaa ttctcatgtt 11700
   tgacagctta tcatcgcaga tccgggcaac gttgttgcca ttgctgcagg cgcagaactg 11760
   gtaggtatgg aagatctata cattgaatca atattggcaa ttagccatat tagtcattgg 11820
   ttatatagca taaatcaata ttggctattg gccattgcat acgttgtatc tatatcataa 11880
   tatgtacatt tatattggct catgtccaat atgaccgcca t                     11921
<210> 226
   <211> 744
   <212> DNA
   <213> Artificial Sequence
<220>
   <223> In vitro synthesized DNA encoding a melanocortin receptor binding alpha-MSH mimetibody without secretory signal (p3391).
<400> 226
   cagatccagt cctactcctg cgagcacttc cgctggtgca agccggtggg aggcggagga 60
   agcggcggag gcggatcctg cccaccgtgc ccagcacctg aactcctggg gggaccgtca 120
   gtcttcctct tccccccaaa acccaaggac accctcatga tctcccggac ccctgaggtc 180
   acatgcgtgg tggtggacgt gagccacgaa gaccctgagg tcaagttcaa ctggtacgtg 240
   gacggcgtgg aggtgcataa tgccaagaca aagccgcggg aggagcagta caacagcacg 300
   taccgtgtgg tcagcgtcct caccgtcctg caccaggact ggctgaatgg caaggagtac 360
   aagtgcaagg tctccaacaa agccctccca gcccccatcg agaaaaccat ctccaaagcc 420
   aaagggcagc cccgagaacc acaggtgtac accctgcccc catcccggga tgagctgacc 480
   aagaaccagg tcagcctgac ctgcctggtc aaaggcttct atcccagcga catcgccgtg 540
   gagtgggaga gcaatgggca gccggagaac aactacaaga ccacgcctcc cgtgctggac 600
   tccgacggct ccttcttcct ctacagcaag ctcaccgtgg acaagagcag gtggcagcag 660
   gggaacgtct tctcatgctc cgtgatgcat gaggctctgc acaaccacta cacgcagaag 720
   agcctctccc tgtctctggg taaa                                        744
<210> 227
   <211> 248
   <212> PRT
   <213> Artificial Sequence
<220>
   <223> Melanocortin receptor binding alpha-MSH mimetibody without secretory signal encoded by an in vitro synthesized DNA (p3391).
<400> 227
   Gln Ile Gln Ser Tyr Ser Cys Glu His Phe Arg Trp Cys Lys Pro Val
    1               5                  10                  15
   Gly Gly Gly Gly Ser Gly Gly Gly Gly Ser Cys Pro Pro Cys Pro Ala
               20                  25                  30
   Pro Glu Leu Leu Gly Gly Pro Ser Val Phe Leu Phe Pro Pro Lys Pro
           35                  40                  45
   Lys Asp Thr Leu Met Ile Ser Arg Thr Pro Glu Val Thr Cys Val Val
       50                  55                  60
   Val Asp Val Ser His Glu Asp Pro Glu Val Lys Phe Asn Trp Tyr Val
   65                  70                  75                  80
   Asp Gly Val Glu Val His Asn Ala Lys Thr Lys Pro Arg Glu Glu Gln
                   85                  90                  95
   Tyr Asn Ser Thr Tyr Arg Val Val Ser Val Leu Thr Val Leu His Gln
               100                 105                 110
   Asp Trp Leu Asn Gly Lys Glu Tyr Lys Cys Lys Val Ser Asn Lys Ala
           115                 120                 125
   Leu Pro Ala Pro Ile Glu Lys Thr Ile Ser Lys Ala Lys Gly Gln Pro
       130                 135                 140
   Arg Glu Pro Gln Val Tyr Thr Leu Pro Pro Ser Arg Asp Glu Leu Thr
   145                 150                 155                 160
   Lys Asn Gln Val Ser Leu Thr Cys Leu Val Lys Gly Phe Tyr Pro Ser
                   165                 170                 175
   Asp Ile Ala Val Glu Trp Glu Ser Asn Gly Gln Pro Glu Asn Asn Tyr
               180                 185                 190
   Lys Thr Thr Pro Pro Val Leu Asp Ser Asp Gly Ser Phe Phe Leu Tyr
           195                 200                 205
   Ser Lys Leu Thr Val Asp Lys Ser Arg Trp Gln Gln Gly Asn Val Phe
       210                 215                 220
   Ser Cys Ser Val Met His Glu Ala Leu His Asn His Tyr Thr Gln Lys
   225                 230                 235                 240
   Ser Leu Ser Leu Ser Leu Gly Lys
                   245
<210> 228
   <211> 801
   <212> DNA
   <213> Artificial Sequence
<220>
   <223> In vitro synthesized DNA encoding a melanocortin receptor binding alpha-MSH mimetibody with secretory signal and V1 (p3391).
<400> 228
   atggcttggg tgtggacctt gctattcctg atggcggccg cccaaagtat acaggcccag 60
   atccagtcct actcctgcga gcacttccgc tggtgcaagc cggtgggagg cggaggaagc 120
   ggcggaggcg gatcctgccc accgtgccca gcacctgaac tcctgggggg accgtcagtc 180
   ttcctcttcc ccccaaaacc caaggacacc ctcatgatct cccggacccc tgaggtcaca 240
   tgcgtggtgg tggacgtgag ccacgaagac cctgaggtca agttcaactg gtacgtggac 300
   ggcgtggagg tgcataatgc caagacaaag ccgcgggagg agcagtacaa cagcacgtac 360
   cgtgtggtca gcgtcctcac cgtcctgcac caggactggc tgaatggcaa ggagtacaag 420
   tgcaaggtct ccaacaaagc cctcccagcc cccatcgaga aaaccatctc caaagccaaa 480
   gggcagcccc gagaaccaca ggtgtacacc ctgcccccat cccgggatga gctgaccaag 540
   aaccaggtca gcctgacctg cctggtcaaa ggcttctatc ccagcgacat cgccgtggag 600
   tgggagagca atgggcagcc ggagaacaac tacaagacca cgcctcccgt gctggactcc 660
   gacggctcct tcttcctcta cagcaagctc accgtggaca agagcaggtg gcagcagggg 720
   aacgtcttct catgctccgt gatgcatgag gctctgcaca accactacac gcagaagagc 780
   ctctccctgt ctctgggtaa a                                           801
<210> 229
   <211> 267
   <212> PRT
   <213> Artificial Sequence
<220>
   <223> Melanocortin receptor binding alpha-MSH mimetibody with secretory signal and V1 encoded by an in vitro synthesized DNA (p3391).
<400> 229
   Met Ala Trp Val Trp Thr Leu Leu Phe Leu Met Ala Ala Ala Gln Ser
    1               5                  10                  15
   Ile Gln Ala Gln Ile Gln Ser Tyr Ser Cys Glu His Phe Arg Trp Cys
               20                  25                  30
   Lys Pro Val Gly Gly Gly Gly Ser Gly Gly Gly Gly Ser Cys Pro Pro
           35                  40                  45
   Cys Pro Ala Pro Glu Leu Leu Gly Gly Pro Ser Val Phe Leu Phe Pro
       50                  55                  60
   Pro Lys Pro Lys Asp Thr Leu Met Ile Ser Arg Thr Pro Glu Val Thr
   65                  70                  75                  80
   Cys Val Val Val Asp Val Ser His Glu Asp Pro Glu Val Lys Phe Asn
                   85                  90                  95
   Trp Tyr Val Asp Gly Val Glu Val His Asn Ala Lys Thr Lys Pro Arg
               100                 105                 110
   Glu Glu Gln Tyr Asn Ser Thr Tyr Arg Val Val Ser Val Leu Thr Val
           115                 120                 125
   Leu His Gln Asp Trp Leu Asn Gly Lys Glu Tyr Lys Cys Lys Val Ser
       130                 135                 140
   Asn Lys Ala Leu Pro Ala Pro Ile Glu Lys Thr Ile Ser Lys Ala Lys
   145                 150                 155                 160
   Gly Gln Pro Arg Glu Pro Gln Val Tyr Thr Leu Pro Pro Ser Arg Asp
                   165                 170                 175
   Glu Leu Thr Lys Asn Gln Val Ser Leu Thr Cys Leu Val Lys Gly Phe
               180                 185                 190
   Tyr Pro Ser Asp Ile Ala Val Glu Trp Glu Ser Asn Gly Gln Pro Glu
           195                 200                 205
   Asn Asn Tyr Lys Thr Thr Pro Pro Val Leu Asp Ser Asp Gly Ser Phe
       210                 215                 220
   Phe Leu Tyr Ser Lys Leu Thr Val Asp Lys Ser Arg Trp Gln Gln Gly
   225                 230                 235                 240
   Asn Val Phe Ser Cys Ser Val Met His Glu Ala Leu His Asn His Tyr
                   245                 250                 255
   Thr Gln Lys Ser Leu Ser Leu Ser Leu Gly Lys
               260                 265
<210> 230
   <211> 11936
   <212> DNA
   <213> Artificial Sequence
<220>
   <223> In vitro synthesized DNA with expression vector functions that encodes an alpha-MSH mimetibody (p3391).
<400> 230
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcccag atccagtcct actcctgcga 1680
   gcacttccgc tggtgcaagc cggtgggagg cggaggaagc ggcggaggcg gatcctgccc 1740
   accgtgccca gcacctgaac tcctgggggg accgtcagtc ttcctcttcc ccccaaaacc 1800
   caaggacacc ctcatgatct cccggacccc tgaggtcaca tgcgtggtgg tggacgtgag 1860
   ccacgaagac cctgaggtca agttcaactg gtacgtggac ggcgtggagg tgcataatgc 1920
   caagacaaag ccgcgggagg agcagtacaa cagcacgtac cgtgtggtca gcgtcctcac 1980
   cgtcctgcac caggactggc tgaatggcaa ggagtacaag tgcaaggtct ccaacaaagc 2040
   cctcccagcc cccatcgaga aaaccatctc caaagccaaa gggcagcccc gagaaccaca 2100
   ggtgtacacc ctgcccccat cccgggatga gctgaccaag aaccaggtca gcctgacctg 2160
   cctggtcaaa ggcttctatc ccagcgacat cgccgtggag tgggagagca atgggcagcc 2220
   ggagaacaac tacaagacca cgcctcccgt gctggactcc gacggctcct tcttcctcta 2280
   cagcaagctc accgtggaca agagcaggtg gcagcagggg aacgtcttct catgctccgt 2340
   gatgcatgag gctctgcaca accactacac gcagaagagc ctctccctgt ctctgggtaa 2400
   atgagggccc gtttaaacag atccagacat gataagatac attgatgagt ttggacaaac 2460
   cacaactaga atgcagtgaa aaaaatgctt tatttgtgaa atttgtgatg ctattgcttt 2520
   atttgtaacc attataagct gcaataaaca agttaacaac aacaattgca ttcattttat 2580
   gtttcaggtt cagggggagg tgtgggaggt tttttaaagc aagtaaaacc tctacaaatg 2640
   tggtatggct gattatgatc cggctgcctc gcgcgtttcg gtgatgacgg tgaaaacctc 2700
   tgacacatgc agctcccgga gacggtcaca gcttgtctgt aagcggatgc cgggagcaga 2760
   caagcccgtc aggcgtcagc gggtgttggc gggtgtcggg gcgcagccat gaggtcgact 2820
   ctagaggatc gatgccccgc cccggacgaa ctaaacctga ctacgacatc tctgcccctt 2880
   cttcgcgggg cagtgcatgt aatcccttca gttggttggt acaacttgcc aactgggccc 2940
   tgttccacat gtgacacggg gggggaccaa acacaaaggg gttctctgac tgtagttgac 3000
   atccttataa atggatgtgc acatttgcca acactgagtg gctttcatcc tggagcagac 3060
   tttgcagtct gtggactgca acacaacatt gcctttatgt gtaactcttg gctgaagctc 3120
   ttacaccaat gctgggggac atgtacctcc caggggccca ggaagactac gggaggctac 3180
   accaacgtca atcagagggg cctgtgtagc taccgataag cggaccctca agagggcatt 3240
   agcaatagtg tttataaggc ccccttgtta accctaaacg ggtagcatat gcttcccggg 3300
   tagtagtata tactatccag actaacccta attcaatagc atatgttacc caacgggaag 3360
   catatgctat cgaattaggg ttagtaaaag ggtcctaagg aacagcgata tctcccaccc 3420
   catgagctgt cacggtttta tttacatggg gtcaggattc cacgagggta gtgaaccatt 3480
   ttagtcacaa gggcagtggc tgaagatcaa ggagcgggca gtgaactctc ctgaatcttc 3540
   gcctgcttct tcattctcct tcgtttagct aatagaataa ctgctgagtt gtgaacagta 3600
   aggtgtatgt gaggtgctcg aaaacaaggt ttcaggtgac gcccccagaa taaaatttgg 3660
   acggggggtt cagtggtggc attgtgctat gacaccaata taaccctcac aaaccccttg 3720
   ggcaataaat actagtgtag gaatgaaaca ttctgaatat ctttaacaat agaaatccat 3780
   ggggtgggga caagccgtaa agactggatg tccatctcac acgaatttat ggctatgggc 3840
   aacacataat cctagtgcaa tatgatactg gggttattaa gatgtgtccc aggcagggac 3900
   caagacaggt gaaccatgtt gttacactct atttgtaaca aggggaaaga gagtggacgc 3960
   cgacagcagc ggactccact ggttgtctct aacacccccg aaaattaaac ggggctccac 4020
   gccaatgggg cccataaaca aagacaagtg gccactcttt tttttgaaat tgtggagtgg 4080
   gggcacgcgt cagcccccac acgccgccct gcggttttgg actgtaaaat aagggtgtaa 4140
   taacttggct gattgtaacc ccgctaacca ctgcggtcaa accacttgcc cacaaaacca 4200
   ctaatggcac cccggggaat acctgcataa gtaggtgggc gggccaagat aggggcgcga 4260
   ttgctgcgat ctggaggaca aattacacac acttgcgcct gagcgccaag cacagggttg 4320
   ttggtcctca tattcacgag gtcgctgaga gcacggtggg ctaatgttgc catgggtagc 4380
   atatactacc caaatatctg gatagcatat gctatcctaa tctatatctg ggtagcatag 4440
   gctatcctaa tctatatctg ggtagcatat gctatcctaa tctatatctg ggtagtatat 4500
   gctatcctaa tttatatctg ggtagcatag gctatcctaa tctatatctg ggtagcatat 4560
   gctatcctaa tctatatctg ggtagtatat gctatcctaa tctgtatccg ggtagcatat 4620
   gctatcctaa tagagattag ggtagtatat gctatcctaa tttatatctg ggtagcatat 4680
   actacccaaa tatctggata gcatatgcta tcctaatcta tatctgggta gcatatgcta 4740
   tcctaatcta tatctgggta gcataggcta tcctaatcta tatctgggta gcatatgcta 4800
   tcctaatcta tatctgggta gtatatgcta tcctaattta tatctgggta gcataggcta 4860
   tcctaatcta tatctgggta gcatatgcta tcctaatcta tatctgggta gtatatgcta 4920
   tcctaatctg tatccgggta gcatatgcta tcctcatgca tatacagtca gcatatgata 4980
   cccagtagta gagtgggagt gctatccttt gcatatgccg ccacctccca agggggcgtg 5040
   aattttcgct gcttgtcctt ttcctgctgg ttgctcccat tcttaggtga atttaaggag 5100
   gccaggctaa agccgtcgca tgtctgattg ctcaccaggt aaatgtcgct aatgttttcc 5160
   aacgcgagaa ggtgttgagc gcggagctga gtgacgtgac aacatgggta tgcccaattg 5220
   ccccatgttg ggaggacgaa aatggtgaca agacagatgg ccagaaatac accaacagca 5280
   cgcatgatgt ctactgggga tttattcttt agtgcggggg aatacacggc ttttaatacg 5340
   attgagggcg tctcctaaca agttacatca ctcctgccct tcctcaccct catctccatc 5400
   acctccttca tctccgtcat ctccgtcatc accctccgcg gcagcccctt ccaccatagg 5460
   tggaaaccag ggaggcaaat ctactccatc gtcaaagctg cacacagtca ccctgatatt 5520
   gcaggtagga gcgggctttg tcataacaag gtccttaatc gcatccttca aaacctcagc 5580
   aaatatatga gtttgtaaaa agaccatgaa ataacagaca atggactccc ttagcgggcc 5640
   aggttgtggg ccgggtccag gggccattcc aaaggggaga cgactcaatg gtgtaagacg 5700
   acattgtgga atagcaaggg cagttcctcg ccttaggttg taaagggagg tcttactacc 5760
   tccatatacg aacacaccgg cgacccaagt tccttcgtcg gtagtccttt ctacgtgact 5820
   cctagccagg agagctctta aaccttctgc aatgttctca aatttcgggt tggaacctcc 5880
   ttgaccacga tgcttttcca aaccaccctc cttttttgcg ccctgcctcc atcaccctga 5940
   ccccggggtc cagtgcttgg gccttctcct gggtcatctg cggggccctg ctctatcgct 6000
   cccgggggca cgtcaggctc accatctggg ccaccttctt ggtggtattc aaaataatcg 6060
   gcttccccta cagggtggaa aaatggcctt ctacctggag ggggcctgcg cggtggagac 6120
   ccggatgatg atgactgact actgggactc ctgggcctct tttctccacg tccacgacct 6180
   ctccccctgg ctctttcacg acttcccccc ctggctcttt cacgtcctct accccggcgg 6240
   cctccactac ctcctcgacc ccggcctcca ctacctcctc gaccccggcc tccactgcct 6300
   cctcgacccc ggcctccacc tcctgctcct gcccctcctg ctcctgcccc tcctcctgct 6360
   cctgcccctc ctgcccctcc tgctcctgcc cctcctgccc ctcctgctcc tgcccctcct 6420
   gcccctcctg ctcctgcccc tcctgcccct cctcctgctc ctgcccctcc tgcccctcct 6480
   cctgctcctg cccctcctgc ccctcctgct cctgcccctc ctgcccctcc tgctcctgcc 6540
   cctcctgccc ctcctgctcc tgcccctcct gctcctgccc ctcctgctcc tgcccctcct 6600
   gctcctgccc ctcctgcccc tcctgcccct cctcctgctc ctgcccctcc tgctcctgcc 6660
   cctcctgccc ctcctgcccc tcctgctcct gcccctcctc ctgctcctgc ccctcctgcc 6720
   cctcctgccc ctcctcctgc tcctgcccct cctgcccctc ctcctgctcc tgcccctcct 6780
   cctgctcctg cccctcctgc ccctcctgcc cctcctcctg ctcctgcccc tcctgcccct 6840
   cctcctgctc ctgcccctcc tcctgctcct gcccctcctg cccctcctgc ccctcctcct 6900
   gctcctgccc ctcctcctgc tcctgcccct cctgcccctc ctgcccctcc tgcccctcct 6960
   cctgctcctg cccctcctcc tgctcctgcc cctcctgctc ctgcccctcc cgctcctgct 7020
   cctgctcctg ttccaccgtg ggtccctttg cagccaatgc aacttggacg tttttggggt 7080
   ctccggacac catctctatg tcttggccct gatcctgagc cgcccggggc tcctggtctt 7140
   ccgcctcctc gtcctcgtcc tcttccccgt cctcgtccat ggttatcacc ccctcttctt 7200
   tgaggtccac tgccgccgga gccttctggt ccagatgtgt ctcccttctc tcctaggcca 7260
   tttccaggtc ctgtacctgg cccctcgtca gacatgattc acactaaaag agatcaatag 7320
   acatctttat tagacgacgc tcagtgaata cagggagtgc agactcctgc cccctccaac 7380
   agccccccca ccctcatccc cttcatggtc gctgtcagac agatccaggt ctgaaaattc 7440
   cccatcctcc gaaccatcct cgtcctcatc accaattact cgcagcccgg aaaactcccg 7500
   ctgaacatcc tcaagatttg cgtcctgagc ctcaagccag gcctcaaatt cctcgtcccc 7560
   ctttttgctg gacggtaggg atggggattc tcgggacccc tcctcttcct cttcaaggtc 7620
   accagacaga gatgctactg gggcaacgga agaaaagctg ggtgcggcct gtgaggatca 7680
   gcttatcgat gataagctgt caaacatgag aattcttgaa gacgaaaggg cctcgtgata 7740
   cgcctatttt tataggttaa tgtcatgata ataatggttt cttagacgtc aggtggcact 7800
   tttcggggaa atgtgcgcgg aacccctatt tgtttatttt tctaaataca ttcaaatatg 7860
   tatccgctca tgagacaata accctgataa atgcttcaat aatattgaaa aaggaagagt 7920
   atgagtattc aacatttccg tgtcgccctt attccctttt ttgcggcatt ttgccttcct 7980
   gtttttgctc acccagaaac gctggtgaaa gtaaaagatg ctgaagatca gttgggtgca 8040
   cgagtgggtt acatcgaact ggatctcaac agcggtaaga tccttgagag ttttcgcccc 8100
   gaagaacgtt ttccaatgat gagcactttt aaagttctgc tatgtggcgc ggtattatcc 8160
   cgtgttgacg ccgggcaaga gcaactcggt cgccgcatac actattctca gaatgacttg 8220
   gttgagtact caccagtcac agaaaagcat cttacggatg gcatgacagt aagagaatta 8280
   tgcagtgctg ccataaccat gagtgataac actgcggcca acttacttct gacaacgatc 8340
   ggaggaccga aggagctaac cgcttttttg cacaacatgg gggatcatgt aactcgcctt 8400
   gatcgttggg aaccggagct gaatgaagcc ataccaaacg acgagcgtga caccacgatg 8460
   cctgcagcaa tggcaacaac gttgcgcaaa ctattaactg gcgaactact tactctagct 8520
   tcccggcaac aattaataga ctggatggag gcggataaag ttgcaggacc acttctgcgc 8580
   tcggcccttc cggctggctg gtttattgct gataaatctg gagccggtga gcgtgggtct 8640
   cgcggtatca ttgcagcact ggggccagat ggtaagccct cccgtatcgt agttatctac 8700
   acgacgggga gtcaggcaac tatggatgaa cgaaatagac agatcgctga gataggtgcc 8760
   tcactgatta agcattggta actgtcagac caagtttact catatatact ttagattgat 8820
   ttaaaacttc atttttaatt taaaaggatc taggtgaaga tcctttttga taatctcatg 8880
   accaaaatcc cttaacgtga gttttcgttc cactgagcgt cagaccccgt agaaaagatc 8940
   aaaggatctt cttgagatcc tttttttctg cgcgtaatct gctgcttgca aacaaaaaaa 9000
   ccaccgctac cagcggtggt ttgtttgccg gatcaagagc taccaactct ttttccgaag 9060
   gtaactggct tcagcagagc gcagatacca aatactgtcc ttctagtgta gccgtagtta 9120
   ggccaccact tcaagaactc tgtagcaccg cctacatacc tcgctctgct aatcctgtta 9180
   ccagtggctg ctgccagtgg cgataagtcg tgtcttaccg ggttggactc aagacgatag 9240
   ttaccggata aggcgcagcg gtcgggctga acggggggtt cgtgcacaca gcccagcttg 9300
   gagcgaacga cctacaccga actgagatac ctacagcgtg agctatgaga aagcgccacg 9360
   cttcccgaag ggagaaaggc ggacaggtat ccggtaagcg gcagggtcgg aacaggagag 9420
   cgcacgaggg agcttccagg gggaaacgcc tggtatcttt atagtcctgt cgggtttcgc 9480
   cacctctgac ttgagcgtcg atttttgtga tgctcgtcag gggggcggag cctatggaaa 9540
   aacgccagca acgcggcctt tttacggttc ctggcctttt gctggccttg aagctgtccc 9600
   tgatggtcgt catctacctg cctggacagc atggcctgca acgcgggcat cccgatgccg 9660
   ccggaagcga gaagaatcat aatggggaag gccatccagc ctcgcgtcgc gaacgccagc 9720
   aagacgtagc ccagcgcgtc ggccccgaga tgcgccgcgt gcggctgctg gagatggcgg 9780
   acgcgatgga tatgttctgc caagggttgg tttgcgcatt cacagttctc cgcaagaatt 9840
   gattggctcc aattcttgga gtggtgaatc cgttagcgag gtgccgccct gcttcatccc 9900
   cgtggcccgt tgctcgcgtt tgctggcggt gtccccggaa gaaatatatt tgcatgtctt 9960
   tagttctatg atgacacaaa ccccgcccag cgtcttgtca ttggcgaatt cgaacacgca 10020
   gatgcagtcg gggcggcgcg gtccgaggtc cacttcgcat attaaggtga cgcgtgtggc 10080
   ctcgaacacc gagcgaccct gcagcgaccc gcttaacagc gtcaacagcg tgccgcagat 10140
   cccggggggc aatgagatat gaaaaagcct gaactcaccg cgacgtctgt cgagaagttt 10200
   ctgatcgaaa agttcgacag cgtctccgac ctgatgcagc tctcggaggg cgaagaatct 10260
   cgtgctttca gcttcgatgt aggagggcgt ggatatgtcc tgcgggtaaa tagctgcgcc 10320
   gatggtttct acaaagatcg ttatgtttat cggcactttg catcggccgc gctcccgatt 10380
   ccggaagtgc ttgacattgg ggaattcagc gagagcctga cctattgcat ctcccgccgt 10440
   gcacagggtg tcacgttgca agacctgcct gaaaccgaac tgcccgctgt tctgcagccg 10500
   gtcgcggagg ccatggatgc gatcgctgcg gccgatctta gccagacgag cgggttcggc 10560
   ccattcggac cgcaaggaat cggtcaatac actacatggc gtgatttcat atgcgcgatt 10620
   gctgatcccc atgtgtatca ctggcaaact gtgatggacg acaccgtcag tgcgtccgtc 10680
   gcgcaggctc tcgatgagct gatgctttgg gccgaggact gccccgaagt ccggcacctc 10740
   gtgcacgcgg atttcggctc caacaatgtc ctgacggaca atggccgcat aacagcggtc 10800
   attgactgga gcgaggcgat gttcggggat tcccaatacg aggtcgccaa catcttcttc 10860
   tggaggccgt ggttggcttg tatggagcag cagacgcgct acttcgagcg gaggcatccg 10920
   gagcttgcag gatcgccgcg gctccgggcg tatatgctcc gcattggtct tgaccaactc 10980
   tatcagagct tggttgacgg caatttcgat gatgcagctt gggcgcaggg tcgatgcgac 11040
   gcaatcgtcc gatccggagc cgggactgtc gggcgtacac aaatcgcccg cagaagcgcg 11100
   gccgtctgga ccgatggctg tgtagaagta ctcgccgata gtggaaaccg acgccccagc 11160
   actcgtccgg atcgggagat gggggaggct aactgaaaca cggaaggaga caataccgga 11220
   aggaacccgc gctatgacgg caataaaaag acagaataaa acgcacgggt gttgggtcgt 11280
   ttgttcataa acgcggggtt cggtcccagg gctggcactc tgtcgatacc ccaccgagac 11340
   cccattgggg ccaatacgcc cgcgtttctt ccttttcccc accccacccc ccaagttcgg 11400
   gtgaaggccc agggctcgca gccaacgtcg gggcggcagg ccctgccata gccactggcc 11460
   ccgtgggtta gggacggggt cccccatggg gaatggttta tggttcgtgg gggttattat 11520
   tttgggcgtt gcgtggggtc aggtccacga ctggactgag cagacagacc catggttttt 11580
   ggatggcctg ggcatggacc gcatgtactg gcgcgacacg aacaccgggc gtctgtggct 11640
   gccaaacacc cccgaccccc aaaaaccacc gcgcggattt ctggcgtgcc aagctagtcg 11700
   accaattctc atgtttgaca gcttatcatc gcagatccgg gcaacgttgt tgccattgct 11760
   gcaggcgcag aactggtagg tatggaagat ctatacattg aatcaatatt ggcaattagc 11820
   catattagtc attggttata tagcataaat caatattggc tattggccat tgcatacgtt 11880
   gtatctatat cataatatgt acatttatat tggctcatgt ccaatatgac cgccat     11936
<210> 231
   <211> 792
   <212> DNA
   <213> Artificial Sequence
<220>
   <223> In vitro synthesized DNA encoding a melanocortin receptor binding alpha-MSH mimetibody without secretory signal (p3458).
<400> 231
   cagatccagg gaggctccta ctcctgcgag cacttccgct ggtgcaagcc ggtgggatcc 60
   ggtggaggct ccggtacctt agtcgccgtc tcctcagagc ccaaatctgc cgacaaaact 120
   cacgcctgcc caccgtgccc agcacctgaa ctcctggggg gaccgtcagt cttcctcttc 180
   cccccaaaac ccaaggacac cctcatgatc tcccggaccc ctgaggtcac atgcgtggtg 240
   gtggacgtga gccacgaaga ccctgaggtc aagttcaact ggtacgtgga cggcgtggag 300
   gtgcataatg ccaagacaaa gccgcgggag gagcagtaca acagcacgta ccgtgtggtc 360
   agcgtcctca ccgtcctgca ccaggactgg ctgaatggca aggagtacaa gtgcaaggtc 420
   tccaacaaag ccctcccagc ccccatcgag aaaaccatct ccaaagccaa agggcagccc 480
   cgagaaccac aggtgtacac cctgccccca tcccgggatg agctgaccaa gaaccaggtc 540
   agcctgacct gcctggtcaa aggcttctat cccagcgaca tcgccgtgga gtgggagagc 600
   aatgggcagc cggagaacaa ctacaagacc acgcctcccg tgctggactc cgacggctcc 660
   ttcttcctct acagcaagct caccgtggac aagagcaggt ggcagcaggg gaacgtcttc 720
   tcatgctccg tgatgcatga ggctctgcac aaccactaca cgcagaagag cctctccctg 780
   tctccgggta aa                                                     792
<210> 232
   <211> 264
   <212> PRT
   <213> Artificial Sequence
<220>
   <223> Melanocortin receptor binding alpha-MSH mimetibody without secretory signal encoded by an in vitro synthesized DNA (p3458).
<400> 232
   Gln Ile Gln Gly Gly Ser Tyr Ser Cys Glu His Phe Arg Trp Cys Lys
    1               5                  10                  15
   Pro Val Gly Ser Gly Gly Gly Ser Gly Thr Leu Val Ala Val Ser Ser
               20                  25                  30
   Glu Pro Lys Ser Ala Asp Lys Thr His Ala Cys Pro Pro Cys Pro Ala
           35                  40                  45
   Pro Glu Leu Leu Gly Gly Pro Ser Val Phe Leu Phe Pro Pro Lys Pro
       50                  55                  60
   Lys Asp Thr Leu Met Ile Ser Arg Thr Pro Glu Val Thr Cys Val Val
   65                  70                  75                  80
   Val Asp Val Ser His Glu Asp Pro Glu Val Lys Phe Asn Trp Tyr Val
                   85                  90                  95
   Asp Gly Val Glu Val His Asn Ala Lys Thr Lys Pro Arg Glu Glu Gln
               100                 105                 110
   Tyr Asn Ser Thr Tyr Arg Val Val Ser Val Leu Thr Val Leu His Gln
           115                 120                 125
   Asp Trp Leu Asn Gly Lys Glu Tyr Lys Cys Lys Val Ser Asn Lys Ala
       130                 135                 140
   Leu Pro Ala Pro Ile Glu Lys Thr Ile Ser Lys Ala Lys Gly Gln Pro
   145                 150                 155                 160
   Arg Glu Pro Gln Val Tyr Thr Leu Pro Pro Ser Arg Asp Glu Leu Thr
                   165                 170                 175
   Lys Asn Gln Val Ser Leu Thr Cys Leu Val Lys Gly Phe Tyr Pro Ser
               180                 185                 190
   Asp Ile Ala Val Glu Trp Glu Ser Asn Gly Gln Pro Glu Asn Asn Tyr
           195                 200                 205
   Lys Thr Thr Pro Pro Val Leu Asp Ser Asp Gly Ser Phe Phe Leu Tyr
       210                 215                 220
   Ser Lys Leu Thr Val Asp Lys Ser Arg Trp Gln Gln Gly Asn Val Phe
   225                 230                 235                 240
   Ser Cys Ser Val Met His Glu Ala Leu His Asn His Tyr Thr Gln Lys
                   245                 250                 255
   Ser Leu Ser Leu Ser Pro Gly Lys
               260
<210> 233
   <211> 849
   <212> DNA
   <213> Artificial Sequence
<220>
   <223> In vitro synthesized DNA encoding a melanocortin receptor binding alpha-MSH mimetibody with secretory signal and V1 (p3458).
<400> 233
   atggcttggg tgtggacctt gctattcctg atggcggccg cccaaagtat acaggcccag 60
   atccagggag gctcctactc ctgcgagcac ttccgctggt gcaagccggt gggatccggt 120
   ggaggctccg gtaccttagt cgccgtctcc tcagagccca aatctgccga caaaactcac 180
   gcctgcccac cgtgcccagc acctgaactc ctggggggac cgtcagtctt cctcttcccc 240
   ccaaaaccca aggacaccct catgatctcc cggacccctg aggtcacatg cgtggtggtg 300
   gacgtgagcc acgaagaccc tgaggtcaag ttcaactggt acgtggacgg cgtggaggtg 360
   cataatgcca agacaaagcc gcgggaggag cagtacaaca gcacgtaccg tgtggtcagc 420
   gtcctcaccg tcctgcacca ggactggctg aatggcaagg agtacaagtg caaggtctcc 480
   aacaaagccc tcccagcccc catcgagaaa accatctcca aagccaaagg gcagccccga 540
   gaaccacagg tgtacaccct gcccccatcc cgggatgagc tgaccaagaa ccaggtcagc 600
   ctgacctgcc tggtcaaagg cttctatccc agcgacatcg ccgtggagtg ggagagcaat 660
   gggcagccgg agaacaacta caagaccacg cctcccgtgc tggactccga cggctccttc 720
   ttcctctaca gcaagctcac cgtggacaag agcaggtggc agcaggggaa cgtcttctca 780
   tgctccgtga tgcatgaggc tctgcacaac cactacacgc agaagagcct ctccctgtct 840
   ccgggtaaa                                                         849
<210> 234
   <211> 283
   <212> PRT
   <213> Artificial Sequence
<220>
   <223> Melanocortin receptor binding alpha-MSH mimetibody with secretory signal and V1 encoded by an in vitro synthesized DNA (p3458).
<400> 234
   Met Ala Trp Val Trp Thr Leu Leu Phe Leu Met Ala Ala Ala Gln Ser
    1               5                  10                  15
   Ile Gln Ala Gln Ile Gln Gly Gly Ser Tyr Ser Cys Glu His Phe Arg
               20                  25                  30
   Trp Cys Lys Pro Val Gly Ser Gly Gly Gly Ser Gly Thr Leu Val Ala
           35                  40                  45
   Val Ser Ser Glu Pro Lys Ser Ala Asp Lys Thr His Ala Cys Pro Pro
       50                  55                  60
   Cys Pro Ala Pro Glu Leu Leu Gly Gly Pro Ser Val Phe Leu Phe Pro
   65                  70                  75                  80
   Pro Lys Pro Lys Asp Thr Leu Met Ile Ser Arg Thr Pro Glu Val Thr
                       85                  90                  95
   Cys Val Val Val Asp Val Ser His Glu Asp Pro Glu Val Lys Phe Asn
                   100                 105                 110
   Trp Tyr Val Asp Gly Val Glu Val His Asn Ala Lys Thr Lys Pro Arg
               115                 120                 125
   Glu Glu Gln Tyr Asn Ser Thr Tyr Arg Val Val Ser Val Leu Thr Val
       130                 135                 140
   Leu His Gln Asp Trp Leu Asn Gly Lys Glu Tyr Lys Cys Lys Val Ser
   145                 150                 155                 160
   Asn Lys Ala Leu Pro Ala Pro Ile Glu Lys Thr Ile Ser Lys Ala Lys
                   165                 170                 175
   Gly Gln Pro Arg Glu Pro Gln Val Tyr Thr Leu Pro Pro Ser Arg Asp
               180                 185                 190
   Glu Leu Thr Lys Asn Gln Val Ser Leu Thr Cys Leu Val Lys Gly Phe
           195                 200                 205
   Tyr Pro Ser Asp Ile Ala Val Glu Trp Glu Ser Asn Gly Gln Pro Glu
       210                 215                 220
   Asn Asn Tyr Lys Thr Thr Pro Pro Val Leu Asp Ser Asp Gly Ser Phe
   225                 230                 235                 240
   Phe Leu Tyr Ser Lys Leu Thr Val Asp Lys Ser Arg Trp Gln Gln Gly
                   245                 250                 255
   Asn Val Phe Ser Cys Ser Val Met His Glu Ala Leu His Asn His Tyr
               260                 265                 270
   Thr Gln Lys Ser Leu Ser Leu Ser Pro Gly Lys
           275                 280
<210> 235
   <211> 11984
   <212> DNA
   <213> Artificial Sequence
<220>
   <223> In vitro synthesized DNA with expression vector functions that encodes an alpha-MSH mimetibody (p3458).
<400> 235
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcccag atccagggag gctcctactc 1680
   ctgcgagcac ttccgctggt gcaagccggt gggatccggt ggaggctccg gtaccttagt 1740
   cgccgtctcc tcagagccca aatctgccga caaaactcac gcctgcccac cgtgcccagc 1800
   acctgaactc ctggggggac cgtcagtctt cctcttcccc ccaaaaccca aggacaccct 1860
   catgatctcc cggacccctg aggtcacatg cgtggtggtg gacgtgagcc acgaagaccc 1920
   tgaggtcaag ttcaactggt acgtggacgg cgtggaggtg cataatgcca agacaaagcc 1980
   gcgggaggag cagtacaaca gcacgtaccg tgtggtcagc gtcctcaccg tcctgcacca 2040
   ggactggctg aatggcaagg agtacaagtg caaggtctcc aacaaagccc tcccagcccc 2100
   catcgagaaa accatctcca aagccaaagg gcagccccga gaaccacagg tgtacaccct 2160
   gcccccatcc cgggatgagc tgaccaagaa ccaggtcagc ctgacctgcc tggtcaaagg 2220
   cttctatccc agcgacatcg ccgtggagtg ggagagcaat gggcagccgg agaacaacta 2280
   caagaccacg cctcccgtgc tggactccga cggctccttc ttcctctaca gcaagctcac 2340
   cgtggacaag agcaggtggc agcaggggaa cgtcttctca tgctccgtga tgcatgaggc 2400
   tctgcacaac cactacacgc agaagagcct ctccctgtct ccgggtaaat gagggcccgt 2460
   ttaaacagat ccagacatga taagatacat tgatgagttt ggacaaacca caactagaat 2520
   gcagtgaaaa aaatgcttta tttgtgaaat ttgtgatgct attgctttat ttgtaaccat 2580
   tataagctgc aataaacaag ttaacaacaa caattgcatt cattttatgt ttcaggttca 2640
   gggggaggtg tgggaggttt tttaaagcaa gtaaaacctc tacaaatgtg gtatggctga 2700
   ttatgatccg gctgcctcgc gcgtttcggt gatgacggtg aaaacctctg acacatgcag 2760
   ctcccggaga cggtcacagc ttgtctgtaa gcggatgccg ggagcagaca agcccgtcag 2820
   gcgtcagcgg gtgttggcgg gtgtcggggc gcagccatga ggtcgactct agaggatcga 2880
   tgccccgccc cggacgaact aaacctgact acgacatctc tgccccttct tcgcggggca 2940
   gtgcatgtaa tcccttcagt tggttggtac aacttgccaa ctgggccctg ttccacatgt 3000
   gacacggggg gggaccaaac acaaaggggt tctctgactg tagttgacat ccttataaat 3060
   ggatgtgcac atttgccaac actgagtggc tttcatcctg gagcagactt tgcagtctgt 3120
   ggactgcaac acaacattgc ctttatgtgt aactcttggc tgaagctctt acaccaatgc 3180
   tgggggacat gtacctccca ggggcccagg aagactacgg gaggctacac caacgtcaat 3240
   cagaggggcc tgtgtagcta ccgataagcg gaccctcaag agggcattag caatagtgtt 3300
   tataaggccc ccttgttaac cctaaacggg tagcatatgc ttcccgggta gtagtatata 3360
   ctatccagac taaccctaat tcaatagcat atgttaccca acgggaagca tatgctatcg 3420
   aattagggtt agtaaaaggg tcctaaggaa cagcgatatc tcccacccca tgagctgtca 3480
   cggttttatt tacatggggt caggattcca cgagggtagt gaaccatttt agtcacaagg 3540
   gcagtggctg aagatcaagg agcgggcagt gaactctcct gaatcttcgc ctgcttcttc 3600
   attctccttc gtttagctaa tagaataact gctgagttgt gaacagtaag gtgtatgtga 3660
   ggtgctcgaa aacaaggttt caggtgacgc ccccagaata aaatttggac ggggggttca 3720
   gtggtggcat tgtgctatga caccaatata accctcacaa accccttggg caataaatac 3780
   tagtgtagga atgaaacatt ctgaatatct ttaacaatag aaatccatgg ggtggggaca 3840
   agccgtaaag actggatgtc catctcacac gaatttatgg ctatgggcaa cacataatcc 3900
   tagtgcaata tgatactggg gttattaaga tgtgtcccag gcagggacca agacaggtga 3960
   accatgttgt tacactctat ttgtaacaag gggaaagaga gtggacgccg acagcagcgg 4020
   actccactgg ttgtctctaa cacccccgaa aattaaacgg ggctccacgc caatggggcc 4080
   cataaacaaa gacaagtggc cactcttttt tttgaaattg tggagtgggg gcacgcgtca 4140
   gcccccacac gccgccctgc ggttttggac tgtaaaataa gggtgtaata acttggctga 4200
   ttgtaacccc gctaaccact gcggtcaaac cacttgccca caaaaccact aatggcaccc 4260
   cggggaatac ctgcataagt aggtgggcgg gccaagatag gggcgcgatt gctgcgatct 4320
   ggaggacaaa ttacacacac ttgcgcctga gcgccaagca cagggttgtt ggtcctcata 4380
   ttcacgaggt cgctgagagc acggtgggct aatgttgcca tgggtagcat atactaccca 4440
   aatatctgga tagcatatgc tatcctaatc tatatctggg tagcataggc tatcctaatc 4500
   tatatctggg tagcatatgc tatcctaatc tatatctggg tagtatatgc tatcctaatt 4560
   tatatctggg tagcataggc tatcctaatc tatatctggg tagcatatgc tatcctaatc 4620
   tatatctggg tagtatatgc tatcctaatc tgtatccggg tagcatatgc tatcctaata 4680
   gagattaggg tagtatatgc tatcctaatt tatatctggg tagcatatac tacccaaata 4740
   tctggatagc atatgctatc ctaatctata tctgggtagc atatgctatc ctaatctata 4800
   tctgggtagc ataggctatc ctaatctata tctgggtagc atatgctatc ctaatctata 4860
   tctgggtagt atatgctatc ctaatttata tctgggtagc ataggctatc ctaatctata 4920
   tctgggtagc atatgctatc ctaatctata tctgggtagt atatgctatc ctaatctgta 4980
   tccgggtagc atatgctatc ctcatgcata tacagtcagc atatgatacc cagtagtaga 5040
   gtgggagtgc tatcctttgc atatgccgcc acctcccaag ggggcgtgaa ttttcgctgc 5100
   ttgtcctttt cctgctggtt gctcccattc ttaggtgaat ttaaggaggc caggctaaag 5160
   ccgtcgcatg tctgattgct caccaggtaa atgtcgctaa tgttttccaa cgcgagaagg 5220
   tgttgagcgc ggagctgagt gacgtgacaa catgggtatg cccaattgcc ccatgttggg 5280
   aggacgaaaa tggtgacaag acagatggcc agaaatacac caacagcacg catgatgtct 5340
   actggggatt tattctttag tgcgggggaa tacacggctt ttaatacgat tgagggcgtc 5400
   tcctaacaag ttacatcact cctgcccttc ctcaccctca tctccatcac ctccttcatc 5460
   tccgtcatct ccgtcatcac cctccgcggc agccccttcc accataggtg gaaaccaggg 5520
   aggcaaatct actccatcgt caaagctgca cacagtcacc ctgatattgc aggtaggagc 5580
   gggctttgtc ataacaaggt ccttaatcgc atccttcaaa acctcagcaa atatatgagt 5640
   ttgtaaaaag accatgaaat aacagacaat ggactccctt agcgggccag gttgtgggcc 5700
   gggtccaggg gccattccaa aggggagacg actcaatggt gtaagacgac attgtggaat 5760
   agcaagggca gttcctcgcc ttaggttgta aagggaggtc ttactacctc catatacgaa 5820
   cacaccggcg acccaagttc cttcgtcggt agtcctttct acgtgactcc tagccaggag 5880
   agctcttaaa ccttctgcaa tgttctcaaa tttcgggttg gaacctcctt gaccacgatg 5940
   cttttccaaa ccaccctcct tttttgcgcc ctgcctccat caccctgacc ccggggtcca 6000
   gtgcttgggc cttctcctgg gtcatctgcg gggccctgct ctatcgctcc cgggggcacg 6060
   tcaggctcac catctgggcc accttcttgg tggtattcaa aataatcggc ttcccctaca 6120
   gggtggaaaa atggccttct acctggaggg ggcctgcgcg gtggagaccc ggatgatgat 6180
   gactgactac tgggactcct gggcctcttt tctccacgtc cacgacctct ccccctggct 6240
   ctttcacgac ttccccccct ggctctttca cgtcctctac cccggcggcc tccactacct 6300
   cctcgacccc ggcctccact acctcctcga ccccggcctc cactgcctcc tcgaccccgg 6360
   cctccacctc ctgctcctgc ccctcctgct cctgcccctc ctcctgctcc tgcccctcct 6420
   gcccctcctg ctcctgcccc tcctgcccct cctgctcctg cccctcctgc ccctcctgct 6480
   cctgcccctc ctgcccctcc tcctgctcct gcccctcctg cccctcctcc tgctcctgcc 6540
   cctcctgccc ctcctgctcc tgcccctcct gcccctcctg ctcctgcccc tcctgcccct 6600
   cctgctcctg cccctcctgc tcctgcccct cctgctcctg cccctcctgc tcctgcccct 6660
   cctgcccctc ctgcccctcc tcctgctcct gcccctcctg ctcctgcccc tcctgcccct 6720
   cctgcccctc ctgctcctgc ccctcctcct gctcctgccc ctcctgcccc tcctgcccct 6780
   cctcctgctc ctgcccctcc tgcccctcct cctgctcctg cccctcctcc tgctcctgcc 6840
   cctcctgccc ctcctgcccc tcctcctgct cctgcccctc ctgcccctcc tcctgctcct 6900
   gcccctcctc ctgctcctgc ccctcctgcc cctcctgccc ctcctcctgc tcctgcccct 6960
   cctcctgctc ctgcccctcc tgcccctcct gcccctcctg cccctcctcc tgctcctgcc 7020
   cctcctcctg ctcctgcccc tcctgctcct gcccctcccg ctcctgctcc tgctcctgtt 7080
   ccaccgtggg tccctttgca gccaatgcaa cttggacgtt tttggggtct ccggacacca 7140
   tctctatgtc ttggccctga tcctgagccg cccggggctc ctggtcttcc gcctcctcgt 7200
   cctcgtcctc ttccccgtcc tcgtccatgg ttatcacccc ctcttctttg aggtccactg 7260
   ccgccggagc cttctggtcc agatgtgtct cccttctctc ctaggccatt tccaggtcct 7320
   gtacctggcc cctcgtcaga catgattcac actaaaagag atcaatagac atctttatta 7380
   gacgacgctc agtgaataca gggagtgcag actcctgccc cctccaacag cccccccacc 7440
   ctcatcccct tcatggtcgc tgtcagacag atccaggtct gaaaattccc catcctccga 7500
   accatcctcg tcctcatcac caattactcg cagcccggaa aactcccgct gaacatcctc 7560
   aagatttgcg tcctgagcct caagccaggc ctcaaattcc tcgtccccct ttttgctgga 7620
   cggtagggat ggggattctc gggacccctc ctcttcctct tcaaggtcac cagacagaga 7680
   tgctactggg gcaacggaag aaaagctggg tgcggcctgt gaggatcagc ttatcgatga 7740
   taagctgtca aacatgagaa ttcttgaaga cgaaagggcc tcgtgatacg cctattttta 7800
   taggttaatg tcatgataat aatggtttct tagacgtcag gtggcacttt tcggggaaat 7860
   gtgcgcggaa cccctatttg tttatttttc taaatacatt caaatatgta tccgctcatg 7920
   agacaataac cctgataaat gcttcaataa tattgaaaaa ggaagagtat gagtattcaa 7980
   catttccgtg tcgcccttat tccctttttt gcggcatttt gccttcctgt ttttgctcac 8040
   ccagaaacgc tggtgaaagt aaaagatgct gaagatcagt tgggtgcacg agtgggttac 8100
   atcgaactgg atctcaacag cggtaagatc cttgagagtt ttcgccccga agaacgtttt 8160
   ccaatgatga gcacttttaa agttctgcta tgtggcgcgg tattatcccg tgttgacgcc 8220
   gggcaagagc aactcggtcg ccgcatacac tattctcaga atgacttggt tgagtactca 8280
   ccagtcacag aaaagcatct tacggatggc atgacagtaa gagaattatg cagtgctgcc 8340
   ataaccatga gtgataacac tgcggccaac ttacttctga caacgatcgg aggaccgaag 8400
   gagctaaccg cttttttgca caacatgggg gatcatgtaa ctcgccttga tcgttgggaa 8460
   ccggagctga atgaagccat accaaacgac gagcgtgaca ccacgatgcc tgcagcaatg 8520
   gcaacaacgt tgcgcaaact attaactggc gaactactta ctctagcttc ccggcaacaa 8580
   ttaatagact ggatggaggc ggataaagtt gcaggaccac ttctgcgctc ggcccttccg 8640
   gctggctggt ttattgctga taaatctgga gccggtgagc gtgggtctcg cggtatcatt 8700
   gcagcactgg ggccagatgg taagccctcc cgtatcgtag ttatctacac gacggggagt 8760
   caggcaacta tggatgaacg aaatagacag atcgctgaga taggtgcctc actgattaag 8820
   cattggtaac tgtcagacca agtttactca tatatacttt agattgattt aaaacttcat 8880
   ttttaattta aaaggatcta ggtgaagatc ctttttgata atctcatgac caaaatccct 8940
   taacgtgagt tttcgttcca ctgagcgtca gaccccgtag aaaagatcaa aggatcttct 9000
   tgagatcctt tttttctgcg cgtaatctgc tgcttgcaaa caaaaaaacc accgctacca 9060
   gcggtggttt gtttgccgga tcaagagcta ccaactcttt ttccgaaggt aactggcttc 9120
   agcagagcgc agataccaaa tactgtcctt ctagtgtagc cgtagttagg ccaccacttc 9180
   aagaactctg tagcaccgcc tacatacctc gctctgctaa tcctgttacc agtggctgct 9240
   gccagtggcg ataagtcgtg tcttaccggg ttggactcaa gacgatagtt accggataag 9300
   gcgcagcggt cgggctgaac ggggggttcg tgcacacagc ccagcttgga gcgaacgacc 9360
   tacaccgaac tgagatacct acagcgtgag ctatgagaaa gcgccacgct tcccgaaggg 9420
   agaaaggcgg acaggtatcc ggtaagcggc agggtcggaa caggagagcg cacgagggag 9480
   cttccagggg gaaacgcctg gtatctttat agtcctgtcg ggtttcgcca cctctgactt 9540
   gagcgtcgat ttttgtgatg ctcgtcaggg gggcggagcc tatggaaaaa cgccagcaac 9600
   gcggcctttt tacggttcct ggccttttgc tggccttgaa gctgtccctg atggtcgtca 9660
   tctacctgcc tggacagcat ggcctgcaac gcgggcatcc cgatgccgcc ggaagcgaga 9720
   agaatcataa tggggaaggc catccagcct cgcgtcgcga acgccagcaa gacgtagccc 9780
   agcgcgtcgg ccccgagatg cgccgcgtgc ggctgctgga gatggcggac gcgatggata 9840
   tgttctgcca agggttggtt tgcgcattca cagttctccg caagaattga ttggctccaa 9900
   ttcttggagt ggtgaatccg ttagcgaggt gccgccctgc ttcatccccg tggcccgttg 9960
   ctcgcgtttg ctggcggtgt ccccggaaga aatatatttg catgtcttta gttctatgat 10020
   gacacaaacc ccgcccagcg tcttgtcatt ggcgaattcg aacacgcaga tgcagtcggg 10080
   gcggcgcggt ccgaggtcca cttcgcatat taaggtgacg cgtgtggcct cgaacaccga 10140
   gcgaccctgc agcgacccgc ttaacagcgt caacagcgtg ccgcagatcc cggggggcaa 10200
   tgagatatga aaaagcctga actcaccgcg acgtctgtcg agaagtttct gatcgaaaag 10260
   ttcgacagcg tctccgacct gatgcagctc tcggagggcg aagaatctcg tgctttcagc 10320
   ttcgatgtag gagggcgtgg atatgtcctg cgggtaaata gctgcgccga tggtttctac 10380
   aaagatcgtt atgtttatcg gcactttgca tcggccgcgc tcccgattcc ggaagtgctt 10440
   gacattgggg aattcagcga gagcctgacc tattgcatct cccgccgtgc acagggtgtc 10500
   acgttgcaag acctgcctga aaccgaactg cccgctgttc tgcagccggt cgcggaggcc 10560
   atggatgcga tcgctgcggc cgatcttagc cagacgagcg ggttcggccc attcggaccg 10620
   caaggaatcg gtcaatacac tacatggcgt gatttcatat gcgcgattgc tgatccccat 10680
   gtgtatcact ggcaaactgt gatggacgac accgtcagtg cgtccgtcgc gcaggctctc 10740
   gatgagctga tgctttgggc cgaggactgc cccgaagtcc ggcacctcgt gcacgcggat 10800
   ttcggctcca acaatgtcct gacggacaat ggccgcataa cagcggtcat tgactggagc 10860
   gaggcgatgt tcggggattc ccaatacgag gtcgccaaca tcttcttctg gaggccgtgg 10920
   ttggcttgta tggagcagca gacgcgctac ttcgagcgga ggcatccgga gcttgcagga 10980
   tcgccgcggc tccgggcgta tatgctccgc attggtcttg accaactcta tcagagcttg 11040
   gttgacggca atttcgatga tgcagcttgg gcgcagggtc gatgcgacgc aatcgtccga 11100
   tccggagccg ggactgtcgg gcgtacacaa atcgcccgca gaagcgcggc cgtctggacc 11160
   gatggctgtg tagaagtact cgccgatagt ggaaaccgac gccccagcac tcgtccggat 11220
   cgggagatgg gggaggctaa ctgaaacacg gaaggagaca ataccggaag gaacccgcgc 11280
   tatgacggca ataaaaagac agaataaaac gcacgggtgt tgggtcgttt gttcataaac 11340
   gcggggttcg gtcccagggc tggcactctg tcgatacccc accgagaccc cattggggcc 11400
   aatacgcccg cgtttcttcc ttttccccac cccacccccc aagttcgggt gaaggcccag 11460
   ggctcgcagc caacgtcggg gcggcaggcc ctgccatagc cactggcccc gtgggttagg 11520
   gacggggtcc cccatgggga atggtttatg gttcgtgggg gttattattt tgggcgttgc 11580
   gtggggtcag gtccacgact ggactgagca gacagaccca tggtttttgg atggcctggg 11640
   catggaccgc atgtactggc gcgacacgaa caccgggcgt ctgtggctgc caaacacccc 11700
   cgacccccaa aaaccaccgc gcggatttct ggcgtgccaa gctagtcgac caattctcat 11760
   gtttgacagc ttatcatcgc agatccgggc aacgttgttg ccattgctgc aggcgcagaa 11820
   ctggtaggta tggaagatct atacattgaa tcaatattgg caattagcca tattagtcat 11880
   tggttatata gcataaatca atattggcta ttggccattg catacgttgt atctatatca 11940
   taatatgtac atttatattg gctcatgtcc aatatgaccg ccat                  11984
<210> 236
   <211> 798
   <212> DNA
   <213> Artificial Sequence
<220>
   <223> In vitro synthesized DNA encoding a melanocortin receptor binding alpha-MSH mimetibody without secretory signal (p3459).
<400> 236
   cagatccagg gaggcggagg ctcctactcc tgcgagcact tccgctggtg caagccggtg 60
   ggatccggtg gaggctccgg taccttagtc gccgtctcct cagagcccaa atctgccgac 120
   aaaactcacg cctgcccacc gtgcccagca cctgaactcc tggggggacc gtcagtcttc 180
   ctcttccccc caaaacccaa ggacaccctc atgatctccc ggacccctga ggtcacatgc 240
   gtggtggtgg acgtgagcca cgaagaccct gaggtcaagt tcaactggta cgtggacggc 300
   gtggaggtgc ataatgccaa gacaaagccg cgggaggagc agtacaacag cacgtaccgt 360
   gtggtcagcg tcctcaccgt cctgcaccag gactggctga atggcaagga gtacaagtgc 420
   aaggtctcca acaaagccct cccagccccc atcgagaaaa ccatctccaa agccaaaggg 480
   cagccccgag aaccacaggt gtacaccctg cccccatccc gggatgagct gaccaagaac 540
   caggtcagcc tgacctgcct ggtcaaaggc ttctatccca gcgacatcgc cgtggagtgg 600
   gagagcaatg ggcagccgga gaacaactac aagaccacgc ctcccgtgct ggactccgac 660
   ggctccttct tcctctacag caagctcacc gtggacaaga gcaggtggca gcaggggaac 720
   gtcttctcat gctccgtgat gcatgaggct ctgcacaacc actacacgca gaagagcctc 780
   tccctgtctc cgggtaaa                                                798
<210> 237
   <211> 266
   <212> PRT
   <213> Artificial Sequence
<220>
   <223> Melanocortin receptor binding alpha-MSH mimetibody without secretory signal encoded by an in vitro synthesized DNA (p3459).
<400> 237
   Gln Ile Gln Gly Gly Gly Gly Ser Tyr Ser Cys Glu His Phe Arg Trp
    1               5                  10                  15
   Cys Lys Pro Val Gly Ser Gly Gly Gly Ser Gly Thr Leu Val Ala Val
               20                  25                  30
   Ser Ser Glu Pro Lys Ser Ala Asp Lys Thr His Ala Cys Pro Pro Cys
           35                  40                  45
   Pro Ala Pro Glu Leu Leu Gly Gly Pro Ser Val Phe Leu Phe Pro Pro
       50                  55                  60
   Lys Pro Lys Asp Thr Leu Met Ile Ser Arg Thr Pro Glu Val Thr Cys
   65                  70                  75                  80
   Val Val Val Asp Val Ser His Glu Asp Pro Glu Val Lys Phe Asn Trp
                   85                  90                  95
   Tyr Val Asp Gly Val Glu Val His Asn Ala Lys Thr Lys Pro Arg Glu
               100                 105                 110
   Glu Gln Tyr Asn Ser Thr Tyr Arg Val Val Ser Val Leu Thr Val Leu
           115                 120                 125
   His Gln Asp Trp Leu Asn Gly Lys Glu Tyr Lys Cys Lys Val Ser Asn
       130                 135                 140
   Lys Ala Leu Pro Ala Pro Ile Glu Lys Thr Ile Ser Lys Ala Lys Gly
   145                 150                 155                 160
   Gln Pro Arg Glu Pro Gln Val Tyr Thr Leu Pro Pro Ser Arg Asp Glu
                   165                 170                 175
   Leu Thr Lys Asn Gln Val Ser Leu Thr Cys Leu Val Lys Gly Phe Tyr
               180                 185                 190
   Pro Ser Asp Ile Ala Val Glu Trp Glu Ser Asn Gly Gln Pro Glu Asn
           195                 200                 205
   Asn Tyr Lys Thr Thr Pro Pro Val Leu Asp Ser Asp Gly Ser Phe Phe
       210                 215                 220
   Leu Tyr Ser Lys Leu Thr Val Asp Lys Ser Arg Trp Gln Gln Gly Asn
   225                 230                 235                 240
   Val Phe Ser Cys Ser Val Met His Glu Ala Leu His Asn His Tyr Thr
                   245                 250                 255
   Gln Lys Ser Leu Ser Leu Ser Pro Gly Lys
               260                 265
<210> 238
   <211> 855
   <212> DNA
   <213> Artificial Sequence
<220>
   <223> In vitro synthesized DNA encoding a melanocortin receptor binding alpha-MSH mimetibody with secretory signal and V1 (p3459).
<400> 238
   atggcttggg tgtggacctt gctattcctg atggcggccg cccaaagtat acaggcccag 60
   atccagggag gcggaggctc ctactcctgc gagcacttcc gctggtgcaa gccggtggga 120
   tccggtggag gctccggtac cttagtcgcc gtctcctcag agcccaaatc tgccgacaaa 180
   actcacgcct gcccaccgtg cccagcacct gaactcctgg ggggaccgtc agtcttcctc 240
   ttccccccaa aacccaagga caccctcatg atctcccgga cccctgaggt cacatgcgtg 300
   gtggtggacg tgagccacga agaccctgag gtcaagttca actggtacgt ggacggcgtg 360
   gaggtgcata atgccaagac aaagccgcgg gaggagcagt acaacagcac gtaccgtgtg 420
   gtcagcgtcc tcaccgtcct gcaccaggac tggctgaatg gcaaggagta caagtgcaag 480
   gtctccaaca aagccctccc agcccccatc gagaaaacca tctccaaagc caaagggcag 540
   ccccgagaac cacaggtgta caccctgccc ccatcccggg atgagctgac caagaaccag 600
   gtcagcctga cctgcctggt caaaggcttc tatcccagcg acatcgccgt ggagtgggag 660
   agcaatgggc agccggagaa caactacaag accacgcctc ccgtgctgga ctccgacggc 720
   tccttcttcc tctacagcaa gctcaccgtg gacaagagca ggtggcagca ggggaacgtc 780
   ttctcatgct ccgtgatgca tgaggctctg cacaaccact acacgcagaa gagcctctcc 840
   ctgtctccgg gtaaa                                                  855
<210> 239
   <211> 285
   <212> PRT
   <213> Artificial Sequence
<220>
   <223> Melanocortin receptor binding alpha-MSH mimetibody with secretory signal and V1 encoded by an in vitro synthesized DNA (p3459).
<400> 239
   Met Ala Trp Val Trp Thr Leu Leu Phe Leu Met Ala Ala Ala Gln Ser
   1               5                   10                  15
   Ile Gln Ala Gln Ile Gln Gly Gly Gly Gly Ser Tyr Ser Cys Glu His
               20                  25                  30
   Phe Arg Trp Cys Lys Pro Val Gly Ser Gly Gly Gly Ser Gly Thr Leu
           35                  40                  45
   Val Ala Val Ser Ser Glu Pro Lys Ser Ala Asp Lys Thr His Ala Cys
       50                  55                  60
   Pro Pro Cys Pro Ala Pro Glu Leu Leu Gly Gly Pro Ser Val Phe Leu
   65                  70                  75                  80
   Phe Pro Pro Lys Pro Lys Asp Thr Leu Met Ile Ser Arg Thr Pro Glu
                   85                  90                  95
   Val Thr Cys Val Val Val Asp Val Ser His Glu Asp Pro Glu Val Lys
               100                 105                 110
   Phe Asn Trp Tyr Val Asp Gly Val Glu Val His Asn Ala Lys Thr Lys
           115                 120                 125
   Pro Arg Glu Glu Gln Tyr Asn Ser Thr Tyr Arg Val Val Ser Val Leu
       130                 135                 140
   Thr Val Leu His Gln Asp Trp Leu Asn Gly Lys Glu Tyr Lys Cys Lys
   145                 150                 155                 160
   Val Ser Asn Lys Ala Leu Pro Ala Pro Ile Glu Lys Thr Ile Ser Lys
                   165                 170                 175
   Ala Lys Gly Gln Pro Arg Glu Pro Gln Val Tyr Thr Leu Pro Pro Ser
               180                 185                 190
   Arg Asp Glu Leu Thr Lys Asn Gln Val Ser Leu Thr Cys Leu Val Lys
           195                 200                 205
   Gly Phe Tyr Pro Ser Asp Ile Ala Val Glu Trp Glu Ser Asn Gly Gln
       210                 215                 220
   Pro Glu Asn Asn Tyr Lys Thr Thr Pro Pro Val Leu Asp Ser Asp Gly
   225                 230                 235                 240
   Ser Phe Phe Leu Tyr Ser Lys Leu Thr Val Asp Lys Ser Arg Trp Gln
                   245                 250                 255
   Gln Gly Asn Val Phe Ser Cys Ser Val Met His Glu Ala Leu His Asn
               260                 265                 270
   His Tyr Thr Gln Lys Ser Leu Ser Leu Ser Pro Gly Lys
           275                 280                 285
<210> 240
   <211> 11990
   <212> DNA
   <213> Artificial Sequence
<220>
   <223> In vitro synthesized DNA with expression vector functions that encodes an alpha-MSH mimetibody (p3459).
<400> 240
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcccag atccagggag gcggaggctc 1680
   ctactcctgc gagcacttcc gctggtgcaa gccggtggga tccggtggag gctccggtac 1740
   cttagtcgcc gtctcctcag agcccaaatc tgccgacaaa actcacgcct gcccaccgtg 1800
   cccagcacct gaactcctgg ggggaccgtc agtcttcctc ttccccccaa aacccaagga 1860
   caccctcatg atctcccgga cccctgaggt cacatgcgtg gtggtggacg tgagccacga 1920
   agaccctgag gtcaagttca actggtacgt ggacggcgtg gaggtgcata atgccaagac 1980
   aaagccgcgg gaggagcagt acaacagcac gtaccgtgtg gtcagcgtcc tcaccgtcct 2040
   gcaccaggac tggctgaatg gcaaggagta caagtgcaag gtctccaaca aagccctccc 2100
   agcccccatc gagaaaacca tctccaaagc caaagggcag ccccgagaac cacaggtgta 2160
   caccctgccc ccatcccggg atgagctgac caagaaccag gtcagcctga cctgcctggt 2220
   caaaggcttc tatcccagcg acatcgccgt ggagtgggag agcaatgggc agccggagaa 2280
   caactacaag accacgcctc ccgtgctgga ctccgacggc tccttcttcc tctacagcaa 2340
   gctcaccgtg gacaagagca ggtggcagca ggggaacgtc ttctcatgct ccgtgatgca 2400
   tgaggctctg cacaaccact acacgcagaa gagcctctcc ctgtctccgg gtaaatgagg 2460
   gcccgtttaa acagatccag acatgataag atacattgat gagtttggac aaaccacaac 2520
   tagaatgcag tgaaaaaaat gctttatttg tgaaatttgt gatgctattg ctttatttgt 2580
   aaccattata agctgcaata aacaagttaa caacaacaat tgcattcatt ttatgtttca 2640
   ggttcagggg gaggtgtggg aggtttttta aagcaagtaa aacctctaca aatgtggtat 2700
   ggctgattat gatccggctg cctcgcgcgt ttcggtgatg acggtgaaaa cctctgacac 2760
   atgcagctcc cggagacggt cacagcttgt ctgtaagcgg atgccgggag cagacaagcc 2820
   cgtcaggcgt cagcgggtgt tggcgggtgt cggggcgcag ccatgaggtc gactctagag 2880
   gatcgatgcc ccgccccgga cgaactaaac ctgactacga catctctgcc ccttcttcgc 2940
   ggggcagtgc atgtaatccc ttcagttggt tggtacaact tgccaactgg gccctgttcc 3000
   acatgtgaca cgggggggga ccaaacacaa aggggttctc tgactgtagt tgacatcctt 3060
   ataaatggat gtgcacattt gccaacactg agtggctttc atcctggagc agactttgca 3120
   gtctgtggac tgcaacacaa cattgccttt atgtgtaact cttggctgaa gctcttacac 3180
   caatgctggg ggacatgtac ctcccagggg cccaggaaga ctacgggagg ctacaccaac 3240
   gtcaatcaga ggggcctgtg tagctaccga taagcggacc ctcaagaggg cattagcaat 3300
   agtgtttata aggcccccct gttaacccta aacgggtagc atatgcttcc cgggtagtag 3360
   tatatactat ccagactaac cctaattcaa tagcatatgt tacccaacgg gaagcatatg 3420
   ctatcgaatt agggttagta aaagggtcct aaggaacagc gatatctccc accccatgag 3480
   ctgtcacggt tttatttaca tggggtcagg attccacgag ggtagtgaac cattttagtc 3540
   acaagggcag tggctgaaga tcaaggagcg ggcagtgaac tctcctgaat cttcgcctgc 3600
   ttcttcattc tccttcgttt agctaataga ataactgctg agttgtgaac agtaaggtgt 3660
   atgtgaggtg ctcgaaaaca aggtttcagg tgacgccccc agaataaaat ttggacgggg 3720
   ggttcagtgg tggcattgtg ctatgacacc aatataaccc tcacaaaccc cttgggcaat 3780
   aaatactagt gtaggaatga aacattctga atatctttaa caatagaaat ccatggggtg 3840
   gggacaagcc gtaaagactg gatgtccatc tcacacgaat ttatggctat gggcaacaca 3900
   taatcctagt gcaatatgat actggggtta ttaagatgtg tcccaggcag ggaccaagac 3960
   aggtgaacca tgttgttaca ctctatttgt aacaagggga aagagagtgg acgccgacag 4020
   cagcggactc cactggttgt ctctaacacc cccgaaaatt aaacggggct ccacgccaat 4080
   ggggcccata aacaaagaca agtggccact cttttttttg aaattgtgga gtgggggcac 4140
   gcgtcagccc ccacacgccg ccctgcggtt ttggactgta aaataagggt gtaataactt 4200
   ggctgattgt aaccccgcta accactgcgg tcaaaccact tgcccacaaa accactaatg 4260
   gcaccccggg gaatacctgc ataagtaggt gggcgggcca agataggggc gcgattgctg 4320
   cgatctggag gacaaattac acacacttgc gcctgagcgc caagcacagg gttgttggtc 4380
   ctcatattca cgaggtcgct gagagcacgg tgggctaatg ttgccatggg tagcatatac 4440
   tacccaaata tctggatagc atatgctatc ctaatctata tctgggtagc ataggctatc 4500
   ctaatctata tctgggtagc atatgctatc ctaatctata tctgggtagt atatgctatc 4560
   ctaatttata tctgggtagc ataggctatc ctaatctata tctgggtagc atatgctatc 4620
   ctaatctata tctgggtagt atatgctatc ctaatctgta tccgggtagc atatgctatc 4680
   ctaatagaga ttagggtagt atatgctatc ctaatttata tctgggtagc atatactacc 4740
   caaatatctg gatagcatat gctatcctaa tctatatctg ggtagcatat gctatcctaa 4800
   tctatatctg ggtagcatag gctatcctaa tctatatctg ggtagcatat gctatcctaa 4860
   tctatatctg ggtagtatat gctatcctaa tttatatctg ggtagcatag gctatcctaa 4920
   tctatatctg ggtagcatat gctatcctaa tctatatctg ggtagtatat gctatcctaa 4980
   tctgtatccg ggtagcatat gctatcctca tgcatataca gtcagcatat gatacccagt 5040
   agtagagtgg gagtgctatc ctttgcatat gccgccacct cccaaggggg cgtgaatttt 5100
   cgctgcttgt ccttttcctg ctggttgctc ccattcttag gtgaatttaa ggaggccagg 5160
   ctaaagccgt cgcatgtctg attgctcacc aggtaaatgt cgctaatgtt ttccaacgcg 5220
   agaaggtgtt gagcgcggag ctgagtgacg tgacaacatg ggtatgccca attgccccat 5280
   gttgggagga cgaaaatggt gacaagacag atggccagaa atacaccaac agcacgcatg 5340
   atgtctactg gggatttatt ctttagtgcg ggggaataca cggcttttaa tacgattgag 5400
   ggcgtctcct aacaagttac atcactcctg cccttcctca ccctcatctc catcacctcc 5460
   ttcatctccg tcatctccgt catcaccctc cgcggcagcc ccttccacca taggtggaaa 5520
   ccagggaggc aaatctactc catcgtcaaa gctgcacaca gtcaccctga tattgcaggt 5580
   aggagcgggc tttgtcataa caaggtcctt aatcgcatcc ttcaaaacct cagcaaatat 5640
   atgagtttgt aaaaagacca tgaaataaca gacaatggac tcccttagcg ggccaggttg 5700
   tgggccgggt ccaggggcca ttccaaaggg gagacgactc aatggtgtaa gacgacattg 5760
   tggaatagca agggcagttc ctcgccttag gttgtaaagg gaggtcttac tacctccata 5820
   tacgaacaca ccggcgaccc aagttccttc gtcggtagtc ctttctacgt gactcctagc 5880
   caggagagct cttaaacctt ctgcaatgtt ctcaaatttc gggttggaac ctccttgacc 5940
   acgatgcttt tccaaaccac cctccttttt tgcgccctgc ctccatcacc ctgaccccgg 6000
   ggtccagtgc ttgggccttc tcctgggtca tctgcggggc cctgctctat cgctcccggg 6060
   ggcacgtcag gctcaccatc tgggccacct tcttggtggt attcaaaata atcggcttcc 6120
   cctacagggt ggaaaaatgg ccttctacct ggagggggcc tgcgcggtgg agacccggat 6180
   gatgatgact gactactggg actcctgggc ctcttttctc cacgtccacg acctctcccc 6240
   ctggctcttt cacgacttcc ccccctggct ctttcacgtc ctctaccccg gcggcctcca 6300
   ctacctcctc gaccccggcc tccactacct cctcgacccc ggcctccact gcctcctcga 6360
   ccccggcctc cacctcctgc tcctgcccct cctgctcctg cccctcctcc tgctcctgcc 6420
   cctcctgccc ctcctgctcc tgcccctcct gcccctcctg ctcctgcccc tcctgcccct 6480
   cctgctcctg cccctcctgc ccctcctcct gctcctgccc ctcctgcccc tcctcctgct 6540
   cctgcccctc ctgcccctcc tgctcctgcc cctcctgccc ctcctgctcc tgcccctcct 6600
   gcccctcctg ctcctgcccc tcctgctcct gcccctcctg ctcctgcccc tcctgctcct 6660
   gcccctcctg cccctcctgc ccctcctcct gctcctgccc ctcctgctcc tgcccctcct 6720
   gcccctcctg cccctcctgc tcctgcccct cctcctgctc ctgcccctcc tgcccctcct 6780
   gcccctcctc ctgctcctgc ccctcctgcc cctcctcctg ctcctgcccc tcctcctgct 6840
   cctgcccctc ctgcccctcc tgcccctcct cctgctcctg cccctcctgc ccctcctcct 6900
   gctcctgccc ctcctcctgc tcctgcccct cctgcccctc ctgcccctcc tcctgctcct 6960
   gcccctcctc ctgctcctgc ccctcctgcc cctcctgccc ctcctgcccc tcctcctgct 7020
   cctgcccctc ctcctgctcc tgcccctcct gctcctgccc ctcccgctcc tgctcctgct 7080
   cctgttccac cgtgggtccc tttgcagcca atgcaacttg gacgtttttg gggtctccgg 7140
   acaccatctc tatgtcttgg ccctgatcct gagccgcccg gggctcctgg tcttccgcct 7200
   cctcgtcctc gtcctcttcc ccgtcctcgt ccatggttat caccccctct tctttgaggt 7260
   ccactgccgc cggagccttc tggtccagat gtgtctccct tctctcctag gccatttcca 7320
   ggtcctgtac ctggcccctc gtcagacatg attcacacta aaagagatca atagacatct 7380
   ttattagacg acgctcagtg aatacaggga gtgcagactc ctgccccctc caacagcccc 7440
   cccaccctca tccccttcat ggtcgctgtc agacagatcc aggtctgaaa attccccatc 7500
   ctccgaacca tcctcgtcct catcaccaat tactcgcagc ccggaaaact cccgctgaac 7560
   atcctcaaga tttgcgtcct gagcctcaag ccaggcctca aattcctcgt cccccttttt 7620
   gctggacggt agggatgggg attctcggga cccctcctct tcctcttcaa ggtcaccaga 7680
   cagagatgct actggggcaa cggaagaaaa gctgggtgcg gcctgtgagg atcagcttat 7740
   cgatgataag ctgtcaaaca tgagaattct tgaagacgaa agggcctcgt gatacgccta 7800
   tttttatagg ttaatgtcat gataataatg gtttcttaga cgtcaggtgg cacttttcgg 7860
   ggaaatgtgc gcggaacccc tatttgttta tttttctaaa tacattcaaa tatgtatccg 7920
   ctcatgagac aataaccctg ataaatgctt caataatatt gaaaaaggaa gagtatgagt 7980
   attcaacatt tccgtgtcgc ccttattccc ttttttgcgg cattttgcct tcctgttttt 8040
   gctcacccag aaacgctggt gaaagtaaaa gatgctgaag atcagttggg tgcacgagtg 8100
   ggttacatcg aactggatct caacagcggt aagatccttg agagttttcg ccccgaagaa 8160
   cgttttccaa tgatgagcac ttttaaagtt ctgctatgtg gcgcggtatt atcccgtgtt 8220
   gacgccgggc aagagcaact cggtcgccgc atacactatt ctcagaatga cttggttgag 8280
   tactcaccag tcacagaaaa gcatcttacg gatggcatga cagtaagaga attatgcagt 8340
   gctgccataa ccatgagtga taacactgcg gccaacttac ttctgacaac gatcggagga 8400
   ccgaaggagc taaccgcttt tttgcacaac atgggggatc atgtaactcg ccttgatcgt 8460
   tgggaaccgg agctgaatga agccatacca aacgacgagc gtgacaccac gatgcctgca 8520
   gcaatggcaa caacgttgcg caaactatta actggcgaac tacttactct agcttcccgg 8580
   caacaattaa tagactggat ggaggcggat aaagttgcag gaccacttct gcgctcggcc 8640
   cttccggctg gctggtttat tgctgataaa tctggagccg gtgagcgtgg gtctcgcggt 8700
   atcattgcag cactggggcc agatggtaag ccctcccgta tcgtagttat ctacacgacg 8760
   gggagtcagg caactatgga tgaacgaaat agacagatcg ctgagatagg tgcctcactg 8820
   attaagcatt ggtaactgtc agaccaagtt tactcatata tactttagat tgatttaaaa 8880
   cttcattttt aatttaaaag gatctaggtg aagatccttt ttgataatct catgaccaaa 8940
   atcccttaac gtgagttttc gttccactga gcgtcagacc ccgtagaaaa gatcaaagga 9000
   tcttcttgag atcctttttt tctgcgcgta atctgctgct tgcaaacaaa aaaaccaccg 9060
   ctaccagcgg tggtttgttt gccggatcaa gagctaccaa ctctttttcc gaaggtaact 9120
   ggcttcagca gagcgcagat accaaatact gtccttctag tgtagccgta gttaggccac 9180
   cacttcaaga actctgtagc accgcctaca tacctcgctc tgctaatcct gttaccagtg 9240
   gctgctgcca gtggcgataa gtcgtgtctt accgggttgg actcaagacg atagttaccg 9300
   gataaggcgc agcggtcggg ctgaacgggg ggttcgtgca cacagcccag cttggagcga 9360
   acgacctaca ccgaactgag atacctacag cgtgagctat gagaaagcgc cacgcttccc 9420
   gaagggagaa aggcggacag gtatccggta agcggcaggg tcggaacagg agagcgcacg 9480
   agggagcttc cagggggaaa cgcctggtat ctttatagtc ctgtcgggtt tcgccacctc 9540
   tgacttgagc gtcgattttt gtgatgctcg tcaggggggc ggagcctatg gaaaaacgcc 9600
   agcaacgcgg cctttttacg gttcctggcc ttttgctggc cttgaagctg tccctgatgg 9660
   tcgtcatcta cctgcctgga cagcatggcc tgcaacgcgg gcatcccgat gccgccggaa 9720
   gcgagaagaa tcataatggg gaaggccatc cagcctcgcg tcgcgaacgc cagcaagacg 9780
   tagcccagcg cgtcggcccc gagatgcgcc gcgtgcggct gctggagatg gcggacgcga 9840
   tggatatgtt ctgccaaggg ttggtttgcg cattcacagt tctccgcaag aattgattgg 9900
   ctccaattct tggagtggtg aatccgttag cgaggtgccg ccctgcttca tccccgtggc 9960
   ccgttgctcg cgtttgctgg cggtgtcccc ggaagaaata tatttgcatg tctttagttc 10020
   tatgatgaca caaaccccgc ccagcgtctt gtcattggcg aattcgaaca cgcagatgca 10080
   gtcggggcgg cgcggtccga ggtccacttc gcatattaag gtgacgcgtg tggcctcgaa 10140
   caccgagcga ccctgcagcg acccgcttaa cagcgtcaac agcgtgccgc agatcccggg 10200
   gggcaatgag atatgaaaaa gcctgaactc accgcgacgt ctgtcgagaa gtttctgatc 10260
   gaaaagttcg acagcgtctc cgacctgatg cagctctcgg agggcgaaga atctcgtgct 10320
   ttcagcttcg atgtaggagg gcgtggatat gtcctgcggg taaatagctg cgccgatggt 10380
   ttctacaaag atcgttatgt ttatcggcac tttgcatcgg ccgcgctccc gattccggaa 10440
   gtgcttgaca ttggggaatt cagcgagagc ctgacctatt gcatctcccg ccgtgcacag 10500
   ggtgtcacgt tgcaagacct gcctgaaacc gaactgcccg ctgttctgca gccggtcgcg 10560
   gaggccatgg atgcgatcgc tgcggccgat cttagccaga cgagcgggtt cggcccattc 10620
   ggaccgcaag gaatcggtca atacactaca tggcgtgatt tcatatgcgc gattgctgat 10680
   ccccatgtgt atcactggca aactgtgatg gacgacaccg tcagtgcgtc cgtcgcgcag 10740
   gctctcgatg agctgatgct ttgggccgag gactgccccg aagtccggca cctcgtgcac 10800
   gcggatttcg gctccaacaa tgtcctgacg gacaatggcc gcataacagc ggtcattgac 10860
   tggagcgagg cgatgttcgg ggattcccaa tacgaggtcg ccaacatctt cttctggagg 10920
   ccgtggttgg cttgtatgga gcagcagacg cgctacttcg agcggaggca tccggagctt 10980
   gcaggatcgc cgcggctccg ggcgtatatg ctccgcattg gtcttgacca actctatcag 11040
   agcttggttg acggcaattt cgatgatgca gcttgggcgc agggtcgatg cgacgcaatc 11100
   gtccgatccg gagccgggac tgtcgggcgt acacaaatcg cccgcagaag cgcggccgtc 11160
   tggaccgatg gctgtgtaga agtactcgcc gatagtggaa accgacgccc cagcactcgt 11220
   ccggatcggg agatggggga ggctaactga aacacggaag gagacaatac cggaaggaac 11280
   ccgcgctatg acggcaataa aaagacagaa taaaacgcac gggtgttggg tcgtttgttc 11340
   ataaacgcgg ggttcggtcc cagggctggc actctgtcga taccccaccg agaccccatt 11400
   ggggccaata cgcccgcgtt tcttcctttt ccccacccca ccccccaagt tcgggtgaag 11460
   gcccagggct cgcagccaac gtcggggcgg caggccctgc catagccact ggccccgtgg 11520
   gttagggacg gggtccccca tggggaatgg tttatggttc gtgggggtta ttattttggg 11580
   cgttgcgtgg ggtcaggtcc acgactggac tgagcagaca gacccatggt ttttggatgg 11640
   cctgggcatg gaccgcatgt actggcgcga cacgaacacc gggcgtctgt ggctgccaaa 11700
   cacccccgac cccaaaaaac caccgcgcgg atttctggcg tgccaagcta gtcgaccaat 11760
   tctcatgttt gacagcttat catcgcagat ccgggcaacg ttgttgccat tgctgcaggc 11820
   gcagaactgg taggtatgga agatctatac attgaatcaa tattggcaat tagccatatt 11880
   agtcattggt tatatagcat aaatcaatat tggctattgg ccattgcata cgttgtatct 11940
   atatcataat atgtacattt atattggctc atgtccaata tgaccgccat 11990
<210> 241
   <211> 777
   <212> DNA
   <213> Artificial Sequence
<220>
   <223> In vitro synthesized DNA encoding a melanocortin receptor binding alpha-MSH mimetibody without secretory signal (p3560).
<400> 241
   tcctactgcg gcgagcactt ccgctggggc tgcccggtgg gatccggtgg aggctccggt 60
   accttagtcg ccgtctcctc agagcccaaa tctgccgaca aaactcacgc ctgcccaccg 120
   tgcccagcac ctgaactcct ggggggaccg tcagtcttcc tcttcccccc aaaacccaag 180
   gacaccctca tgatctcccg gacccctgag gtcacatgcg tggtggtgga cgtgagccac 240
   gaagaccctg aggtcaagtt caactggtac gtggacggcg tggaggtgca taatgccaag 300
   acaaagccgc gggaggagca gtacaacagc acgtaccgtg tggtcagcgt cctcaccgtc 360
   ctgcaccagg actggctgaa tggcaaggag tacaagtgca aggtctccaa caaagccctc 420
   ccagccccca tcgagaaaac catctccaaa gccaaagggc agccccgaga accacaggtg 480
   tacaccctgc ccccatcccg ggatgagctg accaagaacc aggtcagcct gacctgcctg 540
   gtcaaaggct tctatcccag cgacatcgcc gtggagtggg agagcaatgg gcagccggag 600
   aacaactaca agaccacgcc tcccgtgctg gactccgacg gctccttctt cctctacagc 660
   aagctcaccg tggacaagag caggtggcag caggggaacg tcttctcatg ctccgtgatg 720
   catgaggctc tgcacaacca ctacacgcag aagagcctct ccctgtctcc gggtaaa    777
<210> 242
   <211> 259
   <212> PRT
   <213> Artificial Sequence
<220>
   <223> Melanocortin receptor binding alpha-MSH mimetibody without secretory signal encoded by an in vitro synthesized DNA (p3560).
<400> 242
   Ser Tyr Cys Gly Glu His Phe Arg Trp Gly Cys Pro Val Gly Ser Gly
    1               5                  10                  15
   Gly Gly Ser Gly Thr Leu Val Ala Val Ser Ser Glu Pro Lys Ser Ala
               20                  25                  30
   Asp Lys Thr His Ala Cys Pro Pro Cys Pro Ala Pro Glu Leu Leu Gly
           35                  40                  45
   Gly Pro Ser Val Phe Leu Phe Pro Pro Lys Pro Lys Asp Thr Leu Met
       50                  55                  60
   Ile Ser Arg Thr Pro Glu Val Thr Cys Val Val Val Asp Val Ser His
   65                  70                  75                  80
   Glu Asp Pro Glu Val Lys Phe Asn Trp Tyr Val Asp Gly Val Glu Val
                   85                  90                  95
   His Asn Ala Lys Thr Lys Pro Arg Glu Glu Gln Tyr Asn Ser Thr Tyr
               100                 105                 110
   Arg Val Val Ser Val Leu Thr Val Leu His Gln Asp Trp Leu Asn Gly
           115                 120                 125
   Lys Glu Tyr Lys Cys Lys Val Ser Asn Lys Ala Leu Pro Ala Pro Ile
       130                 135                 140
   Glu Lys Thr Ile Ser Lys Ala Lys Gly Gln Pro Arg Glu Pro Gln Val
   145                 150                 155                 160
   Tyr Thr Leu Pro Pro Ser Arg Asp Glu Leu Thr Lys Asn Gln Val Ser
                   165                 170                 175
   Leu Thr Cys Leu Val Lys Gly Phe Tyr Pro Ser Asp Ile Ala Val Glu
               180                 185                 190
   Trp Glu Ser Asn Gly Gln Pro Glu Asn Asn Tyr Lys Thr Thr Pro Pro
           195                 200                 205
   Val Leu Asp Ser Asp Gly Ser Phe Phe Leu Tyr Ser Lys Leu Thr Val
       210                 215                 220
   Asp Lys Ser Arg Trp Gln Gln Gly Asn Val Phe Ser Cys Ser Val Met
   225                 230                 235                 240
   His Glu Ala Leu His Asn His Tyr Thr Gln Lys Ser Leu Ser Leu Ser
                   245                 250                 255
   Pro Gly Lys
<210> 243
   <211> 834
   <212> DNA
   <213> Artificial Sequence
<220>
   <223> In vitro synthesized DNA encoding a melanocortin receptor binding alpha-MSH mimetibody with secretory signal (p3560).
<400> 243
   atggcttggg tgtggacctt gctattcctg atggcggccg cccaaagtat acaggcctcc 60
   tactgcggcg agcacttccg ctggggctgc ccggtgggat ccggtggagg ctccggtacc 120
   ttagtcgccg tctcctcaga gcccaaatct gccgacaaaa ctcacgcctg cccaccgtgc 180
   ccagcacctg aactcctggg gggaccgtca gtcttcctct tccccccaaa acccaaggac 240
   accctcatga tctcccggac ccctgaggtc acatgcgtgg tggtggacgt gagccacgaa 300
   gaccctgagg tcaagttcaa ctggtacgtg gacggcgtgg aggtgcataa tgccaagaca 360
   aagccgcggg aggagcagta caacagcacg taccgtgtgg tcagcgtcct caccgtcctg 420
   caccaggact ggctgaatgg caaggagtac aagtgcaagg tctccaacaa agccctccca 480
   gcccccatcg agaaaaccat ctccaaagcc aaagggcagc cccgagaacc acaggtgtac 540
   accctgcccc catcccggga tgagctgacc aagaaccagg tcagcctgac ctgcctggtc 600
   aaaggcttct atcccagcga catcgccgtg gagtgggaga gcaatgggca gccggagaac 660
   aactacaaga ccacgcctcc cgtgctggac tccgacggct ccttcttcct ctacagcaag 720
   ctcaccgtgg acaagagcag gtggcagcag gggaacgtct tctcatgctc cgtgatgcat 780
   gaggctctgc acaaccacta cacgcagaag agcctctccc tgtctccggg taaa       834
<210> 244
   <211> 278
   <212> PRT
   <213> Artificial Sequence
<220>
   <223> Melanocortin receptor binding alpha-MSH mimetibody with secretory signal encoded by an in vitro synthesized DNA (p3560).
<400> 244
   Met Ala Trp Val Trp Thr Leu Leu Phe Leu Met Ala Ala Ala Gln Ser
    1               5                  10                  15
   Ile Gln Ala Ser Tyr Cys Gly Glu His Phe Arg Trp Gly Cys Pro Val
               20                  25                  30
   Gly Ser Gly Gly Gly Ser Gly Thr Leu Val Ala Val Ser Ser Glu Pro
           35                  40                  45
   Lys Ser Ala Asp Lys Thr His Ala Cys Pro Pro Cys Pro Ala Pro Glu
       50                  55                  60
   Leu Leu Gly Gly Pro Ser Val Phe Leu Phe Pro Pro Lys Pro Lys Asp
   65                  70                  75                  80
   Thr Leu Met Ile Ser Arg Thr Pro Glu Val Thr Cys Val Val Val Asp
                   85                  90                  95
   Val Ser His Glu Asp Pro Glu Val Lys Phe Asn Trp Tyr Val Asp Gly
               100                 105                 110
   Val Glu Val His Asn Ala Lys Thr Lys Pro Arg Glu Glu Gln Tyr Asn
           115                 120                 125
   Ser Thr Tyr Arg Val Val Ser Val Leu Thr Val Leu His Gln Asp Trp
       130                 135                 140
   Leu Asn Gly Lys Glu Tyr Lys Cys Lys Val Ser Asn Lys Ala Leu Pro
   145                 150                 155                 160
   Ala Pro Ile Glu Lys Thr Ile Ser Lys Ala Lys Gly Gln Pro Arg Glu
                   165                 170                 175
   Pro Gln Val Tyr Thr Leu Pro Pro Ser Arg Asp Glu Leu Thr Lys Asn
               180                 185                 190
   Gln Val Ser Leu Thr Cys Leu Val Lys Gly Phe Tyr Pro Ser Asp Ile
           195                 200                 205
   Ala Val Glu Trp Glu Ser Asn Gly Gln Pro Glu Asn Asn Tyr Lys Thr
       210                 215                 220
   Thr Pro Pro Val Leu Asp Ser Asp Gly Ser Phe Phe Leu Tyr Ser Lys
   225                 230                 235                 240
   Leu Thr Val Asp Lys Ser Arg Trp Gln Gln Gly Asn Val Phe Ser Cys
                   245                 250                 255
   Ser Val Met His Glu Ala Leu His Asn His Tyr Thr Gln Lys Ser Leu
               260                 265                 270
   Ser Leu Ser Pro Gly Lys
           275
<210> 245
   <211> 11969
   <212> DNA
   <213> Artificial Sequence
<220>
   <223> In vitro synthesized DNA with expression vector functions that encodes an alpha-MSH mimetibody (p3560).
<400> 245
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggcctcc tactgcggcg agcacttccg 1680
   ctggggctgc ccggtgggat ccggtggagg ctccggtacc ttagtcgccg tctcctcaga 1740
   gcccaaatct gccgacaaaa ctcacgcctg cccaccgtgc ccagcacctg aactcctggg 1800
   gggaccgtca gtcttcctct tccccccaaa acccaaggac accctcatga tctcccggac 1860
   ccctgaggtc acatgcgtgg tggtggacgt gagccacgaa gaccctgagg tcaagttcaa 1920
   ctggtacgtg gacggcgtgg aggtgcataa tgccaagaca aagccgcggg aggagcagta 1980
   caacagcacg taccgtgtgg tcagcgtcct caccgtcctg caccaggact ggctgaatgg 2040
   caaggagtac aagtgcaagg tctccaacaa agccctccca gcccccatcg agaaaaccat 2100
   ctccaaagcc aaagggcagc cccgagaacc acaggtgtac accctgcccc catcccggga 2160
   tgagctgacc aagaaccagg tcagcctgac ctgcctggtc aaaggcttct atcccagcga 2220
   catcgccgtg gagtgggaga gcaatgggca gccggagaac aactacaaga ccacgcctcc 2280
   cgtgctggac tccgacggct ccttcttcct ctacagcaag ctcaccgtgg acaagagcag 2340
   gtggcagcag gggaacgtct tctcatgctc cgtgatgcat gaggctctgc acaaccacta 2400
   cacgcagaag agcctctccc tgtctccggg taaatgaggg cccgtttaaa cagatccaga 2460
   catgataaga tacattgatg agtttggaca aaccacaact agaatgcagt gaaaaaaatg 2520
   ctttatttgt gaaatttgtg atgctattgc tttatttgta accattataa gctgcaataa 2580
   acaagttaac aacaacaatt gcattcattt tatgtttcag gttcaggggg aggtgtggga 2640
   ggttttttaa agcaagtaaa acctctacaa atgtggtatg gctgattatg atccggctgc 2700
   ctcgcgcgtt tcggtgatga cggtgaaaac ctctgacaca tgcagctccc ggagacggtc 2760
   acagcttgtc tgtaagcgga tgccgggagc agacaagccc gtcaggcgtc agcgggtgtt 2820
   ggcgggtgtc ggggcgcagc catgaggtcg actctagagg atcgatgccc cgccccggac 2880
   gaactaaacc tgactacgac atctctgccc cttcttcgcg gggcagtgca tgtaatccct 2940
   tcagttggtt ggtacaactt gccaactggg ccctgttcca catgtgacac ggggggggac 3000
   caaacacaaa ggggttctct gactgtagtt gacatcctta taaatggatg tgcacatttg 3060
   ccaacactga gtggctttca tcctggagca gactttgcag tctgtggact gcaacacaac 3120
   attgccttta tgtgtaactc ttggctgaag ctcttacacc aatgctgggg gacatgtacc 3180
   tcccaggggc ccaggaagac tacgggaggc tacaccaacg tcaatcagag gggcctgtgt 3240
   agctaccgat aagcggaccc tcaagagggc attagcaata gtgtttataa ggcccccttg 3300
   ttaaccctaa acgggtagca tatgcttccc gggtagtagt atatactatc cagactaacc 3360
   ctaattcaat agcatatgtt acccaacggg aagcatatgc tatcgaatta gggttagtaa 3420
   aagggtccta aggaacagcg atatctccca ccccatgagc tgtcacggtt ttatttacat 3480
   ggggtcagga ttccacgagg gtagtgaacc attttagtca caagggcagt ggctgaagat 3540
   caaggagcgg gcagtgaact ctcctgaatc ttcgcctgct tcttcattct ccttcgttta 3600
   gctaatagaa taactgctga gttgtgaaca gtaaggtgta tgtgaggtgc tcgaaaacaa 3660
   ggtttcaggt gacgccccca gaataaaatt tggacggggg gttcagtggt ggcattgtgc 3720
   tatgacacca atataaccct cacaaacccc ttgggcaata aatactagtg taggaatgaa 3780
   acattctgaa tatctttaac aatagaaatc catggggtgg ggacaagccg taaagactgg 3840
   atgtccatct cacacgaatt tatggctatg ggcaacacat aatcctagtg caatatgata 3900
   ctggggttat taagatgtgt cccaggcagg gaccaagaca ggtgaaccat gttgttacac 3960
   tctatttgta acaaggggaa agagagtgga cgccgacagc agcggactcc actggttgtc 4020
   tctaacaccc ccgaaaatta aacggggctc cacgccaatg gggcccataa acaaagacaa 4080
   gtggccactc ttttttttga aattgtggag tgggggcacg cgtcagcccc cacacgccgc 4140
   cctgcggttt tggactgtaa aataagggtg taataacttg gctgattgta accccgctaa 4200
   ccactgcggt caaaccactt gcccacaaaa ccactaatgg caccccgggg aatacctgca 4260
   taagtaggtg ggcgggccaa gataggggcg cgattgctgc gatctggagg acaaattaca 4320
   cacacttgcg cctgagcgcc aagcacaggg ttgttggtcc tcatattcac gaggtcgctg 4380
   agagcacggt gggctaatgt tgccatgggt agcatatact acccaaatat ctggatagca 4440
   tatgctatcc taatctatat ctgggtagca taggctatcc taatctatat ctgggtagca 4500
   tatgctatcc taatctatat ctgggtagta tatgctatcc taatttatat ctgggtagca 4560
   taggctatcc taatctatat ctgggtagca tatgctatcc taatctatat ctgggtagta 4620
   tatgctatcc taatctgtat ccgggtagca tatgctatcc taatagagat tagggtagta 4680
   tatgctatcc taatttatat ctgggtagca tatactaccc aaatatctgg atagcatatg 4740
   ctatcctaat ctatatctgg gtagcatatg ctatcctaat ctatatctgg gtagcatagg 4800
   ctatcctaat ctatatctgg gtagcatatg ctatcctaat ctatatctgg gtagtatatg 4860
   ctatcctaat ttatatctgg gtagcatagg ctatcctaat ctatatctgg gtagcatatg 4920
   ctatcctaat ctatatctgg gtagtatatg ctatcctaat ctgtatccgg gtagcatatg 4980
   ctatcctcat gcatatacag tcagcatatg atacccagta gtagagtggg agtgctatcc 5040
   tttgcatatg ccgccacctc ccaagggggc gtgaattttc gctgcttgtc cttttcctgc 5100
   tggttgctcc cattcttagg tgaatttaag gaggccaggc taaagccgtc gcatgtctga 5160
   ttgctcacca ggtaaatgtc gctaatgttt tccaacgcga gaaggtgttg agcgcggagc 5220
   tgagtgacgt gacaacatgg gtatgcccaa ttgccccatg ttgggaggac gaaaatggtg 5280
   acaagacaga tggccagaaa tacaccaaca gcacgcatga tgtctactgg ggatttattc 5340
   tttagtgcgg gggaatacac ggcttttaat acgattgagg gcgtctccta acaagttaca 5400
   tcactcctgc ccttcctcac cctcatctcc atcacctcct tcatctccgt catctccgtc 5460
   atcaccctcc gcggcagccc cttccaccat aggtggaaac cagggaggca aatctactcc 5520
   atcgtcaaag ctgcacacag tcaccctgat attgcaggta ggagcgggct ttgtcataac 5580
   aaggtcctta atcgcatcct tcaaaacctc agcaaatata tgagtttgta aaaagaccat 5640
   gaaataacag acaatggact cccttagcgg gccaggttgt gggccgggtc caggggccat 5700
   tccaaagggg agacgactca atggtgtaag acgacattgt ggaatagcaa gggcagttcc 5760
   tcgccttagg ttgtaaaggg aggtcttact acctccatat acgaacacac cggcgaccca 5820
   agttccttcg tcggtagtcc tttctacgtg actcctagcc aggagagctc ttaaaccttc 5880
   tgcaatgttc tcaaatttcg ggttggaacc tccttgacca cgatgctttt ccaaaccacc 5940
   ctcctttttt gcgccctgcc tccatcaccc tgaccccggg gtccagtgct tgggccttct 6000
   cctgggtcat ctgcggggcc ctgctctatc gctcccgggg gcacgtcagg ctcaccatct 6060
   gggccacctt cttggtggta ttcaaaataa tcggcttccc ctacagggtg gaaaaatggc 6120
   cttctacctg gagggggcct gcgcggtgga gacccggatg atgatgactg actactggga 6180
   ctcctgggcc tcttttctcc acgtccacga cctctccccc tggctctttc acgacttccc 6240
   cccctggctc tttcacgtcc tctaccccgg cggcctccac tacctcctcg accccggcct 6300
   ccactacctc ctcgaccccg gcctccactg cctcctcgac cccggcctcc acctcctgct 6360
   cctgcccctc ctgctcctgc ccctcctcct gctcctgccc ctcctgcccc tcctgctcct 6420
   gcccctcctg cccctcctgc tcctgcccct cctgcccctc ctgctcctgc ccctcctgcc 6480
   cctcctcctg ctcctgcccc tcctgcccct cctcctgctc ctgcccctcc tgcccctcct 6540
   gctcctgccc ctcctgcccc tcctgctcct gcccctcctg cccctcctgc tcctgcccct 6600
   cctgctcctg cccctcctgc tcctgcccct cctgctcctg cccctcctgc ccctcctgcc 6660
   cctcctcctg ctcctgcccc tcctgctcct gcccctcctg cccctcctgc ccctcctgct 6720
   cctgcccctc ctcctgctcc tgcccctcct gcccctcctg cccctcctcc tgctcctgcc 6780
   cctcctgccc ctcctcctgc tcctgcccct cctcctgctc ctgcccctcc tgcccctcct 6840
   gcccctcctc ctgctcctgc ccctcctgcc cctcctcctg ctcctgcccc tcctcctgct 6900
   cctgcccctc ctgcccctcc tgcccctcct cctgctcctg cccctcctcc tgctcctgcc 6960
   cctcctgccc ctcctgcccc tcctgcccct cctcctgctc ctgcccctcc tcctgctcct 7020
   gcccctcctg ctcctgcccc tcccgctcct gctcctgctc ctgttccacc gtgggtccct 7080
   ttgcagccaa tgcaacttgg acgtttttgg ggtctccgga caccatctct atgtcttggc 7140
   cctgatcctg agccgcccgg ggctcctggt cttccgcctc ctcgtcctcg tcctcttccc 7200
   cgtcctcgtc catggttatc accccctctt ctttgaggtc cactgccgcc ggagccttct 7260
   ggtccagatg tgtctccctt ctctcctagg ccatttccag gtcctgtacc tggcccctcg 7320
   tcagacatga ttcacactaa aagagatcaa tagacatctt tattagacga cgctcagtga 7380
   atacagggag tgcagactcc tgccccctcc aacagccccc ccaccctcat ccccttcatg 7440
   gtcgctgtca gacagatcca ggtctgaaaa ttccccatcc tccgaaccat cctcgtcctc 7500
   atcaccaatt actcgcagcc cggaaaactc ccgctgaaca tcctcaagat ttgcgtcctg 7560
   agcctcaagc caggcctcaa attcctcgtc cccctttttg ctggacggta gggatgggga 7620
   ttctcgggac ccctcctctt cctcttcaag gtcaccagac agagatgcta ctggggcaac 7680
   ggaagaaaag ctgggtgcgg cctgtgagga tcagcttatc gatgataagc tgtcaaacat 7740
   gagaattctt gaagacgaaa gggcctcgtg atacgcctat ttttataggt taatgtcatg 7800
   ataataatgg tttcttagac gtcaggtggc acttttcggg gaaatgtgcg cggaacccct 7860
   atttgtttat ttttctaaat acattcaaat atgtatccgc tcatgagaca ataaccctga 7920
   taaatgcttc aataatattg aaaaaggaag agtatgagta ttcaacattt ccgtgtcgcc 7980
   cttattccct tttttgcggc attttgcctt cctgtttttg ctcacccaga aacgctggtg 8040
   aaagtaaaag atgctgaaga tcagttgggt gcacgagtgg gttacatcga actggatctc 8100
   aacagcggta agatccttga gagttttcgc cccgaagaac gttttccaat gatgagcact 8160
   tttaaagttc tgctatgtgg cgcggtatta tcccgtgttg acgccgggca agagcaactc 8220
   ggtcgccgca tacactattc tcagaatgac ttggttgagt actcaccagt cacagaaaag 8280
   catcttacgg atggcatgac agtaagagaa ttatgcagtg ctgccataac catgagtgat 8340
   aacactgcgg ccaacttact tctgacaacg atcggaggac cgaaggagct aaccgctttt 8400
   ttgcacaaca tgggggatca tgtaactcgc cttgatcgtt gggaaccgga gctgaatgaa 8460
   gccataccaa acgacgagcg tgacaccacg atgcctgcag caatggcaac aacgttgcgc 8520
   aaactattaa ctggcgaact acttactcta gcttcccggc aacaattaat agactggatg 8580
   gaggcggata aagttgcagg accacttctg cgctcggccc ttccggctgg ctggtttatt 8640
   gctgataaat ctggagccgg tgagcgtggg tctcgcggta tcattgcagc actggggcca 8700
   gatggtaagc cctcccgtat cgtagttatc tacacgacgg ggagtcaggc aactatggat 8760
   gaacgaaata gacagatcgc tgagataggt gcctcactga ttaagcattg gtaactgtca 8820
   gaccaagttt actcatatat actttagatt gatttaaaac ttcattttta atttaaaagg 8880
   atctaggtga agatcctttt tgataatctc atgaccaaaa tcccttaacg tgagttttcg 8940
   ttccactgag cgtcagaccc cgtagaaaag atcaaaggat cttcttgaga tccttttttt 9000
   ctgcgcgtaa tctgctgctt gcaaacaaaa aaaccaccgc taccagcggt ggtttgtttg 9060
   ccggatcaag agctaccaac tctttttccg aaggtaactg gcttcagcag agcgcagata 9120
   ccaaatactg tccttctagt gtagccgtag ttaggccacc acttcaagaa ctctgtagca 9180
   ccgcctacat acctcgctct gctaatcctg ttaccagtgg ctgctgccag tggcgataag 9240
   tcgtgtctta ccgggttgga ctcaagacga tagttaccgg ataaggcgca gcggtcgggc 9300
   tgaacggggg gttcgtgcac acagcccagc ttggagcgaa cgacctacac cgaactgaga 9360
   tacctacagc gtgagctatg agaaagcgcc acgcttcccg aagggagaaa ggcggacagg 9420
   tatccggtaa gcggcagggt cggaacagga gagcgcacga gggagcttcc agggggaaac 9480
   gcctggtatc tttatagtcc tgtcgggttt cgccacctct gacttgagcg tcgatttttg 9540
   tgatgctcgt caggggggcg gagcctatgg aaaaacgcca gcaacgcggc ctttttacgg 9600
   ttcctggcct tttgctggcc ttgaagctgt ccctgatggt cgtcatctac ctgcctggac 9660
   agcatggcct gcaacgcggg catcccgatg ccgccggaag cgagaagaat cataatgggg 9720
   aaggccatcc agcctcgcgt cgcgaacgcc agcaagacgt agcccagcgc gtcggccccg 9780
   agatgcgccg cgtgcggctg ctggagatgg cggacgcgat ggatatgttc tgccaagggt 9840
   tggtttgcgc attcacagtt ctccgcaaga attgattggc tccaattctt ggagtggtga 9900
   atccgttagc gaggtgccgc cctgcttcat ccccgtggcc cgttgctcgc gtttgctggc 9960
   ggtgtccccg gaagaaatat atttgcatgt ctttagttct atgatgacac aaaccccgcc 10020
   cagcgtcttg tcattggcga attcgaacac gcagatgcag tcggggcggc gcggtccgag 10080
   gtccacttcg catattaagg tgacgcgtgt ggcctcgaac accgagcgac cctgcagcga 10140
   cccgcttaac agcgtcaaca gcgtgccgca gatcccgggg ggcaatgaga tatgaaaaag 10200
   cctgaactca ccgcgacgtc tgtcgagaag tttctgatcg aaaagttcga cagcgtctcc 10260
   gacctgatgc agctctcgga gggcgaagaa tctcgtgctt tcagcttcga tgtaggaggg 10320
   cgtggatatg tcctgcgggt aaatagctgc gccgatggtt tctacaaaga tcgttatgtt 10380
   tatcggcact ttgcatcggc cgcgctcccg attccggaag tgcttgacat tggggaattc 10440
   agcgagagcc tgacctattg catctcccgc cgtgcacagg gtgtcacgtt gcaagacctg 10500
   cctgaaaccg aactgcccgc tgttctgcag ccggtcgcgg aggccatgga tgcgatcgct 10560
   gcggccgatc ttagccagac gagcgggttc ggcccattcg gaccgcaagg aatcggtcaa 10620
   tacactacat ggcgtgattt catatgcgcg attgctgatc cccatgtgta tcactggcaa 10680
   actgtgatgg acgacaccgt cagtgcgtcc gtcgcgcagg ctctcgatga gctgatgctt 10740
   tgggccgagg actgccccga agtccggcac ctcgtgcacg cggatttcgg ctccaacaat 10800
   gtcctgacgg acaatggccg cataacagcg gtcattgact ggagcgaggc gatgttcggg 10860
   gattcccaat acgaggtcgc caacatcttc ttctggaggc cgtggttggc ttgtatggag 10920
   cagcagacgc gctacttcga gcggaggcat ccggagcttg caggatcgcc gcggctccgg 10980
   gcgtatatgc tccgcattgg tcttgaccaa ctctatcaga gcttggttga cggcaatttc 11040
   gatgatgcag cttgggcgca gggtcgatgc gacgcaatcg tccgatccgg agccgggact 11100
   gtcgggcgta cacaaatcgc ccgcagaagc gcggccgtct ggaccgatgg ctgtgtagaa 11160
   gtactcgccg atagtggaaa ccgacgcccc agcactcgtc cggatcggga gatgggggag 11220
   gctaactgaa acacggaagg agacaatacc ggaaggaacc cgcgctatga cggcaataaa 11280
   aagacagaat aaaacgcacg ggtgttgggt cgtttgttca taaacgcggg gttcggtccc 11340
   agggctggca ctctgtcgat accccaccga gaccccattg gggccaatac gcccgcgttt 11400
   cttccttttc cccaccccac cccccaagtt cgggtgaagg cccagggctc gcagccaacg 11460
   tcggggcggc aggccctgcc atagccactg gccccgtggg ttagggacgg ggtcccccat 11520
   ggggaatggt ttatggttcg tgggggttat tattttgggc gttgcgtggg gtcaggtcca 11580
   cgactggact gagcagacag acccatggtt tttggatggc ctgggcatgg accgcatgta 11640
   ctggcgcgac acgaacaccg ggcgtctgtg gctgccaaac acccccgacc cccaaaaacc 11700
   accgcgcgga tttctggcgt gccaagctag tcgaccaatt ctcatgtttg acagcttatc 11760
   atcgcagatc cgggcaacgt tgttgccatt gctgcaggcg cagaactggt aggtatggaa 11820
   gatctataca ttgaatcaat attggcaatt agccatatta gtcattggtt atatagcata 11880
   aatcaatatt ggctattggc cattgcatac gttgtatcta tatcataata tgtacattta 11940
   tattggctca tgtccaatat gaccgccat                                   11969
<210> 246
   <211> 330
   <212> DNA
   <213> Homo sapiens
<220>
   <223> gcacctgaagccgccgggggaccgtcagtcttcctcttccccccaaaacc caaggacaccctcatgatctcccggacccctgaggtcacatgcgtggtgg tggacgt
<400> 246
   gcacctgaag ccgccggggg accgtcagtc ttcctcttcc ccccaaaacc caaggacacc 60
   ctcatgatct cccggacccc tgaggtcaca tgcgtggtgg tggacgtgag ccacgaagac 120
   cctgaggtca agttcaactg gtacgtggac ggcgtggagg tgcataatgc caagacaaag 180
   ccgcgggagg agcagtacaa cagcacgtac cgtgtggtca gcgtcctcac cgtcctgcac 240
   caggactggc tgaatggcaa ggagtacaag tgcaaggtct ccaacaaagc cctcccagcc 300
   cccatcgaga aaaccatctc caaagccaaa                                  330
<210> 247
   <211> 110
   <212> PRT
   <213> Homo sapiens
<400> 247
   Ala Pro Glu Ala Ala Gly Gly Pro Ser Val Phe Leu Phe Pro Pro Lys
    1               5                  10                  15
   Pro Lys Asp Thr Leu Met Ile Ser Arg Thr Pro Glu Val Thr Cys Val
               20                  25                  30
   Val Val Asp Val Ser His Glu Asp Pro Glu Val Lys Phe Asn Trp Tyr
           35                  40                  45
   Val Asp Gly Val Glu Val His Asn Ala Lys Thr Lys Pro Arg Glu Glu
       50                  55                  60
   Gln Tyr Asn Ser Thr Tyr Arg Val Val Ser Val Leu Thr Val Leu His
   65                  70                  75                  80
   Gln Asp Trp Leu Asn Gly Lys Glu Tyr Lys Cys Lys Val Ser Asn Lys
                   85                  90                  95
   Ala Leu Pro Ala Pro Ile Glu Lys Thr Ile Ser Lys Ala Lys
               100                 105                 110
<210> 248
   <211> 21
   <212> DNA
   <213> Artificial Sequence
<220>
   <223> In vitro mutagenized Homo sapien DNA encoding a V2 peptide sequence comprising a T to A substitution to limit 0-linked glycosylation.
<400> 248
   accttagtca ccgtctcctc a 21
<210> 249
   <211> 7
   <212> PRT
   <213> Artificial Sequence
<220>
   <223> Mutagenized Homo sapien V2 peptide comprising a T to A substitution to limit 0-linked glycosylation and encoded by an in vitro mutagenized Homo sapien DNA.
<400> 249
   Thr Leu Val Thr Val Ser Ser
    1               5
<210> 250
   <211> 732
   <212> DNA
   <213> Artificial Sequence
<220>
   <223> In vitro synthesized DNA encoding a melanocortin receptor binding alpha-MSH mimetibody without secretory signal (p4362).
<400> 250
   ggcggatgcg agcacttccg ctggtgcaag ccggtgggag gcggaggaag cggcggaggc 60
   ggatcctgcc caccatgccc ggcgcctgag gccgccgggg gaccatcagt cttcctgttc 120
   cccccaaaac ccaaggacac tctcatgatc tcccggaccc ctgaggtcac gtgcgtggtg 180
   gtggacgtga gccaggaaga ccccgaggtc cagttcaact ggtacgtgga tggcgtggag 240
   gtgcataatg ccaagacaaa gccgcgggag gagcagttca acagcacgta ccgtgtggtc 300
   agcgtcctca ccgtcctgca ccaggactgg ctgaacggca aggagtacaa gtgcaaggtc 360
   tccaacaaag gcctcccgtc ctccatcgag aaaaccatct ccaaagccaa agggcagcct 420
   cgagagccac aggtgtacac cctgccccca tcccaggagg agatgaccaa gaaccaggtc 480
   agcctgacct gcctggtcaa aggcttctac cccagcgaca tcgccgtgga gtgggagagc 540
   aatgggcagc cggagaacaa ctacaagacc acgcctcccg tgctggactc cgacggctcc 600
   ttcttcctct acagcaggct aaccgtggac aagagcaggt ggcaggaggg gaatgtcttc 660
   tcatgctccg tgatgcatga ggctctgcac aaccactaca cacagaaaag cttgtccctg 720
   tctctgggta aa                                                     732
<210> 251
   <211> 244
   <212> PRT
   <213> Artificial Sequence
<220>
   <223> Melanocortin receptor binding alpha-MSH mimetibody without secretory signal encoded by an in vitro synthesized DNA (p4362).
<400> 251
   Gly Gly Cys Glu His Phe Arg Trp Cys Lys Pro Val Gly Gly Gly Gly
    1               5                  10                  15
   Ser Gly Gly Gly Gly Ser Cys Pro Pro Cys Pro Ala Pro Glu Ala Ala
               20                  25                  30
   Gly Gly Pro Ser Val Phe Leu Phe Pro Pro Lys Pro Lys Asp Thr Leu
           35                  40                  45
   Met Ile Ser Arg Thr Pro Glu Val Thr Cys Val Val Val Asp Val Ser
       50                  55                  60
   Gln Glu Asp Pro Glu Val Gln Phe Asn Trp Tyr Val Asp Gly Val Glu
   65                  70                  75                  80
   Val His Asn Ala Lys Thr Lys Pro Arg Glu Glu Gln Phe Asn Ser Thr
                   85                  90                  95
   Tyr Arg Val Val Ser Val Leu Thr Val Leu His Gln Asp Trp Leu Asn
               100                 105                 110
   Gly Lys Glu Tyr Lys Cys Lys Val Ser Asn Lys Gly Leu Pro Ser Ser
           115                 120                 125
   Ile Glu Lys Thr Ile Ser Lys Ala Lys Gly Gln Pro Arg Glu Pro Gln
       130                 135                 140
   Val Tyr Thr Leu Pro Pro Ser Gln Glu Glu Met Thr Lys Asn Gln Val
   145                 150                 155                 160
   Ser Leu Thr Cys Leu Val Lys Gly Phe Tyr Pro Ser Asp Ile Ala Val
                   165                 170                 175
   Glu Trp Glu Ser Asn Gly Gln Pro Glu Asn Asn Tyr Lys Thr Thr Pro
               180                 185                 190
   Pro Val Leu Asp Ser Asp Gly Ser Phe Phe Leu Tyr Ser Arg Leu Thr
           195                 200                 205
   Val Asp Lys Ser Arg Trp Gln Glu Gly Asn Val Phe Ser Cys Ser Val
       210                 215                 220
   Met His Glu Ala Leu His Asn His Tyr Thr Gln Lys Ser Leu Ser Leu
   225                 230                 235                 240
   Ser Leu Gly Lys
<210> 252
   <211> 789
   <212> DNA
   <213> Artificial Sequence
<220>
   <223> In vitro synthesized DNA encoding a melanocortin receptor binding alpha-MSH mimetibody with secretory signal (p4362).
<400> 252
   atggcttggg tgtggacctt gctattcctg atggcggccg cccaaagtat acaggccggc 60
   ggatgcgagc acttccgctg gtgcaagccg gtgggaggcg gaggaagcgg cggaggcgga 120
   tcctgcccac catgcccggc gcctgaggcc gccgggggac catcagtctt cctgttcccc 180
   ccaaaaccca aggacactct catgatctcc cggacccctg aggtcacgtg cgtggtggtg 240
   gacgtgagcc aggaagaccc cgaggtccag ttcaactggt acgtggatgg cgtggaggtg 300
   cataatgcca agacaaagcc gcgggaggag cagttcaaca gcacgtaccg tgtggtcagc 360
   gtcctcaccg tcctgcacca ggactggctg aacggcaagg agtacaagtg caaggtctcc 420
   aacaaaggcc tcccgtcctc catcgagaaa accatctcca aagccaaagg gcagcctcga 480
   gagccacagg tgtacaccct gcccccatcc caggaggaga tgaccaagaa ccaggtcagc 540
   ctgacctgcc tggtcaaagg cttctacccc agcgacatcg ccgtggagtg ggagagcaat 600
   gggcagccgg agaacaacta caagaccacg cctcccgtgc tggactccga cggctccttc 660
   ttcctctaca gcaggctaac cgtggacaag agcaggtggc aggaggggaa tgtcttctca 720
   tgctccgtga tgcatgaggc tctgcacaac cactacacac agaaaagctt gtccctgtct 780
   ctgggtaaa                                                          789
<210> 253
   <211> 263
   <212> PRT
   <213> Artificial Sequence
<220>
   <223> Melanocortin receptor binding alpha-MSH mimetibody with secretory signal encoded by an in vitro synthesized DNA (p4362).
<400> 253
   Met Ala Trp Val Trp Thr Leu Leu Phe Leu Met Ala Ala Ala Gln Ser
    1               5                  10                  15
   Ile Gln Ala Gly Gly Cys Glu His Phe Arg Trp Cys Lys Pro Val Gly
               20                  25                  30
   Gly Gly Gly Ser Gly Gly Gly Gly Ser Cys Pro Pro Cys Pro Ala Pro
           35                  40                  45
   Glu Ala Ala Gly Gly Pro Ser Val Phe Leu Phe Pro Pro Lys Pro Lys
       50                  55                  60
   Asp Thr Leu Met Ile Ser Arg Thr Pro Glu Val Thr Cys Val Val Val
   65                  70                  75                  80
   Asp Val Ser Gln Glu Asp Pro Glu Val Gln Phe Asn Trp Tyr Val Asp
                   85                  90                  95
   Gly Val Glu Val His Asn Ala Lys Thr Lys Pro Arg Glu Glu Gln Phe
               100                 105                 110
   Asn Ser Thr Tyr Arg Val Val Ser Val Leu Thr Val Leu His Gln Asp
           115                 120                 125
   Trp Leu Asn Gly Lys Glu Tyr Lys Cys Lys Val Ser Asn Lys Gly Leu
       130                 135                 140
   Pro Ser Ser Ile Glu Lys Thr Ile Ser Lys Ala Lys Gly Gln Pro Arg
   145                 150                 155                 160
   Glu Pro Gln Val Tyr Thr Leu Pro Pro Ser Gln Glu Glu Met Thr Lys
                   165                 170                 175
   Asn Gln Val Ser Leu Thr Cys Leu Val Lys Gly Phe Tyr Pro Ser Asp
               180                 185                 190
   Ile Ala Val Glu Trp Glu Ser Asn Gly Gln Pro Glu Asn Asn Tyr Lys
           195                 200                 205
   Thr Thr Pro Pro Val Leu Asp Ser Asp Gly Ser Phe Phe Leu Tyr Ser
       210                 215                 220
   Arg Leu Thr Val Asp Lys Ser Arg Trp Gln Glu Gly Asn Val Phe Ser
   225                 230                 235                 240
   Cys Ser Val Met His Glu Ala Leu His Asn His Tyr Thr Gln Lys Ser
                   245                 250                 255
   Leu Ser Leu Ser Leu Gly Lys
               260
<210> 254
   <211> 11924
   <212> DNA
   <213> Artificial Sequence
<220>
   <223> In vitro synthesized DNA with expression vector functions that encodes an alpha-MSH mimetibody (p4362).
<400> 254
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggccggc ggatgcgagc acttccgctg 1680
   gtgcaagccg gtgggaggcg gaggaagcgg cggaggcgga tcctgcccac catgcccggc 1740
   gcctgaggcc gccgggggac catcagtctt cctgttcccc ccaaaaccca aggacactct 1800
   catgatctcc cggacccctg aggtcacgtg cgtggtggtg gacgtgagcc aggaagaccc 1860
   cgaggtccag ttcaactggt acgtggatgg cgtggaggtg cataatgcca agacaaagcc 1920
   gcgggaggag cagttcaaca gcacgtaccg tgtggtcagc gtcctcaccg tcctgcacca 1980
   ggactggctg aacggcaagg agtacaagtg caaggtctcc aacaaaggcc tcccgtcctc 2040
   catcgagaaa accatctcca aagccaaagg gcagcctcga gagccacagg tgtacaccct 2100
   gcccccatcc caggaggaga tgaccaagaa ccaggtcagc ctgacctgcc tggtcaaagg 2160
   cttctacccc agcgacatcg ccgtggagtg ggagagcaat gggcagccgg agaacaacta 2220
   caagaccacg cctcccgtgc tggactccga cggctccttc ttcctctaca gcaggctaac 2280
   cgtggacaag agcaggtggc aggaggggaa tgtcttctca tgctccgtga tgcatgaggc 2340
   tctgcacaac cactacacac agaaaagctt gtccctgtct ctgggtaaat gagggcccgt 2400
   ttaaacagat ccagacatga taagatacat tgatgagttt ggacaaacca caactagaat 2460
   gcagtgaaaa aaatgcttta tttgtgaaat ttgtgatgct attgctttat ttgtaaccat 2520
   tataagctgc aataaacaag ttaacaacaa caattgcatt cattttatgt ttcaggttca 2580
   gggggaggtg tgggaggttt tttaaagcaa gtaaaacctc tacaaatgtg gtatggctga 2640
   ttatgatccg gctgcctcgc gcgtttcggt gatgacggtg aaaacctctg acacatgcag 2700
   ctcccggaga cggtcacagc ttgtctgtaa gcggatgccg ggagcagaca agcccgtcag 2760
   gcgtcagcgg gtgttggcgg gtgtcggggc gcagccatga ggtcgactct agaggatcga 2820
   tgccccgccc cggacgaact aaacctgact acgacatctc tgccccttct tcgcggggca 2880
   gtgcatgtaa tcccttcagt tggttggtac aacttgccaa ctgggccctg ttccacatgt 2940
   gacacggggg gggaccaaac acaaaggggt tctctgactg tagttgacat ccttataaat 3000
   ggatgtgcac atttgccaac actgagtggc tttcatcctg gagcagactt tgcagtctgt 3060
   ggactgcaac acaacattgc ctttatgtgt aactcttggc tgaagctctt acaccaatgc 3120
   tgggggacat gtacctccca ggggcccagg aagactacgg gaggctacac caacgtcaat 3180
   cagaggggcc tgtgtagcta ccgataagcg gaccctcaag agggcattag caatagtgtt 3240
   tataaggccc ccttgttaac cctaaacggg tagcatatgc ttcccgggta gtagtatata 3300
   ctatccagac taaccctaat tcaatagcat atgttaccca acgggaagca tatgctatcg 3360
   aattagggtt agtaaaaggg tcctaaggaa cagcgatatc tcccacccca tgagctgtca 3420
   cggttttatt tacatggggt caggattcca cgagggtagt gaaccatttt agtcacaagg 3480
   gcagtggctg aagatcaagg agcgggcagt gaactctcct gaatcttcgc ctgcttcttc 3540
   attctccttc gtttagctaa tagaataact gctgagttgt gaacagtaag gtgtatgtga 3600
   ggtgctcgaa aacaaggttt caggtgacgc ccccagaata aaatttggac ggggggttca 3660
   gtggtggcat tgtgctatga caccaatata accctcacaa accccttggg caataaatac 3720
   tagtgtagga atgaaacatt ctgaatatct ttaacaatag aaatccatgg ggtggggaca 3780
   agccgtaaag actggatgtc catctcacac gaatttatgg ctatgggcaa cacataatcc 3840
   tagtgcaata tgatactggg gttattaaga tgtgtcccag gcagggacca agacaggtga 3900
   accatgttgt tacactctat ttgtaacaag gggaaagaga gtggacgccg acagcagcgg 3960
   actccactgg ttgtctctaa cacccccgaa aattaaacgg ggctccacgc caatggggcc 4020
   cataaacaaa gacaagtggc cactcttttt tttgaaattg tggagtgggg gcacgcgtca 4080
   gcccccacac gccgccctgc ggttttggac tgtaaaataa gggtgtaata acttggctga 4140
   ttgtaacccc gctaaccact gcggtcaaac cacttgccca caaaaccact aatggcaccc 4200
   cggggaatac ctgcataagt aggtgggcgg gccaagatag gggcgcgatt gctgcgatct 4260
   ggaggacaaa ttacacacac ttgcgcctga gcgccaagca cagggttgtt ggtcctcata 4320
   ttcacgaggt cgctgagagc acggtgggct aatgttgcca tgggtagcat atactaccca 4380
   aatatctgga tagcatatgc tatcctaatc tatatctggg tagcataggc tatcctaatc 4440
   tatatctggg tagcatatgc tatcctaatc tatatctggg tagtatatgc tatcctaatt 4500
   tatatctggg tagcataggc tatcctaatc tatatctggg tagcatatgc tatcctaatc 4560
   tatatctggg tagtatatgc tatcctaatc tgtatccggg tagcatatgc tatcctaata 4620
   gagattaggg tagtatatgc tatcctaatt tatatctggg tagcatatac tacccaaata 4680
   tctggatagc atatgctatc ctaatctata tctgggtagc atatgctatc ctaatctata 4740
   tctgggtagc ataggctatc ctaatctata tctgggtagc atatgctatc ctaatctata 4800
   tctgggtagt atatgctatc ctaatttata tctgggtagc ataggctatc ctaatctata 4860
   tctgggtagc atatgctatc ctaatctata tctgggtagt atatgctatc ctaatctgta 4920
   tccgggtagc atatgctatc ctcatgcata tacagtcagc atatgatacc cagtagtaga 4980
   gtgggagtgc tatcctttgc atatgccgcc acctcccaag ggggcgtgaa ttttcgctgc 5040
   ttgtcctttt cctgctggtt gctcccattc ttaggtgaat ttaaggaggc caggctaaag 5100
   ccgtcgcatg tctgattgct caccaggtaa atgtcgctaa tgttttccaa cgcgagaagg 5160
   tgttgagcgc ggagctgagt gacgtgacaa catgggtatg cccaattgcc ccatgttggg 5220
   aggacgaaaa tggtgacaag acagatggcc agaaatacac caacagcacg catgatgtct 5280
   actggggatt tattctttag tgcgggggaa tacacggctt ttaatacgat tgagggcgtc 5340
   tcctaacaag ttacatcact cctgcccttc ctcaccctca tctccatcac ctccttcatc 5400
   tccgtcatct ccgtcatcac cctccgcggc agccccttcc accataggtg gaaaccaggg 5460
   aggcaaatct actccatcgt caaagctgca cacagtcacc ctgatattgc aggtaggagc 5520
   gggctttgtc ataacaaggt ccttaatcgc atccttcaaa acctcagcaa atatatgagt 5580
   ttgtaaaaag accatgaaat aacagacaat ggactccctt agcgggccag gttgtgggcc 5640
   gggtccaggg gccattccaa aggggagacg actcaatggt gtaagacgac attgtggaat 5700
   agcaagggca gttcctcgcc ttaggttgta aagggaggtc ttactacctc catatacgaa 5760
   cacaccggcg acccaagttc cttcgtcggt agtcctttct acgtgactcc tagccaggag 5820
   agctcttaaa ccttctgcaa tgttctcaaa tttcgggttg gaacctcctt gaccacgatg 5880
   cttttccaaa ccaccctcct tttttgcgcc ctgcctccat caccctgacc ccggggtcca 5940
   gtgcttgggc cttctcctgg gtcatctgcg gggccctgct ctatcgctcc cgggggcacg 6000
   tcaggctcac catctgggcc accttcttgg tggtattcaa aataatcggc ttcccctaca 6060
   gggtggaaaa atggccttct acctggaggg ggcctgcgcg gtggagaccc ggatgatgat 6120
   gactgactac tgggactcct gggcctcttt tctccacgtc cacgacctct ccccctggct 6180
   ctttcacgac ttccccccct ggctctttca cgtcctctac cccggcggcc tccactacct 6240
   cctcgacccc ggcctccact acctcctcga ccccggcctc cactgcctcc tcgaccccgg 6300
   cctccacctc ctgctcctgc ccctcctgct cctgcccctc ctcctgctcc tgcccctcct 6360
   gcccctcctg ctcctgcccc tcctgcccct cctgctcctg cccctcctgc ccctcctgct 6420
   cctgcccctc ctgcccctcc tcctgctcct gcccctcctg cccctcctcc tgctcctgcc 6480
   cctcctgccc ctcctgctcc tgcccctcct gcccctcctg ctcctgcccc tcctgcccct 6540
   cctgctcctg cccctcctgc tcctgcccct cctgctcctg cccctcctgc tcctgcccct 6600
   cctgcccctc ctgcccctcc tcctgctcct gcccctcctg ctcctgcccc tcctgcccct 6660
   cctgcccctc ctgctcctgc ccctcctcct gctcctgccc ctcctgcccc tcctgcccct 6720
   cctcctgctc ctgcccctcc tgcccctcct cctgctcctg cccctcctcc tgctcctgcc 6780
   cctcctgccc ctcctgcccc tcctcctgct cctgcccctc ctgcccctcc tcctgctcct 6840
   gcccctcctc ctgctcctgc ccctcctgcc cctcctgccc ctcctcctgc tcctgcccct 6900
   cctcctgctc ctgcccctcc tgcccctcct gcccctcctg cccctcctcc tgctcctgcc 6960
   cctcctcctg ctcctgcccc tcctgctcct gcccctcccg ctcctgctcc tgctcctgtt 7020
   ccaccgtggg tccctttgca gccaatgcaa cttggacgtt tttggggtct ccggacacca 7080
   tctctatgtc ttggccctga tcctgagccg cccggggctc ctggtcttcc gcctcctcgt 7140
   cctcgtcctc ttccccgtcc tcgtccatgg ttatcacccc ctcttctttg aggtccactg 7200
   ccgccggagc cttctggtcc agatgtgtct cccttctctc ctaggccatt tccaggtcct 7260
   gtacctggcc cctcgtcaga catgattcac actaaaagag atcaatagac atctttatta 7320
   gacgacgctc agtgaataca gggagtgcag actcctgccc cctccaacag cccccccacc 7380
   ctcatcccct tcatggtcgc tgtcagacag atccaggtct gaaaattccc catcctccga 7440
   accatcctcg tcctcatcac caattactcg cagcccggaa aactcccgct gaacatcctc 7500
   aagatttgcg tcctgagcct caagccaggc ctcaaattcc tcgtccccct ttttgctgga 7560
   cggtagggat ggggattctc gggacccctc ctcttcctct tcaaggtcac cagacagaga 7620
   tgctactggg gcaacggaag aaaagctggg tgcggcctgt gaggatcagc ttatcgatga 7680
   taagctgtca aacatgagaa ttcttgaaga cgaaagggcc tcgtgatacg cctattttta 7740
   taggttaatg tcatgataat aatggtttct tagacgtcag gtggcacttt tcggggaaat 7800
   gtgcgcggaa cccctatttg tttatttttc taaatacatt caaatatgta tccgctcatg 7860
   agacaataac cctgataaat gcttcaataa tattgaaaaa ggaagagtat gagtattcaa 7920
   catttccgtg tcgcccttat tccctttttt gcggcatttt gccttcctgt ttttgctcac 7980
   ccagaaacgc tggtgaaagt aaaagatgct gaagatcagt tgggtgcacg agtgggttac 8040
   atcgaactgg atctcaacag cggtaagatc cttgagagtt ttcgccccga agaacgtttt 8100
   ccaatgatga gcacttttaa agttctgcta tgtggcgcgg tattatcccg tgttgacgcc 8160
   gggcaagagc aactcggtcg ccgcatacac tattctcaga atgacttggt tgagtactca 8220
   ccagtcacag aaaagcatct tacggatggc atgacagtaa gagaattatg cagtgctgcc 8280
   ataaccatga gtgataacac tgcggccaac ttacttctga caacgatcgg aggaccgaag 8340
   gagctaaccg cttttttgca caacatgggg gatcatgtaa ctcgccttga tcgttgggaa 8400
   ccggagctga atgaagccat accaaacgac gagcgtgaca ccacgatgcc tgcagcaatg 8460
   gcaacaacgt tgcgcaaact attaactggc gaactactta ctctagcttc ccggcaacaa 8520
   ttaatagact ggatggaggc ggataaagtt gcaggaccac ttctgcgctc ggcccttccg 8580
   gctggctggt ttattgctga taaatctgga gccggtgagc gtgggtctcg cggtatcatt 8640
   gcagcactgg ggccagatgg taagccctcc cgtatcgtag ttatctacac gacggggagt 8700
   caggcaacta tggatgaacg aaatagacag atcgctgaga taggtgcctc actgattaag 8760
   cattggtaac tgtcagacca agtttactca tatatacttt agattgattt aaaacttcat 8820
   ttttaattta aaaggatcta ggtgaagatc ctttttgata atctcatgac caaaatccct 8880
   taacgtgagt tttcgttcca ctgagcgtca gaccccgtag aaaagatcaa aggatcttct 8940
   tgagatcctt tttttctgcg cgtaatctgc tgcttgcaaa caaaaaaacc accgctacca 9000
   gcggtggttt gtttgccgga tcaagagcta ccaactcttt ttccgaaggt aactggcttc 9060
   agcagagcgc agataccaaa tactgtcctt ctagtgtagc cgtagttagg ccaccacttc 9120
   aagaactctg tagcaccgcc tacatacctc gctctgctaa tcctgttacc agtggctgct 9180
   gccagtggcg ataagtcgtg tcttaccggg ttggactcaa gacgatagtt accggataag 9240
   gcgcagcggt cgggctgaac ggggggttcg tgcacacagc ccagcttgga gcgaacgacc 9300
   tacaccgaac tgagatacct acagcgtgag ctatgagaaa gcgccacgct tcccgaaggg 9360
   agaaaggcgg acaggtatcc ggtaagcggc agggtcggaa caggagagcg cacgagggag 9420
   cttccagggg gaaacgcctg gtatctttat agtcctgtcg ggtttcgcca cctctgactt 9480
   gagcgtcgat ttttgtgatg ctcgtcaggg gggcggagcc tatggaaaaa cgccagcaac 9540
   gcggcctttt tacggttcct ggccttttgc tggccttgaa gctgtccctg atggtcgtca 9600
   tctacctgcc tggacagcat ggcctgcaac gcgggcatcc cgatgccgcc ggaagcgaga 9660
   agaatcataa tggggaaggc catccagcct cgcgtcgcga acgccagcaa gacgtagccc 9720
   agcgcgtcgg ccccgagatg cgccgcgtgc ggctgctgga gatggcggac gcgatggata 9780
   tgttctgcca agggttggtt tgcgcattca cagttctccg caagaattga ttggctccaa 9840
   ttcttggagt ggtgaatccg ttagcgaggt gccgccctgc ttcatccccg tggcccgttg 9900
   ctcgcgtttg ctggcggtgt ccccggaaga aatatatttg catgtcttta gttctatgat 9960
   gacacaaacc ccgcccagcg tcttgtcatt ggcgaattcg aacacgcaga tgcagtcggg 10020
   gcggcgcggt ccgaggtcca cttcgcatat taaggtgacg cgtgtggcct cgaacaccga 10080
   gcgaccctgc agcgacccgc ttaacagcgt caacagcgtg ccgcagatcc cggggggcaa 10140
   tgagatatga aaaagcctga actcaccgcg acgtctgtcg agaagtttct gatcgaaaag 10200
   ttcgacagcg tctccgacct gatgcagctc tcggagggcg aagaatctcg tgctttcagc 10260
   ttcgatgtag gagggcgtgg atatgtcctg cgggtaaata gctgcgccga tggtttctac 10320
   aaagatcgtt atgtttatcg gcactttgca tcggccgcgc tcccgattcc ggaagtgctt 10380
   gacattgggg aattcagcga gagcctgacc tattgcatct cccgccgtgc acagggtgtc 10440
   acgttgcaag acctgcctga aaccgaactg cccgctgttc tgcagccggt cgcggaggcc 10500
   atggatgcga tcgctgcggc cgatcttagc cagacgagcg ggttcggccc attcggaccg 10560
   caaggaatcg gtcaatacac tacatggcgt gatttcatat gcgcgattgc tgatccccat 10620
   gtgtatcact ggcaaactgt gatggacgac accgtcagtg cgtccgtcgc gcaggctctc 10680
   gatgagctga tgctttgggc cgaggactgc cccgaagtcc ggcacctcgt gcacgcggat 10740
   ttcggctcca acaatgtcct gacggacaat ggccgcataa cagcggtcat tgactggagc 10800
   gaggcgatgt tcggggattc ccaatacgag gtcgccaaca tcttcttctg gaggccgtgg 10860
   ttggcttgta tggagcagca gacgcgctac ttcgagcgga ggcatccgga gcttgcagga 10920
   tcgccgcggc tccgggcgta tatgctccgc attggtcttg accaactcta tcagagcttg 10980
   gttgacggca atttcgatga tgcagcttgg gcgcagggtc gatgcgacgc aatcgtccga 11040
   tccggagccg ggactgtcgg gcgtacacaa atcgcccgca gaagcgcggc cgtctggacc 11100
   gatggctgtg tagaagtact cgccgatagt ggaaaccgac gccccagcac tcgtccggat 11160
   cgggagatgg gggaggctaa ctgaaacacg gaaggagaca ataccggaag gaacccgcgc 11220
   tatgacggca ataaaaagac agaataaaac gcacgggtgt tgggtcgttt gttcataaac 11280
   gcggggttcg gtcccagggc tggcactctg tcgatacccc accgagaccc cattggggcc 11340
   aatacgcccg cgtttcttcc ttttccccac cccacccccc aagttcgggt gaaggcccag 11400
   ggctcgcagc caacgtcggg gcggcaggcc ctgccatagc cactggcccc gtgggttagg 11460
   gacggggtcc cccatgggga atggtttatg gttcgtgggg gttattattt tgggcgttgc 11520
   gtggggtcag gtccacgact ggactgagca gacagaccca tggtttttgg atggcctggg 11580
   catggaccgc atgtactggc gcgacacgaa caccgggcgt ctgtggctgc caaacacccc 11640
   cgacccccaa aaaccaccgc gcggatttct ggcgtgccaa gctagtcgac caattctcat 11700
   gtttgacagc ttatcatcgc agatccgggc aacgttgttg ccattgctgc aggcgcagaa 11760
   ctggtaggta tggaagatct atacattgaa tcaatattgg caattagcca tattagtcat 11820
   tggttatata gcataaatca atattggcta ttggccattg catacgttgt atctatatca 11880
   taatatgtac atttatattg gctcatgtcc aatatgaccg ccat                 11924
<210> 255
   <211> 753
   <212> DNA
   <213> Artificial Sequence
<220>
   <223> In vitro synthesized DNA encoding a melanocortin receptor binding alpha-MSH mimetibody without secretory signal (p4371).
<400> 255
   ggcggatgcg agcacttccg ctggtgcaag ccggtgggat ccggtacctt agtcaccaac 60
   tccaccgagt ccaaatatgg tcccccatgc ccaccatgcc cggcgcctga ggccgccggg 120
   ggaccatcag tcttcctgtt ccccccaaaa cccaaggaca ctctcatgat ctcccggacc 180
   cctgaggtca cgtgcgtggt ggtggacgtg agccaggaag accccgaggt ccagttcaac 240
   tggtacgtgg atggcgtgga ggtgcataat gccaagacaa agccgcggga ggagcagttc 300
   aacagcacgt accgtgtggt cagcgtcctc accgtcctgc accaggactg gctgaacggc 360
   aaggagtaca agtgcaaggt ctccaacaaa ggcctcccgt cctccatcga gaaaaccatc 420
   tccaaagcca aagggcagcc tcgagagcca caggtgtaca ccctgccccc atcccaggag 480
   gagatgacca agaaccaggt cagcctgacc tgcctggtca aaggcttcta ccccagcgac 540
   atcgccgtgg agtgggagag caatgggcag ccggagaaca actacaagac cacgcctccc 600
   gtgctggact ccgacggctc cttcttcctc tacagcaggc taaccgtgga caagagcagg 660
   tggcaggagg ggaatgtctt ctcatgctcc gtgatgcatg aggctctgca caaccactac 720
   acacagaaaa gcttgtccct gtctctgggt aaa                              753
<210> 256
   <211> 251
   <212> PRT
   <213> Artificial Sequence
<220>
   <223> Melanocortin receptor binding alpha-MSH mimetibody without secretory signal encoded by an in vitro synthesized DNA (p4371).
<400> 256
   Gly Gly Cys Glu His Phe Arg Trp Cys Lys Pro Val Gly Ser Gly Thr
    1               5                  10                  15
   Leu Val Thr Asn Ser Thr Glu Ser Lys Tyr Gly Pro Pro Cys Pro Pro
               20                  25                  30
   Cys Pro Ala Pro Glu Ala Ala Gly Gly Pro Ser Val Phe Leu Phe Pro
       35                  40                  45
   Pro Lys Pro Lys Asp Thr Leu Met Ile Ser Arg Thr Pro Glu Val Thr
       50                  55                  60
   Cys Val Val Val Asp Val Ser Gln Glu Asp Pro Glu Val Gln Phe Asn
   65                  70                  75                  80
   Trp Tyr Val Asp Gly Val Glu Val His Asn Ala Lys Thr Lys Pro Arg
                   85                  90                  95
   Glu Glu Gln Phe Asn Ser Thr Tyr Arg Val Val Ser Val Leu Thr Val
               100                 105                 110
   Leu His Gln Asp Trp Leu Asn Gly Lys Glu Tyr Lys Cys Lys Val Ser
           115                 120                 125
   Asn Lys Gly Leu Pro Ser Ser Ile Glu Lys Thr Ile Ser Lys Ala Lys
       130                 135                 140
   Gly Gln Pro Arg Glu Pro Gln Val Tyr Thr Leu Pro Pro Ser Gln Glu
   145                 150                 155                 160
   Glu Met Thr Lys Asn Gln Val Ser Leu Thr Cys Leu Val Lys Gly Phe
                   165                 170                 175
   Tyr Pro Ser Asp Ile Ala Val Glu Trp Glu Ser Asn Gly Gln Pro Glu
               180                 185                 190
   Asn Asn Tyr Lys Thr Thr Pro Pro Val Leu Asp Ser Asp Gly Ser Phe
           195                 200                 205
   Phe Leu Tyr Ser Arg Leu Thr Val Asp Lys Ser Arg Trp Gln Glu Gly
       210                 215                 220
   Asn Val Phe Ser Cys Ser Val Met His Glu Ala Leu His Asn His Tyr
   225                 230                 235                 240
   Thr Gln Lys Ser Leu Ser Leu Ser Leu Gly Lys
                   245                 250
<210> 257
   <211> 810
   <212> DNA
   <213> Artificial Sequence
<220>
   <223> In vitro synthesized DNA encoding a melanocortin receptor binding alpha-MSH mimetibody with secretory signal (p4371).
<400> 257
   atggcttggg tgtggacctt gctattcctg atggcggccg cccaaagtat acaggccggc 60
   ggatgcgagc acttccgctg gtgcaagccg gtgggatccg gtaccttagt caccaactcc 120
   accgagtcca aatatggtcc cccatgccca ccatgcccgg cgcctgaggc cgccggggga 180
   ccatcagtct tcctgttccc cccaaaaccc aaggacactc tcatgatctc ccggacccct 240
   gaggtcacgt gcgtggtggt ggacgtgagc caggaagacc ccgaggtcca gttcaactgg 300
   tacgtggatg gcgtggaggt gcataatgcc aagacaaagc cgcgggagga gcagttcaac 360
   agcacgtacc gtgtggtcag cgtcctcacc gtcctgcacc aggactggct gaacggcaag 420
   gagtacaagt gcaaggtctc caacaaaggc ctcccgtcct ccatcgagaa aaccatctcc 480
   aaagccaaag ggcagcctcg agagccacag gtgtacaccc tgcccccatc ccaggaggag 540
   atgaccaaga accaggtcag cctgacctgc ctggtcaaag gcttctaccc cagcgacatc 600
   gccgtggagt gggagagcaa tgggcagccg gagaacaact acaagaccac gcctcccgtg 660
   ctggactccg acggctcctt cttcctctac agcaggctaa ccgtggacaa gagcaggtgg 720
   caggagggga atgtcttctc atgctccgtg atgcatgagg ctctgcacaa ccactacaca 780
   cagaaaagct tgtccctgtc tctgggtaaa                                 810
<210> 258
   <211> 270
   <212> PRT
   <213> Artificial Sequence
<220>
   <223> Melanocortin receptor binding alpha-MSH mimetibody with secretory signal encoded by an in vitro synthesized DNA (p4371).
<400> 258
   Met Ala Trp Val Trp Thr Leu Leu Phe Leu Met Ala Ala Ala Gln Ser
    1               5                  10                  15
   Ile Gln Ala Gly Gly Cys Glu His Phe Arg Trp Cys Lys Pro Val Gly
               20                  25                  30
   Ser Gly Thr Leu Val Thr Asn Ser Thr Glu Ser Lys Tyr Gly Pro Pro
           35                  40                  45
   Cys Pro Pro Cys Pro Ala Pro Glu Ala Ala Gly Gly Pro Ser Val Phe
       50                  55                  60
   Leu Phe Pro Pro Lys Pro Lys Asp Thr Leu Met Ile Ser Arg Thr Pro
   65                  70                  75                  80
   Glu Val Thr Cys Val Val Val Asp Val Ser Gln Glu Asp Pro Glu Val
                   85                  90                  95
   Gln Phe Asn Trp Tyr Val Asp Gly Val Glu Val His Asn Ala Lys Thr
               100                 105                 110
   Lys Pro Arg Glu Glu Gln Phe Asn Ser Thr Tyr Arg Val Val Ser Val
           115                 120                 125
   Leu Thr Val Leu His Gln Asp Trp Leu Asn Gly Lys Glu Tyr Lys Cys
       130                 135                 140
   Lys Val Ser Asn Lys Gly Leu Pro Ser Ser Ile Glu Lys Thr Ile Ser
   145                 150                 155                 160
   Lys Ala Lys Gly Gln Pro Arg Glu Pro Gln Val Tyr Thr Leu Pro Pro
                   165                 170                 175
   Ser Gln Glu Glu Met Thr Lys Asn Gln Val Ser Leu Thr Cys Leu Val
               180                 185                 190
   Lys Gly Phe Tyr Pro Ser Asp Ile Ala Val Glu Trp Glu Ser Asn Gly
           195                 200                 205
   Gln Pro Glu Asn Asn Tyr Lys Thr Thr Pro Pro Val Leu Asp Ser Asp
       210                 215                 220
   Gly Ser Phe Phe Leu Tyr Ser Arg Leu Thr Val Asp Lys Ser Arg Trp
   225                 230                 235                 240
   Gln Glu Gly Asn Val Phe Ser Cys Ser Val Met His Glu Ala Leu His
                   245                 250                 255
   Asn His Tyr Thr Gln Lys Ser Leu Ser Leu Ser Leu Gly Lys
               260                 265                 270
<210> 259
   <211> 11945
   <212> DNA
   <213> Artificial Sequence
<220>
   <223> In vitro synthesized DNA with expression vector functions that encodes an alpha-MSH mimetibody (p4371).
<400> 259
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggccggc ggatgcgagc acttccgctg 1680
   gtgcaagccg gtgggatccg gtaccttagt caccaactcc accgagtcca aatatggtcc 1740
   cccatgccca ccatgcccgg cgcctgaggc cgccggggga ccatcagtct tcctgttccc 1800
   cccaaaaccc aaggacactc tcatgatctc ccggacccct gaggtcacgt gcgtggtggt 1860
   ggacgtgagc caggaagacc ccgaggtcca gttcaactgg tacgtggatg gcgtggaggt 1920
   gcataatgcc aagacaaagc cgcgggagga gcagttcaac agcacgtacc gtgtggtcag 1980
   cgtcctcacc gtcctgcacc aggactggct gaacggcaag gagtacaagt gcaaggtctc 2040
   caacaaaggc ctcccgtcct ccatcgagaa aaccatctcc aaagccaaag ggcagcctcg 2100
   agagccacag gtgtacaccc tgcccccatc ccaggaggag atgaccaaga accaggtcag 2160
   cctgacctgc ctggtcaaag gcttctaccc cagcgacatc gccgtggagt gggagagcaa 2220
   tgggcagccg gagaacaact acaagaccac gcctcccgtg ctggactccg acggctcctt 2280
   cttcctctac agcaggctaa ccgtggacaa gagcaggtgg caggagggga atgtcttctc 2340
   atgctccgtg atgcatgagg ctctgcacaa ccactacaca cagaaaagct tgtccctgtc 2400
   tctgggtaaa tgagggcccg tttaaacaga tccagacatg ataagataca ttgatgagtt 2460
   tggacaaacc acaactagaa tgcagtgaaa aaaatgcttt atttgtgaaa tttgtgatgc 2520
   tattgcttta tttgtaacca ttataagctg caataaacaa gttaacaaca acaattgcat 2580
   tcattttatg tttcaggttc agggggaggt gtgggaggtt ttttaaagca agtaaaacct 2640
   ctacaaatgt ggtatggctg attatgatcc ggctgcctcg cgcgtttcgg tgatgacggt 2700
   gaaaacctct gacacatgca gctcccggag acggtcacag cttgtctgta agcggatgcc 2760
   gggagcagac aagcccgtca ggcgtcagcg ggtgttggcg ggtgtcgggg cgcagccatg 2820
   aggtcgactc tagaggatcg atgccccgcc ccggacgaac taaacctgac tacgacatct 2880
   ctgccccttc ttcgcggggc agtgcatgta atcccttcag ttggttggta caacttgcca 2940
   actgggccct gttccacatg tgacacgggg ggggaccaaa cacaaagggg ttctctgact 3000
   gtagttgaca tccttataaa tggatgtgca catttgccaa cactgagtgg ctttcatcct 3060
   ggagcagact ttgcagtctg tggactgcaa cacaacattg cctttatgtg taactcttgg 3120
   ctgaagctct tacaccaatg ctgggggaca tgtacctccc aggggcccag gaagactacg 3180
   ggaggctaca ccaacgtcaa tcagaggggc ctgtgtagct accgataagc ggaccctcaa 3240
   gagggcatta gcaatagtgt ttataaggcc cccttgttaa ccctaaacgg gtagcatatg 3300
   cttcccgggt agtagtatat actatccaga ctaaccctaa ttcaatagca tatgttaccc 3360
   aacgggaagc atatgctatc gaattagggt tagtaaaagg gtcctaagga acagcgatat 3420
   ctcccacccc atgagctgtc acggttttat ttacatgggg tcaggattcc acgagggtag 3480
   tgaaccattt tagtcacaag ggcagtggct gaagatcaag gagcgggcag tgaactctcc 3540
   tgaatcttcg cctgcttctt cattctcctt cgtttagcta atagaataac tgctgagttg 3600
   tgaacagtaa ggtgtatgtg aggtgctcga aaacaaggtt tcaggtgacg cccccagaat 3660
   aaaatttgga cggggggttc agtggtggca ttgtgctatg acaccaatat aaccctcaca 3720
   aaccccttgg gcaataaata ctagtgtagg aatgaaacat tctgaatatc tttaacaata 3780
   gaaatccatg gggtggggac aagccgtaaa gactggatgt ccatctcaca cgaatttatg 3840
   gctatgggca acacataatc ctagtgcaat atgatactgg ggttattaag atgtgtccca 3900
   ggcagggacc aagacaggtg aaccatgttg ttacactcta tttgtaacaa ggggaaagag 3960
   agtggacgcc gacagcagcg gactccactg gttgtctcta acacccccga aaattaaacg 4020
   gggctccacg ccaatggggc ccataaacaa agacaagtgg ccactctttt ttttgaaatt 4080
   gtggagtggg ggcacgcgtc agcccccaca cgccgccctg cggttttgga ctgtaaaata 4140
   agggtgtaat aacttggctg attgtaaccc cgctaaccac tgcggtcaaa ccacttgccc 4200
   acaaaaccac taatggcacc ccggggaata cctgcataag taggtgggcg ggccaagata 4260
   ggggcgcgat tgctgcgatc tggaggacaa attacacaca cttgcgcctg agcgccaagc 4320
   acagggttgt tggtcctcat attcacgagg tcgctgagag cacggtgggc taatgttgcc 4380
   atgggtagca tatactaccc aaatatctgg atagcatatg ctatcctaat ctatatctgg 4440
   gtagcatagg ctatcctaat ctatatctgg gtagcatatg ctatcctaat ctatatctgg 4500
   gtagtatatg ctatcctaat ttatatctgg gtagcatagg ctatcctaat ctatatctgg 4560
   gtagcatatg ctatcctaat ctatatctgg gtagtatatg ctatcctaat ctgtatccgg 4620
   gtagcatatg ctatcctaat agagattagg gtagtatatg ctatcctaat ttatatctgg 4680
   gtagcatata ctacccaaat atctggatag catatgctat cctaatctat atctgggtag 4740
   catatgctat cctaatctat atctgggtag cataggctat cctaatctat atctgggtag 4800
   catatgctat cctaatctat atctgggtag tatatgctat cctaatttat atctgggtag 4860
   cataggctat cctaatctat atctgggtag catatgctat cctaatctat atctgggtag 4920
   tatatgctat cctaatctgt atccgggtag catatgctat cctcatgcat atacagtcag 4980
   catatgatac ccagtagtag agtgggagtg ctatcctttg catatgccgc cacctcccaa 5040
   gggggcgtga attttcgctg cttgtccttt tcctgctggt tgctcccatt cttaggtgaa 5100
   tttaaggagg ccaggctaaa gccgtcgcat gtctgattgc tcaccaggta aatgtcgcta 5160
   atgttttcca acgcgagaag gtgttgagcg cggagctgag tgacgtgaca acatgggtat 5220
   gcccaattgc cccatgttgg gaggacgaaa atggtgacaa gacagatggc cagaaataca 5280
   ccaacagcac gcatgatgtc tactggggat ttattcttta gtgcggggga atacacggct 5340
   tttaatacga ttgagggcgt ctcctaacaa gttacatcac tcctgccctt cctcaccctc 5400
   atctccatca cctccttcat ctccgtcatc tccgtcatca ccctccgcgg cagccccttc 5460
   caccataggt ggaaaccagg gaggcaaatc tactccatcg tcaaagctgc acacagtcac 5520
   cctgatattg caggtaggag cgggctttgt cataacaagg tccttaatcg catccttcaa 5580
   aacctcagca aatatatgag tttgtaaaaa gaccatgaaa taacagacaa tggactccct 5640
   tagcgggcca ggttgtgggc cgggtccagg ggccattcca aaggggagac gactcaatgg 5700
   tgtaagacga cattgtggaa tagcaagggc agttcctcgc cttaggttgt aaagggaggt 5760
   cttactacct ccatatacga acacaccggc gacccaagtt ccttcgtcgg tagtcctttc 5820
   tacgtgactc ctagccagga gagctcttaa accttctgca atgttctcaa atttcgggtt 5880
   ggaacctcct tgaccacgat gcttttccaa accaccctcc ttttttgcgc cctgcctcca 5940
   tcaccctgac cccggggtcc agtgcttggg ccttctcctg ggtcatctgc ggggccctgc 6000
   tctatcgctc ccgggggcac gtcaggctca ccatctgggc caccttcttg gtggtattca 6060
   aaataatcgg cttcccctac agggtggaaa aatggccttc tacctggagg gggcctgcgc 6120
   ggtggagacc cggatgatga tgactgacta ctgggactcc tgggcctctt ttctccacgt 6180
   ccacgacctc tccccctggc tctttcacga cttccccccc tggctctttc acgtcctcta 6240
   ccccggcggc ctccactacc tcctcgaccc cggcctccac tacctcctcg accccggcct 6300
   ccactgcctc ctcgaccccg gcctccacct cctgctcctg cccctcctgc tcctgcccct 6360
   cctcctgctc ctgcccctcc tgcccctcct gctcctgccc ctcctgcccc tcctgctcct 6420
   gcccctcctg cccctcctgc tcctgcccct cctgcccctc ctcctgctcc tgcccctcct 6480
   gcccctcctc ctgctcctgc ccctcctgcc cctcctgctc ctgcccctcc tgcccctcct 6540
   gctcctgccc ctcctgcccc tcctgctcct gcccctcctg ctcctgcccc tcctgctcct 6600
   gcccctcctg ctcctgcccc tcctgcccct cctgcccctc ctcctgctcc tgcccctcct 6660
   gctcctgccc ctcctgcccc tcctgcccct cctgctcctg cccctcctcc tgctcctgcc 6720
   cctcctgccc ctcctgcccc tcctcctgct cctgcccctc ctgcccctcc tcctgctcct 6780
   gcccctcctc ctgctcctgc ccctcctgcc cctcctgccc ctcctcctgc tcctgcccct 6840
   cctgcccctc ctcctgctcc tgcccctcct cctgctcctg cccctcctgc ccctcctgcc 6900
   cctcctcctg ctcctgcccc tcctcctgct cctgcccctc ctgcccctcc tgcccctcct 6960
   gcccctcctc ctgctcctgc ccctcctcct gctcctgccc ctcctgctcc tgcccctccc 7020
   gctcctgctc ctgctcctgt tccaccgtgg gtccctttgc agccaatgca acttggacgt 7080
   ttttggggtc tccggacacc atctctatgt cttggccctg atcctgagcc gcccggggct 7140
   cctggtcttc cgcctcctcg tcctcgtcct cttccccgtc ctcgtccatg gttatcaccc 7200
   cctcttcttt gaggtccact gccgccggag ccttctggtc cagatgtgtc tcccttctct 7260
   cctaggccat ttccaggtcc tgtacctggc ccctcgtcag acatgattca cactaaaaga 7320
   gatcaataga catctttatt agacgacgct cagtgaatac agggagtgca gactcctgcc 7380
   ccctccaaca gcccccccac cctcatcccc ttcatggtcg ctgtcagaca gatccaggtc 7440
   tgaaaattcc ccatcctccg aaccatcctc gtcctcatca ccaattactc gcagcccgga 7500
   aaactcccgc tgaacatcct caagatttgc gtcctgagcc tcaagccagg cctcaaattc 7560
   ctcgtccccc tttttgctgg acggtaggga tggggattct cgggacccct cctcttcctc 7620
   ttcaaggtca ccagacagag atgctactgg ggcaacggaa gaaaagctgg gtgcggcctg 7680
   tgaggatcag cttatcgatg ataagctgtc aaacatgaga attcttgaag acgaaagggc 7740
   ctcgtgatac gcctattttt ataggttaat gtcatgataa taatggtttc ttagacgtca 7800
   ggtggcactt ttcggggaaa tgtgcgcgga acccctattt gtttattttt ctaaatacat 7860
   tcaaatatgt atccgctcat gagacaataa ccctgataaa tgcttcaata atattgaaaa 7920
   aggaagagta tgagtattca acatttccgt gtcgccctta ttcccttttt tgcggcattt 7980
   tgccttcctg tttttgctca cccagaaacg ctggtgaaag taaaagatgc tgaagatcag 8040
   ttgggtgcac gagtgggtta catcgaactg gatctcaaca gcggtaagat ccttgagagt 8100
   tttcgccccg aagaacgttt tccaatgatg agcactttta aagttctgct atgtggcgcg 8160
   gtattatccc gtgttgacgc cgggcaagag caactcggtc gccgcataca ctattctcag 8220
   aatgacttgg ttgagtactc accagtcaca gaaaagcatc ttacggatgg catgacagta 8280
   agagaattat gcagtgctgc cataaccatg agtgataaca ctgcggccaa cttacttctg 8340
   acaacgatcg gaggaccgaa ggagctaacc gcttttttgc acaacatggg ggatcatgta 8400
   actcgccttg atcgttggga accggagctg aatgaagcca taccaaacga cgagcgtgac 8460
   accacgatgc ctgcagcaat ggcaacaacg ttgcgcaaac tattaactgg cgaactactt 8520
   actctagctt cccggcaaca attaatagac tggatggagg cggataaagt tgcaggacca 8580
   cttctgcgct cggcccttcc ggctggctgg tttattgctg ataaatctgg agccggtgag 8640
   cgtgggtctc gcggtatcat tgcagcactg gggccagatg gtaagccctc ccgtatcgta 8700
   gttatctaca cgacggggag tcaggcaact atggatgaac gaaatagaca gatcgctgag 8760
   ataggtgcct cactgattaa gcattggtaa ctgtcagacc aagtttactc atatatactt 8820
   tagattgatt taaaacttca tttttaattt aaaaggatct aggtgaagat cctttttgat 8880
   aatctcatga ccaaaatccc ttaacgtgag ttttcgttcc actgagcgtc agaccccgta 8940
   gaaaagatca aaggatcttc ttgagatcct ttttttctgc gcgtaatctg ctgcttgcaa 9000
   acaaaaaaac caccgctacc agcggtggtt tgtttgccgg atcaagagct accaactctt 9060
   tttccgaagg taactggctt cagcagagcg cagataccaa atactgtcct tctagtgtag 9120
   ccgtagttag gccaccactt caagaactct gtagcaccgc ctacatacct cgctctgcta 9180
   atcctgttac cagtggctgc tgccagtggc gataagtcgt gtcttaccgg gttggactca 9240
   agacgatagt taccggataa ggcgcagcgg tcgggctgaa cggggggttc gtgcacacag 9300
   cccagcttgg agcgaacgac ctacaccgaa ctgagatacc tacagcgtga gctatgagaa 9360
   agcgccacgc ttcccgaagg gagaaaggcg gacaggtatc cggtaagcgg cagggtcgga 9420
   acaggagagc gcacgaggga gcttccaggg ggaaacgcct ggtatcttta tagtcctgtc 9480
   gggtttcgcc acctctgact tgagcgtcga tttttgtgat gctcgtcagg ggggcggagc 9540
   ctatggaaaa acgccagcaa cgcggccttt ttacggttcc tggccttttg ctggccttga 9600
   agctgtccct gatggtcgtc atctacctgc ctggacagca tggcctgcaa cgcgggcatc 9660
   ccgatgccgc cggaagcgag aagaatcata atggggaagg ccatccagcc tcgcgtcgcg 9720
   aacgccagca agacgtagcc cagcgcgtcg gccccgagat gcgccgcgtg cggctgctgg 9780
   agatggcgga cgcgatggat atgttctgcc aagggttggt ttgcgcattc acagttctcc 9840
   gcaagaattg attggctcca attcttggag tggtgaatcc gttagcgagg tgccgccctg 9900
   cttcatcccc gtggcccgtt gctcgcgttt gctggcggtg tccccggaag aaatatattt 9960
   gcatgtcttt agttctatga tgacacaaac cccgcccagc gtcttgtcat tggcgaattc 10020
   gaacacgcag atgcagtcgg ggcggcgcgg tccgaggtcc acttcgcata ttaaggtgac 10080
   gcgtgtggcc tcgaacaccg agcgaccctg cagcgacccg cttaacagcg tcaacagcgt 10140
   gccgcagatc ccgggggcca atgagatatg aaaaagcctg aactcaccgc gacgtctgtc 10200
   gagaagtttc tgatcgaaaa gttcgacagc gtctccgacc tgatgcagct ctcggagggc 10260
   gaagaatctc gtgctttcag cttcgatgta ggagggcgtg gatatgtcct gcgggtaaat 10320
   agctgcgccg atggtttcta caaagatcgt tatgtttatc ggcactttgc atcggccgcg 10380
   ctcccgattc cggaagtgct tgacattggg gaattcagcg agagcctgac ctattgcatc 10440
   tcccgccgtg cacagggtgt cacgttgcaa gacctgcctg aaaccgaact gcccgctgtt 10500
   ctgcagccgg tcgcggaggc catggatgcg atcgctgcgg ccgatcttag ccagacgagc 10560
   gggttcggcc cattcggacc gcaaggaatc ggtcaataca ctacatggcg tgatttcata 10620
   tgcgcgattg ctgatcccca tgtgtatcac tggcaaactg tgatggacga caccgtcagt 10680
   gcgtccgtcg cgcaggctct cgatgagctg atgctttggg ccgaggactg ccccgaagtc 10740
   cggcacctcg tgcacgcgga tttcggctcc aacaatgtcc tgacggacaa tggccgcata 10800
   acagcggtca ttgactggag cgaggcgatg ttcggggatt cccaatacga ggtcgccaac 10860
   atcttcttct ggaggccgtg gttggcttgt atggagcagc agacgcgcta cttcgagcgg 10920
   aggcatccgg agcttgcagg atcgccgcgg ctccgggcgt atatgctccg cattggtctt 10980
   gaccaactct atcagagctt ggttgacggc aatttcgatg atgcagcttg ggcgcagggt 11040
   cgatgcgacg caatcgtccg atccggagcc gggactgtcg ggcgtacaca aatcgcccgc 11100
   agaagcgcgg ccgtctggac cgatggctgt gtagaagtac tcgccgatag tggaaaccga 11160
   cgccccagca ctcgtccgga tcgggagatg ggggaggcta actgaaacac ggaaggagac 11220
   aataccggaa ggaacccgcg ctatgacggc aataaaaaga cagaataaaa cgcacgggtg 11280
   ttgggtcgtt tgttcataaa cgcggggttc ggtcccaggg ctggcactct gtcgataccc 11340
   caccgagacc ccattggggc caatacgccc gcgtttcttc cttttcccca ccccaccccc 11400
   caagttcggg tgaaggccca gggctcgcag ccaacgtcgg ggcggcaggc cctgccatag 11460
   ccactggccc cgtgggttag ggacggggtc ccccatgggg aatggtttat ggttcgtggg 11520
   ggttattatt ttgggcgttg cgtggggtca ggtccacgac tggactgagc agacagaccc 11580
   atggtttttg gatggcctgg gcatggaccg catgtactgg cgcgacacga acaccgggcg 11640
   tctgtggctg ccaaacaccc ccgaccccca aaaaccaccg cgcggatttc tggcgtgcca 11700
   agctagtcga ccaattctca tgtttgacag cttatcatcg cagatccggg caacgttgtt 11760
   gccattgctg caggcgcaga actggtaggt atggaagatc tatacattga atcaatattg 11820
   gcaattagcc atattagtca ttggttatat agcataaatc aatattggct attggccatt 11880
   gcatacgttg tatctatatc ataatatgta catttatatt ggctcatgtc caatatgacc 11940
   gccat                                                             11945
<210> 260
   <211> 747
   <212> DNA
   <213> Artificial Sequence
<220>
   <223> In vitro synthesized DNA encoding a melanocortin receptor binding alpha-MSH mimetibody without secretory signal (p4372).
<400> 260
   ggcggatgcg agcacttccg ctggtgcaag ccggtgggag gcggatccgg taccttagtc 60
   accaactcca ccggtccccc atgcccacca tgcccggcgc ctgaggccgc cgggggacca 120
   tcagtcttcc tgttcccccc aaaacccaag gacactctca tgatctcccg gacccctgag 180
   gtcacgtgcg tggtggtgga cgtgagccag gaagaccccg aggtccagtt caactggtac 240
   gtggatggcg tggaggtgca taatgccaag acaaagccgc gggaggagca gttcaacagc 300
   acgtaccgtg tggtcagcgt cctcaccgtc ctgcaccagg actggctgaa cggcaaggag 360
   tacaagtgca aggtctccaa caaaggcctc ccgtcctcca tcgagaaaac catctccaaa 420
   gccaaagggc agcctcgaga gccacaggtg tacaccctgc ccccatccca ggaggagatg 480
   accaagaacc aggtcagcct gacctgcctg gtcaaaggct tctaccccag cgacatcgcc 540
   gtggagtggg agagcaatgg gcagccggag aacaactaca agaccacgcc tcccgtgctg 600
   gactccgacg gctccttctt cctctacagc aggctaaccg tggacaagag caggtggcag 660
   gaggggaatg tcttctcatg ctccgtgatg catgaggctc tgcacaacca ctacacacag 720
   aaaagcttgt ccctgtctct gggtaaa                                     747
<210> 261
   <211> 249
   <212> PRT
   <213> Artificial Sequence
<220>
   <223> In vitro synthesized DNA encoding a melanocortin receptor binding alpha-MSH mimetibody without secretory signal (p4372).
<400> 261
   Gly Gly Cys Glu His Phe Arg Trp Cys Lys Pro Val Gly Gly Gly Ser
    1               5                  10                  15
   Gly Thr Leu Val Thr Asn Ser Thr Gly Pro Pro Cys Pro Pro Cys Pro
               20                  25                  30
   Ala Pro Glu Ala Ala Gly Gly Pro Ser Val Phe Leu Phe Pro Pro Lys
           35                  40                  45
   Pro Lys Asp Thr Leu Met Ile Ser Arg Thr Pro Glu Val Thr Cys Val
       50                  55                  60
   Val Val Asp Val Ser Gln Glu Asp Pro Glu Val Gln Phe Asn Trp Tyr
   65                  70                  75                  80
   Val Asp Gly Val Glu Val His Asn Ala Lys Thr Lys Pro Arg Glu Glu
                   85                  90                  95
   Gln Phe Asn Ser Thr Tyr Arg Val Val Ser Val Leu Thr Val Leu His
               100                 105                 110
   Gln Asp Trp Leu Asn Gly Lys Glu Tyr Lys Cys Lys Val Ser Asn Lys
           115                 120                 125
   Gly Leu Pro Ser Ser Ile Glu Lys Thr Ile Ser Lys Ala Lys Gly Gln
       130                 135                 140
   Pro Arg Glu Pro Gln Val Tyr Thr Leu Pro Pro Ser Gln Glu Glu Met
   145                 150                 155                 160
   Thr Lys Asn Gln Val Ser Leu Thr Cys Leu Val Lys Gly Phe Tyr Pro
                   165                 170                 175
   Ser Asp Ile Ala Val Glu Trp Glu Ser Asn Gly Gln Pro Glu Asn Asn
               180                 185                 190
   Tyr Lys Thr Thr Pro Pro Val Leu Asp Ser Asp Gly Ser Phe Phe Leu
           195                 200                 205
   Tyr Ser Arg Leu Thr Val Asp Lys Ser Arg Trp Gln Glu Gly Asn Val
       210                 215                 220
   Phe Ser Cys Ser Val Met His Glu Ala Leu His Asn His Tyr Thr Gln
   225                 230                 235                 240
   Lys Ser Leu Ser Leu Ser Leu Gly Lys
                   245
<210> 262
   <211> 804
   <212> DNA
   <213> Artificial Sequence
<220>
   <223> In vitro synthesized DNA encoding a melanocortin receptor binding alpha-MSH mimetibody with secretory signal (p4372).
<400> 262
   atggcttggg tgtggacctt gctattcctg atggcggccg cccaaagtat acaggccggc 60
   ggatgcgagc acttccgctg gtgcaagccg gtgggaggcg gatccggtac cttagtcacc 120
   aactccaccg gtcccccatg cccaccatgc ccggcgcctg aggccgccgg gggaccatca 180
   gtcttcctgt tccccccaaa acccaaggac actctcatga tctcccggac ccctgaggtc 240
   acgtgcgtgg tggtggacgt gagccaggaa gaccccgagg tccagttcaa ctggtacgtg 300
   gatggcgtgg aggtgcataa tgccaagaca aagccgcggg aggagcagtt caacagcacg 360
   taccgtgtgg tcagcgtcct caccgtcctg caccaggact ggctgaacgg caaggagtac 420
   aagtgcaagg tctccaacaa aggcctcccg tcctccatcg agaaaaccat ctccaaagcc 480
   aaagggcagc ctcgagagcc acaggtgtac accctgcccc catcccagga ggagatgacc 540
   aagaaccagg tcagcctgac ctgcctggtc aaaggcttct accccagcga catcgccgtg 600
   gagtgggaga gcaatgggca gccggagaac aactacaaga ccacgcctcc cgtgctggac 660
   tccgacggct ccttcttcct ctacagcagg ctaaccgtgg acaagagcag gtggcaggag 720
   gggaatgtct tctcatgctc cgtgatgcat gaggctctgc acaaccacta cacacagaaa 780
   agcttgtccc tgtctctggg taaa                                        804
<210> 263
   <211> 268
   <212> PRT
   <213> Artificial Sequence
<220>
   <223> Melanocortin receptor binding alpha-MSH mimetibody with secretory signal encoded by an in vitro synthesized DNA (p4372).
<400> 263
   Met Ala Trp Val Trp Thr Leu Leu Phe Leu Met Ala Ala Ala Gln Ser
    1               5                  10                  15
   Ile Gln Ala Gly Gly Cys Glu His Phe Arg Trp Cys Lys Pro Val Gly
               20                  25                  30
   Gly Gly Ser Gly Thr Leu Val Thr Asn Ser Thr Gly Pro Pro Cys Pro
           35                  40                  45
   Pro Cys Pro Ala Pro Glu Ala Ala Gly Gly Pro Ser Val Phe Leu Phe
       50                  55                  60
   Pro Pro Lys Pro Lys Asp Thr Leu Met Ile Ser Arg Thr Pro Glu Val
   65                  70                  75                  80
   Thr Cys Val Val Val Asp Val Ser Gln Glu Asp Pro Glu Val Gln Phe
                   85                  90                  95
   Asn Trp Tyr Val Asp Gly Val Glu Val His Asn Ala Lys Thr Lys Pro
               100                 105                 110
   Arg Glu Glu Gln Phe Asn Ser Thr Tyr Arg Val Val Ser Val Leu Thr
           115                 120                 125
   Val Leu His Gln Asp Trp Leu Asn Gly Lys Glu Tyr Lys Cys Lys Val
       130                 135                 140
   Ser Asn Lys Gly Leu Pro Ser Ser Ile Glu Lys Thr Ile Ser Lys Ala
   145                 150                 155                 160
   Lys Gly Gln Pro Arg Glu Pro Gln Val Tyr Thr Leu Pro Pro Ser Gln
                   165                 170                 175
   Glu Glu Met Thr Lys Asn Gln Val Ser Leu Thr Cys Leu Val Lys Gly
               180                 185                 190
   Phe Tyr Pro Ser Asp Ile Ala Val Glu Trp Glu Ser Asn Gly Gln Pro
           195                 200                 205
   Glu Asn Asn Tyr Lys Thr Thr Pro Pro Val Leu Asp Ser Asp Gly Ser
       210                 215                 220
   Phe Phe Leu Tyr Ser Arg Leu Thr Val Asp Lys Ser Arg Trp Gln Glu
   225                 230                 235                 240
   Gly Asn Val Phe Ser Cys Ser Val Met His Glu Ala Leu His Asn His
                   245                 250                 255
   Tyr Thr Gln Lys Ser Leu Ser Leu Ser Leu Gly Lys
               260                 265
<210> 264
   <211> 11939
   <212> DNA
   <213> Artificial Sequence
<220>
   <223> In vitro synthesized DNA with expression vector functions that encodes an alpha-MSH mimetibody (p4372).
<400> 264
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggccggc ggatgcgagc acttccgctg 1680
   gtgcaagccg gtgggaggcg gatccggtac cttagtcacc aactccaccg gtcccccatg 1740
   cccaccatgc ccggcgcctg aggccgccgg gggaccatca gtcttcctgt tccccccaaa 1800
   acccaaggac actctcatga tctcccggac ccctgaggtc acgtgcgtgg tggtggacgt 1860
   gagccaggaa gaccccgagg tccagttcaa ctggtacgtg gatggcgtgg aggtgcataa 1920
   tgccaagaca aagccgcggg aggagcagtt caacagcacg taccgtgtgg tcagcgtcct 1980
   caccgtcctg caccaggact ggctgaacgg caaggagtac aagtgcaagg tctccaacaa 2040
   aggcctcccg tcctccatcg agaaaaccat ctccaaagcc aaagggcagc ctcgagagcc 2100
   acaggtgtac accctgcccc catcccagga ggagatgacc aagaaccagg tcagcctgac 2160
   ctgcctggtc aaaggcttct accccagcga catcgccgtg gagtgggaga gcaatgggca 2220
   gccggagaac aactacaaga ccacgcctcc cgtgctggac tccgacggct ccttcttcct 2280
   ctacagcagg ctaaccgtgg acaagagcag gtggcaggag gggaatgtct tctcatgctc 2340
   cgtgatgcat gaggctctgc acaaccacta cacacagaaa agcttgtccc tgtctctggg 2400
   taaatgaggg cccgtttaaa cagatccaga catgataaga tacattgatg agtttggaca 2460
   aaccacaact agaatgcagt gaaaaaaatg ctttatttgt gaaatttgtg atgctattgc 2520
   tttatttgta accattataa gctgcaataa acaagttaac aacaacaatt gcattcattt 2580
   tatgtttcag gttcaggggg aggtgtggga ggttttttaa agcaagtaaa acctctacaa 2640
   atgtggtatg gctgattatg atccggctgc ctcgcgcgtt tcggtgatga cggtgaaaac 2700
   ctctgacaca tgcagctccc ggagacggtc acagcttgtc tgtaagcgga tgccgggagc 2760
   agacaagccc gtcaggcgtc agcgggtgtt ggcgggtgtc ggggcgcagc catgaggtcg 2820
   actctagagg atcgatgccc cgccccggac gaactaaacc tgactacgac atctctgccc 2880
   cttcttcgcg gggcagtgca tgtaatccct tcagttggtt ggtacaactt gccaactggg 2940
   ccctgttcca catgtgacac ggggggggac caaacacaaa ggggttctct gactgtagtt 3000
   gacatcctta taaatggatg tgcacatttg ccaacactga gtggctttca tcctggagca 3060
   gactttgcag tctgtggact gcaacacaac attgccttta tgtgtaactc ttggctgaag 3120
   ctcttacacc aatgctgggg gacatgtacc tcccaggggc ccaggaagac tacgggaggc 3180
   tacaccaacg tcaatcagag gggcctgtgt agctaccgat aagcggaccc tcaagagggc 3240
   attagcaata gtgtttataa ggcccccttg ttaaccctaa acgggtagca tatgcttccc 3300
   gggtagtagt atatactatc cagactaacc ctaattcaat agcatatgtt acccaacggg 3360
   aagcatatgc tatcgaatta gggttagtaa aagggtccta aggaacagcg atatctccca 3420
   ccccatgagc tgtcacggtt ttatttacat ggggtcagga ttccacgagg gtagtgaacc 3480
   attttagtca caagggcagt ggctgaagat caaggagcgg gcagtgaact ctcctgaatc 3540
   ttcgcctgct tcttcattct ccttcgttta gctaatagaa taactgctga gttgtgaaca 3600
   gtaaggtgta tgtgaggtgc tcgaaaacaa ggtttcaggt gacgccccca gaataaaatt 3660
   tggacggggg gttcagtggt ggcattgtgc tatgacacca atataaccct cacaaacccc 3720
   ttgggcaata aatactagtg taggaatgaa acattctgaa tatctttaac aatagaaatc 3780
   catggggtgg ggacaagccg taaagactgg atgtccatct cacacgaatt tatggctatg 3840
   ggcaacacat aatcctagtg caatatgata ctggggttat taagatgtgt cccaggcagg 3900
   gaccaagaca ggtgaaccat gttgttacac tctatttgta acaaggggaa agagagtgga 3960
   cgccgacagc agcggactcc actggttgtc tctaacaccc ccgaaaatta aacggggctc 4020
   cacgccaatg gggcccataa acaaagacaa gtggccactc ttttttttga aattgtggag 4080
   tgggggcacg cgtcagcccc cacacgccgc cctgcggttt tggactgtaa aataagggtg 4140
   taataacttg gctgattgta accccgctaa ccactgcggt caaaccactt gcccacaaaa 4200
   ccactaatgg caccccgggg aatacctgca taagtaggtg ggcgggccaa gataggggcg 4260
   cgattgctgc gatctggagg acaaattaca cacacttgcg cctgagcgcc aagcacaggg 4320
   ttgttggtcc tcatattcac gaggtcgctg agagcacggt gggctaatgt tgccatgggt 4380
   agcatatact acccaaatat ctggatagca tatgctatcc taatctatat ctgggtagca 4440
   taggctatcc taatctatat ctgggtagca tatgctatcc taatctatat ctgggtagta 4500
   tatgctatcc taatttatat ctgggtagca taggctatcc taatctatat ctgggtagca 4560
   tatgctatcc taatctatat ctgggtagta tatgctatcc taatctgtat ccgggtagca 4620
   tatgctatcc taatagagat tagggtagta tatgctatcc taatttatat ctgggtagca 4680
   tatactaccc aaatatctgg atagcatatg ctatcctaat ctatatctgg gtagcatatg 4740
   ctatcctaat ctatatctgg gtagcatagg ctatcctaat ctatatctgg gtagcatatg 4800
   ctatcctaat ctatatctgg gtagtatatg ctatcctaat ttatatctgg gtagcatagg 4860
   ctatcctaat ctatatctgg gtagcatatg ctatcctaat ctatatctgg gtagtatatg 4920
   ctatcctaat ctgtatccgg gtagcatatg ctatcctcat gcatatacag tcagcatatg 4980
   atacccagta gtagagtggg agtgctatcc tttgcatatg ccgccacctc ccaagggggc 5040
   gtgaattttc gctgcttgtc cttttcctgc tggttgctcc cattcttagg tgaatttaag 5100
   gaggccaggc taaagccgtc gcatgtctga ttgctcacca ggtaaatgtc gctaatgttt 5160
   tccaacgcga gaaggtgttg agcgcggagc tgagtgacgt gacaacatgg gtatgcccaa 5220
   ttgccccatg ttgggaggac gaaaatggtg acaagacaga tggccagaaa tacaccaaca 5280
   gcacgcatga tgtctactgg ggatttattc tttagtgcgg gggaatacac ggcttttaat 5340
   acgattgagg gcgtctccta acaagttaca tcactcctgc ccttcctcac cctcatctcc 5400
   atcacctcct tcatctccgt catctccgtc atcaccctcc gcggcagccc cttccaccat 5460
   aggtggaaac cagggaggca aatctactcc atcgtcaaag ctgcacacag tcaccctgat 5520
   attgcaggta ggagcgggct ttgtcataac aaggtcctta atcgcatcct tcaaaacctc 5580
   agcaaatata tgagtttgta aaaagaccat gaaataacag acaatggact cccttagcgg 5640
   gccaggttgt gggccgggtc caggggccat tccaaagggg agacgactca atggtgtaag 5700
   acgacattgt ggaatagcaa gggcagttcc tcgccttagg ttgtaaaggg aggtcttact 5760
   acctccatat acgaacacac cggcgaccca agttccttcg tcggtagtcc tttctacgtg 5820
   actcctagcc aggagagctc ttaaaccttc tgcaatgttc tcaaatttcg ggttggaacc 5880
   tccttgacca cgatgctttt ccaaaccacc ctcctttttt gcgccctgcc tccatcaccc 5940
   tgaccccggg gtccagtgct tgggccttct cctgggtcat ctgcggggcc ctgctctatc 6000
   gctcccgggg gcacgtcagg ctcaccatct gggccacctt cttggtggta ttcaaaataa 6060
   tcggcttccc ctacagggtg gaaaaatggc cttctacctg gagggggcct gcgcggtgga 6120
   gacccggatg atgatgactg actactggga ctcctgggcc tcttttctcc acgtccacga 6180
   cctctccccc tggctctttc acgacttccc cccctggctc tttcacgtcc tctaccccgg 6240
   cggcctccac tacctcctcg accccggcct ccactacctc ctcgaccccg gcctccactg 6300
   cctcctcgac cccggcctcc acctcctgct cctgcccctc ctgctcctgc ccctcctcct 6360
   gctcctgccc ctcctgcccc tcctgctcct gcccctcctg cccctcctgc tcctgcccct 6420
   cctgcccctc ctgctcctgc ccctcctgcc cctcctcctg ctcctgcccc tcctgcccct 6480
   cctcctgctc ctgcccctcc tgcccctcct gctcctgccc ctcctgcccc tcctgctcct 6540
   gcccctcctg cccctcctgc tcctgcccct cctgctcctg cccctcctgc tcctgcccct 6600
   cctgctcctg cccctcctgc ccctcctgcc cctcctcctg ctcctgcccc tcctgctcct 6660
   gcccctcctg cccctcctgc ccctcctgct cctgcccctc ctcctgctcc tgcccctcct 6720
   gcccctcctg cccctcctcc tgctcctgcc cctcctgccc ctcctcctgc tcctgcccct 6780
   cctcctgctc ctgcccctcc tgcccctcct gcccctcctc ctgctcctgc ccctcctgcc 6840
   cctcctcctg ctcctgcccc tcctcctgct cctgcccctc ctgcccctcc tgcccctcct 6900
   cctgctcctg cccctcctcc tgctcctgcc cctcctgccc ctcctgcccc tcctgcccct 6960
   cctcctgctc ctgcccctcc tcctgctcct gcccctcctg ctcctgcccc tcccgctcct 7020
   gctcctgctc ctgttccacc gtgggtccct ttgcagccaa tgcaacttgg acgtttttgg 7080
   ggtctccgga caccatctct atgtcttggc cctgatcctg agccgcccgg ggctcctggt 7140
   cttccgcctc ctcgtcctcg tcctcttccc cgtcctcgtc catggttatc accccctctt 7200
   ctttgaggtc cactgccgcc ggagccttct ggtccagatg tgtctccctt ctctcctagg 7260
   ccatttccag gtcctgtacc tggcccctcg tcagacatga ttcacactaa aagagatcaa 7320
   tagacatctt tattagacga cgctcagtga atacagggag tgcagactcc tgccccctcc 7380
   aacagccccc ccaccctcat ccccttcatg gtcgctgtca gacagatcca ggtctgaaaa 7440
   ttccccatcc tccgaaccat cctcgtcctc atcaccaatt actcgcagcc cggaaaactc 7500
   ccgctgaaca tcctcaagat ttgcgtcctg agcctcaagc caggcctcaa attcctcgtc 7560
   cccctttttg ctggacggta gggatgggga ttctcgggac ccctcctctt cctcttcaag 7620
   gtcaccagac agagatgcta ctggggcaac ggaagaaaag ctgggtgcgg cctgtgagga 7680
   tcagcttatc gatgataagc tgtcaaacat gagaattctt gaagacgaaa gggcctcgtg 7740
   atacgcctat ttttataggt taatgtcatg ataataatgg tttcttagac gtcaggtggc 7800
   acttttcggg gaaatgtgcg cggaacccct atttgtttat ttttctaaat acattcaaat 7860
   atgtatccgc tcatgagaca ataaccctga taaatgcttc aataatattg aaaaaggaag 7920
   agtatgagta ttcaacattt ccgtgtcgcc cttattccct tttttgcggc attttgcctt 7980
   cctgtttttg ctcacccaga aacgctggtg aaagtaaaag atgctgaaga tcagttgggt 8040
   gcacgagtgg gttacatcga actggatctc aacagcggta agatccttga gagttttcgc 8100
   cccgaagaac gttttccaat gatgagcact tttaaagttc tgctatgtgg cgcggtatta 8160
   tcccgtgttg acgccgggca agagcaactc ggtcgccgca tacactattc tcagaatgac 8220
   ttggttgagt actcaccagt cacagaaaag catcttacgg atggcatgac agtaagagaa 8280
   ttatgcagtg ctgccataac catgagtgat aacactgcgg ccaacttact tctgacaacg 8340
   atcggaggac cgaaggagct aaccgctttt ttgcacaaca tgggggatca tgtaactcgc 8400
   cttgatcgtt gggaaccgga gctgaatgaa gccataccaa acgacgagcg tgacaccacg 8460
   atgcctgcag caatggcaac aacgttgcgc aaactattaa ctggcgaact acttactcta 8520
   gcttcccggc aacaattaat agactggatg gaggcggata aagttgcagg accacttctg 8580
   cgctcggccc ttccggctgg ctggtttatt gctgataaat ctggagccgg tgagcgtggg 8640
   tctcgcggta tcattgcagc actggggcca gatggtaagc cctcccgtat cgtagttatc 8700
   tacacgacgg ggagtcaggc aactatggat gaacgaaata gacagatcgc tgagataggt 8760
   gcctcactga ttaagcattg gtaactgtca gaccaagttt actcatatat actttagatt 8820
   gatttaaaac ttcattttta atttaaaagg atctaggtga agatcctttt tgataatctc 8880
   atgaccaaaa tcccttaacg tgagttttcg ttccactgag cgtcagaccc cgtagaaaag 8940
   atcaaaggat cttcttgaga tccttttttt ctgcgcgtaa tctgctgctt gcaaacaaaa 9000
   aaaccaccgc taccagcggt ggtttgtttg ccggatcaag agctaccaac tctttttccg 9060
   aaggtaactg gcttcagcag agcgcagata ccaaatactg tccttctagt gtagccgtag 9120
   ttaggccacc acttcaagaa ctctgtagca ccgcctacat acctcgctct gctaatcctg 9180
   ttaccagtgg ctgctgccag tggcgataag tcgtgtctta ccgggttgga ctcaagacga 9240
   tagttaccgg ataaggcgca gcggtcgggc tgaacggggg gttcgtgcac acagcccagc 9300
   ttggagcgaa cgacctacac cgaactgaga tacctacagc gtgagctatg agaaagcgcc 9360
   acgcttcccg aagggagaaa ggcggacagg tatccggtaa gcggcagggt cggaacagga 9420
   gagcgcacga gggagcttcc agggggaaac gcctggtatc tttatagtcc tgtcgggttt 9480
   cgccacctct gacttgagcg tcgatttttg tgatgctcgt caggggggcg gagcctatgg 9540
   aaaaacgcca gcaacgcggc ctttttacgg ttcctggcct tttgctggcc ttgaagctgt 9600
   ccctgatggt cgtcatctac ctgcctggac agcatggcct gcaacgcggg catcccgatg 9660
   ccgccggaag cgagaagaat cataatgggg aaggccatcc agcctcgcgt cgcgaacgcc 9720
   agcaagacgt agcccagcgc gtcggccccg agatgcgccg cgtgcggctg ctggagatgg 9780
   cggacgcgat ggatatgttc tgccaagggt tggtttgcgc attcacagtt ctccgcaaga 9840
   attgattggc tccaattctt ggagtggtga atccgttagc gaggtgccgc cctgcttcat 9900
   ccccgtggcc cgttgctcgc gtttgctggc ggtgtccccg gaagaaatat atttgcatgt 9960
   ctttagttct atgatgacac aaaccccgcc cagcgtcttg tcattggcga attcgaacac 10020
   gcagatgcag tcggggcggc gcggtccgag gtccacttcg catattaagg tgacgcgtgt 10080
   ggcctcgaac accgagcgac cctgcagcga cccgcttaac agcgtcaaca gcgtgccgca 10140
   gatcccgggg ggcaatgaga tatgaaaaag cctgaactca ccgcgacgtc tgtcgagaag 10200
   tttctgatcg aaaagttcga cagcgtctcc gacctgatgc agctctcgga gggcgaagaa 10260
   tctcgtgctt tcagcttcga tgtaggaggg cgtggatatg tcctgcgggt aaatagctgc 10320
   gccgatggtt tctacaaaga tcgttatgtt tatcggcact ttgcatcggc cgcgctcccg 10380
   attccggaag tgcttgacat tggggaattc agcgagagcc tgacctattg catctcccgc 10440
   cgtgcacagg gtgtcacgtt gcaagacctg cctgaaaccg aactgcccgc tgttctgcag 10500
   ccggtcgcgg aggccatgga tgcgatcgct gcggccgatc ttagccagac gagcgggttc 10560
   ggcccattcg gaccgcaagg aatcggtcaa tacactacat ggcgtgattt catatgcgcg 10620
   attgctgatc cccatgtgta tcactggcaa actgtgatgg acgacaccgt cagtgcgtcc 10680
   gtcgcgcagg ctctcgatga gctgatgctt tgggccgagg actgccccga agtccggcac 10740
   ctcgtgcacg cggatttcgg ctccaacaat gtcctgacgg acaatggccg cataacagcg 10800
   gtcattgact ggagcgaggc gatgttcggg gattcccaat acgaggtcgc caacatcttc 10860
   ttctggaggc cgtggttggc ttgtatggag cagcagacgc gctacttcga gcggaggcat 10920
   ccggagcttg caggatcgcc gcggctccgg gcgtatatgc tccgcattgg tcttgaccaa 10980
   ctctatcaga gcttggttga cggcaatttc gatgatgcag cttgggcgca gggtcgatgc 11040
   gacgcaatcg tccgatccgg agccgggact gtcgggcgta cacaaatcgc ccgcagaagc 11100
   gcggccgtct ggaccgatgg ctgtgtagaa gtactcgccg atagtggaaa ccgacgcccc 11160
   agcactcgtc cggatcggga gatgggggag gctaactgaa acacggaagg agacaatacc 11220
   ggaaggaacc cgcgctatga cggcaataaa aagacagaat aaaacgcacg ggtgttgggt 11280
   cgtttgttca taaacgcggg gttcggtccc agggctggca ctctgtcgat accccaccga 11340
   gaccccattg gggccaatac gcccgcgttt cttccttttc cccaccccac cccccaagtt 11400
   cgggtgaagg cccagggctc gcagccaacg tcggggcggc aggccctgcc atagccactg 11460
   gccccgtggg ttagggacgg ggtcccccat ggggaatggt ttatggttcg tgggggttat 11520
   tattttgggc gttgcgtggg gtcaggtcca cgactggact gagcagacag acccatggtt 11580
   tttggatggc ctgggcatgg accgcatgta ctggcgcgac acgaacaccg ggcgtctgtg 11640
   gctgccaaac acccccgacc cccaaaaacc accgcgcgga tttctggcgt gccaagctag 11700
   tcgaccaatt ctcatgtttg acagcttatc atcgcagatc cgggcaacgt tgttgccatt 11760
   gctgcaggcg cagaactggt aggtatggaa gatctataca ttgaatcaat attggcaatt 11820
   agccatatta gtcattggtt atatagcata aatcaatatt ggctattggc cattgcatac 11880
   gttgtatcta tatcataata tgtacattta tattggctca tgtccaatat gaccgccat  11939
<210> 265
   <211> 747
   <212> DNA
   <213> Artificial Sequence
<220>
   <223> In vitro synthesized DNA encoding a melanocortin receptor binding alpha-MSH mimetibody without secretory signal (p4383).
<400> 265
   ggcggatgcg agcacttccg ctggtgcaag ccggtgggtg gaggctccgg taccaacgtc 60
   accgtctccg caggtccccc atgcccacca tgcccggcgc ctgaggccgc cgggggacca 120
   tcagtcttcc tgttcccccc aaaacccaag gacactctca tgatctcccg gacccctgag 180
   gtcacgtgcg tggtggtgga cgtgagccag gaagaccccg aggtccagtt caactggtac 240
   gtggatggcg tggaggtgca taatgccaag acaaagccgc gggaggagca gttcaacagc 300
   acgtaccgtg tggtcagcgt cctcaccgtc ctgcaccagg actggctgaa cggcaaggag 360
   tacaagtgca aggtctccaa caaaggcctc ccgtcctcca tcgagaaaac catctccaaa 420
   gccaaagggc agcctcgaga gccacaggtg tacaccctgc ccccatccca ggaggagatg 480
   accaagaacc aggtcagcct gacctgcctg gtcaaaggct tctaccccag cgacatcgcc 540
   gtggagtggg agagcaatgg gcagccggag aacaactaca agaccacgcc tcccgtgctg 600
   gactccgacg gctccttctt cctctacagc aggctaaccg tggacaagag caggtggcag 660
   gaggggaatg tcttctcatg ctccgtgatg catgaggctc tgcacaacca ctacacacag 720
   aaaagcttgt ccctgtctct gggtaaa                                     747
<210> 266
   <211> 249
   <212> PRT
   <213> Artificial Sequence
<220>
   <223> Melanocortin receptor binding alpha-MSH mimetibody without secretory signal encoded by an in vitro synthesized DNA (p4383).
<400> 266
   Gly Gly Cys Glu His Phe Arg Trp Cys Lys Pro Val Gly Gly Gly Ser
    1               5                   10                  15
   Gly Thr Asn Val Thr Val Ser Ala Gly Pro Pro Cys Pro Pro Cys Pro
               20                  25                  30
   Ala Pro Glu Ala Ala Gly Gly Pro Ser Val Phe Leu Phe Pro Pro Lys
           35                  40                  45
   Pro Lys Asp Thr Leu Met Ile Ser Arg Thr Pro Glu Val Thr Cys Val
       50                  55                  60
   Val Val Asp Val Ser Gln Glu Asp Pro Glu Val Gln Phe Asn Trp Tyr
   65                  70                  75                  80
   Val Asp Gly Val Glu Val His Asn Ala Lys Thr Lys Pro Arg Glu Glu
                   85                  90                  95
   Gln Phe Asn Ser Thr Tyr Arg Val Val Ser Val Leu Thr Val Leu His
               100                 105                 110
   Gln Asp Trp Leu Asn Gly Lys Glu Tyr Lys Cys Lys Val Ser Asn Lys
           115                 120                 125
   Gly Leu Pro Ser Ser Ile Glu Lys Thr Ile Ser Lys Ala Lys Gly Gln
       130                 135                 140
   Pro Arg Glu Pro Gln Val Tyr Thr Leu Pro Pro Ser Gln Glu Glu Met
   145                 150                 155                 160
   Thr Lys Asn Gln Val Ser Leu Thr Cys Leu Val Lys Gly Phe Tyr Pro
                   165                 170                 175
   Ser Asp Ile Ala Val Glu Trp Glu Ser Asn Gly Gln Pro Glu Asn Asn
               180                 185                 190
   Tyr Lys Thr Thr Pro Pro Val Leu Asp Ser Asp Gly Ser Phe Phe Leu
           195                 200                 205
   Tyr Ser Arg Leu Thr Val Asp Lys Ser Arg Trp Gln Glu Gly Asn Val
       210                 215                 220
   Phe Ser Cys Ser Val Met His Glu Ala Leu His Asn His Tyr Thr Gln
   225                 230                 235                 240
   Lys Ser Leu Ser Leu Ser Leu Gly Lys
                   245
<210> 267
   <211> 804
   <212> DNA
   <213> Artificial Sequence
<220>
   <223> In vitro synthesized DNA encoding a melanocortin receptor binding alpha-MSH mimetibody with secretory signal (p4383).
<400> 267
   atggcttggg tgtggacctt gctattcctg atggcggccg cccaaagtat acaggccggc 60
   ggatgcgagc acttccgctg gtgcaagccg gtgggtggag gctccggtac caacgtcacc 120
   gtctccgcag gtcccccatg cccaccatgc ccggcgcctg aggccgccgg gggaccatca 180
   gtcttcctgt tccccccaaa acccaaggac actctcatga tctcccggac ccctgaggtc 240
   acgtgcgtgg tggtggacgt gagccaggaa gaccccgagg tccagttcaa ctggtacgtg 300
   gatggcgtgg aggtgcataa tgccaagaca aagccgcggg aggagcagtt caacagcacg 360
   taccgtgtgg tcagcgtcct caccgtcctg caccaggact ggctgaacgg caaggagtac 420
   aagtgcaagg tctccaacaa aggcctcccg tcctccatcg agaaaaccat ctccaaagcc 480
   aaagggcagc ctcgagagcc acaggtgtac accctgcccc catcccagga ggagatgacc 540
   aagaaccagg tcagcctgac ctgcctggtc aaaggcttct accccagcga catcgccgtg 600
   gagtgggaga gcaatgggca gccggagaac aactacaaga ccacgcctcc cgtgctggac 660
   tccgacggct ccttcttcct ctacagcagg ctaaccgtgg acaagagcag gtggcaggag 720
   gggaatgtct tctcatgctc cgtgatgcat gaggctctgc acaaccacta cacacagaaa 780
   agcttgtccc tgtctctggg taaa                                        804
<210> 268
   <211> 268
   <212> PRT
   <213> Artificial Sequence
<220>
   <223> Melanocortin receptor binding alpha-MSH mimetibody with secretory signal encoded by an in vitro synthesized DNA (p4383).
<400> 268
   Met Ala Trp Val Trp Thr Leu Leu Phe Leu Met Ala Ala Ala Gln Ser
    1               5                  10                  15
   Ile Gln Ala Gly Gly Cys Glu His Phe Arg Trp Cys Lys Pro Val Gly
               20                  25                  30
   Gly Gly Ser Gly Thr Asn Val Thr Val Ser Ala Gly Pro Pro Cys Pro
           35                  40                  45
   Pro Cys Pro Ala Pro Glu Ala Ala Gly Gly Pro Ser Val Phe Leu Phe
       50                  55                  60
   Pro Pro Lys Pro Lys Asp Thr Leu Met Ile Ser Arg Thr Pro Glu Val
   65                  70                  75                  80
   Thr Cys Val Val Val Asp Val Ser Gln Glu Asp Pro Glu Val Gln Phe
                   85                  90                  95
   Asn Trp Tyr Val Asp Gly Val Glu Val His Asn Ala Lys Thr Lys Pro
               100                 105                 110
   Arg Glu Glu Gln Phe Asn Ser Thr Tyr Arg Val Val Ser Val Leu Thr
           115                 120                 125
   Val Leu His Gln Asp Trp Leu Asn Gly Lys Glu Tyr Lys Cys Lys Val
       130                 135                 140
   Ser Asn Lys Gly Leu Pro Ser Ser Ile Glu Lys Thr Ile Ser Lys Ala
   145                 150                 155                 160
   Lys Gly Gln Pro Arg Glu Pro Gln Val Tyr Thr Leu Pro Pro Ser Gln
                   165                 170                 175
   Glu Glu Met Thr Lys Asn Gln Val Ser Leu Thr Cys Leu Val Lys Gly
               180                 185                 190
   Phe Tyr Pro Ser Asp Ile Ala Val Glu Trp Glu Ser Asn Gly Gln Pro
           195                 200                 205
   Glu Asn Asn Tyr Lys Thr Thr Pro Pro Val Leu Asp Ser Asp Gly Ser
       210                 215                 220
   Phe Phe Leu Tyr Ser Arg Leu Thr Val Asp Lys Ser Arg Trp Gln Glu
   225                 230                 235                 240
   Gly Asn Val Phe Ser Cys Ser Val Met His Glu Ala Leu His Asn His
                   245                 250                 255
   Tyr Thr Gln Lys Ser Leu Ser Leu Ser Leu Gly Lys
               260                 265
<210> 269
   <211> 11939
   <212> DNA
   <213> Artificial Sequence
<220>
   <223> In vitro synthesized DNA with expression vector functions that encodes an alpha-MSH mimetibody (p4383).
<400> 269
   gttgacattg attattgact agttattaat agtaatcaat tacggggtca ttagttcata 60
   gcccatatat ggagttccgc gttacataac ttacggtaaa tggcccgcct ggctgaccgc 120
   ccaacgaccc ccgcccattg acgtcaataa tgacgtatgt tcccatagta acgccaatag 180
   ggactttcca ttgacgtcaa tgggtggagt atttacggta aactgcccac ttggcagtac 240
   atcaagtgta tcatatgcca agtccgcccc ctattgacgt caatgacggt aaatggcccg 300
   cctggcatta tgcccagtac atgaccttac gggactttcc tacttggcag tacatctacg 360
   tattagtcat cgctattacc atggtgatgc ggttttggca gtacaccaat gggcgtggat 420
   agcggtttga ctcacgggga tttccaagtc tccaccccat tgacgtcaat gggagtttgt 480
   tttggcacca aaatcaacgg gactttccaa aatgtcgtaa taaccccgcc ccgttgacgc 540
   aaatgggcgg taggcgtgta cggtgggagg tctatataag cagagctcgt ttagtgaacc 600
   gtcagatcgc ctggagacgc catccacgct gttttgacct ccatagaaga caccgggacc 660
   gatccagcct ccgcggccgg gaacggtgca ttggaacgcg gattccccgt gccaagagtg 720
   acgtaagtac cgcctataga gtctataggc ccacctcctt ggcttcttat gcatgctata 780
   ctgtttttgg cttggggtct atacaccccc gcttcctcat gttataggtg atggtatagc 840
   ttagcctata ggtgtgggtt attgaccatt attgaccact cccctattgg tgacgatact 900
   ttccattact aatccataac atggctcttt gccacaactc tctttattgg ctatatgcca 960
   atacactgtc cttcagagac tgacacggac tctgtatttt tacaggatgg ggtctcattt 1020
   attatttaca aattcacata tacaacacca ccgtccccag tgcccgcagc ttttattaaa 1080
   cataacgtgg gatctccacg cgaatctcgg gtacgtgttc cggacatggg ctcttctccg 1140
   gtagcggcgg agcttctaca tccgagccct gctcccatgc ctccagcgac tcatggtcgc 1200
   tcggcagctc cttgctccta acagtggagg ccagacttag gcacagcacg atgcccacca 1260
   ccaccagtgt gccgcacaag gccgtggcgg tagggtatgt gtctgaaaat gagctcgggg 1320
   agcgggcttg caccgctgac gcatttggaa gacttaaggc agcggcagaa gaagatgcag 1380
   gcagctgagt tgttgtgttc tgataagagt cagaggtaac tcccgttgcg gtgctgttaa 1440
   cggtggaggg cagtgtagtc tgagcagtac tcgttgctgc cgcgcgcgcc accagacata 1500
   atagctgaca gactaacaga ctgttccttt ccatgggtct tttctgcagt caccgtcctt 1560
   agatctgtct agaagctggg taccagctgc tagcgccacc atggcttggg tgtggacctt 1620
   gctattcctg atggcggccg cccaaagtat acaggccggc ggatgcgagc acttccgctg 1680
   gtgcaagccg gtgggtggag gctccggtac caacgtcacc gtctccgcag gtcccccatg 1740
   cccaccatgc ccggcgcctg aggccgccgg gggaccatca gtcttcctgt tccccccaaa 1800
   acccaaggac actctcatga tctcccggac ccctgaggtc acgtgcgtgg tggtggacgt 1860
   gagccaggaa gaccccgagg tccagttcaa ctggtacgtg gatggcgtgg aggtgcataa 1920
   tgccaagaca aagccgcggg aggagcagtt caacagcacg taccgtgtgg tcagcgtcct 1980
   caccgtcctg caccaggact ggctgaacgg caaggagtac aagtgcaagg tctccaacaa 2040
   aggcctcccg tcctccatcg agaaaaccat ctccaaagcc aaagggcagc ctcgagagcc 2100
   acaggtgtac accctgcccc catcccagga ggagatgacc aagaaccagg tcagcctgac 2160
   ctgcctggtc aaaggcttct accccagcga catcgccgtg gagtgggaga gcaatgggca 2220
   gccggagaac aactacaaga ccacgcctcc cgtgctggac tccgacggct ccttcttcct 2280
   ctacagcagg ctaaccgtgg acaagagcag gtggcaggag gggaatgtct tctcatgctc 2340
   cgtgatgcat gaggctctgc acaaccacta cacacagaaa agcttgtccc tgtctctggg 2400
   taaatgaggg cccgtttaaa cagatccaga catgataaga tacattgatg agtttggaca 2460
   aaccacaact agaatgcagt gaaaaaaatg ctttatttgt gaaatttgtg atgctattgc 2520
   tttatttgta accattataa gctgcaataa acaagttaac aacaacaatt gcattcattt 2580
   tatgtttcag gttcaggggg aggtgtggga ggttttttaa agcaagtaaa acctctacaa 2640
   atgtggtatg gctgattatg atccggctgc ctcgcgcgtt tcggtgatga cggtgaaaac 2700
   ctctgacaca tgcagctccc ggagacggtc acagcttgtc tgtaagcgga tgccgggagc 2760
   agacaagccc gtcaggcgtc agcgggtgtt ggcgggtgtc ggggcgcagc catgaggtcg 2820
   actctagagg atcgatgccc cgccccggac gaactaaacc tgactacgac atctctgccc 2880
   cttcttcgcg gggcagtgca tgtaatccct tcagttggtt ggtacaactt gccaactggg 2940
   ccctgttcca catgtgacac ggggggggac caaacacaaa ggggttctct gactgtagtt 3000
   gacatcctta taaatggatg tgcacatttg ccaacactga gtggctttca tcctggagca 3060
   gactttgcag tctgtggact gcaacacaac attgccttta tgtgtaactc ttggctgaag 3120
   ctcttacacc aatgctgggg gacatgtacc tcccaggggc ccaggaagac tacgggaggc 3180
   tacaccaacg tcaatcagag gggcctgtgt agctaccgat aagcggaccc tcaagagggc 3240
   attagcaata gtgtttataa ggcccccttg ttaaccctaa acgggtagca tatgcttccc 3300
   gggtagtagt atatactatc cagactaacc ctaattcaat agcatatgtt acccaacggg 3360
   aagcatatgc tatcgaatta gggttagtaa aagggtccta aggaacagcg atatctccca 3420
   ccccatgagc tgtcacggtt ttatttacat ggggtcagga ttccacgagg gtagtgaacc 3480
   attttagtca caagggcagt ggctgaagat caaggagcgg gcagtgaact ctcctgaatc 3540
   ttcgcctgct tcttcattct ccttcgttta gctaatagaa taactgctga gttgtgaaca 3600
   gtaaggtgta tgtgaggtgc tcgaaaacaa ggtttcaggt gacgccccca gaataaaatt 3660
   tggacggggg gttcagtggt ggcattgtgc tatgacacca atataaccct cacaaacccc 3720
   ttgggcaata aatactagtg taggaatgaa acattctgaa tatctttaac aatagaaatc 3780
   catggggtgg ggacaagccg taaagactgg atgtccatct cacacgaatt tatggctatg 3840
   ggcaacacat aatcctagtg caatatgata ctggggttat taagatgtgt cccaggcagg 3900
   gaccaagaca ggtgaaccat gttgttacac tctatttgta acaaggggaa agagagtgga 3960
   cgccgacagc agcggactcc actggttgtc tctaacaccc ccgaaaatta aacggggctc 4020
   cacgccaatg gggcccataa acaaagacaa gtggccactc ttttttttga aattgtggag 4080
   tgggggcacg cgtcagcccc cacacgccgc cctgcggttt tggactgtaa aataagggtg 4140
   taataacttg gctgattgta accccgctaa ccactgcggt caaaccactt gcccacaaaa 4200
   ccactaatgg caccccgggg aatacctgca taagtaggtg ggcgggccaa gataggggcg 4260
   cgattgctgc gatctggagg acaaattaca cacacttgcg cctgagcgcc aagcacaggg 4320
   ttgttggtcc tcatattcac gaggtcgctg agagcacggt gggctaatgt tgccatgggt 4380
   agcatatact acccaaatat ctggatagca tatgctatcc taatctatat ctgggtagca 4440
   taggctatcc taatctatat ctgggtagca tatgctatcc taatctatat ctgggtagta 4500
   tatgctatcc taatttatat ctgggtagca taggctatcc taatctatat ctgggtagca 4560
   tatgctatcc taatctatat ctgggtagta tatgctatcc taatctgtat ccgggtagca 4620
   tatgctatcc taatagagat tagggtagta tatgctatcc taatttatat ctgggtagca 4680
   tatactaccc aaatatctgg atagcatatg ctatcctaat ctatatctgg gtagcatatg 4740
   ctatcctaat ctatatctgg gtagcatagg ctatcctaat ctatatctgg gtagcatatg 4800
   ctatcctaat ctatatctgg gtagtatatg ctatcctaat ttatatctgg gtagcatagg 4860
   ctatcctaat ctatatctgg gtagcatatg ctatcctaat ctatatctgg gtagtatatg 4920
   ctatcctaat ctgtatccgg gtagcatatg ctatcctcat gcatatacag tcagcatatg 4980
   atacccagta gtagagtggg agtgctatcc tttgcatatg ccgccacctc ccaagggggc 5040
   gtgaattttc gctgcttgtc cttttcctgc tggttgctcc cattcttagg tgaatttaag 5100
   gaggccaggc taaagccgtc gcatgtctga ttgctcacca ggtaaatgtc gctaatgttt 5160
   tccaacgcga gaaggtgttg agcgcggagc tgagtgacgt gacaacatgg gtatgcccaa 5220
   ttgccccatg ttgggaggac gaaaatggtg acaagacaga tggccagaaa tacaccaaca 5280
   gcacgcatga tgtctactgg ggatttattc tttagtgcgg gggaatacac ggcttttaat 5340
   acgattgagg gcgtctccta acaagttaca tcactcctgc ccttcctcac cctcatctcc 5400
   atcacctcct tcatctccgt catctccgtc atcaccctcc gcggcagccc cttccaccat 5460
   aggtggaaac cagggaggca aatctactcc atcgtcaaag ctgcacacag tcaccctgat 5520
   attgcaggta ggagcgggct ttgtcataac aaggtcctta atcgcatcct tcaaaacctc 5580
   agcaaatata tgagtttgta aaaagaccat gaaataacag acaatggact cccttagcgg 5640
   gccaggttgt gggccgggtc caggggccat tccaaagggg agacgactca atggtgtaag 5700
   acgacattgt ggaatagcaa gggcagttcc tcgccttagg ttgtaaaggg aggtcttact 5760
   acctccatat acgaacacac cggcgaccca agttccttcg tcggtagtcc tttctacgtg 5820
   actcctagcc aggagagctc ttaaaccttc tgcaatgttc tcaaatttcg ggttggaacc 5880
   tccttgacca cgatgctttt ccaaaccacc ctcctttttt gcgccctgcc tccatcaccc 5940
   tgaccccggg gtccagtgct tgggccttct cctgggtcat ctgcggggcc ctgctctatc 6000
   gctcccgggg gcacgtcagg ctcaccatct gggccacctt cttggtggta ttcaaaataa 6060
   tcggcttccc ctacagggtg gaaaaatggc cttctacctg gagggggcct gcgcggtgga 6120
   gacccggatg atgatgactg actactggga ctcctgggcc tcttttctcc acgtccacga 6180
   cctctccccc tggctctttc acgacttccc cccctggctc tttcacgtcc tctaccccgg 6240
   cggcctccac tacctcctcg accccggcct ccactacctc ctcgaccccg gcctccactg 6300
   cctcctcgac cccggcctcc acctcctgct cctgcccctc ctgctcctgc ccctcctcct 6360
   gctcctgccc ctcctgcccc tcctgctcct gcccctcctg cccctcctgc tcctgcccct 6420
   cctgcccctc ctgctcctgc ccctcctgcc cctcctcctg ctcctgcccc tcctgcccct 6480
   cctcctgctc ctgcccctcc tgcccctcct gctcctgccc ctcctgcccc tcctgctcct 6540
   gcccctcctg cccctcctgc tcctgcccct cctgctcctg cccctcctgc tcctgcccct 6600
   cctgctcctg cccctcctgc ccctcctgcc cctcctcctg ctcctgcccc tcctgctcct 6660
   gcccctcctg cccctcctgc ccctcctgct cctgcccctc ctcctgctcc tgcccctcct 6720
   gcccctcctg cccctcctcc tgctcctgcc cctcctgccc ctcctcctgc tcctgcccct 6780
   cctcctgctc ctgcccctcc tgcccctcct gcccctcctc ctgctcctgc ccctcctgcc 6840
   cctcctcctg ctcctgcccc tcctcctgct cctgcccctc ctgcccctcc tgcccctcct 6900
   cctgctcctg cccctcctcc tgctcctgcc cctcctgccc ctcctgcccc tcctgcccct 6960
   cctcctgctc ctgcccctcc tcctgctcct gcccctcctg ctcctgcccc tcccgctcct 7020
   gctcctgctc ctgttccacc gtgggtccct ttgcagccaa tgcaacttgg acgtttttgg 7080
   ggtctccgga caccatctct atgtcttggc cctgatcctg agccgcccgg ggctcctggt 7140
   cttccgcctc ctcgtcctcg tcctcttccc cgtcctcgtc catggttatc accccctctt 7200
   ctttgaggtc cactgccgcc ggagccttct ggtccagatg tgtctccctt ctctcctagg 7260
   ccatttccag gtcctgtacc tggcccctcg tcagacatga ttcacactaa aagagatcaa 7320
   tagacatctt tattagacga cgctcagtga atacagggag tgcagactcc tgccccctcc 7380
   aacagccccc ccaccctcat ccccttcatg gtcgctgtca gacagatcca ggtctgaaaa 7440
   ttccccatcc tccgaaccat cctcgtcctc atcaccaatt actcgcagcc cggaaaactc 7500
   ccgctgaaca tcctcaagat ttgcgtcctg agcctcaagc caggcctcaa attcctcgtc 7560
   cccctttttg ctggacggta gggatgggga ttctcgggac ccctcctctt cctcttcaag 7620
   gtcaccagac agagatgcta ctggggcaac ggaagaaaag ctgggtgcgg cctgtgagga 7680
   tcagcttatc gatgataagc tgtcaaacat gagaattctt gaagacgaaa gggcctcgtg 7740
   atacgcctat ttttataggt taatgtcatg ataataatgg tttcttagac gtcaggtggc 7800
   acttttcggg gaaatgtgcg cggaacccct atttgtttat ttttctaaat acattcaaat 7860
   atgtatccgc tcatgagaca ataaccctga taaatgcttc aataatattg aaaaaggaag 7920
   agtatgagta ttcaacattt ccgtgtcgcc cttattccct tttttgcggc attttgcctt 7980
   cctgtttttg ctcacccaga aacgctggtg aaagtaaaag atgctgaaga tcagttgggt 8040
   gcacgagtgg gttacatcga actggatctc aacagcggta agatccttga gagttttcgc 8100
   cccgaagaac gttttccaat gatgagcact tttaaagttc tgctatgtgg cgcggtatta 8160
   tcccgtgttg acgccgggca agagcaactc ggtcgccgca tacactattc tcagaatgac 8220
   ttggttgagt actcaccagt cacagaaaag catcttacgg atggcatgac agtaagagaa 8280
   ttatgcagtg ctgccataac catgagtgat aacactgcgg ccaacttact tctgacaacg 8340
   atcggaggac cgaaggagct aaccgctttt ttgcacaaca tgggggatca tgtaactcgc 8400
   cttgatcgtt gggaaccgga gctgaatgaa gccataccaa acgacgagcg tgacaccacg 8460
   atgcctgcag caatggcaac aacgttgcgc aaactattaa ctggcgaact acttactcta 8520
   gcttcccggc aacaattaat agactggatg gaggcggata aagttgcagg accacttctg 8580
   cgctcggccc ttccggctgg ctggtttatt gctgataaat ctggagccgg tgagcgtggg 8640
   tctcgcggta tcattgcagc actggggcca gatggtaagc cctcccgtat cgtagttatc 8700
   tacacgacgg ggagtcaggc aactatggat gaacgaaata gacagatcgc tgagataggt 8760
   gcctcactga ttaagcattg gtaactgtca gaccaagttt actcatatat actttagatt 8820
   gatttaaaac ttcattttta atttaaaagg atctaggtga agatcctttt tgataatctc 8880
   atgaccaaaa tcccttaacg tgagttttcg ttccactgag cgtcagaccc cgtagaaaag 8940
   atcaaaggat cttcttgaga tccttttttt ctgcgcgtaa tctgctgctt gcaaacaaaa 9000
   aaaccaccgc taccagcggt ggtttgtttg ccggatcaag agctaccaac tctttttccg 9060
   aaggtaactg gcttcagcag agcgcagata ccaaatactg tccttctagt gtagccgtag 9120
   ttaggccacc acttcaagaa ctctgtagca ccgcctacat acctcgctct gctaatcctg 9180
   ttaccagtgg ctgctgccag tggcgataag tcgtgtctta ccgggttgga ctcaagacga 9240
   tagttaccgg ataaggcgca gcggtcgggc tgaacggggg gttcgtgcac acagcccagc 9300
   ttggagcgaa cgacctacac cgaactgaga tacctacagc gtgagctatg agaaagcgcc 9360
   acgcttcccg aagggagaaa ggcggacagg tatccggtaa gcggcagggt cggaacagga 9420
   gagcgcacga gggagcttcc agggggaaac gcctggtatc tttatagtcc tgtcgggttt 9480
   cgccacctct gacttgagcg tcgatttttg tgatgctcgt caggggggcg gagcctatgg 9540
   aaaaacgcca gcaacgcggc ctttttacgg ttcctggcct tttgctggcc ttgaagctgt 9600
   ccctgatggt cgtcatctac ctgcctggac agcatggcct gcaacgcggg catcccgatg 9660
   ccgccggaag cgagaagaat cataatgggg aaggccatcc agcctcgcgt cgcgaacgcc 9720
   agcaagacgt agcccagcgc gtcggccccg agatgcgccg cgtgcggctg ctggagatgg 9780
   cggacgcgat ggatatgttc tgccaagggt tggtttgcgc attcacagtt ctccgcaaga 9840
   attgattggc tccaattctt ggagtggtga atccgttagc gaggtgccgc cctgcttcat 9900
   ccccgtggcc cgttgctcgc gtttgctggc ggtgtccccg gaagaaatat atttgcatgt 9960
   ctttagttct atgatgacac aaaccccgcc cagcgtcttg tcattggcga attcgaacac 10020
   gcagatgcag tcggggcggc gcggtccgag gtccacttcg catattaagg tgacgcgtgt 10080
   ggcctcgaac accgagcgac cctgcagcga cccgcttaac agcgtcaaca gcgtgccgca 10140
   gatcccgggg ggcaatgaga tatgaaaaag cctgaactca ccgcgacgtc tgtcgagaag 10200
   tttctgatcg aaaagttcga cagcgtctcc gacctgatgc agctctcgga gggcgaagaa 10260
   tctcgtgctt tcagcttcga tgtaggaggg cgtggatatg tcctgcgggt aaatagctgc 10320
   gccgatggtt tctacaaaga tcgttatgtt tatcggcact ttgcatcggc cgcgctcccg 10380
   attccggaag tgcttgacat tggggaattc agcgagagcc tgacctattg catctcccgc 10440
   cgtgcacagg gtgtcacgtt gcaagacctg cctgaaaccg aactgcccgc tgttctgcag 10500
   ccggtcgcgg aggccatgga tgcgatcgct gcggccgatc ttagccagac gagcgggttc 10560
   ggcccattcg gaccgcaagg aatcggtcaa tacactacat ggcgtgattt catatgcgcg 10620
   attgctgatc cccatgtgta tcactggcaa actgtgatgg acgacaccgt cagtgcgtcc 10680
   gtcgcgcagg ctctcgatga gctgatgctt tgggccgagg actgccccga agtccggcac 10740
   ctcgtgcacg cggatttcgg ctccaacaat gtcctgacgg acaatggccg cataacagcg 10800
   gtcattgact ggagcgaggc gatgttcggg gattcccaat acgaggtcgc caacatcttc 10860
   ttctggaggc cgtggttggc ttgtatggag cagcagacgc gctacttcga gcggaggcat 10920
   ccggagcttg caggatcgcc gcggctccgg gcgtatatgc tccgcattgg tcttgaccaa 10980
   ctctatcaga gcttggttga cggcaatttc gatgatgcag cttgggcgca gggtcgatgc 11040
   gacgcaatcg tccgatccgg agccgggact gtcgggcgta cacaaatcgc ccgcagaagc 11100
   gcggccgtct ggaccgatgg ctgtgtagaa gtactcgccg atagtggaaa ccgacgcccc 11160
   agcactcgtc cggatcggga gatgggggag gctaactgaa acacggaagg agacaatacc 11220
   ggaaggaacc cgcgctatga cggcaataaa aagacagaat aaaacgcacg ggtgttgggt 11280
   cgtttgttca taaacgcggg gttcggtccc agggctggca ctctgtcgat accccaccga 11340
   gaccccattg gggccaatac gcccgcgttt cttccttttc cccaccccac cccccaagtt 11400
   cgggtgaagg cccagggctc gcagccaacg tcggggcggc aggccctgcc atagccactg 11460
   gccccgtggg ttagggacgg ggtcccccat ggggaatggt ttatggttcg tgggggttat 11520
   tattttgggc gttgcgtggg gtcaggtcca cgactggact gagcagacag acccatggtt 11580
   tttggatggc ctgggcatgg accgcatgta ctggcgcgac acgaacaccg ggcgtctgtg 11640
   gctgccaaac acccccgacc cccaaaaacc accgcgcgga tttctggcgt gccaagctag 11700
   tcgaccaatt ctcatgtttg acagcttatc atcgcagatc cgggcaacgt tgttgccatt 11760
   gctgcaggcg cagaactggt aggtatggaa gatctataca ttgaatcaat attggcaatt 11820
   agccatatta gtcattggtt atatagcata aatcaatatt ggctattggc cattgcatac 11880
   gttgtatcta tatcataata tgtacattta tattggctca tgtccaatat gaccgccat  11939
<210> 270
   <211> 36
   <212> DNA
   <213> Artificial Sequence
<220>
   <223> In vitro synthesized DNA encoding a constrained peptide sequence.
<400> 270
   ggcggatgcg agcacttccg ctggtgcaag ccggtg 36
<210> 271
   <211> 12
   <212> PRT
   <213> Artificial Sequence
<220>
   <223> Constrained peptide encoded by an in vitro synthesized DNA.
<400> 271
   Gly Gly Cys Glu His Phe Arg Trp Cys Lys Pro Val
   1               5                   10
<210> 272
   <211> 9
   <212> DNA
   <213> Artificial Sequence
<220>
   <223> In vitro synthesized DNA encoding a flexible peptide sequence.
<400> 272
   ggatccggt 9
<210> 273
   <211> 3
   <212> PRT
   <213> Artificial Sequence
<220>
   <223> Flexible peptide encoded by an in vitro synthesized DNA.
<400> 273
   Gly Ser Gly
   1
<210> 274
   <211> 21
   <212> DNA
   <213> Artificial Sequence
<220>
   <223> In vitro mutagenized Homo sapien DNA encoding a V2 peptide sequence comprising a V to N substitution to increase N-linked glycosylation and a S to T substitution to increase O-linked glycosylation.
<400> 274
   accttagtca ccaactccac c 21
<210> 275
   <211> 7
   <212> PRT
   <213> Artificial Sequence
<220>
   <223> Mutagenized Homo sapien V2 peptide comprising a V to N substitution to increase N-linked glycosylation and a S to T subsutition to improve O-linked glycosylation and encoded by an in vitro mutagenized Homo sapien DNA.
<400> 275
   Thr Leu Val Thr Asn Ser Thr
    1               5
<210> 276
   <211> 15
   <212> DNA
   <213> Artificial Sequence
<220>
   <223> In vitro synthesized DNA encoding a flexible peptide sequence.
<400> 276
   ggaggcggat ccggt 15
<210> 277
   <211> 5
   <212> PRT
   <213> Artificial Sequence
<220>
   <223> Flexible peptide encoded by an in vitro synthesized DNA.
<400> 277
   Gly Gly Gly Ser Gly
    1               5
<210> 278
   <211> 21
   <212> DNA
   <213> Artificial Sequence
<220>
   <223> In vitro mutagenized Homo sapien DNA encoding a V2 peptide sequence comprising a L to N substitution to increase N-linked glycosylation and a S to A substitution to increase N-linked glycosylation.
<400> 278
   accaacgtca ccgtctccgc a 21
<210> 279
   <211> 7
   <212> PRT
   <213> Artificial Sequence
<220>
   <223> Mutagenized Homo sapien V2 peptide comprising a V to N substitution to increase N-linked glycosylation and a S to T subsutition to increase O-linked glycosylation and encoded by an in vitro mutagenized Homo sapien DNA.
<400> 279
   Thr Asn Val Thr Val Ser Ala
   1 5
<210> 280
   <211> 24
   <212> DNA
   <213> Artificial Sequence
<220>
   <223> In vitro synthesized DNA encoding a flexible peptide sequence.
<400> 280
   ggtcccccat gcccaccatg cccg 24
<210> 281
   <211> 8
   <212> PRT
   <213> Artificial Sequence
<220>
   <223> Flexible peptide encoded by an in vitro synthesized DNA.
<400> 281
   Gly Pro Pro Cys Pro Pro Cys Pro
   1 5
<210> 282
   <211> 8
   <212> PRT
   <213> Artificial Sequence
<220>
   <223> Constrained peptide encoded by an in vitro synthesized DNA
<400> 282

## Description

### Field of the Invention

The present invention relates to melanocortin receptor binding mimetibodies, polynucleotides encoding these, cells comprising the polynucleotides or expressing the mimetibodies, and methods of making and using the foregoing.

### Background of the Invention

Obesity is a chronic disease manifested by an excess of fat mass in proportion to body size. Today, every third American is considered over-weight (Body Mass Index (BMI) >25 kg/m²), thus prompting the United States Centers for Disease Control and Prevention (CDC) to declare that obesity is reaching epidemic proportions (Cummings and Schwartz, Annu. Rev. Med. 54:453-471((2003)). The importance of treating obesity is emphasized by the fact that this disease is either the underlying cause, or a risk factor, for developing diseases such as Type 2 Diabetes, congestive heart failure, osteoarthritis and sleep apnea among others.

Additionally, obesity is linked to "Metabolic Syndrome" which is a medical condition characterized by obesity, atherogenic dyslipidemia, elevated blood pressure and insulin resistance. Metabolic Syndrome affects an increasing number of people in the United States. Importantly, it has been shown that even a modest decrease in body weight (5-10% of initial body weight) may significantly improve Metabolic Syndrome conditions and decrease the risk factors for developing obesity-associated disease (Wing et al., Arch. Intern. Med. 147:1749-1753 (1987); Tuomilehto et al., New Engl. J. Med. 344:1343-1350 (2001); Knowler et al., New Engl. J Med. 346:393-403 (2002); Franz et al., Diabetes Care 25:148-198 (2002)). Additionally, treatment of obesity may be important from a mental health perspective due to the social stigma often attached to obese individuals in some cultures.

Melanocortin receptors play a major role in the regulation of overall energy balance and obesity in both humans and rodents. Alpha-melanocyte stimulating hormone (alpha-MSH) is a 13 amino acid peptide hormone that is an important component of the melanocortin system. Alpha-MSH is produced by the proteolytic processing of proopiomelanocortin (POMC) released by the pituitary gland. Alpha-MSH binds with high affinity to the melanocortin 4 receptor (MC4R), but also binds melanocortin receptor 3 (MC3R) and melanocortin receptor 5 (MC5R) with lower affinity. MC4R is a G-coupled protein receptor found in the brain which, when stimulated by alpha-MSH binding, causes decreased food intake and increased fat oxidation. Ultimately, stimulation of melanocortin receptors such as MC4R results in weight loss.

In humans and rodents, loss of function mutations in the different components of the melanocortin system are closely correlated with obesity and related conditions. In mice, mutations within POMC, or MC4R and MC3R produce obesity, insulin resistance and hyperphagia (Goodfellow and Saunders, Curr. Topics Med. Chem. 3: 855-883 (2003); Huszar et al., Cell 88:131-141 (1997); Yaswen et al., Nat. Med. 5: 1066-1070 (1999)). In man mutations within POMC or MC4R lead to the development of obesity associated with increased food intake (Krude et al., Nat. Genet. 19:155-157 (1998); Yeo et al., Nature Genetics 20:111-112 (1998); Branson et al., New Engl. J. Med. 348: 1096-1103 (2003); Vaisse et al., J. Clin. Invest. 106):253-262 (2000); Ho and MacKenzie, J. Biol. Chem. 275: 35816-35822 (1999)).

Weight loss can result from the pharmacological stimulation of melanocortin system activity. In rodents pharmacological stimulation of melanocortin receptors such as MC4R leads to decreased food intake, increased energy expenditure and weight loss (Pierroz et al., Diabetes 51: 1337-1345 (2002)). In man the intranasal administration of alpha-MSH to stimulate MC4R in non-obese men results in decreased body weight due to the loss of fat-but not lean body mass (Fehm et al., J. Clin. Endo. Metabol. 86: 1144-1148 (2001)).

Obesity is currently treated, with only limited success, by several different strategies. These strategies primarily involve "life-style" changes (e.g. diet and exercise), small molecule based pharmaceutical therapies or surgical removal of a portion of the stomach (gastric by-pass surgery). Additionally, weight loss stimulating melanocortin receptor binding peptides such as alpha-MSH are of limited use as pharmaceuticals due to the extremely short serum half-life of such peptides. Thus, a need exists for additional obesity treatments and in particular for melanocortin receptor binding molecules that overcome the short serum half-life of melanocortin receptor binding peptides such as alpha-MSH.

US 2007/082843 relates to a method for suppressing appetite by the administration of an MSH. WO 2006/047535 relates to a mimetibody comprising a melanocortin receptor binding molecule. WO 2005/032460 relates to EPO mimetic hinge core mimetibodies.

Strombergsson et al. (2006) considers Rough set-based proteochemometrics modelling of G-protein-coupled receptor-ligand interactions. Strombergsson et al. (2004) reports a model for the interaction of chimeric melanocortin G-protein coupled receptors with peptide ligands using the rough set approach. Mike Clark (2002) presents an alignment of IgG sequences from human, mouse and rat.

### Brief Description of the Drawings

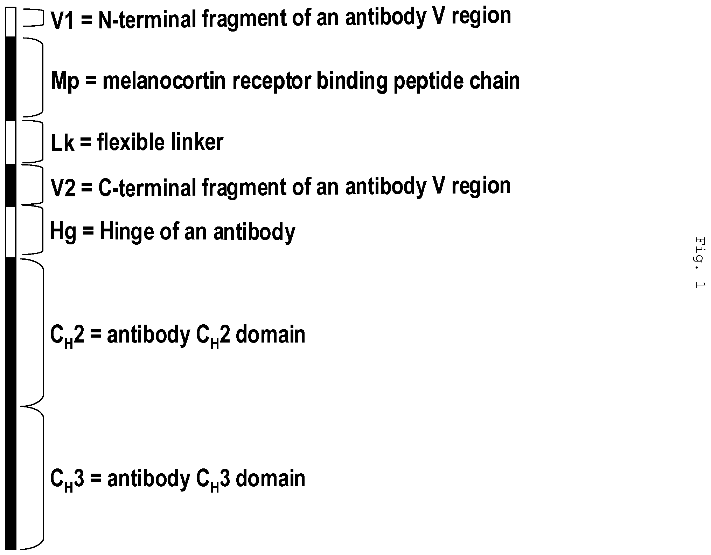

Fig. 1 shows elements of a melanocortin receptor binding mimetibody polypeptide.

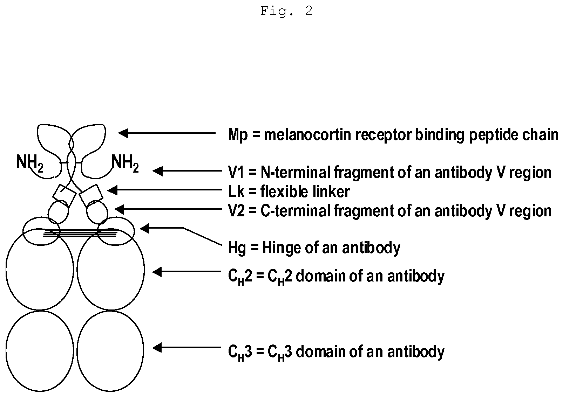

Fig 2 shows a cartoon of a melanocortin receptor binding mimetibody.
Fig. 3 shows the amino acid (SEQ ID NO: 62) and cDNA (SEQ ID NO: 61) sequences of a melanocortin receptor binding alpha-MSH mimetibody. The amino terminal portions of individual mimetibody elements are underlined.
Fig. 4 shows alpha-MSH mimetibody binding to MC4R in a competitive binding assay.
Fig. 5 shows alpha-MSH mimetibody activation of MC4R in cells expressing a high level of MC4R.
Fig. 6 shows alpha-MSH mimetibody activation of MC4R in cells expressing a low level of MC4R.
Fig. 7 shows alpha-MSH mimetibody-mediated decrease in animal food intake.
Fig. 8 shows alpha-MSH mimetibody-mediated decrease in animal body weight.

### Summary of the Invention

The invention provides a polypeptide comprising a polypeptide having the sequence shown in SEQ ID NO: 212.

The invention also provides a polynucleotide: (i) encoding a polypeptide of the invention; or (ii) comprising a polynucleotide having the sequence shown in SEQ ID NO: 211 or a polynucleotide having a sequence complementary to the sequence shown in SEQ ID NO: 211; or (iii) comprising a polynucleotide encoding the polypeptide having the sequence shown in SEQ ID NO: 212.

The invention also provides a vector comprising the polynucleotide of the invention.

The invention also provides a cell expressing: (i) a polypeptide of the invention; or (ii) the vector of the invention. The invention also provides a method to produce a polypeptide comprising the steps of culturing the cell of the invention and purifying the expressed polypeptide.

The invention also provides a pharmaceutical composition comprising an effective amount of at least one polypeptide of the invention and a pharmaceutically acceptable carrier or diluent.

The invention also provides the pharmaceutical composition of the invention, for use in a therapy.

The invention also provides the pharmaceutical composition of the invention, for use in treating a method of modulating at least one melanocortin receptor mediated condition.

The invention also provides the pharmaceutical composition of the invention wherein the melanocortin receptor mediated condition is obesity, male erectile dysfunction, female sexual dysfunction, an inflammatory condition or fibrosis.

### Summary of the disclosure

Disclosed herein is a polypeptide according to formula (I):

(Mp-Lk-(V2)ₓ-Hg-C_{H}2-C_{H}3)₍ₜ₎ (I)

where Mp is a melanocortin receptor binding molecule, Lk is a polypeptide or chemical linkage, V2 is a portion of a C-terminus of an immunoglobulin variable region, Hg is at least a portion of an immunoglobulin variable hinge region, C_{H}2 is an immunoglobulin heavy chain C_{H}2 constant region and C_{H}3 is an immunoglobulin heavy chain C_{H}3 constant region, x is 0 or 1, and t is independently an integer from 1 to 10.

Also disclosed herein is a method of modifying the biological activity of a melanocortin receptor in a cell, tissue or organ, comprising contacting a mimetibody composition of the invention with the cell, tissue or organ.
Also disclosed herein is a method of modulating at least one melanocortin receptor mediated condition comprising administering a mimetibody composition of the invention to a patient in need thereof.

### Detailed Description

Disclosed herein are polypeptides having the properties of binding a melanocortin receptor and mimicking different isotypes of antibody immunoglobulin molecules such as IgA, IgD, IgE, IgG, or IgM, and any subclass thereof, such as IgA₁, IgA₂, IgG₁, IgG₂, IgG₃ or IgG₄, or combinations thereof, herein after generally referred to as "mimetibodies." In some embodiments, the disclosed mimetibody polypeptides contain an alpha melanocyte stimulating hormone peptide (alpha-MSH) sequence and are designated melanocortin receptor binding alpha-MSH mimetibody. Such alpha-MSH mimetibody polypeptides can bind melanocortin receptor 4 (MC4R) and, with equal and lower affinity, for MC3R and MC5R respectively. One result of such melanocortin receptor binding can be the stimulation or inhibition of melanocortin receptor activity. Stimulation can cause weight loss while inhibition may cause weight gain.

Disclosed herein are polypeptides that have the generic formula (I):

(Mp-Lk-(V2)ₓ-Hg-C_{H}2-C_{H}3)₍ₜ₎ (I)

where Mp is a melanocortin receptor binding molecule, Lk is a polypeptide or chemical linkage, V2 is a portion of a C-terminus of an immunoglobulin variable region, Hg is at least a portion of an immunoglobulin variable hinge region, C_{H}2 is an immunoglobulin heavy chain C_{H}2 constant region and C_{H}3 is an immunoglobulin heavy chain C_{H}3 constant region, x is 0 or 1, and t is independently an integer of 1 to 10.

As used herein, "melanocortin receptor binding molecule" means a molecule, which can bind at least one melanocortin receptor such as *Homo sapiens* MC4R (SEQ ID NO: 77). Examples of other *Homo sapiens* melanocortin receptors include MCR1 (SEQ ID NO: 71), MCR2 (SEQ ID NO: 73), MCR3 (SEQ ID NO: 75), and MCR5 (SEQ ID NO: 79). A given peptide chain is a "melanocortin receptor" if it has at least 85% amino acid sequence identity to a known melanocortin receptor sequence or the mature form of a known melanocortin receptor and can function as a G-protein coupled receptor. Percent identity between two peptide chains can be determined by pairwise alignment using the default settings of the AlignX module of Vector NTI v.9.0.0 (Invitrogen Corp., Carslbad, CA). An exemplary melanocortin receptor binding molecule is the 13 amino acid alpha-MSH peptide having the amino acid sequence shown in (SEQ ID NO: 2). Other melanocortin receptor binding molecules include biologically active fragments of SEQ ID NO: 2 and other amino acid sequences that can bind a melanocortin receptor. The term "biologically active fragment" as used herein, refers to a portion of an alpha-MSH peptide that can bind to a melanocortin receptor such as MC4R. The peptide sequence HFRW (SEQ. ID. NO. 81) is an exemplary "biologically active fragment" of the alpha-MSH peptide sequence SYSMEHFRWGKPV (SEQ ID NO: 2). The HFRW fragment has been incorporated into the structure of the synthetic melanocortin receptor activator molecule melanotan II (MTII) (Fan et al., Nature 385: 165-168 (1997)).

Incorporation of melanocortin receptor binding molecules in the mimetibody polypeptides disclosed herein provides for binding to melanocortin receptors with a wide range of affinities. The disclosed mimetibody polypeptides may bind a melanocortin receptor with a K_{d} less than or equal to about 10⁻⁷, 10⁻⁸, 10⁻⁹, 10⁻¹¹, 10⁻¹¹ or 10⁻¹² M. The range of obtained IC₅₀ values for aMSH peptide, MTII peptide and aMSHMMB were 260-400 nM, 5-30 nM and 200-300 nM respectively. The affinity of a mimetibody polypeptide for a melanocortin receptor can be determined experimentally using any suitable method. Such methods may utilize Biacore or KinExA instrumentation, ELISA or competitive binding assays. Mimetibody polypeptides binding specific melanocortin receptors with a desired affinity can be selected from libraries of variants or fragments by techniques known to those skilled in the art.

An alpha-MSH peptide having the amino acid sequence shown in SEQ ID NO: 2 may be modified to obtain other melanocortin receptor binding molecules. Such modifications may comprise the incorporation of C-[X]ₙ-C motifs into the peptide to conformationally constrain the peptide through the formation of disulfide bonds. In a C-[X]ₙ-C motif, C is a cysteine residue, X is a amino acid residues and n is an integer necessary to acheive the required conformational constraint. In this instance n can be as little as 1 residue and as high as 50. Exemplary C-[X]ₙ-C modified peptide sequences are shown in SEQ ID NOs: 4, 6, 8, 10, 89, 91, 93, 95, and 97.

The modification may also comprise the incorporation of a Wa-[X]ₙ-Wa motif into the peptide to conformationally constrain the peptide through the formation of a tryptophan zipper. In a Wa-[X]ₙ-Wa motif W is tryptophan residue, X is an amino acid, a is an integer ususlly 2, but can be from 1 to 10, and n is an integer necessary to acheive the required conformational constraint. In this instance n can be as little a 1 residue and as high as 50. Exemplary Wa-[X]ₙ-Wa peptides are shown in SEQ ID NOs: 12, 14, 16 and 18. Further, the sequence HFRW (SEQ ID NO: 81) present in the alpha-MSH peptide may also be modified by substituting any residue in this sequence with any one of F, H, W and M; for example, HFRW (SEQ ID NO: 81) can be substituted to FHWM (SEQ ID NO: 83).

In the disclosed polypeptides, the linker portion (Lk) provides structural flexibility by allowing the mimetibody to have alternative orientations and binding properties. Exemplary linkers include non-peptide chemical linkages or one to 20 amino acids linked by peptide bonds, wherein the amino acids are selected from the 20 naturally occurring amino acids or other amino acids (e.g. D-amino acids, non-naturally occurring amino acids, or rare naturally occuring amino acids). The linker portion can include a majority of amino acids that are sterically unhindered, such as glycine, alanine and serine and can include GS, poly GS (e.g. GSGS (SEQ ID NO: 20)), GGSG (SEQ ID NO: 22), GSGGGS (SEQ ID NO: 24), GSGGGSG (SEQ ID NO: 26), GSSG (SEQ ID NO: 28), GGGS (SEQ ID NO: 85), GGGGS (SEQ ID NO: 99), GGGGSGGGGS (SEQ ID NO: 101), GGGGSGGGGSGGGGS (SEQ ID NO: 103), GGGGSGGGGSGGGGSGGGGS (SEQ ID NO: 105) or any combination or polymer thereof. Other exemplary linkers disclosed herein may be longer than 20 residues and may include residues other than glycine, alanine and serine.

In the polypeptides of the disclosure, V2 is a portion of a carboxy terminal domain of an immunoglobulin variable region such as a heavy chain variable region. Exemplary V2 amino acid sequences are GTLVTVSS (SEQ ID NO: 32), TLVAVSS (SEQ ID NO: 34), and TLVTVSS (SEQ ID NO: 249).

In the polypeptides of the disclosure, Hg is a portion of the hinge domain of an immunoglobulin variable region such as a heavy chain variable region. Exemplary Hg amino acid sequences include EPKSCDKTHTCPPCP (SEQ ID NO: 36), EPKSADKTHTCPPCP (SEQ ID NO: 38), ESKYGPPCPSCP (SEQ ID NO: 40), ESKYGPPCPPCP (SEQ ID NO: 42), CPPCP (SEQ ID NO: 44) and CPSC (SEQ ID NO: 46).

Hg amino acid sequences can be modified. Such modifications can remove potential sites of O-linked glycosylation. Such modifications can also remove cysteine residues that may cause aggregates or multimers of the polypeptides of the disclosure to form.

One way to minimize O-linked glycosylation in the disclosed mimetibodies is to substitute Ala residues for Thr residues in the Hg portion of the polypeptides. The Hg amino acid sequence EPKSCDKTHACPPCP (SEQ ID NO: 107) is exemplary of such a Thr to Ala substitution; this particular Hg substitution can also be obtained by a Thr to Ala substitution at position 59 of SEQ ID NO: 62.

One way to minimize aggregation or multimerization of the disclosed mimetibodies is to substitute Ala residues for Cys residues in the Hg portion of the polypeptides. The Hg amino acid sequence EPKSADKTHTCPPCP (SEQ ID NO: 109) is exemplary of such a Cys to Ala substitution; this particular Hg substitution can also be obtained by a Cys to Ala substitution at position 54 of SEQ ID NO: 62.

Modifications to the Hg amino acid sequences of the disclosed mimetibody polypeptides can be made singly or in combination. The Hg amino acid sequence EPKSADKTHACPPCP (SEQ ID NO: 111) combines both the aforementioned substitutions; and can be obtained by a Cys to Ala substitution at position 54 and a Thr to Ala substitution at position 59 of SEQ ID NO: 62. Those skilled in the art will recognize other amino acid residues that can be used to make substitutions that remove O-glycosylation sites and aggregation or multimerization associated sites in the disclosed mimetibodies. Such sites can also be deleted by removing amino acid residues.

In the polypeptides disclosed herein, C_{H}2 is an immunoglobulin heavy chain C_{H}2 constant region. Exemplary C_{H}2 amino acid sequences include:

In the polypeptides disclosed herein, C_{H}3 is an immunoglobulin heavy chain C_{H}3 constant region. Exemplary C_{H}3 amino acid sequences include: and It will be recognized by those skilled in the art that the C_{H}3 region of the polypeptides may have its C-terminal amino acid cleaved off when expressed in certain recombinant systems.

In the disclosed mimetibody polypeptides, C_{H}2 or C_{H}3 may be of the IgG₁ or IgG₄ subclass. A sequence is of the IgG₁ or IgG₄ subclass if it is formed or developed from a γ1 or γ4 heavy chain respectively. A given peptide chain is a γ1 or γ4 heavy chain if it is at least 80% identical to a known γ1 or γ4 heavy chain sequence of a given species. Percent identity between two peptide chains can be determined by pairwise alignment using the default settings of the AlignX module of Vector NTI v.9.0.0 (Invitrogen Corp., Carlsbad, CA).

In the disclosed mimetibody polypeptides Hg, C_{H}2 or C_{H}3 may individually be of the IgG₁ or IgG₄ subclass. The mimetibodies may also comprise combinations of Hg, C_{H}2 or C_{H}3 elements from each subclass For example, Hg may be of the IgG₄ subclass while C_{H}2 and C_{H}3 are of the IgG₁ subclass. Alternatively, Hg, C_{H}2 and C_{H}3 may all of the IgG₄ or IgG₁ subclass. The polypeptide EPKSCDKTHTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKGQPREPQVY TLPPSRDELTKNQVSLTCLVKGFYPSDIAVEWESNGQPENNYKTTPPVLDSDGSFFLYSKLTVDKSR WQQGNVFSCSVMHEALHNHYTQKSLSLSPGK (SEQ ID NO: 65) is exemplary of a polypeptide in which Hg (residues 1-15 of SEQ ID NO: 65), C_{H}2 (residues 16-125 of SEQ ID NO: 65), and C_{H}3 (residues 126-232 of SEQ ID NO: 65) are all of the IgG₁ subclass.

The IgG₁ and IgG₄ subclasses differ in the number of cysteines in the hinge region. Most IgG type antibodies, such as IgG₁, are homodimeric molecules made up of two identical heavy (H) chains and two identical light (L) chains, typically abbreviated H₂L₂. Thus, these molecules are generally bivalent with respect to antigen binding due to the formation of inter-heavy chain disulfide bonds and both antigen binding (Fab) arms of the IgG molecule have identical binding specificity. IgG₄ isotype heavy chains, in contrast, contain a CPSC (SEQ ID NO: 46) motif in their hinge regions capable of forming either inter- or intra-heavy chain disulfide bonds, i.e., the two Cys residues in the CPSC motif may disulfide bond with the corresponding Cys residues in the other H chain (inter) or the two Cys residues within a given CPSC motif may disulfide bond with each other (intra). Since the HL pairs in those IgG₄ molecules with intra-heavy chain bonds in the hinge region are not covalently associated with each other, they may dissociate into HL monomers that then reassociate with HL monomers derived from other IgG₄ molecules forming bispecific, heterodimeric IgG₄ molecules. *In vivo* isomerase enzymes may facilitate this process. In a bispecific IgG antibody the two Fab "arms" of the antibody molecule differ in the epitopes that they bind. Substituting Ser residues in the hinge region of IgG₄ with Pro results in "IgG₁-like behavior," i.e., the molecules form stable disulfide bonds between heavy chains and therefore, are not susceptible to HL exchange with other IgG₄ molecules.

The mimetibody polypeptides may be made more IgG₄-like, or IgG₁-like by the modification of sites which are involved in disulfide bond formation and are present in the Hg-C_{H}2-C_{H}3 portion of the mimetibody polypeptides. Such sites may be modified by removal, deletion, insertion or substitution with other amino acids. Typically, the cysteine residues present in disulfide bond associated motifs are removed or substituted. Removal of these sites may avoid covalent disulfide bonding with other cysteine-containing proteins present in the mimetibody producing host cell or intra-heavy chain disulfide bonding in IgG₄-based constructs while still allowing for noncovalent dimerization of mimetibody Hg-C_{H}2-C_{H}3 domains. Modification of such sites can permit the formation of bispecific mimetibody polypeptides with two different M portions or prevent the formation of such bispecific species.

The IgG₁ and IgG₄ subclasses also differ in their ability to mediate complement dependent cytotoxicity (CDC) and antibody-dependent cellular cytotoxicity (ADCC). CDC is the lysing of a target cell in the presence of complement. The complement activation pathway is initiated by the binding of the first component of the complement system (C1q) to a molecule complexed with a cognate antigen. IgG₁ is a strong inducer of the complement cascade and subsequent CDC activity, while IgG₄ has little complement-inducing activity. ADCC is a cell-mediated process in which nonspecific cytotoxic cells that express Fc receptors (FcRs) involved in ADCC (e.g., natural killer (NK) cells, neutrophils, and macrophages) recognize bound antibody on a target cell and subsequently cause lysis of the target cell. The IgG₁ subclass binds with high affinity to Fc receptors involved in ADCC and contributes to ADCC, while IgG₄ binds only weakly to such receptors and has little ADCC inducing activity. The relative inability of IgG₄ to activate effector functions such as ADCC is desirable since delivery of the mimetibody polypeptide to cells without cell killing is possible.

The CDC and ADCC activity of the mimetibody polypeptides disclosed herein may be modified by altering sites involved in CDC and ADCC present in the Hg-C_{H}2-C_{H}3 portion of the mimetibody polypeptide. Such sites may be modified by removal, deletion, insertion or substitution with other amino acids. In the mimetibodies sites involved in CDC, such as the C1q binding site, are typically removed or otherwise modified to minimize CDC activity. Additionally, Fc receptor binding sites involved in ADCC can also be similarly modified in the mimetibodies. In general, such modification will remove Fc receptor binding sites involved in ADCC activity from the mimetibodies. The substitution of Leu residues with Ala residues in the C_{H}2 portion of the polypeptides is one example of a modification which can minimize ADCC activity in the polypeptides disclosed herein. The C_{H}2 amino acid sequences APEAAGGPSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAK (SEQ ID NO: 52) and APEAAGGPSVFLFPPKPKDTLMISRTPEVTCVVVDVSQEDPEVQFNWYVDGVEVHNAKTKPREEQFN STYRVVSVLTVLHQDWLNGKEYKCKVSNKGLSSPIEKTISKAK (SEQ ID NO: 54) are exemplary of such a Leu to Ala substitution at residues 4 and 5 (in these sequences). Further, the V1 domain can be removed such that the N-terminus of the peptide is free following cleavage of the signal peptide, and is accessible to and could be modified by enzymes such as acetylases.

Antibodies of both the IgG₄ and IgG₁ isotypes contain FcRn salvage receptor binding sites. The FcRn salvage receptor helps maintain IgG antibody levels in the body by recycling or transporting IgG type antibodies across enodothelial cell layers such as those lining the inside of body cavities and blood vessels. The FcRn salavage receptor does this by binding IgGs that have entered endothelial cells by nonspecific pinocytosis and preventing these IgG antibody molecules from being degraded in the lysosome of the cell. The result of such FcRn receptor activity is that the serum half-life of a molecule with an FcRn binding site is extended relative to an otherwise identical molecule lacking such a site.

It is desirable that the Hg-C_{H}2-C_{H}3 portion of the mimetibodies disclosed herein contain a FcRn binding site at the junction of the C_{H}2 and C_{H}3 regions. It is expected that such FcRn sites will increase the serum half-life of the mimetibodies as well as improve other pharmacokinetic properties relative to a melanocortin receptor binding molecule, such as alpha-MSH alone. In the mimetibodies disclosed herein FcRn sites may be modified or added by removal, deletion, insertion or substitution of amino acids. Typically, such modifications are used to improve the binding of a given site to the FcRn. One example of a human FcRn binding sites is the sequence MISRTPTVLHQHNHY (SEQ. ID. NO.: 69) found in both IgG₁ and IgG₄ antibodies. Other FcRn binding sites are well known by those skilled in the art.

Antibodies with different isotypes, such as IgG₄ and IgG₁, may contain glycosylation sites. Glycosylation of these sites can alter the properties and activites of antibody molecules. Antibody molecules may be N-glycosylated or O-glycosylated. N-glycosylation of antibody amino acid residue side chains containing nitrogen atoms (e.g., Asn) can modulate antibody Fc effector functions such as ADCC by conferring a cytolytic activity to N-glycosylated antibody molecules. This ADCC associated cytolytic activity causes the lysis of cells effected by such N-glycosylated antibodies. Alternatively, an antibody molecule may be O-glycosylated by modification of amino acid residue side chains containing oxygen atoms (e.g., Ser or Thr). O-glycosylation can decrease the serum half-life of an antibody molecule through increased lectin mediated clearance of O-glycosylated antibody molecules from the serum. Additionally, O-glycosylation can cause undesirable increases in antibody heterogeneity due to differing extents of O-glycosylation between various antibody molecules. Lastly, both O-glycosylation and N-glycosylation can alter the structure dependent properties of antibody molecules such as binding affinity and immunogenicity.

Like the antibody molecules they mimic, the mimetibody polypeptides of the invention may also be post-translationally modified by N-glycosylation and O-glycosylation. In most instances, it is desirable to limit the N-glycosylation of the mimetibodies of the invention to minimize cytolytic activity. N-glycosylation can be limited by the removal or substitution of amino acid residues, such as Asn, which are typically N-glycosylated. It is also desirable to limit mimetibody O-glycosylation to minimize lectin-mediated clearance, mimetibody heterogeneity and the alteration of structure dependent mimetibody properties such as binding affinity and immunogenicity. One way to minimize O-linked glycosylation in the mimetibodies of the invention is to substitute Ala residues for Thr residues in the V2 portion of the polypeptides of the invention. The V2 amino acid sequence TLVAVSS (SEQ ID NO: 34) is exemplary of such a Thr to Ala substitution; this particular V2 substitution can also be obtained by a Thr to Ala substitution at postion 47 of SEQ ID NO: 62. Those skilled in the art also will recognize other ways to control N-linked and O-linked glycosylation including modulation of glycosylase enzyme activity.

The monomeric structure Mp-Lk-(V2)ₓ-Hg-C_{H}2-C_{H}3 of the mimetibody polypeptides of the invention can be linked to "t" other monomers where t is an integer from 1 to 10. Such linking can occur through non-covalent interactions or covalent linkages such as a Cys-Cys disulfide bond. In this way multimeric structures such as dimers and higher order multimers of the polypeptides of the invention can be formed. It is expected that dimerization of the polypeptides of the invention will increase the affinity of these polypeptides to melanocortin receptors such as MC4R. The term "multimers" as used herein means molecules that have quaternary structure and are formed by the association of two or more subunits.

The polypeptides of the invention can optionally comprise at the amino terminus, an amino terminal portion of an immunoglobulin variable region, designated V1 as shown in Formula II:

(V1-Mp-Lk-(V2)ₓ-Hg-C_{H}2-C_{H}3)₍ₜ₎ (II)

Exemplary V1 amino acid sequences include QIQ, QVQ, QIQGG (SEQ ID NO: 113), and QIQGGGG (SEQ ID NO: 115).

The polypeptides of the invention may also comprise secretory signals necessary to facilitate protein secretion or other signals necessary for protein trafficking in the cell. An exemplary secretory signal sequence is MAWVWTLLFLMAAAQSIQA (SEQ ID NO: 69). Those skilled in the art will recognize other secretory signals.

In one embodiment the polypeptides of the invention comprise SEQ ID NO: 212. SEQ ID NO: 62 represents a (V1-Mp-Lk-(V2)ₓ-Hg-C_{H}2-C_{H}3)₍ₜ₎ melanocortin receptor binding alpha-MSH polypetide of generic formula (II) which has the secretory signal MAWVWTLLFLMAAAQSIQA (SEQ ID NO: 69) fused to its amino terminus. SEQ ID NO: 60 represents a (Mp-Lk-(V2)ₓ-Hg-C_{H}2-C_{H}3)₍ₜ₎ melanocortin receptor binding alpha-MSH polypetide of generic formula (I). No secretory signal is present in SEQ ID NO: 60. SEQ ID NOs: 121, 123, 127, 129, 132, 134, 137, 139, 142, 144, 147, 149, 152, 154, 157, 159, 162, 164, 167, 169, 172, 174, 177, 179, 182, 184, 187, 189, 192, 194, 197, 199, 202, 204, 207, 209, 212, 214, 217, 219, 222, 224, 227, 229, 232, 234, 237, 239, 242, or 244 similarly represent additional melanocortin receptor binding alpha-MSH polypetides either having, or lacking, an amino terminal secretory signal sequence.

Disclosed herein is a polynucleotide comprising, complementary to or having significant identity with, a polynucleotide encoding at least one melanocortin receptor binding mimetibody. Also disclosed herein are vectors comprising at least one polynucleotide molecule encoding a melanocortin receptor binding mimetibody. In a different aspect the invention provides a cell comprising a vector of the invention or a cell expressing a mimetibody polypeptide of the invention. The polynucleotides, vectors and cells may be used to produce the mimetibody polypeptides of the invention.

In one embodiment, the polynucleotides of the invention comprise SEQ ID NO: 211 or a polynucleotide complementary to SEQ ID NO: 211. SEQ ID NO: 59 is a cDNA encoding a (Mp-Lk-(V2)ₓ-Hg-C_{H}2-C_{H}3)₍ₜ₎ melanocortin receptor binding alpha-MSH polypetide of generic formula (I) which lacks a signal sequence. SEQ ID NO: 61 is a cDNA encoding a (V1-Mp-Lk-(V2)ₓ-Hg-C_{H}2-C_{H}3)₍ₜ₎ melanocortin receptor binding alpha-MSH polypetide of generic formula (II) which has a secretory signal fused to its amino terminus. SEQ ID NOs: 120, 122, 126, 128, 131, 133, 136, 138, 141, 143, 146, 148, 151, 153, 156, 158, 161, 163, 166, 168, 171, 173, 176, 178, 181, 183, 186, 188, 191, 193, 196, 198, 201, 203, 206, 208, 211, 213, 216, 218, 221, 223, 226, 228, 231, 233, 236, 238, 241, or 243 similarly represent additional melanocortin receptor binding alpha-MSH polypetides either having, or lacking, an amino terminal secretory signal sequence.

In one embodiment, the polynucleotides of the invention comprise a polynucleotide encoding the polypeptide of 212. Exemplary nucleic acid sequences that encode the polypeptide sequences shown in SEQ ID NO 212 are shown in SEQ ID NO: 211. Also disclosed are polynucleotides that are substantially identical to the above described polynucleotides.

The term "substantially identical" in the context of polynucleotides means that a given polynucleotide sequence is identical to a polynucleotide sequence of the invention, or portion thereof, in at least 60% or at least about 70% or at least about 80% or at least about 90% or at least about 95-98% of the nucleotides. Percent identity between two polynucleotide sequences can be determined by pairwise alignment using the default settings of the AlignX module of Vector NTI v.9.0.0 (Invitrogen Corp., Carlsbad, CA).

Typically, the polynucleotides of the invention are used in expression vectors for the preparation of the mimetibody polypeptides of the invention. Vectors within the scope of the invention provide necessary elements for eukaryotic expression and include viral promoter driven vectors, such as CMV promoter driven vectors, e.g., pcDNA3.1, pCEP4, and their derivatives, Baculovirus expression vectors, *Drosophila* expression vectors, and expression vectors that are driven by mammalian gene promoters, such as human Ig gene promoters. Other examples include prokaryotic expression vectors, such as T7 promoter driven vectors, e.g. pET41, lactose promoter driven vectors and arabinose gene promoter driven vectors.

The present invention also relates to a cell that expresses a mimetibody of the invention or comprises a vector of the invention. Open reading frames encoding the mimetibody polypeptides of the disclosure can be identified by translation of the positive strand reading frame beginning with nucleotide residue 1601 of SEQ ID NOs: 63, 125, 130, 135, 140, 145, 150, 155, 160, 165, 170, 175, 180, 185, 190, 195, 200, 205, 210, 215, 220, 225, 230, 235, 240, or 245. The various signal peptide, V1, Mp, V2, Hg, C_{H}2, and C_{H}3 portions of the mimetibody polypeptides disclosed herein, which have been exemplified herein, are also present in these open reading frames and can be identified using standard sequence analysis methods, such as multiple sequence alignment or other methods well know in the art. Such a cell can be prokaryotic or eukaryotic. Exemplary eukaryotic cells are mammalian cells, such as but not limited to, COS-1, COS-7, HEK293, BHK21, CHO, BSC-1, HepG2, 653, SP2/0, NS0, 293, HeLa, myeloma, lymphoma cells or any derivative thereof. Most preferably, the eukaryotic cell is a HEK293, NS0, SP2/0, or CHO cell. *E. coli* is an exempary prokaryotic cell. A cell according to the invention may be generated by transfection, cell fusion, immortalization, or other procedures that are well known in the art. Polynucleotides transfected into a cell may be extrachromasomal or stably integrated into the chromosome of the cell.

Mimetibodies can be made more compatible with a given host cell by modification of the Hg-C_{H}2-C_{H}3 portion of the polypeptide. For example, when a mimetibody is expressed recombinantly in a bacterial cell such as *E*. *coli,* the Pro-Ala sequence in the Hg element may be removed to prevent digestion by the *E*. *coli* enzyme proline iminopeptidase. Similarly, a portion of the Hg element can be deleted or substituted with other amino acids in the mimetibodies prevent heterogeneity in the products expressed in a selected host cell.

The present invention further provides a method to produce a mimetibody polypeptide comprising the steps of culturing a cell of the invention and purifying an expressed mimetibody polypeptide of the invention. Cell components, such as those necessary for *in vitro* transcription and translation, may also be used to express the polypeptides of the invention. The present invention encompasses mimetibodies produced by both methods. Expressed mimetibody polypeptides can be recovered and purified from cells or cell component based systems by methods well known in the art including, but not limited to, protein A purification, ammonium sulfate or ethanol precipitation, acid extraction, anion or cation exchange chromatography, phosphocellulose chromatography, hydrophobic interaction chromatography, affinity chromatography, hydroxylatpatite chromatography and lectin chromatography. High performance liquid chroatography (HPLC) can also be employed for purification. Typically purfication will require a combination of several different methods.

Another aspect of the present invention is a pharmaceutical composition comprising an effective amount of at least one mimetibody polypeptide of the invention and a pharmaceutically acceptable carrier or diluent. The term "effective amount" generally refers to the quantity of mimetibody necessary for effective therapy, i.e., the partial or complete alleviation of the symptom or disorder for which treatment was sought. The composition can optionally comprise at least one further compound, protein or composition useful for treating obesity and the other conditions described below. The parmaceutically acceptable carrier or diluent in the compositions can be a solution, suspension, emulsion, colloid or powder. Those skilled in the art will recognize other parmaceutically acceptable carriers and diluents.

Disclosed herein is a method of modifying the biological activity of a melanocortin receptor in a cell, tissue or organ comprising contacting the pharmaceutical compositions of the invention with the cell, tissue or organ. The method may be used to modify melanocortin receptor activity in the brain, brain tissue, or brain cells. Alternatively, the method may be used to modify melanocortin receptor activity in other peripheral cells or tissues such as muscle, or other organs such as the stomach. Those skilled in the art will recognize other cells, tissues or organs, which may be used.

Disclosed herein is a method of modulating at least one melanocortin receptor-mediated condition comprising administering a pharmaceutical composition of the invention to a patient in need thereof. The pharmaceutical compositions of the invention can be administered by any suitable route. Such routes may be intrathecal, intranasal, peripheral (e.g., subcutaneous, intramuscular, intradermal, intravenous) or by any other means known in the art. As described previously, abnormal melanocortin receptor activity has been implicated in a number of pathological conditions, such as obesity and Type 2 diabetes. The mimetibody polypeptides of the invention may be also be used to modulate other melanocortin receptor mediated conditions such as male and female erectile dysfunction, inflammation, congestive heart failure, central nervous system disorders, nerve damage, infectious disease, pulmonary disease, skin disease, fever and pain.

The present invention is further described with reference to the following examples. These examples are merely to illustrate aspects of the present invention and are not intended as limitations of this invention.

### Example 1

### Alpha-MSH Mimetibody and Expression Vector Construction

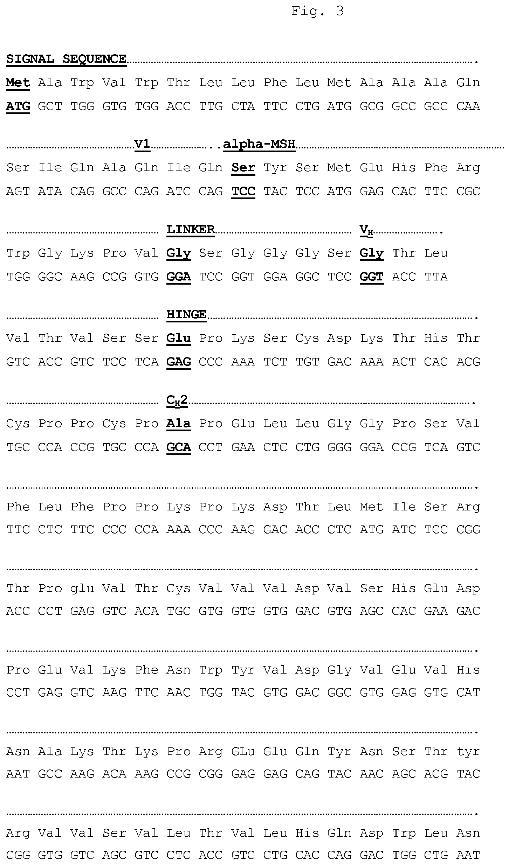

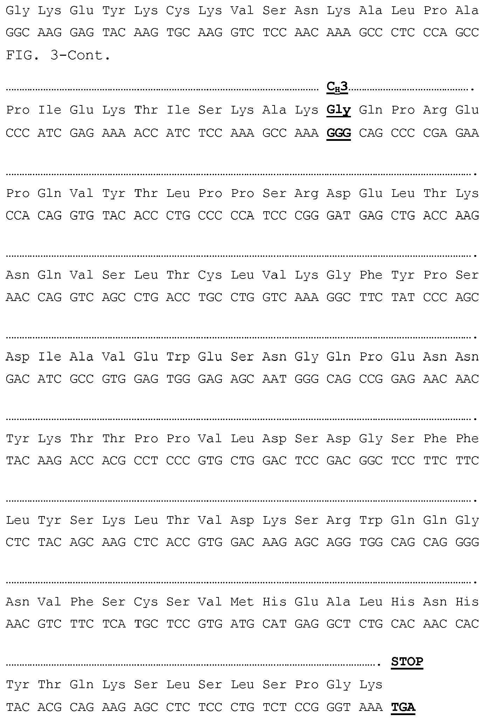

An alpha-MSH mimetibody protein comprising a secretory signal sequence, an alpha-MSH peptide sequence, a linker sequence, V_{H} sequence, a hinge sequence, a human IgG₁ C_{H}2 sequence and a human IgG₁ C_{H}3 sequence was designed (Fig. 3 and SEQ ID NO. 62) Analytical data, *e.g.,* mass spectroscopy, has confirmed that a mature polypeptide is generated (61,344.6 for G1/G1 form). Nucleic acid sequences encoding this alpha-MSH mimetibody protein (Fig. 3; SEQ ID NO: 61) were generated using standard molecular biology techniques. Nucleic acid sequences encoding the alpha-MSH mimetibody sequence were subcloned into the p2389 expression vector to generate an alpha-MSH mimetibody expression vector (SEQ ID NO: 63) .

### Example 2

### Alpha-MSH Mimetibody Expression

The alpha-MSH mimetibody was transiently expressed in HEK293E cells. Cells were cultured using standard conditions and transiently transfected with the alpha-MSH mimetibody expression vector using Lipofectamine 2000 (Invitrogen, Carlsbad, CA) as directed by the manufacturer. 24 h after transfection cells were transferred to a serum free media formulation and cultured for 5 days. The culture media was then removed and centrifuged to remove debris. Clarified media was incubated with Protein A-Sepharose™ (HiTrap rProtein A FF, Amersham Biosciencies, Piscataway, NJ) and proteins were eluted from the Protein A-Sepharose™ conjugate as directed by the manufacturer. The eluted protein solution was then further purified via Superose™ 12 size exclusion chromatography (Superose 12 10/300 GL, Amersham Biosciencies, Piscataway, NJ) using standard methods. Column eluant was then subjected to SDS-PAGE and visualized by silver and Coomassie blue staining. Western blots were then prepared and the blots were probed with either an Fc specific primary antibody or an alpha-MSH specific primary antibody. Together, the Western Blot and SDS-PAGE staining results indicated that a purified alpha-MSH mimetibody, composed of two polypeptide chains, had been obtained from the transiently transfected HEK293 cells.

### Example 3

### Alpha-MSH Mimetibody Binds MC4R

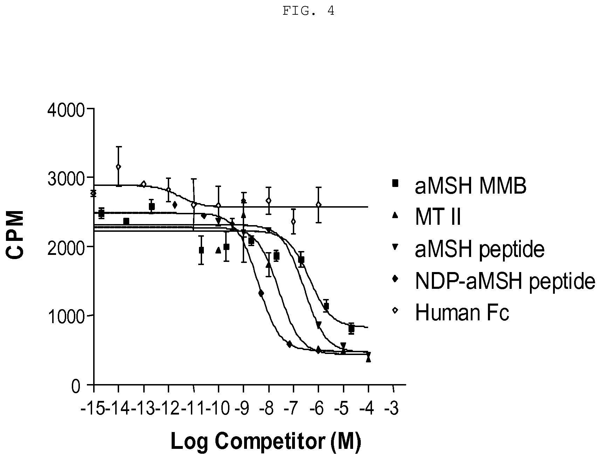

The alpha-MSH mimetibody binds to MC4R and can compete with radiolabeled [Nle(4), D-Phe(7)]-alpha-MSH (NDP-alpha-MSH) agonist molecules for MC4R binding (Fig. 4). MC4R is a receptor for alpha-MSH. alpha-MSH binding to recombinantly expressed MC4R in HEK293 cell membranes (Perkin Elmer Life and Analytical Sciences, Boston, MA) was examined by competive binding assays in which increasing amounts of unlabeled MC4R agonists (positive controls) and the Fc domain of a human antibody (negative control) were added to assay cocktails containing [¹²⁵I]-NDP-alpha-MSH as indicated in Fig. 4. The unlabeled MC4R agonists were melanotan II (MTII; an alpha MSH analog), alpha-MSH, and NDP-alpha-MSH. Alpha-MSH mimetibody binding to MC4R was stable after two weeks of storage at 4°C, - 20°C, and -80°C in PBS (phosphate buffered saline) as assessed by competive binding assays.

Competivive binding assays were performed using Scintillation Proximity Assays® (Amersham Biosciences Corp, Piscataway, NJ) as directed by the assay manufacturer. Assay cocktails contained [¹²⁵I]-NDP-alpha-MSH at EC80, *i.e.,* ∼0.5 nM, 0.1 µg of MC4R membranes, 1 mM MgSO₄, 1.5 mM CaCl₂, 25 mM Hepes, 0.2% BSA, 1 mM 1,10-phenthroline, an assay manufacturer recommended quantity of protease inhibitor cocktail (Roche Diagnostics Corp., Indianapolis, IN) and SPA beads. Light emission from Scintillation Proximity Assay® beads was measured with a Packard Top Count NXT Instrument (Perkin Elmer Life and Analytical Sciences, Boston, MA) for 5 minutes.

### Example 4

### Alpha-MSH Mimetibody Activates MC4R

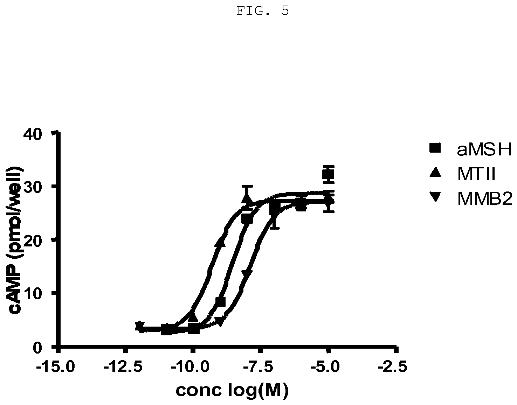

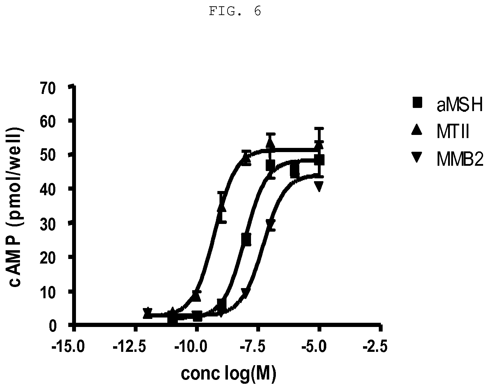

The alpha-MSH mimetibody can activate MC4R signalling to increase cAMP production in CHOK1 cells expressing MC4R (Fig. 5 and Fig. 6). MC4R is a seven transmembrane (7TM) G-protein coupled receptor. Activation of MC4R by ligand or agonist results in an increase in cyclic AMP levels (cAMP).

MC4R receptor activation assays were performed using two different clonal CHOK1 cell lines stably transfected with a MC4R expression vector and expressing MC4R. Clone 1 (Fig. 5) expressed MC4R at high levels relative to Clone 2 (Fig. 6). Clone 1 and Clone 2 cells were grown as a monolayer using standard culture conditions to a density of approximately 100,000 cells/well and then incubated with increasing amounts (0-100 µM) of alpha-MSH, MTII, or alpha-MSH mimetibody for 15 minutes as indicated in Fig. 5 and Fig. 6. Cells were then lysed and cAMP assays were performed using the cAMP-Screen Direct™ Chemiluminescent Immunoassay System (Applied Biosystems, Foster City, CA) as directed by the manufacturer. EC₅₀ values from cAMP assays using Clone 1 (Fig. 5) and Clone 2 (Fig. 6) are listed in Table 1 below

**Table 1**

| | Clone 1 | Clone 2 |
|---|---|---|
| alpha-MSH peptide (Positive control) | EC₅₀ = 3.29 nM | EC₅₀ = 9.46 nM |
| MT II (Positive control) | EC₅₀ = 0.52 nM | EC₅₀ = 0.52 nM |
| alpha-MSH mimetibody | EC₅₀ = 14.36 nM | EC₅₀ = 52.4 nM |

### Example 5

### Alpha-MSH Mimetibody Administration Decreases

### Animal Food Intake and Body Weight

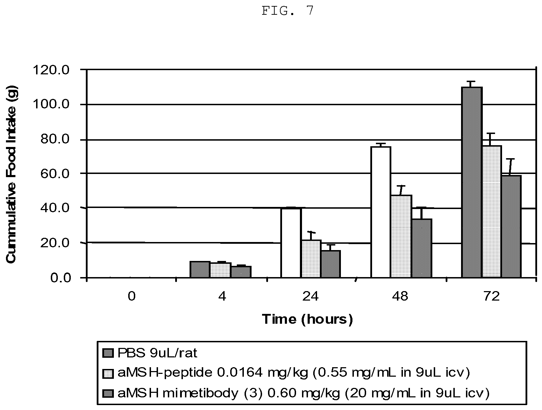

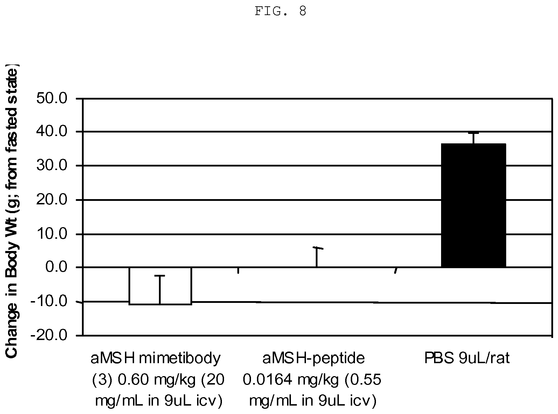

Alpha-MSH mimetibody administration to *Rattus norvegicus* brain ventricules decreases animal food intake (Fig. 7) and body weight (Fig. 8). Alpha-MSH mimetibody was supplied to brain ventricules by intracerebroventricular injections (ICV) via a cannula surgically inserted into the left lateral brain ventricle.

Cannulae were surgically inserted into male Sprague-Dawley or Wistar rats weighing 250 g to 350 g. Cannula placement coordinates were as follows: -0.8 mm from bregma, -4.5 mm ventral and -1.5 posterior-anterior. Animals recovered for 7 to 10 days after surgery. Animals were acclimatized to the experimental procedures by both daily handling and mock injection, in order to minimize stress. In addition animals were submitted to the reversal of dark-light cycle.

Proper cannula placement was confirmed by an angiotensin II test. The test confirmed proper cannula placement if the ICV administration of 10 ng of angiotensin II via the cannula caused the rats to drink 5-10 ml of water in 30 minutes. Only animals that passed this angiotensin II test were used in food intake experiments.

Animals were fasted for 18-24 hours and alpha-MSH mimetibody, alpha-MSH (positive control), or PBS (negative control) were then administered to the brain ventricles via the cannula at an injection rate of 9 µl/min. Each treatment group had a minimum of 7 animals. Treatments and dosages were as indicated in Fig. 7 and Fig. 8.

Food and water was given to the animals after injection. The amount of food and water consumed was measured at 0 h, 4 h, 24 h, 48 h and 72 h (Fig. 7) after injection. Body weight at 72 hours post injection was measured as ahown in Fig. 6.

## Claims

1. A polypeptide comprising a polypeptide having the sequence shown in SEQ ID NO: 212.

2. A polynucleotide:
i) encoding a polypeptide according to claim 1; or
ii) comprising a polynucleotide having the sequence shown in SEQ ID NO: 211 or a polynucleotide having a sequence complementary to the sequence shown in SEQ ID NO: 211; or
iii) comprising a polynucleotide encoding the polypeptide having the sequence shown in SEQ ID NO: 212.

3. A vector comprising the polynucleotide of claim 2.

4. The vector of claim 3 comprising a polynucleotide having the sequence shown in SEQ ID NO: 215.

5. A cell expressing:
i) a polypeptide according to claim 1; or
ii) the vector of claim 3.

6. The cell of claim 5ii) wherein the cell is a Chinese hamster ovary (CHO)-derived cell.

7. A method to produce a polypeptide comprising the steps of culturing the cell of claim 5ii) and purifying the expressed polypeptide.

8. A pharmaceutical composition comprising an effective amount of at least one polypeptide according to claim 1 and a pharmaceutically acceptable carrier or diluent.

9. The pharmaceutical composition of claim 8, for use in a therapy.

10. The pharmaceutical composition of claim 8, for use in treating obesity, male erectile dysfunction, female sexual dysfunction, an inflammatory condition or fibrosis.

11. The pharmaceutical composition for use according to claim 10 wherein the inflammatory condition is allergic inflammation, gouty arthritis, rheumatoid arthritis, inflammatory bowel disease, post-ischemia renal injury, liver inflammation, ischemic brain damage or peripheral neuropathies.

## Patentansprüche

1. Polypeptid, umfassend ein Polypeptid mit der in SEQ ID NO: 212 gezeigten Sequenz.

2. Polynukleotid:
i) das ein Polypeptid gemäß Anspruch 1 kodiert; oder
ii) das ein Polynukleotid mit der in SEQ ID NO: 211 gezeigten Sequenz oder ein Polynukleotid mit einer zu der in SEQ ID NO: 211 gezeigten Sequenz komplementären Sequenz umfasst; oder
iii) das ein Polynukleotid umfasst, das das Polypeptid mit der in SEQ ID NO: 212 gezeigten Sequenz kodiert.

3. Vektor, umfassend das Polynukleotid gemäß Anspruch 2.

4. Vektor gemäß Anspruch 3, umfassend ein Polynukleotid mit der in SEQ ID NO: 215 gezeigten Sequenz.

5. Zelle, exprimierend:
i) ein Polypeptid gemäß Anspruch 1; oder
ii) den Vektor gemäß Anspruch 3.

6. Zelle gemäß Anspruch 5ii), wobei die Zelle eine von Ovarien des chinesischen Hamsters (CHO) abgeleitete Zelle ist.

7. Verfahren zum Herstellen eines Polypeptids, umfassend die Schritte des Kultivierens der Zelle gemäß Anspruch 5ii) und des Reinigens des exprimierten Polypeptids.

8. Pharmazeutische Zusammensetzung, umfassend eine wirksame Menge wenigstens eines Polypeptids gemäß Anspruch 1 und einen pharmazeutisch verträglichen Träger oder ein pharmazeutisch verträgliches Verdünnungsmittel.

9. Pharmazeutische Zusammensetzung gemäß Anspruch 8 für die Verwendung bei einer Therapie.

10. Pharmazeutische Zusammensetzung gemäß Anspruch 8 für die Verwendung bei der Behandlung von Adipositas, erektiler Dysfunktion des Mannes, sexueller Dysfunktion der Frau, eines entzündlichen Zustands oder Fibrose.

11. Pharmazeutische Zusammensetzung für die Verwendung gemäß Anspruch 10, wobei der entzündliche Zustand allergische Entzündung, Gichtarthritis, rheumatoide Arthritis, entzündliche Darmerkrankung, Post-Ischämie-Nierenschädigung, Leberentzündung, ischämische Hirnschädigung oder eine periphere Neuropathie ist.

## Revendications

1. Polypeptide comprenant un polypeptide présentant la séquence montrée dans SEQ ID : 212.

2. Polynucléotide :
i) codant pour un polypeptide selon la revendication 1 ; ou
ii) comprenant un polynucléotide présentant la séquence montrée dans SEQ ID NO : 211 ou un polynucléotide présentant une séquence complémentaire à la séquence représentée dans SEQ ID NO : 211 ; ou
iii) comprenant un polynucléotide codant pour le polypeptide présentant la séquence montrée dans SEQ ID : 212.

3. Vecteur comprenant le polynucléotide selon la revendication 2.

4. Vecteur selon la revendication 3, comprenant un polynucléotide présentant la séquence montrée dans SEQ ID : 215.

5. Cellule exprimant :
i) un polypeptide selon la revendication 1 ; ou
ii) le vecteur selon la revendication 3.

6. Cellule selon la revendication 5ii), la cellule étant une cellule dérivée d'un ovaire de hamster chinois (CHO).

7. Procédé pour produire un polypeptide comprenant les étapes de culture de la cellule selon la revendication 5ii) et de purification du polypeptide exprimé.

8. Composition pharmaceutique comprenant une quantité efficace d'au moins un polypeptide selon la revendication 1 et un support ou un diluant pharmaceutiquement acceptable.

9. Composition pharmaceutique selon la revendication 8, pour une utilisation en thérapie.

10. Composition pharmaceutique selon la revendication 8, pour une utilisation dans le traitement de l'obésité, le dysfonctionnement érectile masculin, le dysfonctionnement sexuel féminin, un état inflammatoire ou la fibrose.

11. Composition pharmaceutique pour une utilisation selon la revendication 10, l'état inflammatoire étant une inflammation allergique, l'arthrite goutteuse, la polyarthrite rhumatoïde, une maladie inflammatoire de l'intestin, une lésion rénale postischémique, une inflammation hépatique, une lésion ischémique du cerveau ou des neuropathies périphériques.
